# EUROPEAN PATENT SPECIFICATION

(11) **EP 4 126 853 B1**
(45) Date of publication and mention of the grant of the patent: **04.03.2026**
(21) Application number: 21719830.8
(22) Date of filing: 30.03.2021
(51) Int. Cl.: C07D 413/10, A61K 31/5375, A61P 31/16

(54) **SMALL MOLECULE INHIBITORS OF INFLUENZA HEMAGGLUTININ**
KLEINMOLEKÜLIGE HEMMER VON INFLUENZA-HÄMAGGLUTININ
INHIBITEURS À PETITES MOLÉCULES DE L'HÉMAGGLUTININE DE LA GRIPPE

(30) Priority: 30.03.2020 US 202063001914 P
(43) Date of publication of application: 08.02.2023
(73) Proprietor: The Scripps Research Institute, La Jolla, CA 92037 (US)
(72) Inventor: WOLAN, Dennis W., La Jolla, CA 92037 (US); WILSON, Ian A., La Jolla, CA 92037 (US); YAO, Yao, Seattle, Washington 98115 (US); KITAMURA, Seiya, San Diego, California 92122 (US); WU, Nicholas C., San Diego, California 92121 (US); KADAM, Rameshwar U., San Diego, California 92122 (US); LEE, Chang-Chun, San Diego, California 92122 (US)
(74) Representative: Hutter, Anton
(86) International application number: PCT/US2021/024985
(87) International publication number: WO 2021/202600

(56) References cited:
- WO-A1-2007/106705
- WO-A1-2010/151577
- WO-A1-2016/156816
- WO-A2-2008/011130
- KOWALSKI JENNIFER A ET AL: "Rapid synthesis of an array of trisubstituted urea-based soluble epoxide hydrolase inhibitors facilitated by a novel solid-phase method", BIOORGANIC & MEDICINAL CHEMISTRY LETTERS, vol. 20, no. 12, 22 April 2010 (2010-04-22) - 22 April 2010 (2010-04-22), pages 3703 - 3707, XP029212964, ISSN: 0960-894X, DOI: 10.1016/J.BMCL.2010.04.078
- YAO YAO ET AL: "An influenza A hemagglutinin small-molecule fusion inhibitor identified by a new high-throughput fluorescence polarization screen", PROCEEDINGS OF THE NATIONAL ACADEMY OF SCIENCES - PNAS, 4 August 2020 (2020-08-04), pages 18431 - 18438, XP055802938, Retrieved from the Internet <URL:https://www.pnas.org/content/pnas/117/31/18431.full.pdf> [retrieved on 20210510], DOI: 10.1073/pnas.2006893117

## Description

### CROSS-REFERENCE TO RELATED APPLICATIONS

This application claims priority to US Application No. 63/001,914 filed March 30, 2020.

### STATEMENT OF GOVERNMENT SUPPORT

This invention was made with government support under Contract No. 1R56AI127371 awarded by the National Institute of Allergy and Infectious Diseases. The government has certain rights in the invention.

### TECHNICAL FIELD

This disclosure relates to novel chemical compounds and methods useful for inhibiting influenza hemagglutinin.

### BACKGROUND

Influenza viruses cause major respiratory disease and are an enormous economic burden. Current therapeutic options to treat acute influenza infection include antiviral drugs directed at blocking virus uncoating (M2 proton channel inhibitors) in cell entry, blocking viral replication (e.g. the cap-dependent endonuclease inhibitor baloxavir marboxil (Xofluza)), and progeny release from infected cells (neuraminidase inhibitors). However, resistance to antiviral drugs is an emerging problem due to the high mutation rate in influenza viruses. Thus, there is a pressing and unmet need for novel broad-spectrum therapeutics to combat pandemics and seasonal epidemics.

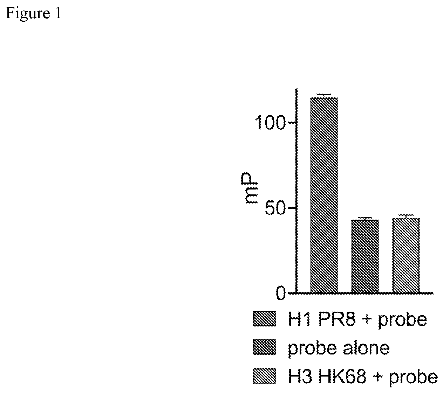

The surface-expressed influenza hemagglutinin (HA) glycoprotein enables viral entry into host cells and is the main target for antibodies in our immune system. HA is a trimeric class I viral fusion protein with a globular membrane-distal head containing the receptor binding site (RBS), where host cell sialosides act as receptors, and a membrane-proximal stem housing the fusion machinery (Fig. 1). The prefusion mature HA is metastable and pH-sensitive; acidification in the endosome after virus entry triggers conformational rearrangements that lead to the post-fusion state. Blocking membrane fusion would be an effective means to prevent infection. The Wilson laboratory has demonstrated that broadly neutralizing human antibodies target conserved HA epitopes and these HA surface regions (e.g., membrane-proximal stem) represent ideal targets for drug discovery, as they are functionally conserved, less susceptible to mutation, and essential for viral entry and fitness. HA is now a validated drug target, but no commercially available therapeutics specifically block HA.

Pathogens that cause influenza are negative-sense, single-stranded RNA viruses, which belong to the family of Orthomyxoviridae. There are three types of influenza viruses: A, B and C. Influenza A viruses are the most common form, which can spread in mammals and birds. The subtypes of influenza A are named by the types of surface proteins hemagglutinin (H) and neuraminidase (N). There are 18 different hemagglutinins (H1-H18) and 11 known neuraminidases (N1-N11), the majority of which are present in avian reservoirs, such as wild aquatic birds. The HA subtypes can be clustered into two groups, 1 and 2, by phylogeny. Current seasonal influenza A viruses found in humans are mainly H1N1 and H3N2 subtypes from group 1 and group 2, respectively. Influenza B viruses are usually found only in humans. They are divided into two antigenically distinct lineages, Yamagata and Victoria. These different groupings can be further broken down into different strains. Circulating influenza viruses are highly variable each year, and both influenza A and B cause seasonal epidemics all over the world. Influenza C viruses give much milder symptoms, which do not cause epidemics.

All three types of viruses have similar genome structures. The genome comprises 8 segments, encoding 9-11 proteins, depending on the type. Influenza A encodes 11 proteins, which includes the surface proteins hemagglutinin (HA) and neuraminidase (NA), the polymerase complex (PA, PB1 and PB2), nucleoprotein (NP), membrane proteins (M 1 and M2), and other proteins (NS1, NS2, NEP). Among the three influenza virus types, influenza A has the highest rate of mutation. Influenza B evolves slower than A but faster than C. The segmented genome allows gene exchanging between different viral strains, which generate new variants of influenza viruses.

No effective antiviral drugs are currently available for preventing entry of influenza viruses into host cells. Recent advances have shown that antibodies can mount broad neutralizing responses against human influenza viruses and common features from these structural studies were found for recognition of key sites of vulnerability on the HA surface in pandemic, emerging, and seasonal influenza viruses. For example, the unexpected recurring motifs for recognition of conserved hydrophobic HA stem sub-pockets can be exploited in assay design and small-molecule optimization. Ultimately, small molecules with affinity to the HA stem will provide paradigm-shifting principles in design, synthesis, and characterization of molecules that broadly inhibit influenza viruses by blocking entry and infection.

WO 2016/156816 A1 describes 1-cyano-pyrrolidine compounds which inhibit deubiquitylating enzymes (DUBs). In particular, the compounds can be used to inhibit ubiquitin C-terminal hydrolase 30 (USP30). WO 2016/156816 A1 explains that the compounds can be used to treat conditions involving mitochondrial dysfunction and cancer.

Kowalski et al. ("Rapid synthesis of an array of trisubstituted urea-based soluble epoxide hydrolase inhibitors facilitated by a novel solid-phase method", Bioorganic & Medicinal Chemistry Letters, Vol. 20, issue 12, 15 June 2010, pages 3703-3707) discloses a 270-membered library of trisubstituted ureas which were synthesized and evaluated for inhibition of soluble epoxide hydrolase.

WO 2008/011130 A2 describes amide compounds which have DGAR inhibitory activity. WO 2008/011130 A2 explains that the compounds may be useful for the prophylaxis, treatment or improvement of diseases or pathologies caused by expression or high activation of DGAT.

WO 2007/106705 A1 describes soluble epoxide hydrolase (sEH) inhibitors. WO 2007/106705 A1 explains that these sEH inhibitors may be used to treat cardiovascular disease.

Yao et al. ("An influenza A hemagglutinin small-molecule fusion inhibitor identified by a new high-throughput fluorescence polarization screen", Proc Natl Acad Sci USA 2020 Aug 4;117(31):18431-18438. doi: 10.1073/pnas.2006893117. Epub 2020 Jul 20) explains how the authors synthesized a fluorescence-polarization probe and an assay compatible with high-throughput screening to identify new antiviral lead candidates. This enabled them to enabled them to identify a small-molecule candidate compound.

WO 2010/151577 A1 describes compounds which can be used to treat or prevent influenza infection by inhibiting influenza virus HA maturation process.

### SUMMARY

The invention is as defined by in the claims.

In a first aspect of the invention, there is provided a compound of Formula (I) or (II): 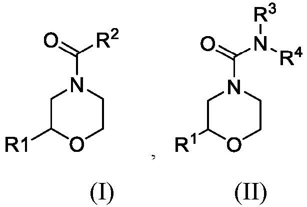 wherein
R¹ is phenyl;
R² is aryl, heterocyclyl, 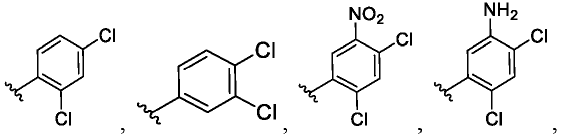 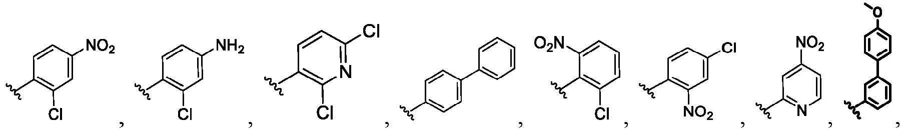 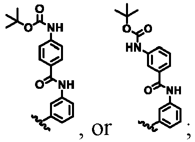
R³ is selected from H, alkyl, -alkylene-(heterocyclyl), -alkylene-(aryl), -alkylene-(cycloalkyl), -alkylene-O-R⁵, -alkylene-C(O)N(R⁵)₂, -alkylene-C(O)R⁵, -alkylene-C(O)₂R⁵, -alkylene-N(R⁵)₂, -alkylene-N(R⁵)C(O)R⁵, -alkylene-N(R⁵)C(O)N(R⁵)₂, -alkylene-N(R⁵)SO₂-R⁵, -alkylene-S-R⁵, -aryl, -arylene-(heterocyclyl), -arylene-alkyl, -arylene-(cycloalkyl), -arylene-O-R⁵, -arylene-C(O)N(R⁵)₂, -arylene-C(O)R⁵, -arylene-C(O)₂R⁵, -arylene-N(R⁵)₂, -arylene-N(R⁵)C(O)R⁵, -arylene-N(R⁵)C(O)N(R⁵)₂, -arylene-N(R⁵)SO₂-R⁵, -arylene-S-R⁵, -heterocyclyl, -heterocyclylene-(alkyl), -heterocyclylene-(aryl), -heterocyclylene-(cycloalkyl), -heterocyclylene-(cycloalkyl), -heterocyclylene-O-R⁵, -heterocyclylene-C(O)N(R⁵)₂, -heterocyclylene-C(O)R⁵, -heterocyclylene-C(O)₂R⁵, - heterocyclylene-N(R⁵)₂, -heterocyclylene-N(R⁵)C(O)R⁵, -heterocyclylene-N(R⁵)C(O)N(R⁵)₂, - heterocyclylene-N(R⁵)SO₂-R⁵, -heterocyclylene-S-R⁵, -cycloalkyl, -cycloalkylene-(alkyl), -cycloalkylene-(aryl), -cycloalkylene-(cycloalkyl), -cycloalkylene(heterocycloalkyl), -cycloalkylene-O-R⁵, -cycloalkylene-C(O)N(R⁵)₂, -cycloalkylene-C(O)R⁵, -cycloalkylene-C(O)₂R⁵, -cycloalkylene-N(R⁵)₂, -cycloalkylene-N(R⁵)C(O)R⁵, -cycloalkylene-N(R⁵)C(O)N(R⁵)₂, -cycloalkylene-N(R⁵)SO₂-R⁵, -alkylene-N(H)-alkylene-C(O)OR⁵, -alkylene-C(O)OR⁵, -arylene-alkylene-aryl, -C(O)R⁵, -C(O)₂R⁵, -alkylene-S-S-R⁵, -C(O)-alkylene-N(R⁵)₂, -C(O)₂R⁵, -aryl-alkylene-N(R⁵)C(O)-R⁵, -cycloalkylene-N(R⁵)S(O)(F)-R⁵, -SO₂-NR⁵ -S(O)(F)-NR⁵;
R⁴ is selected from alkyl, -alkylene-(heterocyclyl), -alkylene-(aryl), -alkylene-(cycloalkyl), -alkylene-O-R⁵, -alkylene-C(O)N(R⁵)₂, -alkylene-C(O)R⁵, -alkylene-C(O)₂R⁵, -alkylene-N(R⁵)₂, -alkylene-N(R⁵)C(O)R⁵, -alkylene-N(R⁵)C(O)N(R⁵)₂, -alkylene-N(R⁵)SO₂-R⁵, -alkylene-S-R⁵, -aryl, -arylene-(heterocyclyl), -arylene-alkyl, -arylene-(cycloalkyl), -arylene-O-R⁵, -arylene-C(O)N(R⁵)₂, -arylene-C(O)R⁵, -arylene-C(O)₂R⁵, -arylene-N(R⁵)₂, -arylene-N(R⁵)C(O)R⁵, -arylene-N(R⁵)C(O)N(R⁵)₂,\ -arylene-N(R⁵)SO₂-R⁵, -arylene-S-R⁵, -heterocyclyl, -heterocyclylene-(alkyl), -heterocyclylene-(aryl), -heterocyclylene-(cycloalkyl), -heterocyclylene-(cycloalkyl), -heterocyclylene-O-R⁵, -heterocyclylene-C(O)N(R⁵)₂, -heterocyclylene-C(O)R⁵, -heterocyclylene-C(O)₂R⁵, *-*heterocyclylene-N(R⁵)₂, heterocyclylene-N(R⁵)C(O)R⁵, -heterocyclylene-N(R⁵)C(O)N(R⁵)₂-, -heterocyclylene-N(R⁵)SO₂-R⁵, -heterocyclylene-S-R⁵, -cycloalkyl, -cycloalkylene-(alkyl), -cycloalkylene-(aryl), -cycloalkylene-(cycloalkyl), -cycloalkylene(heterocycloalkyl), -cycloalkylene-O-R⁵, -cycloalkylene-C(O)N(R⁵)₂, -cycloalkylene-C(O)R⁵, -cycloalkylene-C(O)₂R⁵, -cycloalkylene-N(R⁵)₂, -cycloalkylene-N(R⁵)C(O)R⁵, -cycloalkylene-N(R⁵)C(O)N(R⁵)₂, -cycloalkylene-N(R⁵)SO₂-R⁵, -alkylene-N(H)-alkylene-C(O)OR ⁵, -alkylene-C(O)OR⁵, -arylene-alkylene-aryl, -C(O)R⁵, -C(O)₂R⁵, -alkylene-S-S-R⁵, -C(O)-alkylene-N(R⁵)₂, -C(O)₂R⁵, -aryl-alkylene-N(R⁵)C(O)-R⁵, -cycloalkylene-N(R⁵)S(O)(F)-R⁵, -SO₂-NR⁵ -S(O)(F)-NR⁵; or
R³ and R⁴ can be taken together to form a heterocyclyl or 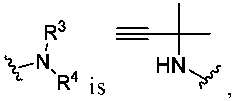 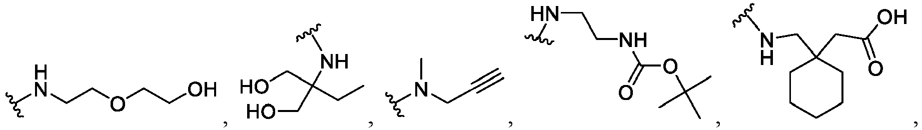 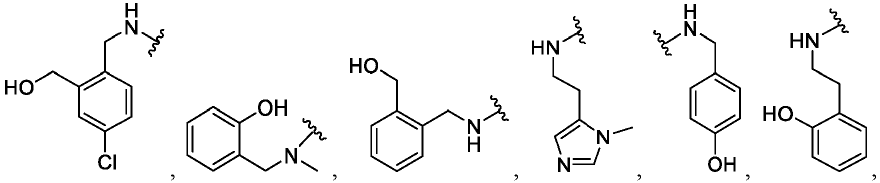 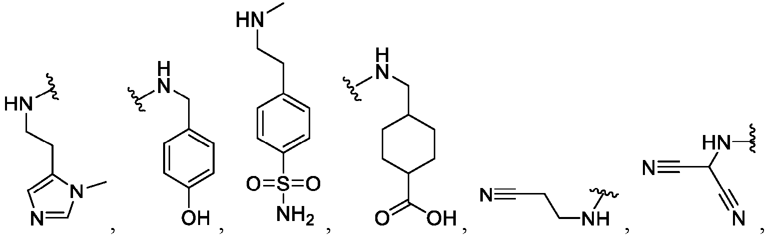 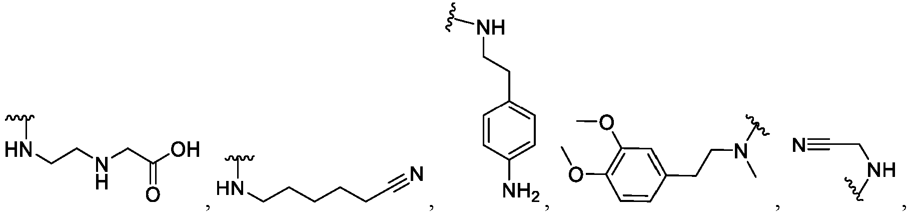 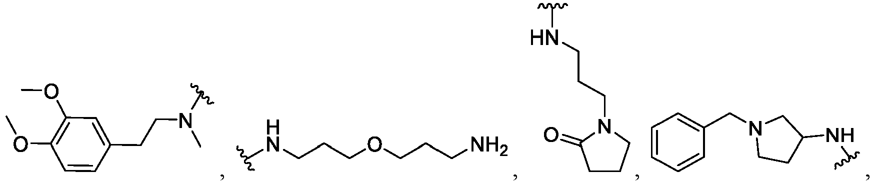 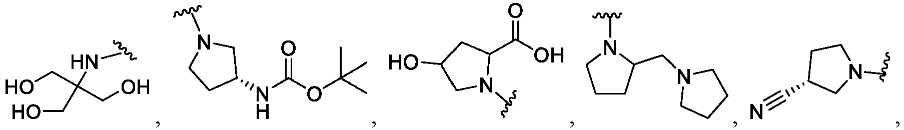 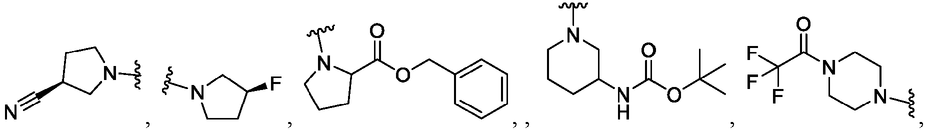 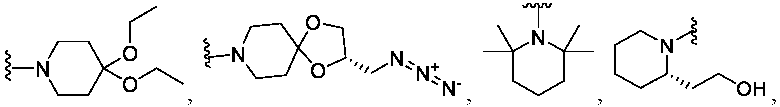 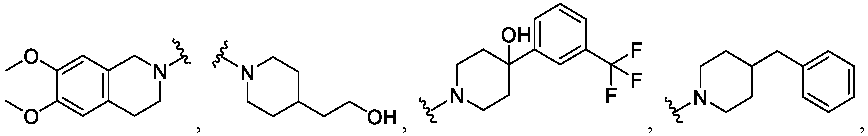 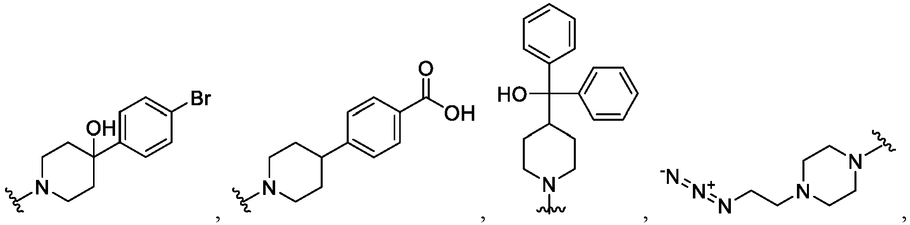 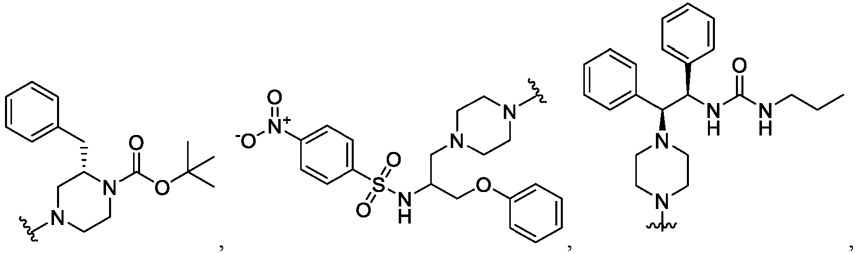 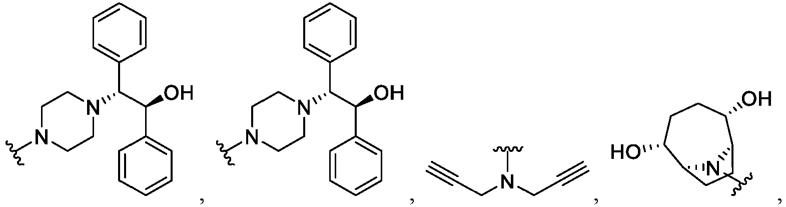 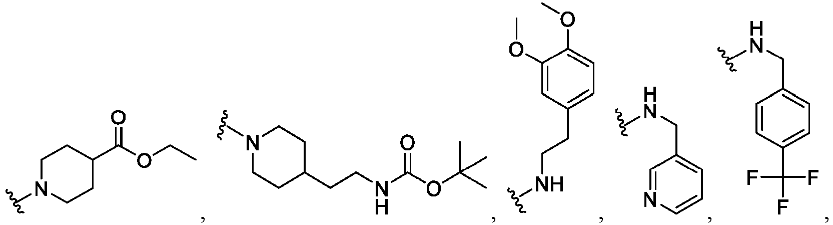 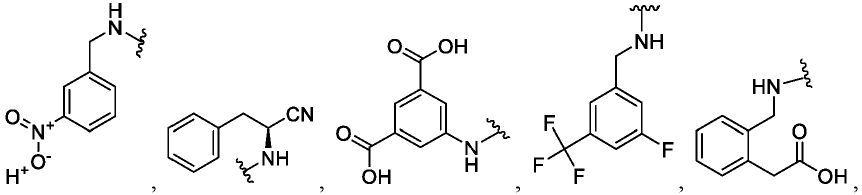 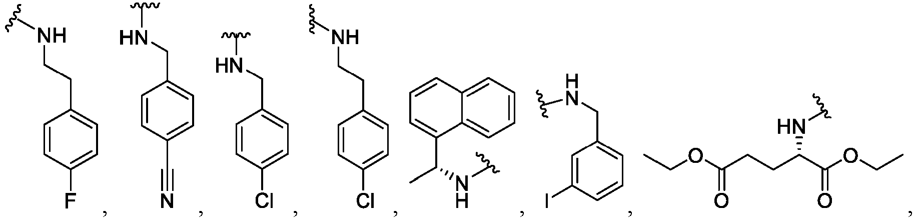 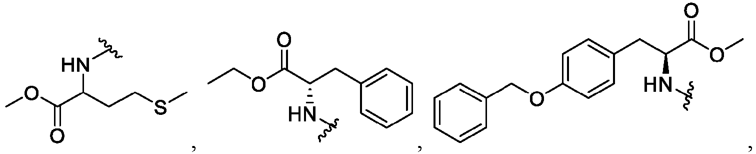 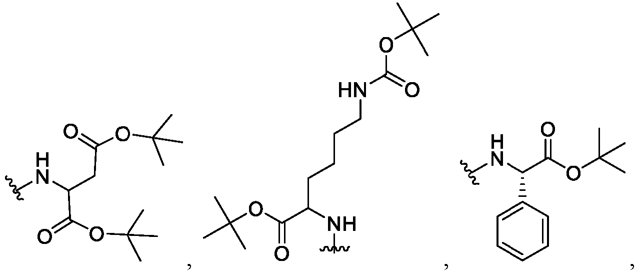 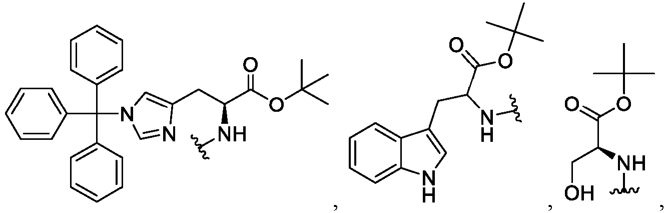 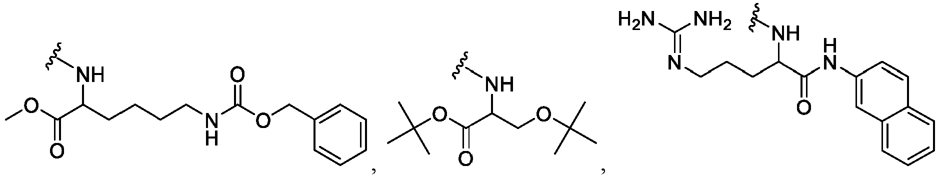 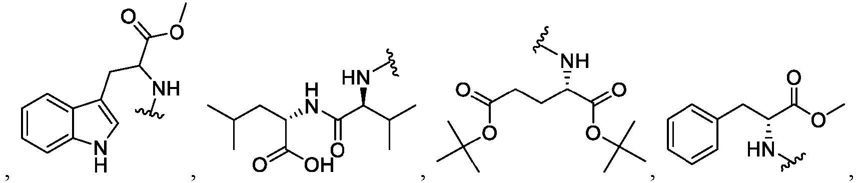 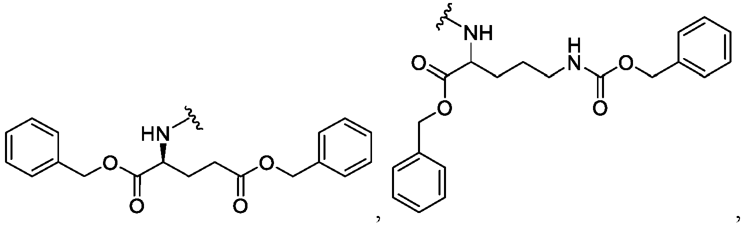 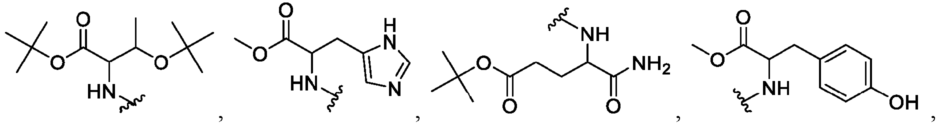 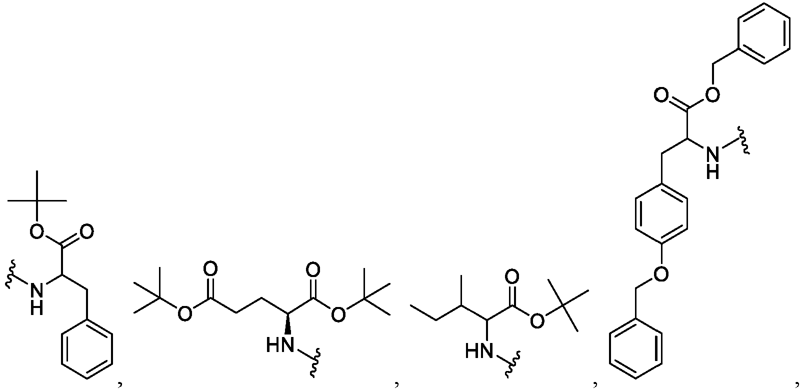 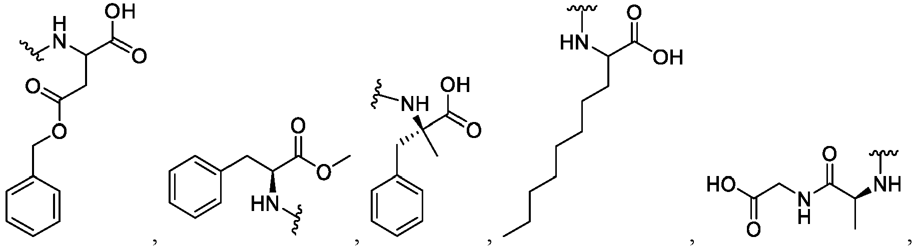 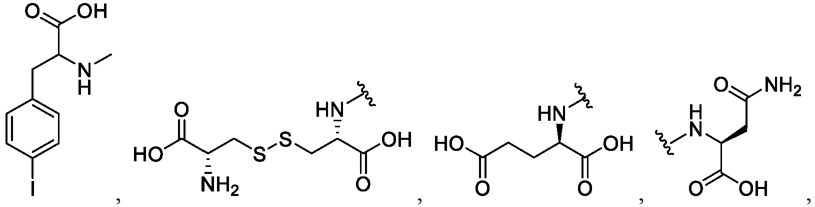 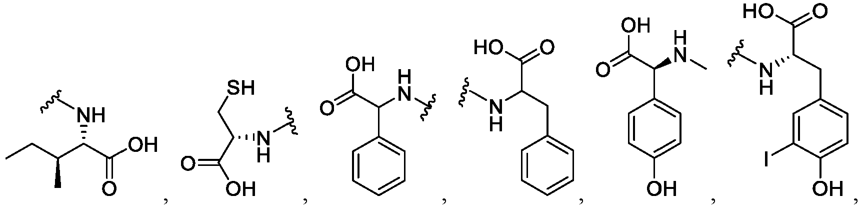 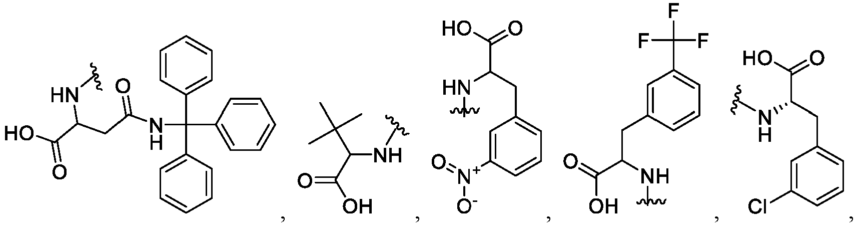 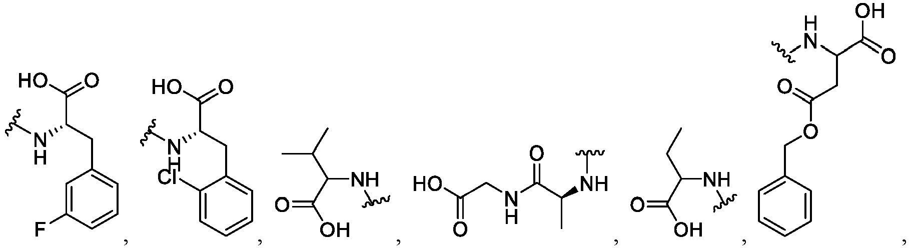 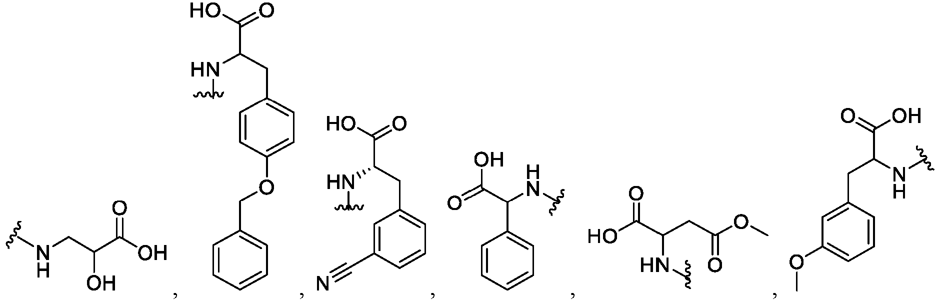 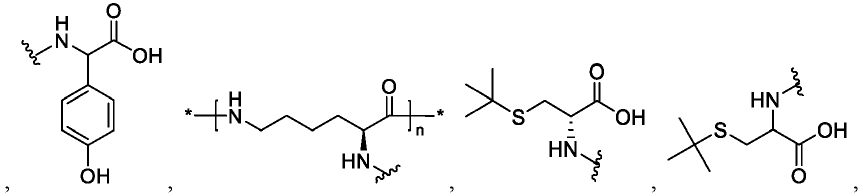 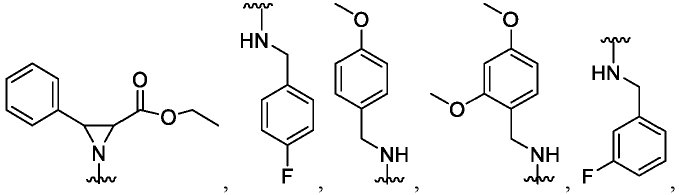 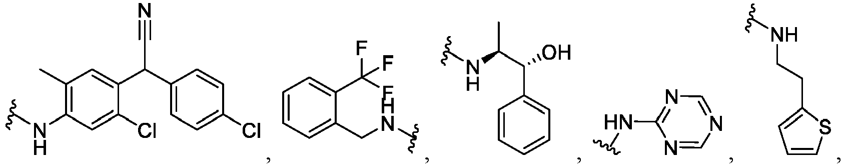 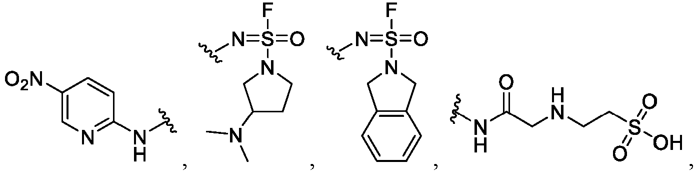 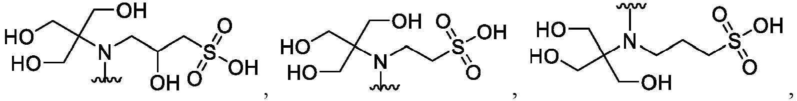 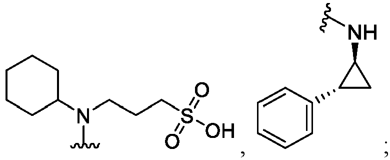 and
R⁵ is H or alkyl or aryl or -alkylene-aryl;
wherein, unless otherwise specified:
   the term alkyl defines a branched or unbranched carbon chain moiety up to 30 carbon atoms;
   the term alkylene defines an alkyl group having from 2 to 12 carbon atoms that contains two points of attachment to the rest of the compound on its longest carbon chain;
   the term aryl defines a 5- to 12-membered aromatic ring, wherein each atom of the ring is carbon or one or more ring atoms are heteroatoms;
   the term heterocyclyl defines a 3- to 12-membered ring structure, wherein one to four ring atoms are heteroatoms; and
   the term cycloalkyl defines a mono-, bicyclic, bridged, spirocyclic or polycyclic saturated carbocyclic ring having from 3 to 12 carbon atoms;
   wherein the compound of formula (II) is not

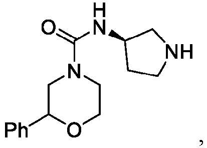

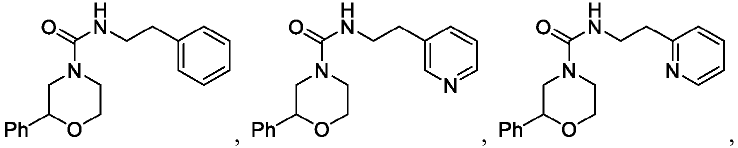

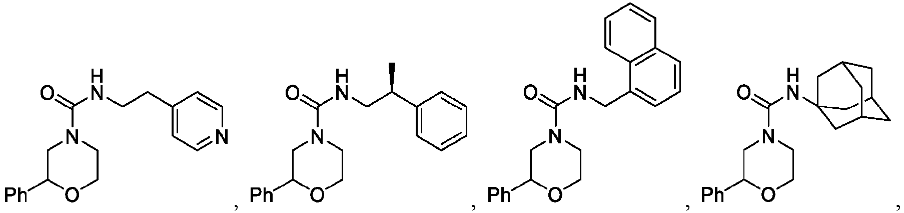

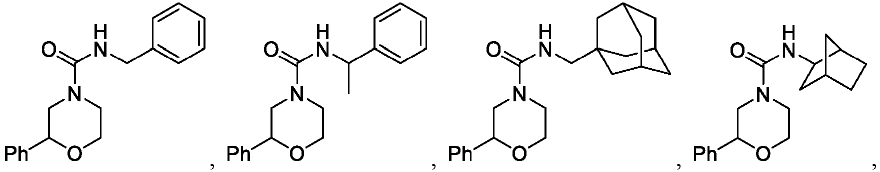

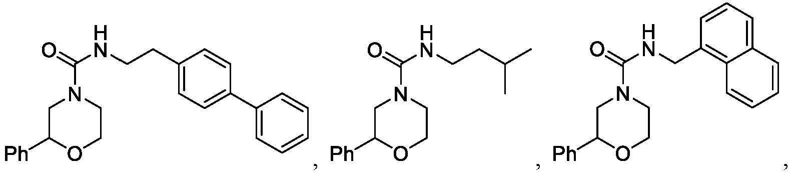

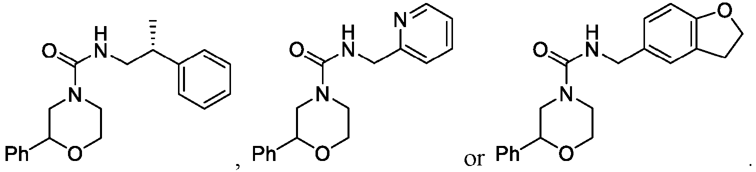

In various embodiments, the disclosure describes a method of treating or preventing an HA-mediated disease or condition, comprising administering a compound of Formula (I) or (II) to a subject in need thereof. It will be appreciated that methods of treatment do not fall within the scope of the claims.

### BRIEF DESCRIPTION OF THE FIGURES

**Figure 1** shows that FP assay and P7-TAMRA probe is specific for group 1 H1 versus group 2 H3 HA. 30 nM HA and 75 nM P7-TAMRA probe were mixed in PBS buffer (pH 7.4, 0.01% Triton X-100) for a few seconds. FP was then measured in 96 well plate by PerkinElmer EnVision plate reader at room temperature. Experiments were performed in at least triplicate to ensure reproducibility.

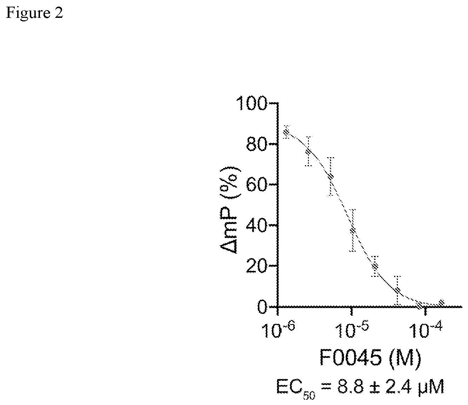

**Figure 2** shows P7-TAMRA probe (75 nM) and H1/PR8 HA (30 nM) competition assay against a dose-dependent increase of a racemic mixture of F0045.

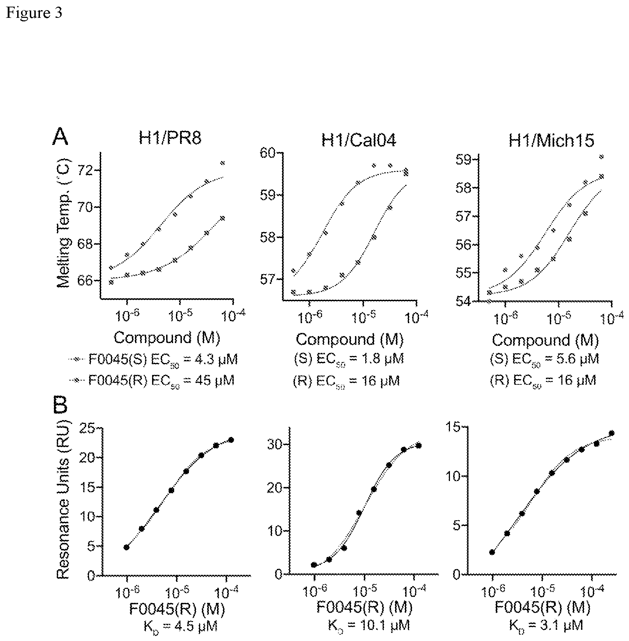

**Figures 3A-3B** show the dose response of F0045(S) and (R) on temperature stability of H1 HAs and SPR of F0045(R) binding to HA. Figure 3A is the dose-response of F0045(S) vs. F0045(R) against different H1 HAs by DSF. F0045(S) and F0045(R) ranging from 0.5 µM to 64 µM were pre-mixed with 0.2 mg/ml HAs (H1/PR8, H1/Cal04, and H1/Mich15) in PBS buffer (pH 7.4), and protein HA unfolding temperature was then measured by nanoDSF. Figure 3B is the dose response of F0045(R) against different H1 HAs (H1/PR8, H1/Cal04, and H1/Mich15) by SPR. Different concentrations (250 µM to 200 nM) of F0045(R) against Resonance units (RU) of different H1 HAs are plotted to determine the steady-state affinity.

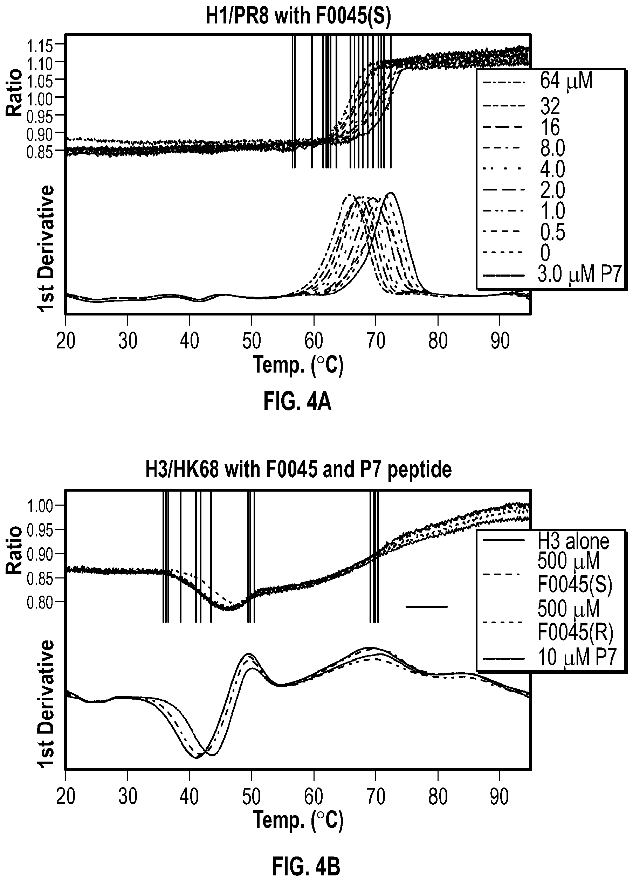

**Figures 4A-4B** show DSF melting curves. Figure 4A shows H1/PR8 HA with increasing concentrations of F0045(S) (0 - 64 µM). Figure 4B shows melting temperature of H3/HK68 HA as control does not change in the presence of 10 µM P7, 500 µM F0045(S), or 500 µM F0045(R).

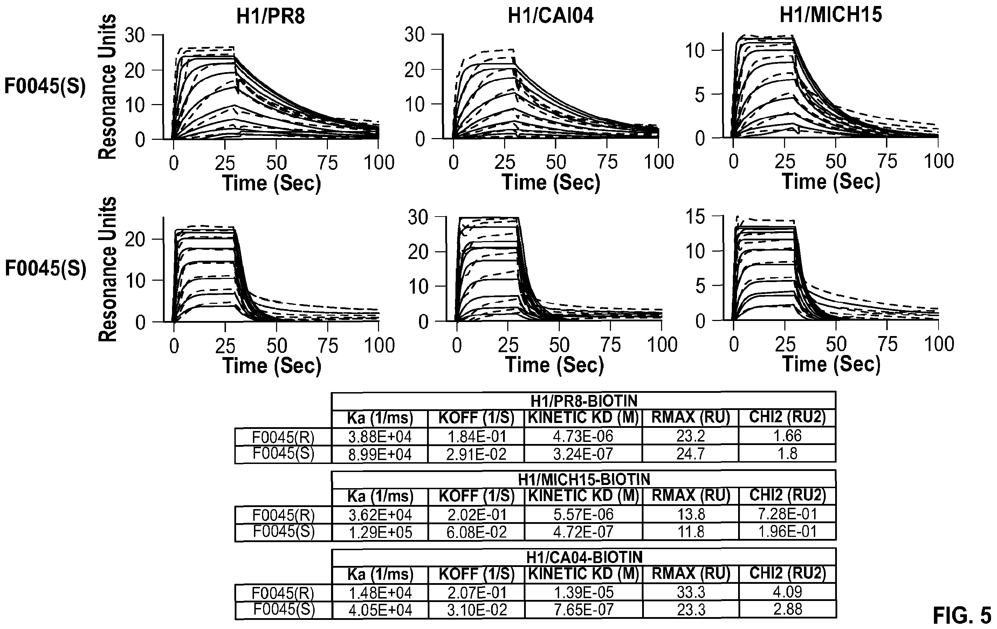

**Figure 5** shows SPR sensorgrams for HA-F0045(R)/(S) binding. Different concentrations of F0045(S) and F0045(R) were passed over immobilized HAs (H1/PR8, H1/Cal04 and H1/Mich15). Representative sensorgrams in resonance units (RU) plotted against time of injection are shown. Black lines are the experimental trace obtained from SPR experiments and the red are the best global fits (1: 1 Langmuir binding model) to the data used to calculate the association rate constants and dissociation rate constants.

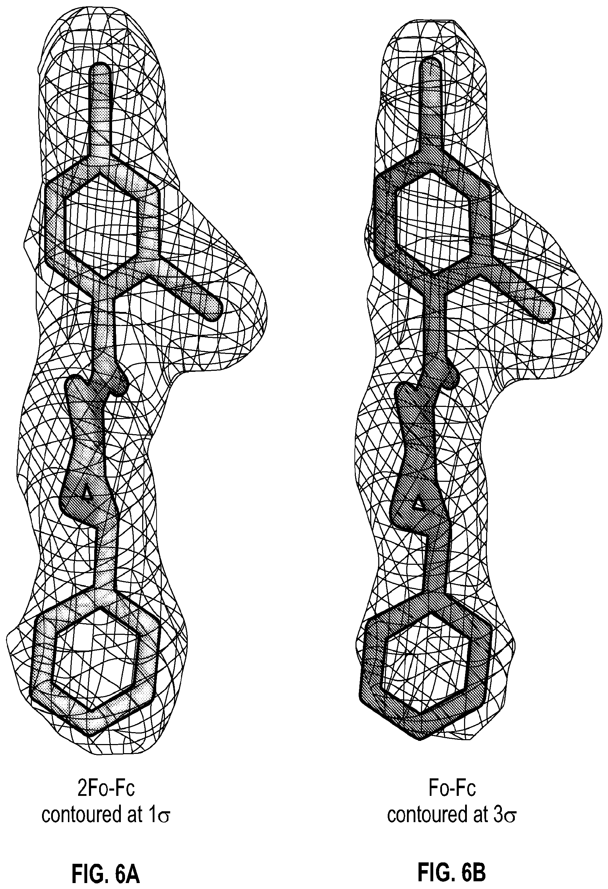

**Figures 6A-6B** show electron density maps for F0045(S) in complex with H1 PR8 HA. Figure 6A show a 2*F*ₒ-F*_{c}* map (sky blue mesh) generated from the final refined crystal structure of F0045(S) in complex with H1 PR8 HA, contoured at 1σ. Carbon, oxygen, nitrogen, and chlorine are represented in yellow, red, blue, and green, respectively. Figure 6B show an *Fₒ-F_{c}* electron density map (sky blue mesh) generated after molecular replacement using apo structures of H1 HA as model, contoured at 3σ. The final refined F0045(S) model is shown superimposed on the unbiased difference maps.

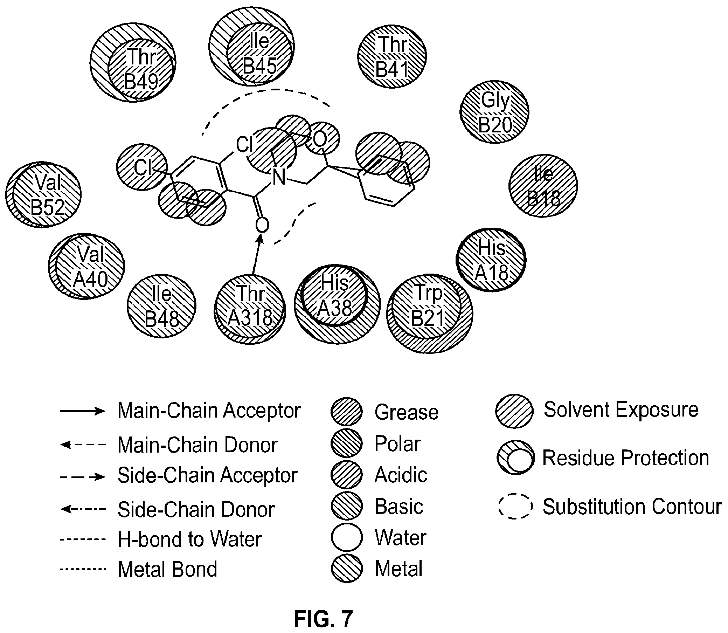

Figure 7 shows two-dimensional depiction of the F0045(S) binding sites on influenza H1/PR8 HA. Interactions of F0045(S) with binding-site residues. Arrow indicates hydrogen bond interaction.

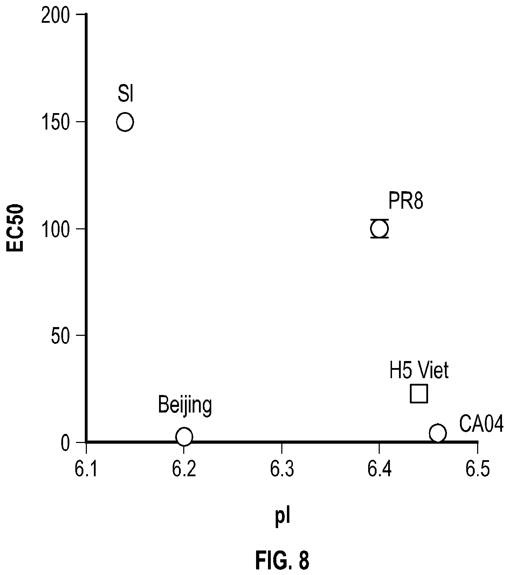

**Figure 8** shows theoretical pI values of influenza group 1 H1 (blue circle) and H5 (purple square) HAs vs. cellular EC₅₀ values.
**Figures 9A-9B** show LC-MS characterization of P7 peptide (Figure 9A) and P7-TAMRA probe (Figure 9B).
**Figures 10A-10B** show LC-MS characterization of F0045(S) (Figure 10A) and F0045(R) (Figure 10B).
**Figures 11A-11B** show NMR characterization of F0045(S) (Figure 11A) and F0045(R) (Figure 11B).

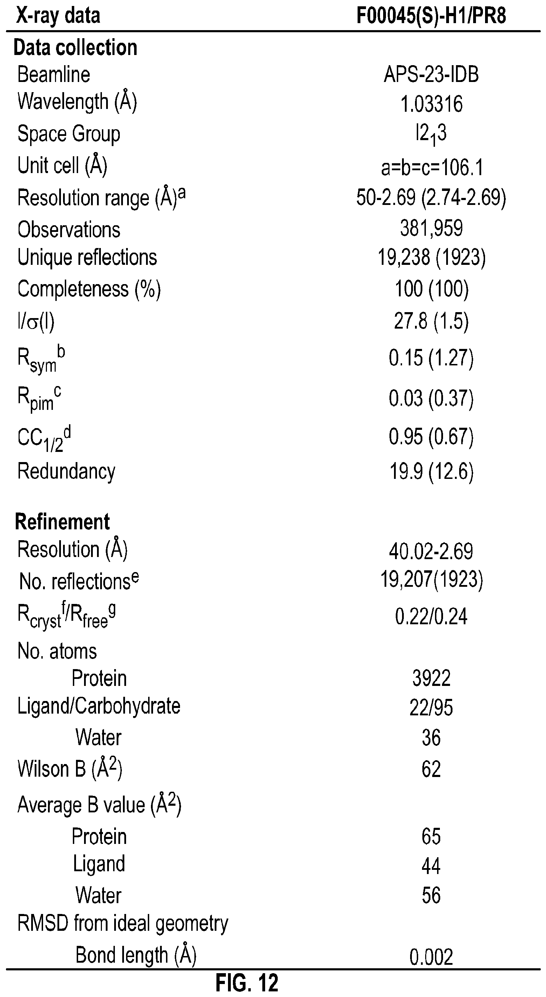

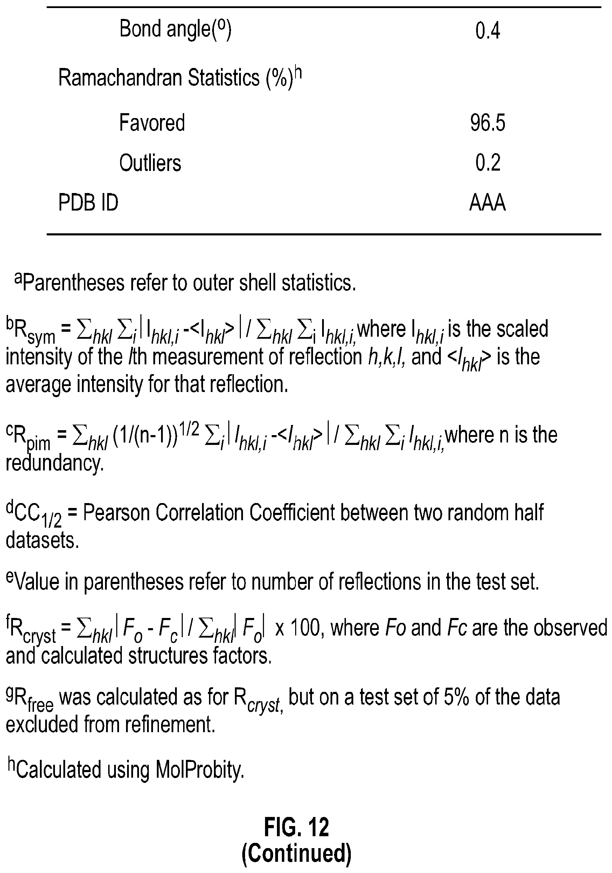

**Figure 12** is a table of data x-ray data collection and refinement statistics for F0045(S) in complex with H1/PR8 HA.

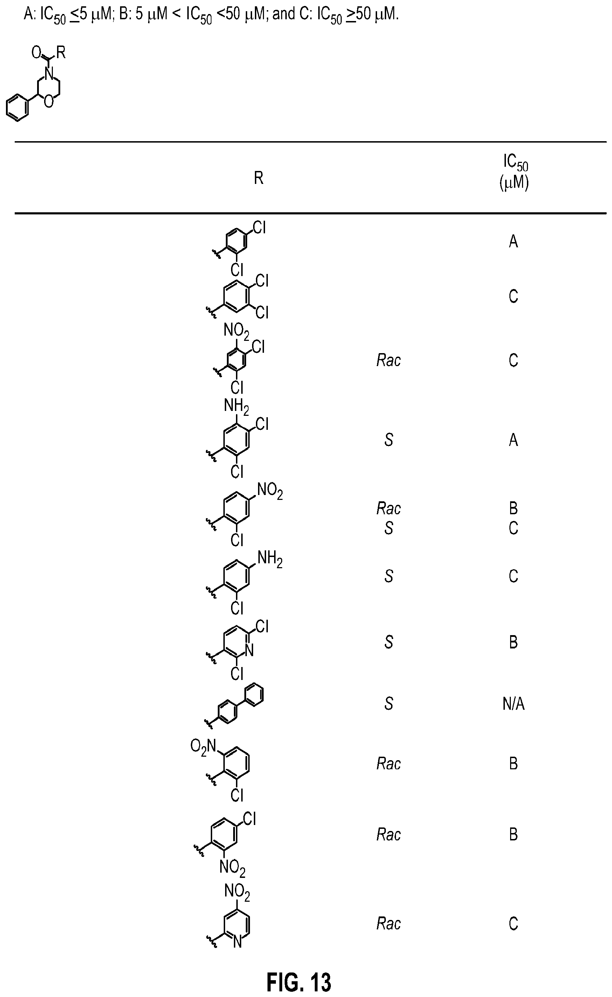

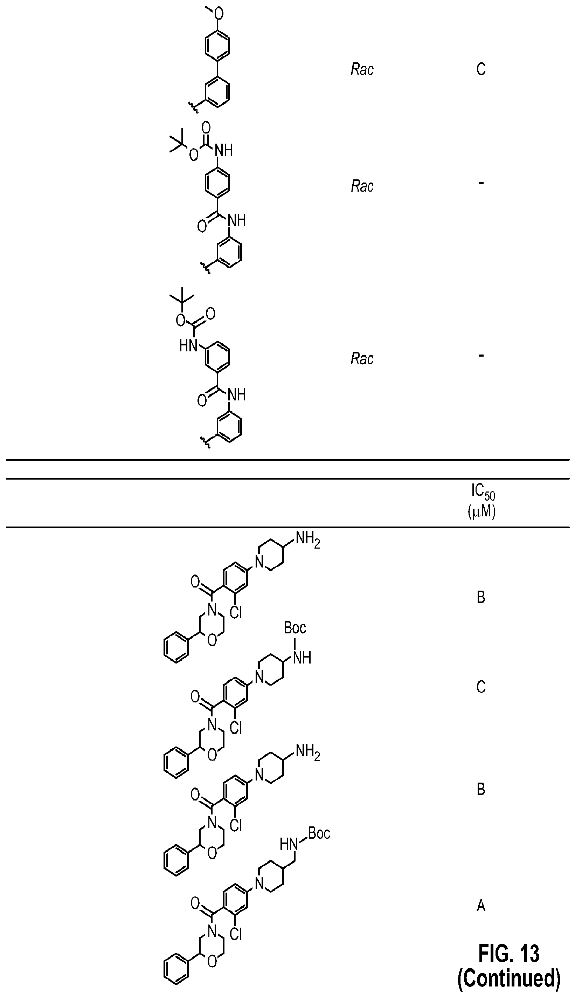

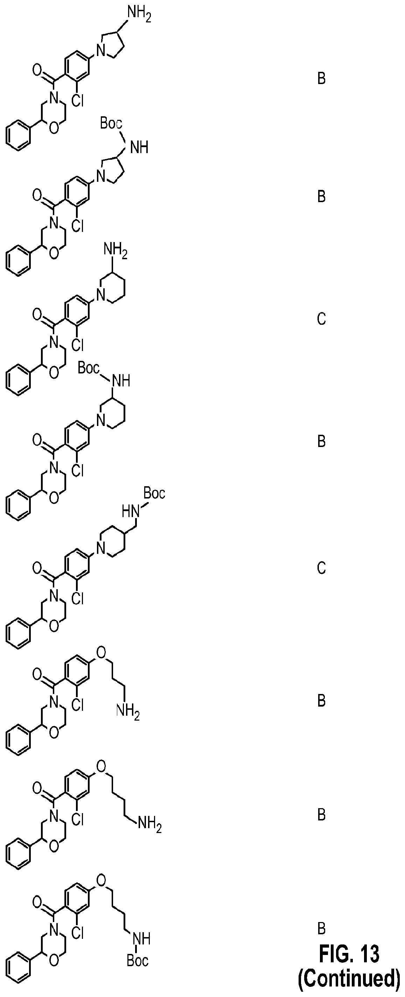

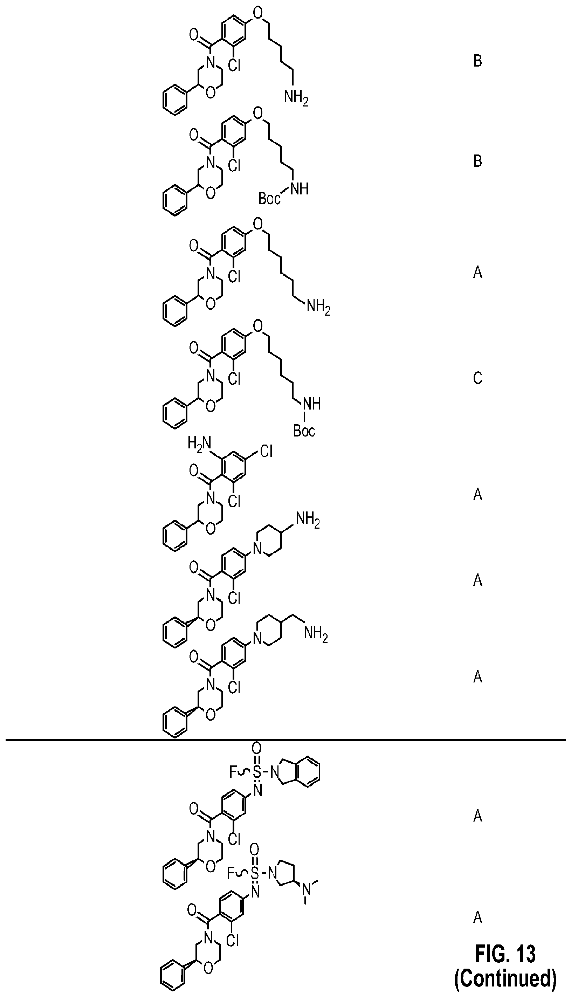

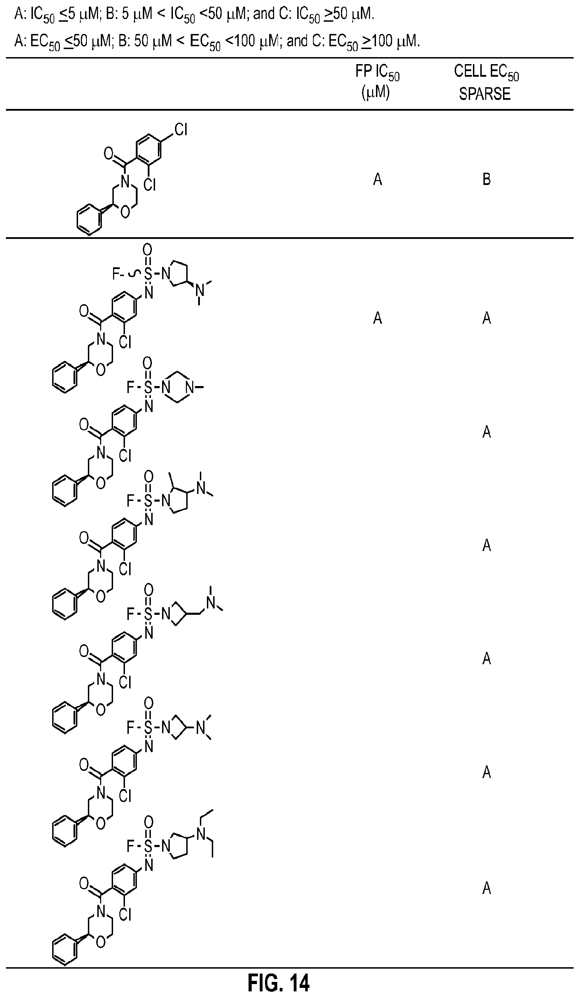

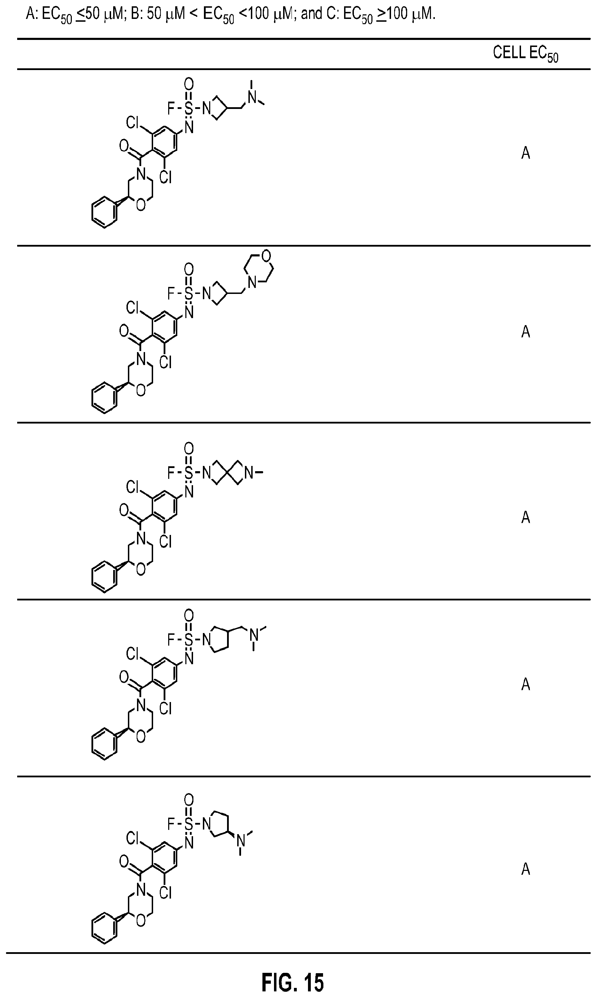

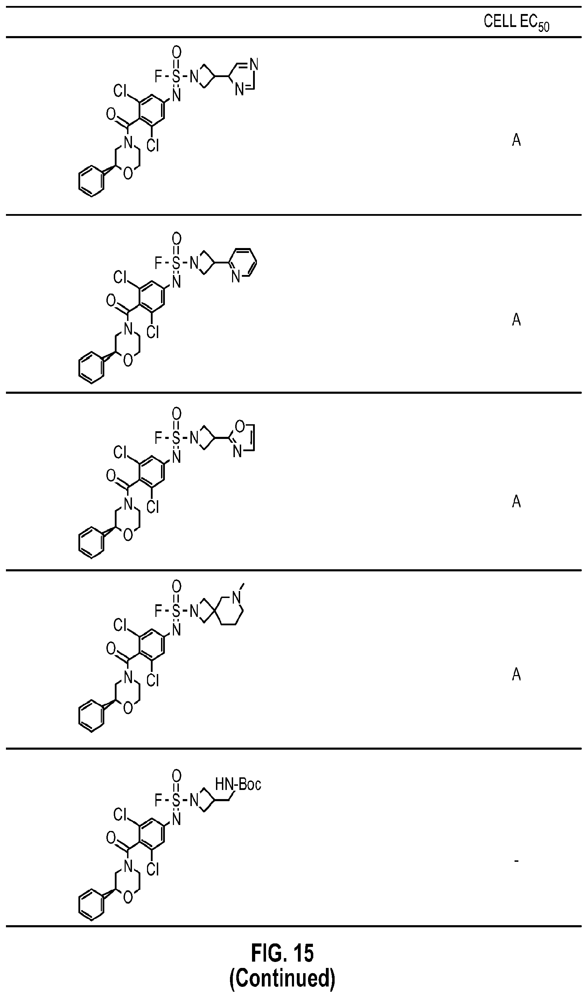

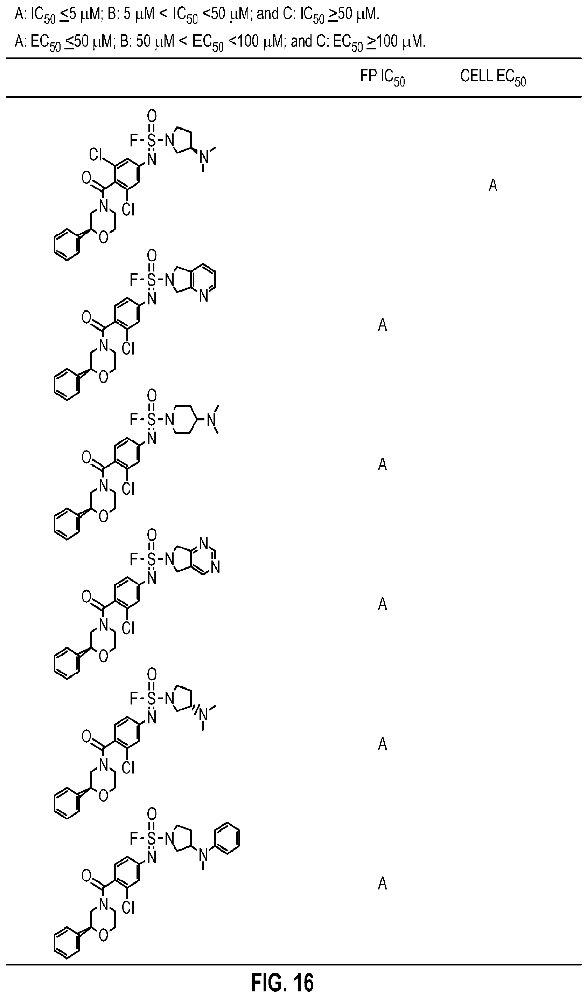

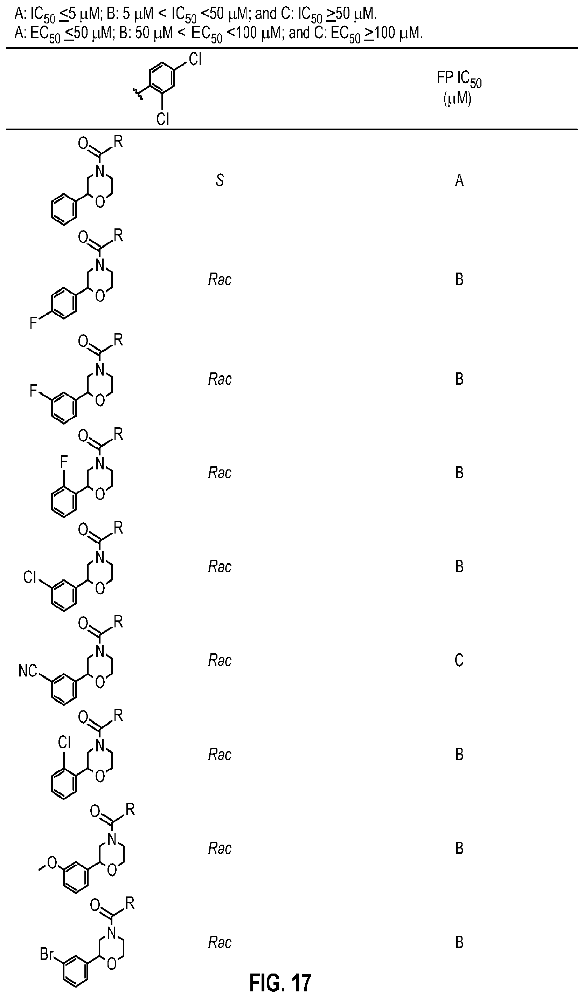

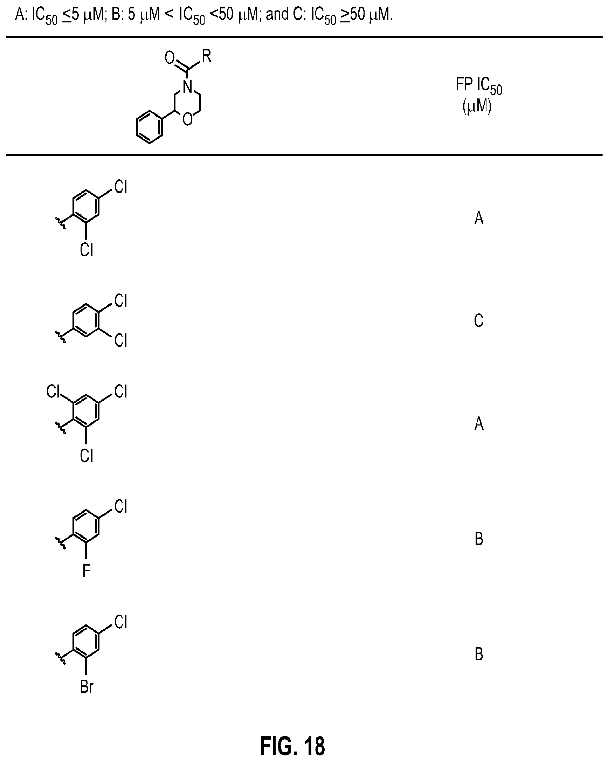

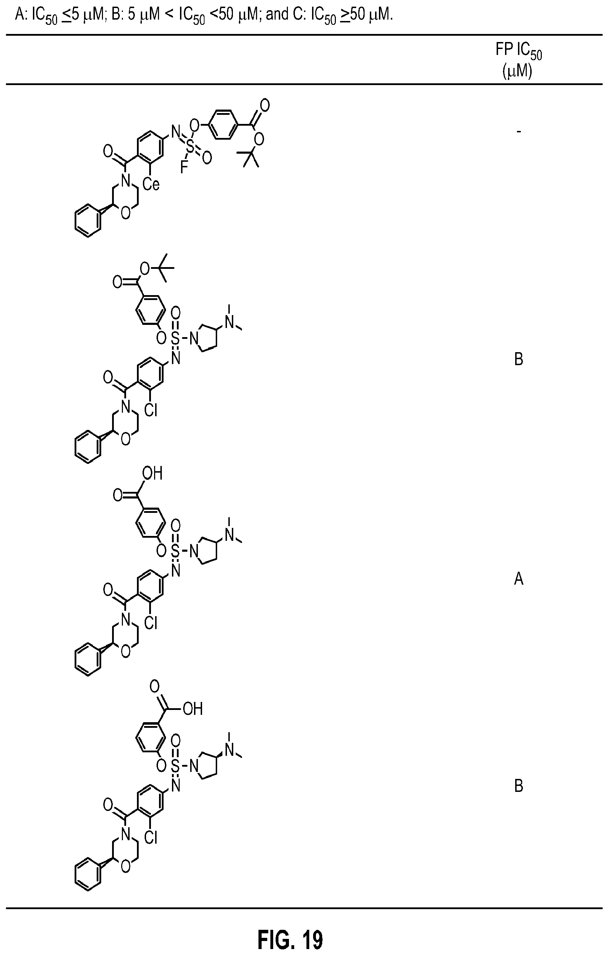

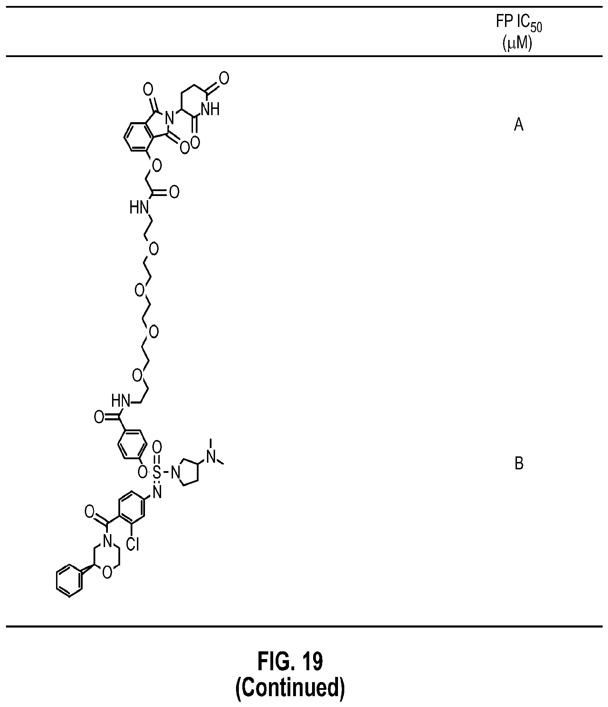

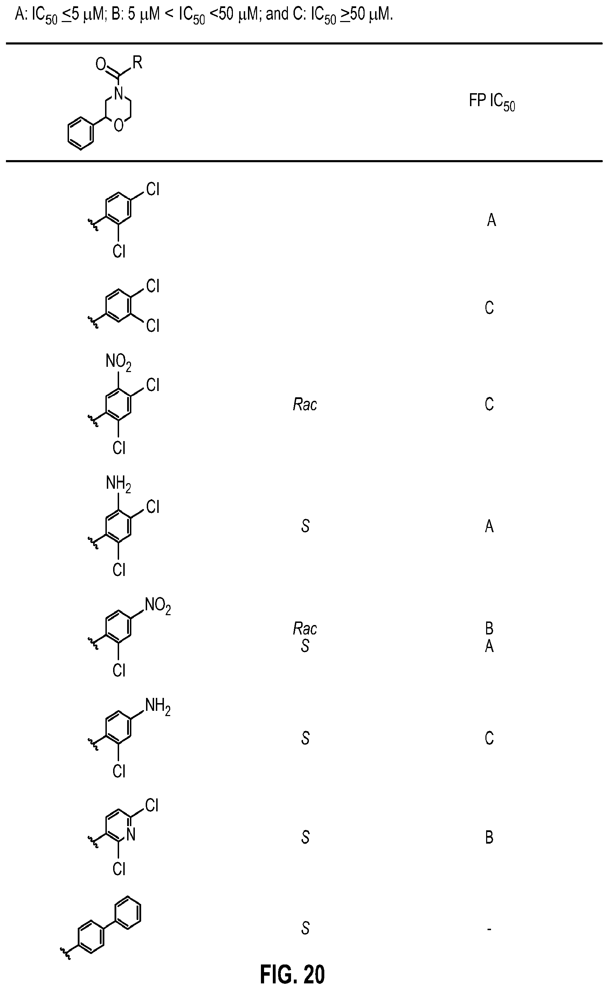

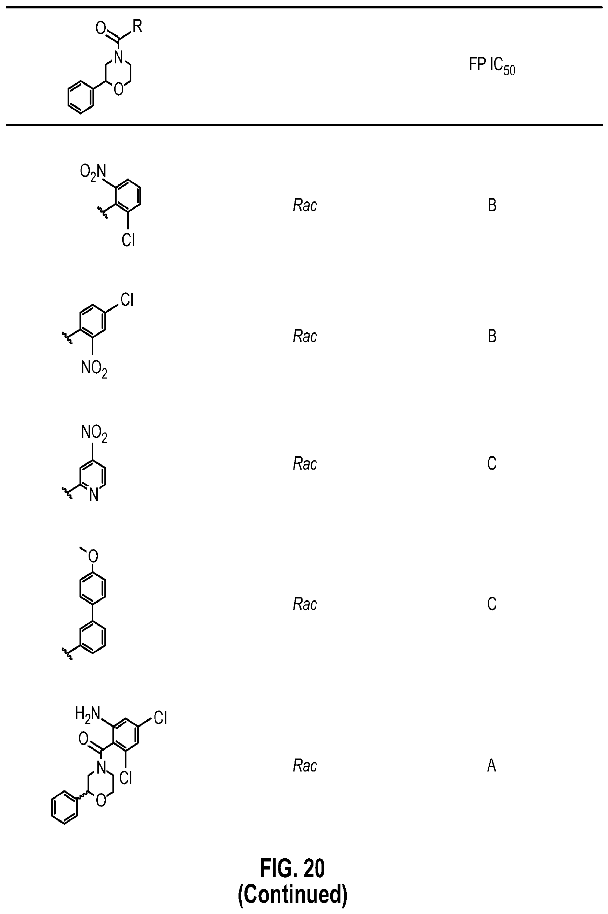

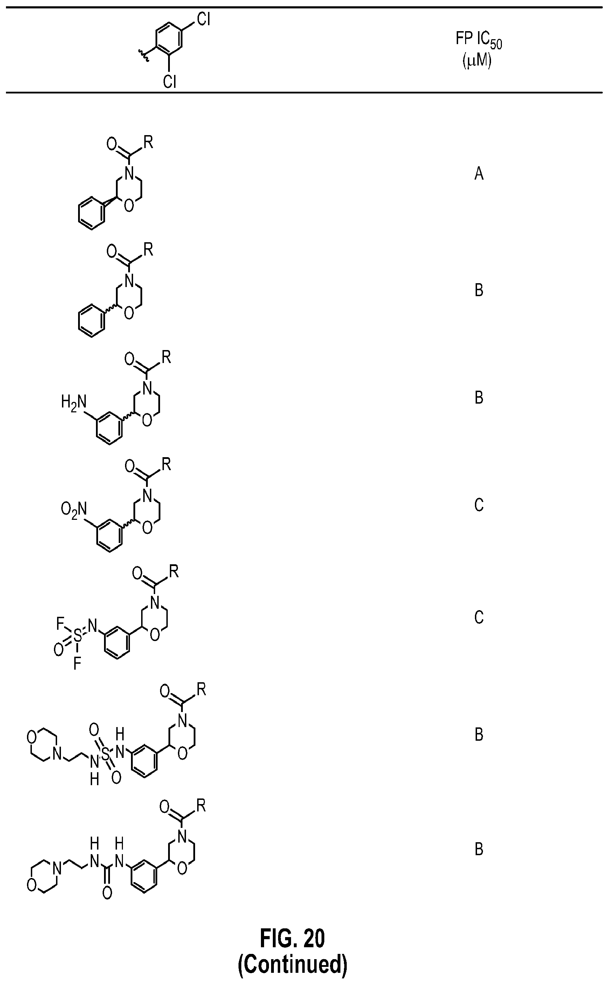

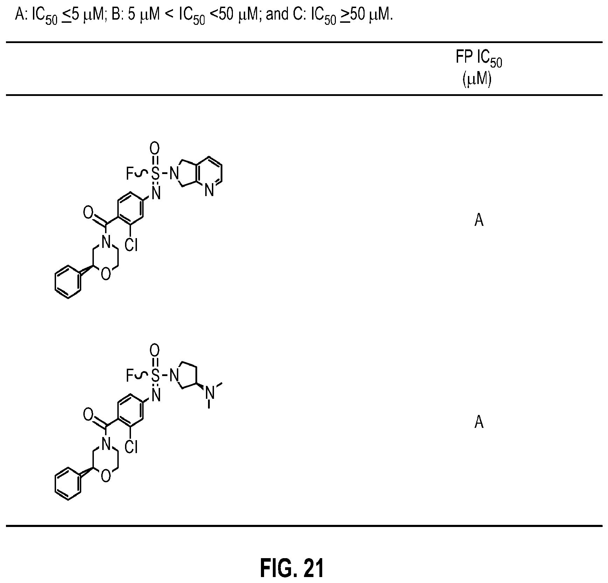

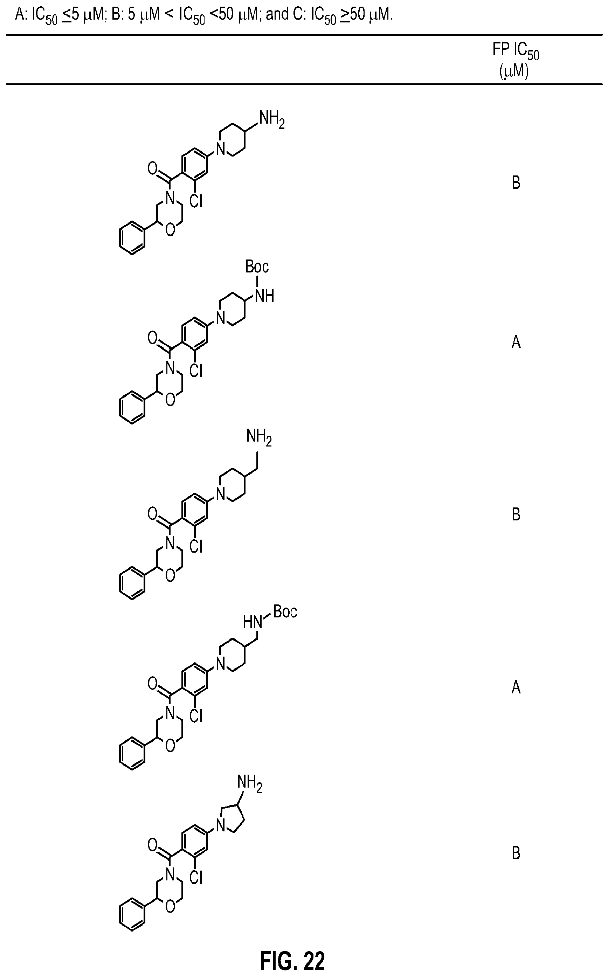

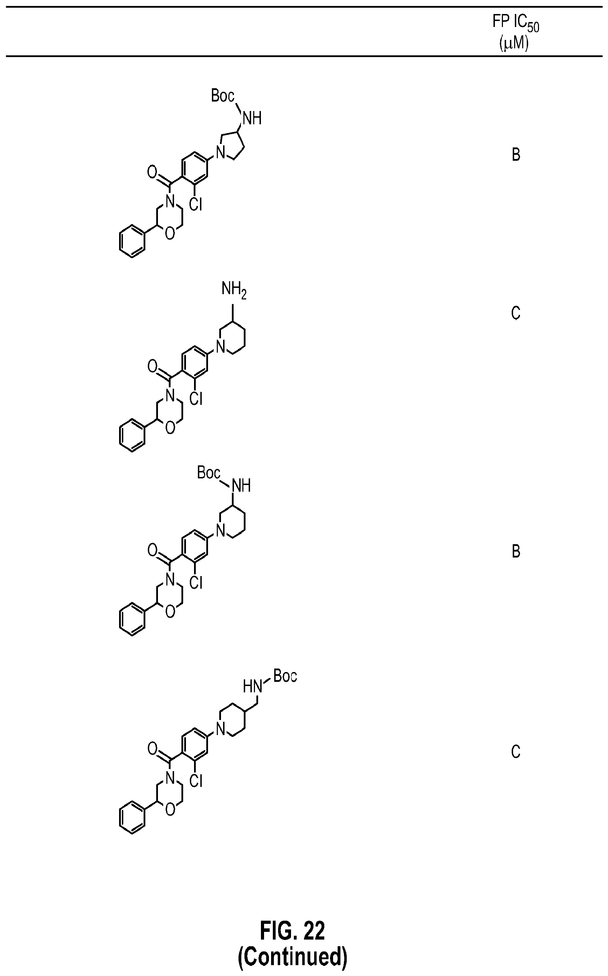

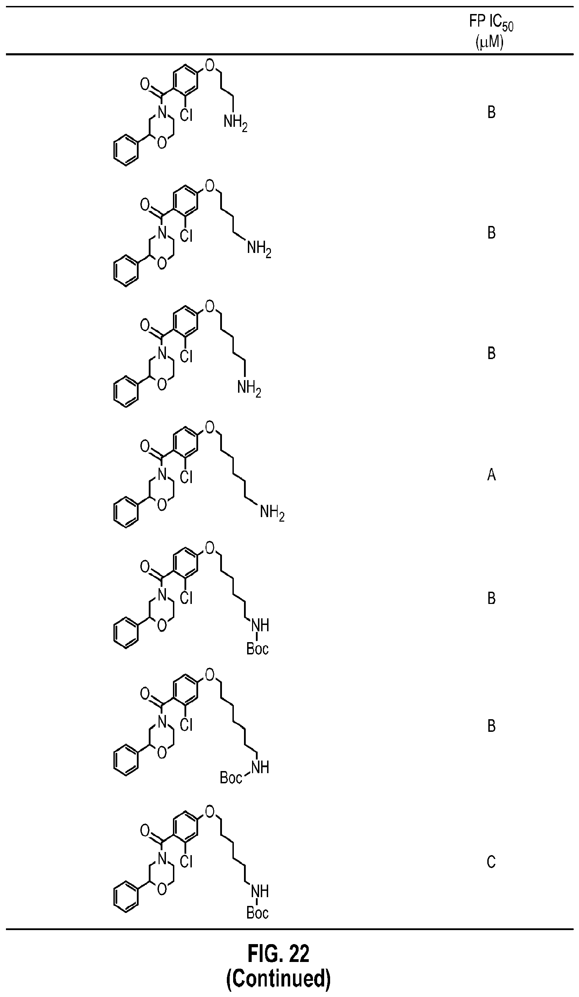

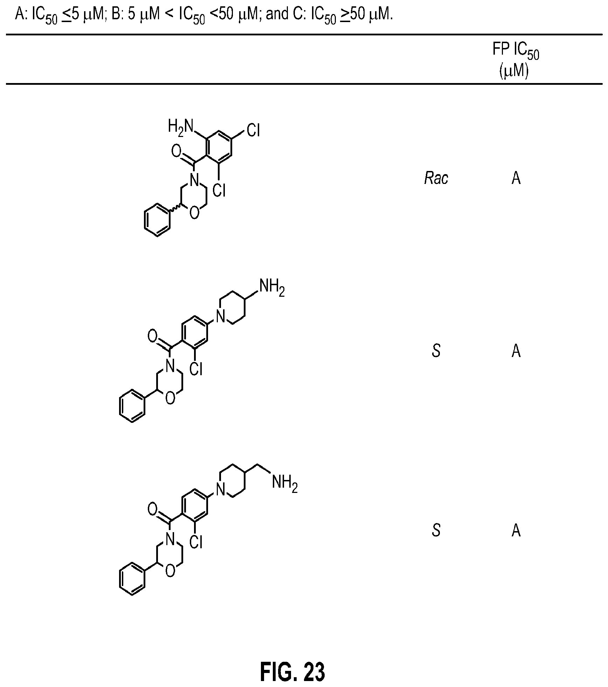

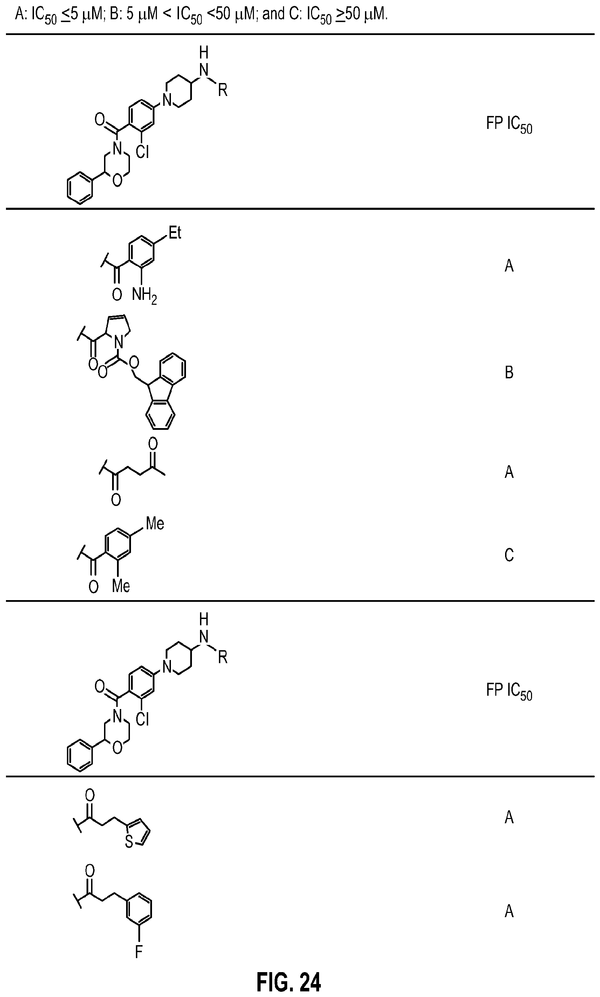

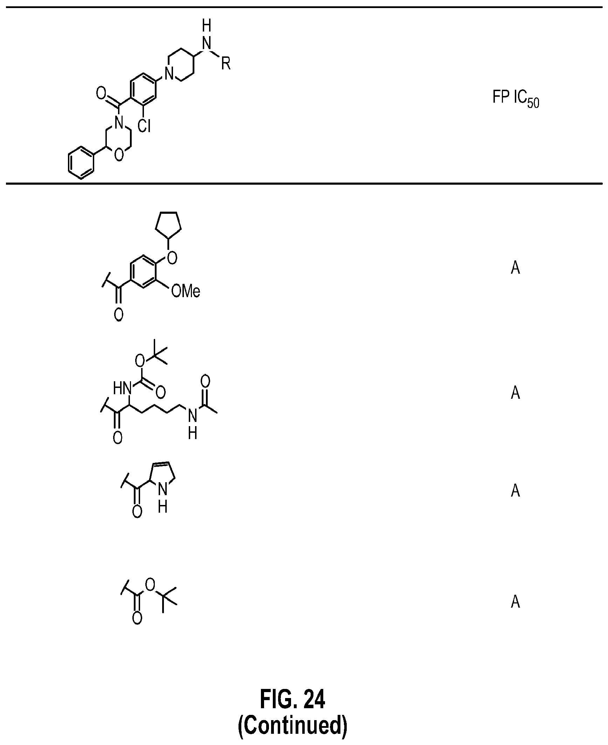

**Figures 13-24** are tables summarizing inhibition of HA by exemplary compounds.

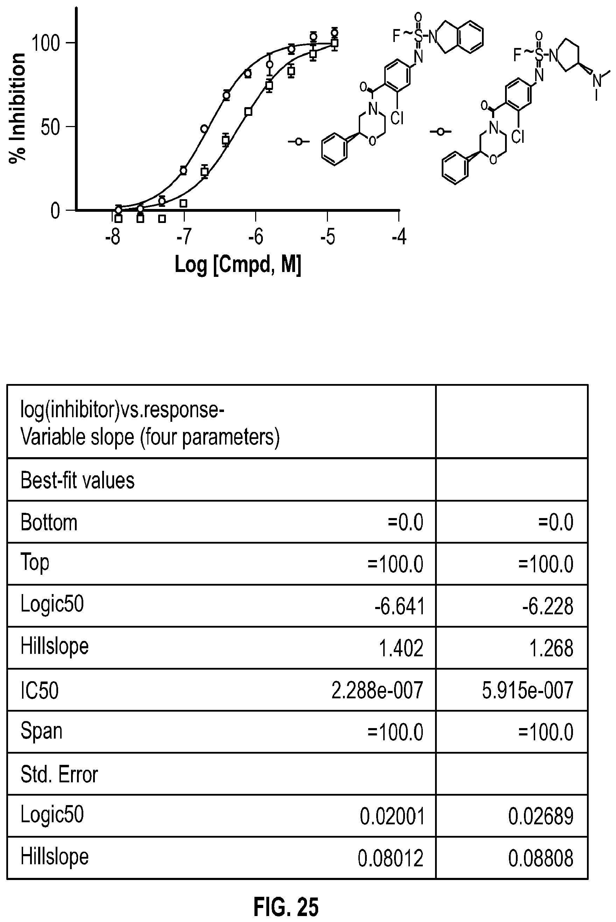

**Figure 25** is a graph summarizing inhibition of HA by exemplary compounds.

### DETAILED DESCRIPTION

In various embodiments, the disclosure relates to compounds that inhibit HA. The compounds will be useful for the treatment or prevention of HA-mediated condition, such as influenza.

### DEFINITIONS

For convenience, before further description of the present disclosure, some terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

In order for the present disclosure to be more readily understood, some terms and phrases are defined below and throughout the specification.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

Various compounds contained in compositions of the present disclosure may exist in particular geometric or stereoisomeric forms. In addition, polymers of the present disclosure may also be optically active. The present disclosure contemplates all such compounds, including cis- and trans-isomers, R- and S-enantiomers, diastereomers, (D)-isomers, (L)-isomers, the racemic mixtures thereof, and other mixtures thereof, as falling within the scope of the disclosure. Additional asymmetric carbon atoms may be present in a substituent such as an alkyl group. All such isomers, as well as mixtures thereof, are intended to be included in this disclosure.

If, for instance, a particular enantiomer of compound of the present disclosure is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

Structures depicted herein are also meant to include compounds that differ only in the presence of one or more isotopically enriched atoms. For example, compounds produced by the replacement of a hydrogen with deuterium or tritium, or of a carbon with a ¹³C- or ¹⁴C-enriched carbon are within the scope of this disclosure.

The term "prodrug" as used herein encompasses compounds that, under physiological conditions, are converted into therapeutically active agents. A common method for making a prodrug is to include selected moieties that are hydrolyzed under physiological conditions to reveal the desired molecule. In other embodiments, the prodrug is converted by an enzymatic activity of the host animal.

The phrase "pharmaceutically acceptable excipient" or "pharmaceutically acceptable carrier" as used herein means a pharmaceutically acceptable material, composition or vehicle, such as a liquid or solid filler, diluent, excipient, solvent or encapsulating material, involved in carrying or transporting the subject chemical from one organ or portion of the body, to another organ or portion of the body. Each carrier must be "acceptable" in the sense of being compatible with the other ingredients of the formulation, not injurious to the patient, and substantially non-pyrogenic. Some examples of materials which can serve as pharmaceutically acceptable carriers include: (1) sugars, such as lactose, glucose, and sucrose; (2) starches, such as corn starch and potato starch; (3) cellulose, and its derivatives, such as sodium carboxymethyl cellulose, ethyl cellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) glycols, such as propylene glycol; (11) polyols, such as glycerin, sorbitol, mannitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. In various embodiments, pharmaceutical compositions of the present disclosure are non-pyrogenic, i.e., do not induce significant temperature elevations when administered to a patient.

The term "pharmaceutically acceptable salts" refers to the relatively non-toxic, inorganic and organic acid addition salts of the compound(s). These salts can be prepared in situ during the final isolation and purification of the compound(s), or by separately reacting a purified compound(s) in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, naphthylate, mesylate, glucoheptonate, lactobionate, and laurylsulphonate salts, and the like. (See, for example, Berge et al. (1977) "Pharmaceutical Salts", J. Pharm. Sci. 66:1-19.)

In other cases, the compounds useful in the methods of the present disclosure may contain one or more acidic functional groups and, thus, are capable of forming pharmaceutically acceptable salts with pharmaceutically acceptable bases. The term "pharmaceutically acceptable salts" in these instances refers to the relatively non-toxic inorganic and organic base addition salts of a compound(s). These salts can likewise be prepared in situ during the final isolation and purification of the compound(s), or by separately reacting the purified compound(s) in its free acid form with a suitable base, such as the hydroxide, carbonate, or bicarbonate of a pharmaceutically acceptable metal cation, with ammonia, or with a pharmaceutically acceptable organic primary, secondary, or tertiary amine. Representative alkali or alkaline earth salts include the lithium, sodium, potassium, calcium, magnesium, and aluminum salts, and the like. Representative organic amines useful for the formation of base addition salts include ethylamine, diethylamine, ethylenediamine, ethanolamine, diethanolamine, piperazine, and the like (see, for example, Berge et al., *supra).*

A "therapeutically effective amount" (or "effective amount") of a compound with respect to use in treatment, refers to an amount of the compound in a preparation which, when administered as part of a desired dosage regimen (to a mammal, such as a human) alleviates a symptom, ameliorates a condition, or slows the onset of disease conditions according to clinically acceptable standards for the disorder or condition to be treated or the cosmetic purpose, e.g., at a reasonable benefit/risk ratio applicable to any medical treatment.

The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic, (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

The term "patient" or "subject" refers to a mammal in need of a particular treatment. In various embodiments, a patient or subject is a primate, canine, feline, or equine. In various embodiments, a patient or subject is a bird. In various embodiments, the bird is a domesticated bird, such as chicken. In various embodiments, the bird is a fowl. In various embodiments, a patient or subject is a human.

An aliphatic chain comprises the classes of alkyl, alkenyl and alkynyl defined below. A straight aliphatic chain is limited to unbranched carbon chain moieties. As used herein, the term "aliphatic group" refers to a straight chain, branched-chain, or cyclic aliphatic hydrocarbon group and includes saturated and unsaturated aliphatic groups, such as an alkyl group, an alkenyl group, or an alkynyl group.

"Alkyl" refers to a fully saturated branched or unbranched carbon chain moiety having the number of carbon atoms specified, or up to 30 carbon atoms if no specification is made. For example, alkyl of 1 to 8 carbon atoms refers to moieties such as methyl, ethyl, propyl, butyl, pentyl, hexyl, heptyl, and octyl, and those moieties which are positional isomers of these moieties. Alkyl of 10 to 30 carbon atoms includes decyl, undecyl, dodecyl, tridecyl, tetradecyl, pentadecyl, hexadecyl, heptadecyl, octadecyl, nonadecyl, eicosyl, heneicosyl, docosyl, tricosyl and tetracosyl. In various embodiments, a straight chain or branched chain alkyl has 30 or fewer carbon atoms in its backbone (e.g., C₁-C₃₀ for straight chains, C₃-C₃₀ for branched chains), and in various embodiments 20 or fewer. Alkyl groups may be substituted or unsubstituted.

As used herein, the term "alkylene" refers to an alkyl group having the specified number of carbons, for example from 2 to 12 carbon atoms, that contains two points of attachment to the rest of the compound on its longest carbon chain. Non-limiting examples of alkylene groups include methylene -(CH₂)-, ethylene -(CH₂CH₂)-, n-propylene - (CH₂CH₂CH₂)-, isopropylene -(CH₂CH(CH₃))-, and the like. Alkylene groups can be branched or unbranched carbon chain moiety, and may be optionally substituted with one or more substituents.

"Cycloalkyl" means mono- or bicyclic or bridged or spirocyclic, or polycyclic saturated carbocyclic rings, each having from 3 to 12 carbon atoms. In various aspects, cycloalkyls have from 3-10 carbon atoms in their ring structure, or 3-6 carbons in the ring structure. Cycloalkyl groups may be substituted or unsubstituted.

Unless the number of carbons is otherwise specified, "lower alkyl," as used herein, means an alkyl group, as defined above, but having from one to ten carbons, or from one to six carbon atoms in its backbone structure such as methyl, ethyl, n-propyl, isopropyl, n-butyl, isobutyl, sec-butyl, and tert-butyl. Likewise, "lower alkenyl" and "lower alkynyl" have similar chain lengths. In various embodiments, a substituent designated herein as alkyl is a lower alkyl.

"Alkenyl" refers to any branched or unbranched unsaturated carbon chain moiety having the number of carbon atoms specified, or up to 26 carbon atoms if no limitation on the number of carbon atoms is specified; and having one or more double bonds in the moiety. Alkenyl of 6 to 26 carbon atoms is exemplified by hexenyl, heptenyl, octenyl, nonenyl, decenyl, undecenyl, dodenyl, tridecenyl, tetradecenyl, pentadecenyl, hexadecenyl, heptadecenyl, octadecenyl, nonadecenyl, eicosenyl, heneicosoenyl, docosenyl, tricosenyl, and tetracosenyl, in their various isomeric forms, where the unsaturated bond(s) can be located anywhere in the moiety and can have either the (Z) or the (E) configuration about the double bond(s).

"Alkynyl" refers to hydrocarbyl moieties of the scope of alkenyl, but having one or more triple bonds in the moiety.

The term "alkylthio" refers to an alkyl group, as defined above, having a sulfur moiety attached thereto. In various embodiments, the "alkylthio" moiety is represented by one of -(S)-alkyl, -(S)-alkenyl, -(S)-alkynyl, and -(S)-(CH₂)ₘ-R¹, wherein m and R¹ are defined below. Representative alkylthio groups include methylthio, ethylthio, and the like. The terms "alkoxyl" or "alkoxy" as used herein refers to an alkyl group, as defined below, having an oxygen moiety attached thereto. Representative alkoxyl groups include methoxy, ethoxy, propoxy, tert-butoxy, and the like. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, -O-alkynyl, -O-(CH₂)ₘ-R₁₀, where m and R₁₀ are described below.

The terms "amine" and "amino" are art-recognized and refer to both unsubstituted and substituted amines, e.g., a moiety that can be represented by the formulae: 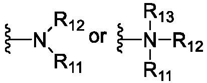 wherein R₁₁, R₁₂ and R₁₃ each independently represent a hydrogen, an alkyl, an alkenyl, -(CH₂)ₘ-R₁₀, or R₁₁ and R₁₂ taken together with the N atom to which they are attached complete a heterocycle having from 4 to 8 atoms in the ring structure; R₁₀ represents an alkenyl, aryl, cycloalkyl, a cycloalkenyl, a heterocyclyl, or a polycyclyl; and m is zero or an integer in the range of 1 to 8. In various embodiments, only one of R₁₁ or R₁₂ can be a carbonyl, e.g., R₁₁, R₁₂, and the nitrogen together do not form an imide. In various embodiments, R₁₁ and R₁₂ (and optionally R₁₃) each independently represent a hydrogen, an alkyl, an alkenyl, or -(CH₂)ₘ- R₁₀. Thus, the term "alkylamine" as used herein means an amine group, as defined above, having a substituted or unsubstituted alkyl attached thereto, i.e., at least one of R₁₁ and R₁₂ is an alkyl group. In various embodiments, an amino group or an alkylamine is basic, meaning it has a conjugate acid with a pKₐ > 7, i.e., the protonated forms of these functional groups have pKₐs relative to water above about 7.

The term "amide", as used herein, refers to a group 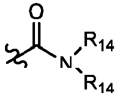 wherein each R₁₄ independently represent a hydrogen or hydrocarbyl group, or two R₁₄ are taken together with the N atom to which they are attached complete a heterocycle having from 4 to 8 atoms in the ring structure.

The term "aryl" as used herein includes 5- to 12-membered unsubstituted aromatic groups in which each atom of the ring is carbon (i.e., carbocyclic aryl) or where one or more atoms are heteroatoms (i.e., heteroaryl). In various aspects, aryl groups include 6- to 10-membered rings The term "aryl" includes polycyclic ring systems having two or more cyclic rings in which two or more carbons are common to two adjoining rings wherein at least one of the rings is aromatic, e.g., the other cyclic rings can be cycloalkyls, cycloalkenyls, cycloalkynyls, aryls, heteroaryls, and/or heterocyclyls. Carbocyclic aryl groups include benzene, naphthalene, phenanthrene, phenol, aniline, and the like. Heteroaryl groups include substituted or unsubstituted aromatic 3- to 12-membered ring structures, 5- to 12-membered rings, or 5- to 10-membered rings, whose ring structures include one to four heteroatoms. Heteroaryl groups include, for example, pyrrole, furan, thiophene, imidazole, oxazole, thiazole, triazole, pyrazole, pyridine, pyrazine, pyridazine and pyrimidine, and the like. Aryl and heteroaryl can be monocyclic, bicyclic, or polycyclic. Each instance of an aryl group may be independently optionally substituted, *i.e.,* unsubstituted (an "unsubstituted aryl") or substituted (a "substituted aryl") with one or more substituents; e.g., for instance from 1 to 5 substituents, 1 to 4 substituents, 1 to 3 substituents, 1 to 2 substituents or just 1 substituent. The aromatic ring may be substituted at one or more ring positions with one or more substituents, such as halogen, azide, alkyl, aryl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, fluoroalkyl (such as trifluromethyl), cyano, or the like. For example, in various embodiments, the aryl group can be an unsubstituted C₅-C₁₂ aryl and in various embodiments, the aryl group can be a substituted C₅-C₁₀ aryl.

The term "halo", "halide", or "halogen" as used herein means halogen and includes, for example, and without being limited thereto, fluoro, chloro, bromo, iodo and the like, in both radioactive and non-radioactive forms. In various embodiments, halo is selected from the group consisting of fluoro, chloro and bromo.

The terms "heterocyclyl" or "heterocyclic group" refer to 3- to 12-membered ring structures, 5- to 12-membered rings, or 5- to 10-membered rings, whose ring structures include one to four heteroatoms. Heterocycles can be monocyclic, bicyclic, spirocyclic, or polycyclic. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, as for example, halogen, alkyl, aryl, alkenyl, alkynyl, cycloalkyl, hydroxyl, amino, nitro, sulfhydryl, imino, amido, phosphate, phosphonate, phosphinate, carbonyl, carboxyl, silyl, sulfamoyl, sulfinyl, ether, alkylthio, sulfonyl, ketone, aldehyde, ester, a heterocyclyl, an aromatic or heteroaromatic moiety, -CF₃, -CN, and the like.

The term "carbonyl" is art-recognized and includes such moieties as can be represented by the formula: 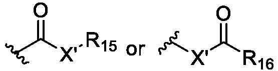 wherein X' represents a carbon, an oxygen, a nitrogen, or a sulfur, and R₁₅ represents a hydrogen, an alkyl, an alkenyl, -(CH₂)ₘ-R₁₀ or a pharmaceutically acceptable salt, R₁₆ represents a hydrogen, an alkyl, an alkenyl or -(CH₂)ₘ-R₁₀, where m and R₁₀ are as defined above. Where X' is an oxygen and R₁₅ or R₁₆ is not hydrogen, the formula represents an "ester." Where X' is an oxygen, and R₁₅ is as defined above, the moiety is referred to herein as a carboxyl group, and particularly when R₁₅ is a hydrogen, the formula represents a "carboxylic acid". Where X' is an oxygen, and R₁₆ is a hydrogen, the formula represents a "formate." In general, where the oxygen atom of the above formula is replaced by a sulfur, the formula represents a "thiocarbonyl" group. Where X' is a sulfur and R₁₅ or R₁₆ is not hydrogen, the formula represents a "thioester" group. Where X' is a sulfur and R₁₅ is a hydrogen, the formula represents a "thiocarboxylic acid" group. Where X' is a sulfur and R₁₆ is a hydrogen, the formula represents a "thioformate" group. On the other hand, where X' is a carbon atom, and R₁₅ is not hydrogen, the above formula represents a "ketone" group. Where X' is a carbon atom, and R₁₅ is a hydrogen, the above formula represents an "aldehyde" group.

As used herein, the term "nitro" means -NO₂; the term "halogen" designates - F, -Cl, -Br, or -I; the term "sulfhydryl" means -SH; the term "hydroxyl" means -OH; the term "sulfonyl" means -SO₂-; the term "azido" means -N₃; the term "cyano" means -CN; the term "isocyanato" means -NCO; the term "thiocyanato" means -SCN; the term "isothiocyanato" means -NCS; and the term "cyanato" means -OCN.

As used herein, the definition of each expression, e.g., alkyl, m, n, etc., when it occurs more than once in any structure, is intended to be independent of its definition elsewhere in the same structure.

The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this disclosure, the heteroatoms such as nitrogen may have hydrogen substituents and/or any permissible substituents of organic compounds described herein which satisfy the valences of the heteroatoms. Substituents can include any substituents described herein, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxy, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aryl, or an aromatic or heteroaromatic moiety. In various embodiments, the substituents on substituted alkyls are selected from C₁₋₆ alkyl, C₃₋₆ cycloalkyl, halogen, carbonyl, cyano, or hydroxyl. In various embodiments, the substituents on substituted alkyls are selected from fluoro, carbonyl, cyano, or hydroxyl. It will be understood by those skilled in the art that substituents can themselves be substituted, if appropriate.

For purposes of this disclosure, the chemical elements are identified in accordance with the Periodic Table of the Elements, CAS version, Handbook of Chemistry and Physics, 67th Ed., 1986-87, inside cover.

### EXEMPLARY COMPOUNDS OF THE DISCLOSURE

In various embodiments, the disclosure relates to a compound of Formula (I) or (II): 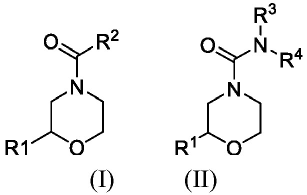 wherein
R¹ is substituted or unsubstituted aryl, or substituted or unsubstituted heteroaryl;
R² is substituted or unsubstituted aryl, or substituted or unsubstituted heterocyclyl;
R³ is selected from absent H, alkyl, -alkylene-(heterocyclyl), -alkylene-(aryl), - alkylene-(cycloalkyl), -alkylene-O-R⁵, -alkylene-C(O)N(R⁵)₂, -alkylene-C(O)R⁵, -alkylene-C(O)₂R⁵, -alkylene-N(R⁵)₂, -alkylene-N(R)C(OR⁵), -alkylene-N(R⁵)C(O)N(R⁵)-, -alkylene-N(R⁵)SO₂-R⁵, -alkylene-S-R⁵, -aryl, -arylene-(heterocyclyl), -arylene-alkyl, -arylene-(cycloalkyl), -arylene-O-R⁵, -arylene-C(O)N(R⁵)₂, -arylene-C(O)R⁵, -arylene-C(O)₂R⁵, - arylene-N(R⁵)₂, -arylene-N(R)C(OR⁵), -arylene-N(R⁵)C(O)N(R⁵)-, -arylene-N(R⁵)SO₂-R⁵ - arylene-S-R⁵, -heterocyclyl, -heterocyclylene-(alkyl), -heterocyclylene-(aryl), - heterocyclylene-(cycloalkyl), -heterocyclylene-(cycloalkyl), -heterocyclylene-O-R⁵, - heterocyclylene-C(O)N(R⁵)₂, -heterocyclylene-C(O)R⁵, -heterocyclylene-C(O)₂R⁵, - heterocyclylene-N(R⁵)₂, -heterocyclylene-N(R)C(OR⁵), -heterocyclylene-N(R⁵)C(O)N(R⁵)-,-heterocyclylene-N(R⁵)SO₂-R⁵, -heterocyclylene-S-R⁵, -cycloalkyl, -cycloalkylene-(alkyl), - cycloalkylene-(aryl), -cycloalkylene-(cycloalkyl), cycloalkylene(heterocycloalkyl), - cycloalkylene -O-R⁵, -cycloalkylene-C(O)N(R⁵)₂, -cycloalkylene-C(O)R⁵, -cycloalkylene-C(O)₂R⁵, -cycloalkylene-N(R⁵)₂, -cycloalkylene-N(R)C(OR⁵), -cycloalkylene-N(R⁵)C(O)N(R⁵)-, -cycloalkylene-N(R⁵)SO₂-R⁵, -alkylene-N(H)-alkylene-C(O)OR⁵, - alkylene-C(O)OR⁵, -arylene-alkylene-aryl, -C(O)R⁵, -C(O)₂R⁵, -alkylene-S-alkyl, -alkylene-S-S-R⁵, -C(O)-alkyl, -C(O)-alkylene-N(R⁵)₂, -C(O)₂R⁵, -aryl-alkylene-N(R⁵)C(O)-R⁵, - cycloalkylene-N(R⁵)S(O)(F)-R⁵; -SO₂-NR⁵, -S(O)(F)-NR⁵;
R⁴ is selected from alkyl, -alkylene-(heterocyclyl), -alkylene-(aryl), -alkylene-(cycloalkyl), -alkylene-O-R⁵, -alkylene-C(O)N(R⁵)₂, -alkylene-C(O)R⁵, -alkylene-C(O)₂R⁵, - alkylene-N(R⁵)₂, -alkylene-N(R)C(OR⁵), -alkylene-N(R⁵)C(O)N(R⁵)-, -alkylene-N(R⁵)SO₂-R⁵*,* -alkylene-S-R⁵, -aryl, -arylene-(heterocyclyl), -arylene-alkyl, -arylene-(cycloalkyl), - arylene-O-R⁵, -arylene-C(O)N(R⁵)₂, -arylene-C(O)R⁵, -arylene-C(O)₂R⁵, -arylene-N(R⁵)₂, - arylene-N(R)C(OR⁵), -arylene-N(R⁵)C(O)N(R⁵)-, -arylene-N(R⁵)SO₂-R⁵, -arylene-S-R⁵, - heterocyclyl, -heterocyclylene-(alkyl), -heterocyclylene-(aryl), -heterocyclylene-(cycloalkyl), -heterocyclylene-(cycloalkyl), -heterocyclylene-O-R⁵, -heterocyclylene-C(O)N(R⁵)₂, - heterocyclylene-C(O)R⁵, -heterocyclylene-C(O)₂R⁵, -heterocyclylene-N(R⁵)₂,-heterocyclylene-N(R)C(OR⁵), -heterocyclylene-N(R⁵)C(O)N(R⁵)-, -heterocyclylene-N(R⁵)SO₂-R⁵, -heterocyclylene-S-R⁵, -cycloalkyl, -cycloalkylene-(alkyl), -cycloalkylene-(aryl), -cycloalkylene-(cycloalkyl), cycloalkylene(heterocycloalkyl), -cycloalkylene -O-R⁵,-cycloalkylene-C(O)N(R⁵)₂, -cycloalkylene-C(O)R⁵, -cycloalkylene-C(O)₂R⁵, -cycloalkylene-N(R⁵)₂, -cycloalkylene-N(R)C(OR⁵), -cycloalkylene-N(R⁵)C(O)N(R⁵)-, -cycloalkylene-N(R⁵)SO₂-R⁵, -alkylene-N(H)-alkylene-C(O)OR⁵, -alkylene-C(O)OR⁵, -arylene-alkylene-aryl, -C(O)R⁵, -C(O)₂R⁵, -alkylene-S-alkyl, -alkylene-S-S-R⁵, -C(O)-alkyl, -C(O)-alkylene-N(R⁵)₂, -C(O)₂R⁵, -aryl-alkylene-N(R⁵)C(O)-R⁵, -cycloalkylene-N(R⁵)S(O)(F)-R⁵; -SO₂-NR⁵ -S(O)(F)-NR⁵; or R³ and R⁴ can be taken together to form a substituted of unsubstituted heterocyclyl; and
R⁵ is H or alkyl or aryl or -alkylene-aryl;
or a salt thereof.

In various aspects of the disclosure, the compound is of formula (II) 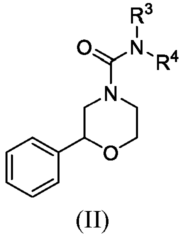 wherein R³and R⁴ are as described above.

In various embodiments, the compound is a compound of Formula (I). In various embodiments, the compound is a compound of Formula (II).

R¹ is unsubstituted phenyl. Other values of R1 are not part of the invention.

In various embodiments, R¹ can be

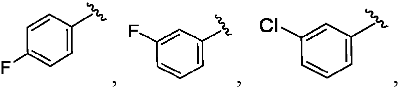

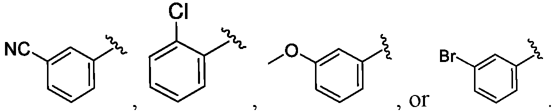

In various embodiments, R¹ can be

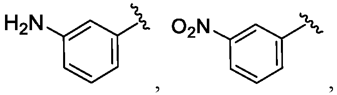

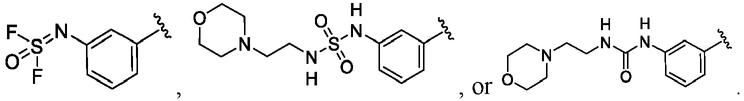

In various embodiments, R¹ can be

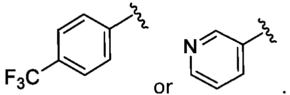

In various embodiments, R² is unsubstituted aryl. various embodiments, aryl is C4-C8-aryl. In various embodiments, aryl is phenyl.

In various embodiments, R² is unsubstituted heterocyclyl. In various embodiments, R² is substituted heterocyclyl. In various embodiments, heterocyclyl is 4-8-membered ring. In various embodiments, heterocyclyl is heteroaryl. In various embodiments, R² is unsubstituted heterocyclyl. In various embodiments, R² is substituted heterocyclyl. In various embodiments, the heterocyclyl is monocyclic. In various embodiments, the heterocyclyl is bicyclic. In various embodiments, the heterocyclyl is tricyclic. In various embodiments, the heterocyclyl is aromatic. In various embodiments, the heterocyclyl is non-aromatic.

In various embodiments, R² is substituted with at least one substituent selected from halogen, alkyl, aryl, alkenyl, alkynyl, cycloalkyl, hydroxyl, amino, nitro, sulfhydryl, imino, amido, sulfamoyl, sulfinyl, alkylthio, sulfonyl, ketone, a heterocyclyl, an aromatic or heteroaromatic moiety, -CHF₂ -CF₃, -CN. If R² is substituted with two or more substituents, the substituents can be the same or different.

In various embodiments, R² is selected from the group consisting of:

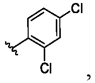

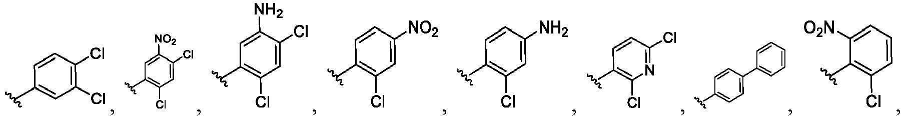

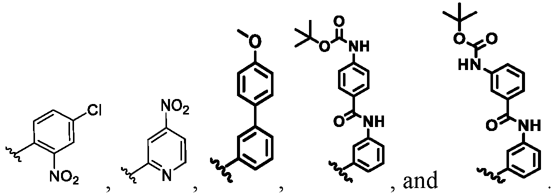

In various embodiments, R² is selected from the group consisting of:

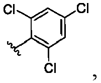

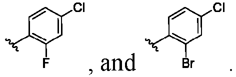

In various embodiments, R² is selected from the group consisting of: 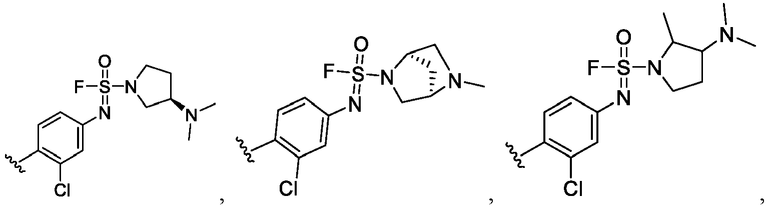 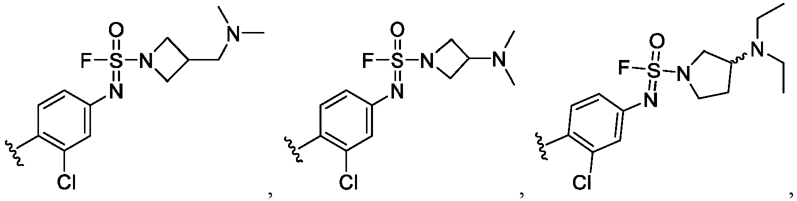 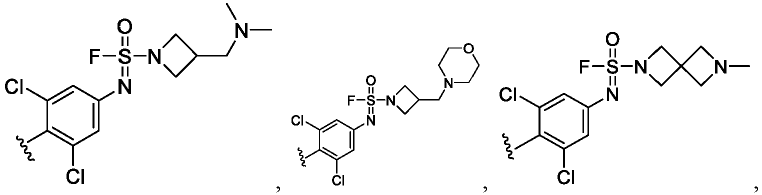 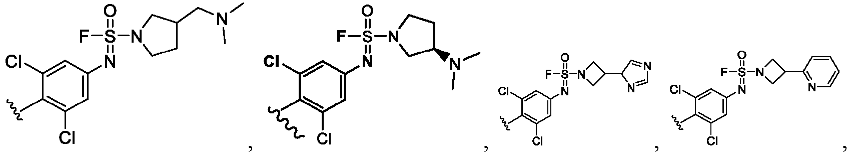 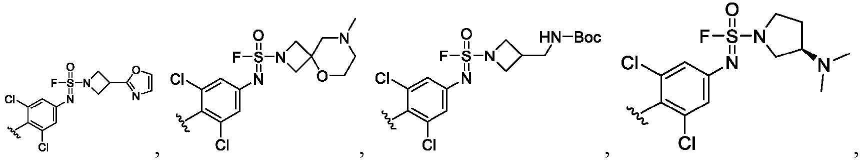 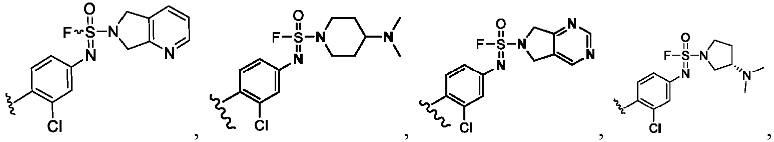 and

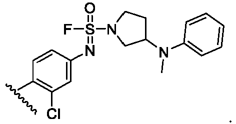

In various embodiments, R² is selected from the group consisting of:

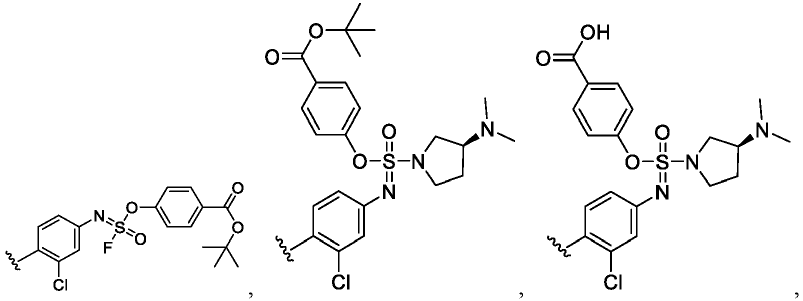

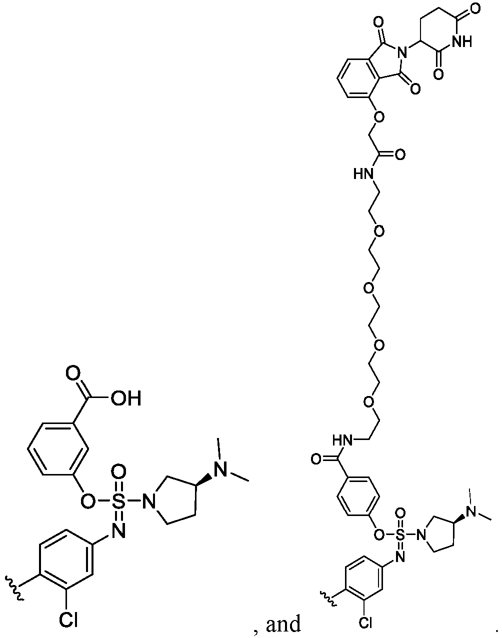

In various embodiments, R² is selected from the group consisting of:

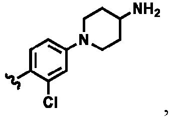

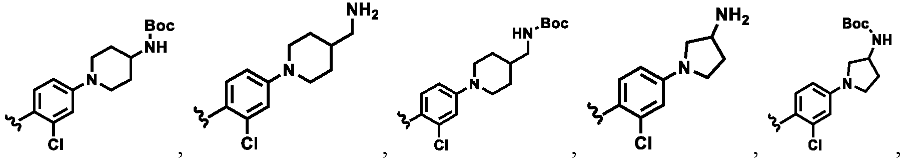

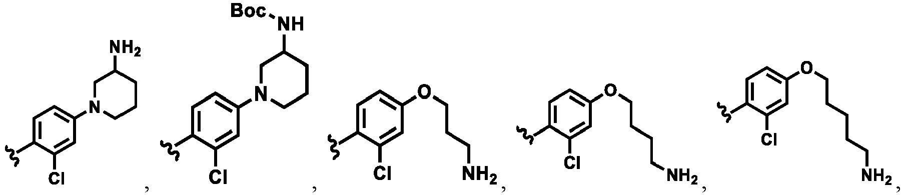

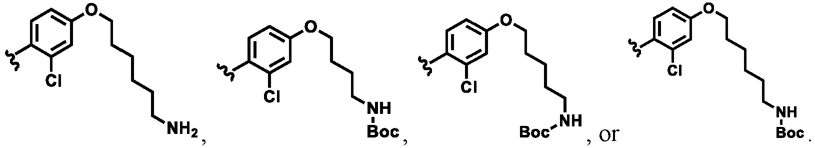

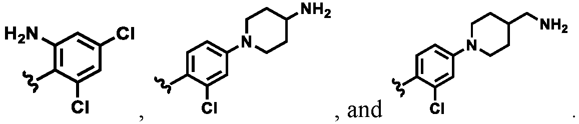

In various embodiments, R² can be: 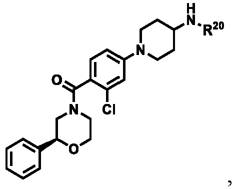 wherein R²⁰ is 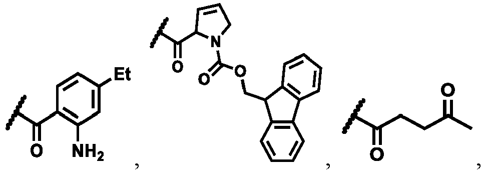 or

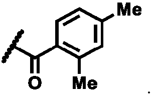

In various embodiments, R² can be: 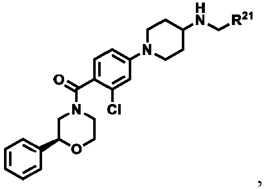 wherein R²¹ is

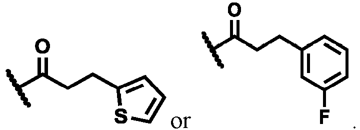

In various embodiments, R² can be: 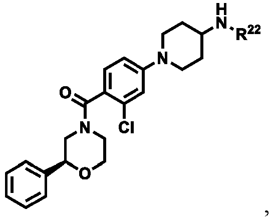 wherein R²² is

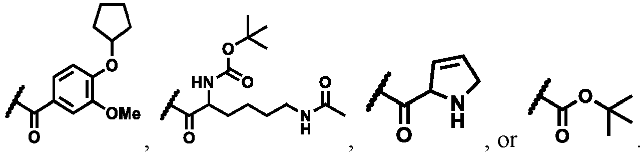

In various embodiments, R³ is absent. In various embodiments, R³ is H. In various embodiments, R³ is alkyl. In various embodiments, R³ is -alkylene-(heterocyclyl). In various embodiments, R³ is -alkylene-(aryl). In various embodiments, R³ is -alkylene-(cycloalkyl). In various embodiments, R³ is -alkylene-O-R⁵. In various embodiments, R³ is -alkylene-C(O)N(R⁵)₂. In various embodiments, R³ is -alkylene-C(O)R⁵. In various embodiments, R³ is -alkylene-C(O)₂R⁵. In various embodiments, R³ is -alkylene-N(R⁵)₂. In various embodiments, R³ is -alkylene-N(R)C(OR⁵). In various embodiments, R³ is -alkylene-N(R⁵)C(O)N(R⁵)-. In various embodiments, R³ is -alkylene-N(R⁵)SO₂-R⁵. In various embodiments, R³ is -alkylene-S-R⁵. In various embodiments, R³ is -aryl. In various embodiments, R³ is -arylene-(heterocyclyl). In various embodiments, R³ is -arylene-alkyl. In various embodiments, R³ is - arylene-(cycloalkyl). In various embodiments, R³ is -arylene-O-R⁵. In various embodiments, R³ is -arylene-C(O)N(R⁵)₂. In various embodiments, R³ is -arylene-C(O)R⁵. In various embodiments, R³ is -arylene-C(O)₂R⁵. In various embodiments, R³ is -arylene-N(R⁵)₂. In various embodiments, R³ is -arylene-N(R)C(OR⁵). In various embodiments, R³ is -arylene-N(R⁵)C(O)N(R⁵)-. In various embodiments, R³ is -arylene-N(R⁵)SO₂-R⁵. In various embodiments, R³ is -arylene-S-R⁵. In various embodiments, R³ is -heterocyclyl. In various embodiments, R³ is -heterocyclylene-(alkyl). In various embodiments, R³ is -heterocyclylene-(aryl). In various embodiments, R³ is -heterocyclylene-(cycloalkyl). In various embodiments, R³ is -heterocyclylene-(cycloalkyl). In various embodiments, R³ is - heterocyclylene-O-R⁵. In various embodiments, R³ is -heterocyclylene-C(O)N(R⁵)₂. In various embodiments, R³ is - heterocyclylene-C(O)R⁵. In various embodiments, R³ is -heterocyclylene-C(O)₂R⁵. In various embodiments, R³ is - heterocyclylene-N(R⁵)₂. In various embodiments, R³ is - heterocyclylene-N(R)C(OR⁵). In various embodiments, R³ is -heterocyclylene-N(R⁵)C(O)N(R⁵)-. In various embodiments, R³ is - heterocyclylene-N(R⁵)SO₂-R⁵. In various embodiments, R³ is -heterocyclylene-S-R⁵. In various embodiments, R³ is -cycloalkyl. In various embodiments, R³ is -cycloalkylene-(alkyl). In various embodiments, R³ is - cycloalkylene-(aryl). In various embodiments, R³ is -cycloalkylene-(cycloalkyl). In various embodiments, R³ is cycloalkylene(heterocycloalkyl). In various embodiments, R³ is - cycloalkylene-O-R⁵. In various embodiments, R³ is -cycloalkylene-C(O)N(R⁵)₂. In various embodiments, R³ is -cycloalkylene-C(O)R⁵. In various embodiments, R³ is -cycloalkylene-C(O)₂R⁵. In various embodiments, R³ is -cycloalkylene-N(R⁵)₂. In various embodiments, R³ is -cycloalkylene-N(R)C(OR⁵). In various embodiments, R³ is -cycloalkylene-N(R⁵)C(O)N(R⁵). In various embodiments, R³ is -cycloalkylene-N(R⁵)SO₂-R⁵. In various embodiments, R³ is -alkylene-N(H)-alkylene-C(O)OR⁵. In various embodiments, R³ is - alkylene-C(O)OR⁵. In various embodiments, R³ is -arylene-alkylene-aryl. In various embodiments, R³ is -C(O)R⁵. In various embodiments, R³ is -C(O)₂R⁵. In various embodiments, R³ is -alkylene-S-alkyl. In various embodiments, R³ is -alkylene-S-S-R⁵. In various embodiments, R³ is -C(O)-alkyl. In various embodiments, R³ is -C(O)-alkylene-N(R⁵)₂. In various embodiments, R³ is -C(O)₂R⁵. In various embodiments, R³ is -aryl-alkylene-N(R⁵)C(O)-R⁵. In various embodiments, R³ is -cycloalkylene-N(R⁵)S(O)(F)-R⁵. In various embodiments, R³ is -SO₂-NR⁵. In various embodiments, R³ is -S(O)(F)-NR⁵. In various embodiments, alkyl, alkylene, aryl, arylene, heterocyclyl, heterocyclylene, cycloalkyl, or cycloalkylene are unsubstituted. In various embodiments, alkyl, alkylene, aryl, arylene, heterocyclyl, heterocyclylene, cycloalkyl, or cycloalkylene are substituted.

In various embodiments, R⁴ is alkyl. In various embodiments, R⁴ is -alkylene-(heterocyclyl). In various embodiments, R⁴ is -alkylene-(aryl). In various embodiments, R⁴ is -alkylene-(cycloalkyl). In various embodiments, R⁴ is -alkylene-O-R⁵. In various embodiments, R⁴ is -alkylene-C(O)N(R⁵)₂. In various embodiments, R⁴ is -alkylene-C(O)R⁵. In various embodiments, R⁴ is -alkylene-C(O)₂R⁵. In various embodiments, R⁴ is -alkylene-N(R⁵)₂. In various embodiments, R⁴ is -alkylene-N(R)C(OR⁵). In various embodiments, R⁴ is -alkylene-N(R⁵)C(O)N(R⁵)-. In various embodiments, R⁴ is -alkylene-N(R⁵)SO₂-R⁵. In various embodiments, R⁴ is -alkylene-S-R⁵. In various embodiments, R⁴ is -aryl. In various embodiments, R⁴ is -arylene-(heterocyclyl). In various embodiments, R⁴ is -arylene-alkyl. In various embodiments, R⁴ is -arylene-(cycloalkyl). In various embodiments, R⁴ is -arylene-OR⁵*.* In various embodiments, R⁴ is -arylene-C(O)N(R⁵)₂. In various embodiments, R⁴ is - arylene-C(O)R⁵. In various embodiments, R⁴ is -arylene-C(O)₂R⁵. In various embodiments, R⁴ is -arylene-N(R⁵)₂. In various embodiments, R⁴ is -arylene-N(R)C(OR⁵). In various embodiments, R⁴ is -arylene-N(R⁵)C(O)N(R⁵)-. In various embodiments, R⁴ is -arylene-N(R⁵)SO₂-R⁵. In various embodiments, R⁴ is -arylene-S-R⁵. In various embodiments, R⁴ is - heterocyclyl. In various embodiments, R⁴ is -heterocyclylene-(alkyl). In various embodiments, R⁴ is -heterocyclylene-(aryl). In various embodiments, R⁴ is -heterocyclylene-(cycloalkyl). In various embodiments, R⁴ is -heterocyclylene-(cycloalkyl). In various embodiments, R⁴ is - heterocyclylene-O-R⁵. In various embodiments, R⁴ is -heterocyclylene-C(O)N(R⁵)₂. In various embodiments, R⁴ is -heterocyclylene-C(O)R⁵. In various embodiments, R⁴ is -heterocyclylene-C(O)₂R⁵. In various embodiments, R⁴ is - heterocyclylene-N(R⁵)₂. In various embodiments, R⁴ is - heterocyclylene-N(R)C(OR⁵). In various embodiments, R⁴ is -heterocyclylene-N(R⁵)C(O)N(R⁵)-. In various embodiments, R⁴ is - heterocyclylene-N(R⁵)SO₂-R⁵. In various embodiments, R⁴ is -heterocyclylene-S-R⁵. In various embodiments, R⁴ is -cycloalkyl. In various embodiments, R⁴ is -cycloalkylene-(alkyl). In various embodiments, R⁴ is -cycloalkylene-(aryl). In various embodiments, R⁴ is - cycloalkylene-(cycloalkyl). In various embodiments, R⁴ is cycloalkylene(heterocycloalkyl). In various embodiments, R⁴ is -cycloalkylene-O-R⁵. In various embodiments, R⁴ is - cycloalkylene-C(O)N(R⁵)₂. In various embodiments, R⁴ is -cycloalkylene-C(O)R⁵. In various embodiments, R⁴ is -cycloalkylene-C(O)₂R⁵. In various embodiments, R⁴ is -cycloalkylene-N(R⁵)₂. In various embodiments, R⁴ is -cycloalkylene-N(R)C(OR⁵). In various embodiments, R⁴ is -cycloalkylene-N(R⁵)C(O)N(R⁵). In various embodiments, R⁴ is -cycloalkylene-N(R⁵)SO₂-R⁵. In various embodiments, R⁴ is -alkylene-N(H)-alkylene-C(O)OR⁵. In various embodiments, R⁴ is -alkylene-C(O)OR⁵. In various embodiments, R⁴ is -arylene-alkylene-aryl. In various embodiments, R⁴ is -C(O)R⁵. In various embodiments, R⁴ is -C(O)₂R⁵. In various embodiments, R⁴ is -alkylene-S-alkyl. In various embodiments, R⁴ is -alkylene-S-S-R⁵*.* In various embodiments, R⁴ is -C(O)-alkyl. In various embodiments, R⁴ is -C(O)-alkylene-N(R⁵)₂. In various embodiments, R⁴ is -C(O)₂R⁵. In various embodiments, R⁴ is - aryl-alkylene-N(R⁵)C(O)-R⁵. In various embodiments, R⁴ is -cycloalkylene-N(R⁵)S(O)(F)-R⁵. In various embodiments, R⁴ is -SO₂-NR⁵. In various embodiments, R⁴ is -S(O)(F)-NR⁵. In various embodiments, alkyl, alkylene, aryl, arylene, heterocyclyl, heterocyclylene, cycloalkyl, or cycloalkylene are unsubstituted. In various embodiments, alkyl, alkylene, aryl, arylene, heterocyclyl, heterocyclylene, cycloalkyl, or cycloalkylene are substituted.

In various embodiments, R³ and R⁴ are taken together to form an unsubstituted heterocyclyl. In various embodiments, R³ and R⁴ are taken together to form a substituted heterocyclyl. In various embodiments, heterocyclyl is 4-8-membered ring. In various embodiments, heterocyclyl is heteroaryl. In various embodiments, the heterocyclyl is monocyclic. In various embodiments, the heterocyclyl is bicyclic. In various embodiments, the heterocyclyl is tricyclic. In various embodiments, the heterocyclyl is aromatic. In various embodiments, the heterocyclyl is non-aromatic. In various embodiments, the heterocyclyl is substituted with at least one substituent selected from halogen, alkyl, alkylene-aryl, aryl alkenyl, alkynyl, cycloalkyl, hydroxyl, amino, nitro, sulfhydryl, imino, amido, sulfamoyl, sulfinyl, alkylthio, sulfonyl, ketone, a heterocyclyl, an aromatic or heteroaromatic moiety, - CHF₂ -CF₃, -CN. If the heterocyclyl is substituted with two or more substituents, the substituents can be the same or different.

In various embodiments, R⁵ is H. In various embodiments, R⁵ is alkyl. In various embodiments, alkyl is C1-C4-alkyl. In various embodiments, R⁵ is aryl. In various embodiments, R⁵ is phenyl. In various embodiments, R⁵ is -alkylene-aryl. In various embodiments, R⁵ is benzyl. In various embodiments, all instances of R⁵ are different. In various embodiments, all instances of R⁵ are the same.

In various aspects of the invention, 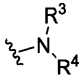 of formula (I) or (II) is selected from the group consisting of:

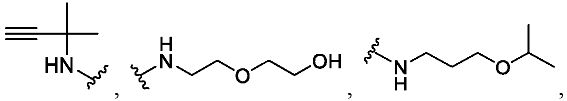

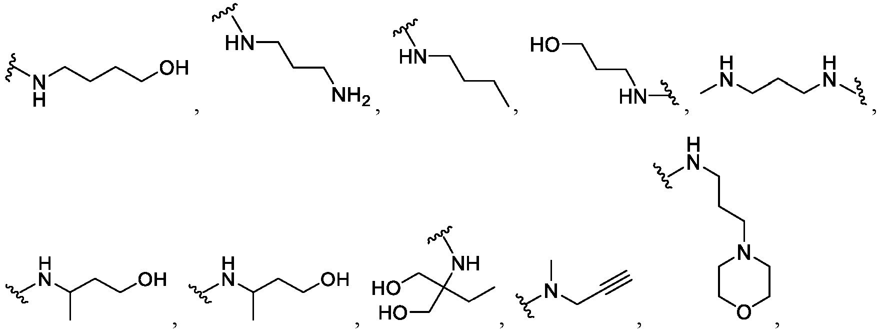

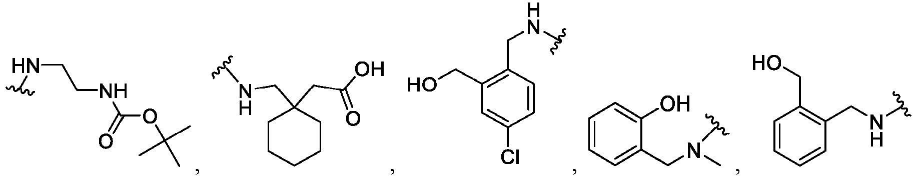

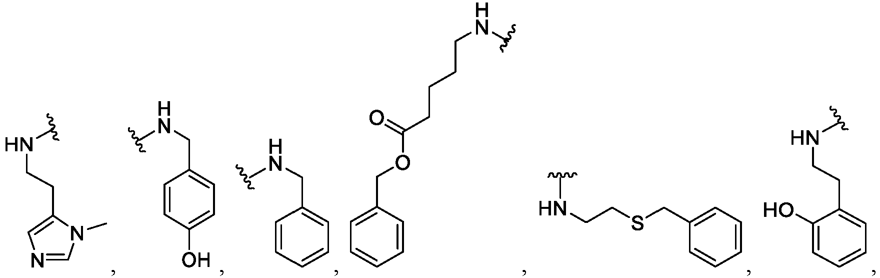

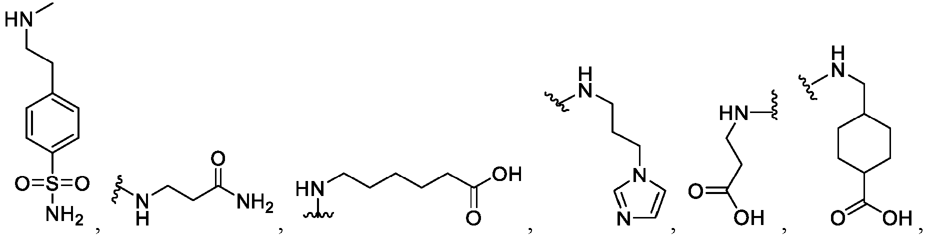

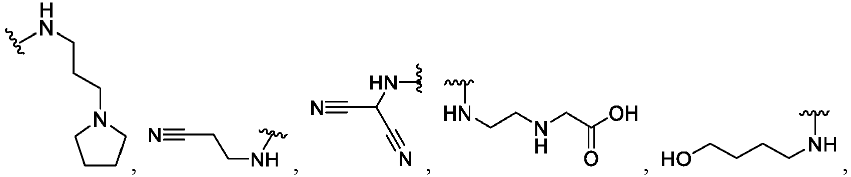

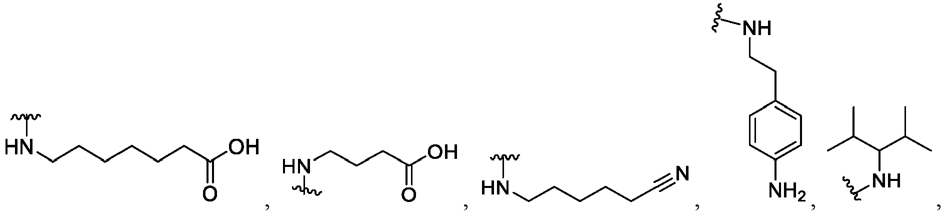

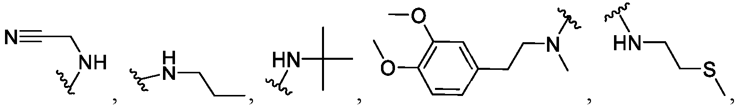

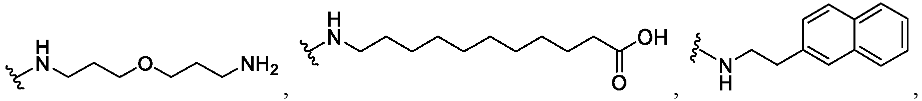

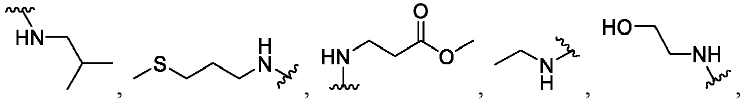

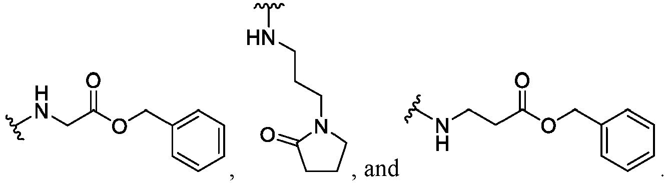

In various aspects of the invention, 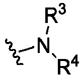 of formula (I) or (II) is selected from the group consisting of:

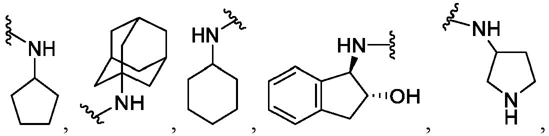

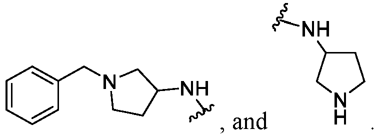

In various aspects of the invention, 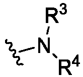 of formula (I) or (II) is 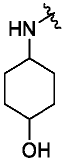 or

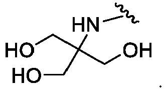

In various aspects of the invention, 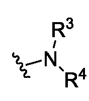 of formula (I) or (II) is selected from the group consisting of: 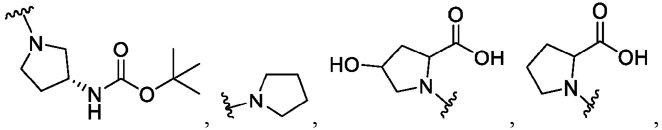 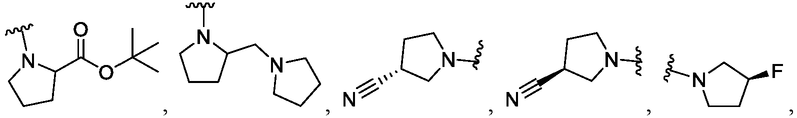 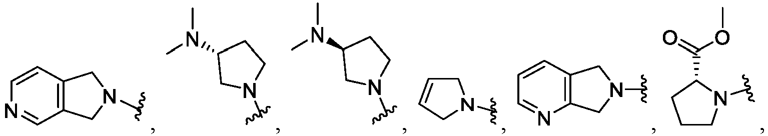 and

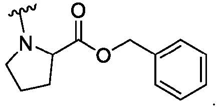

In various aspects of the invention, 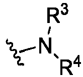 of formula (I) or (II) is selected from the group consisting of:

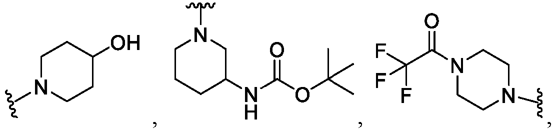

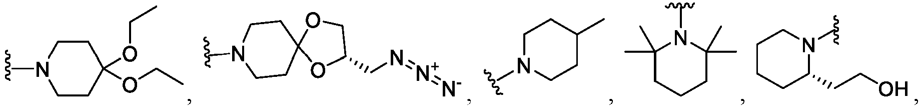

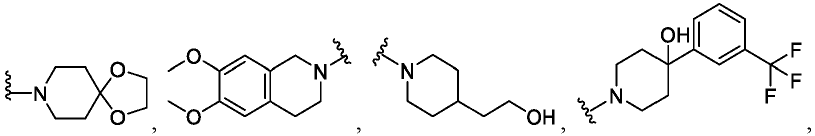

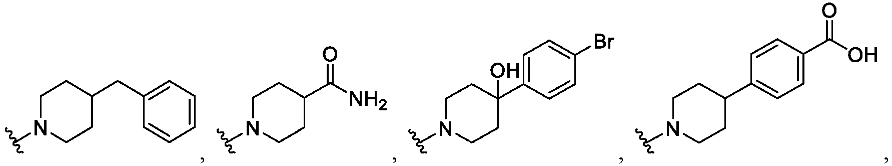

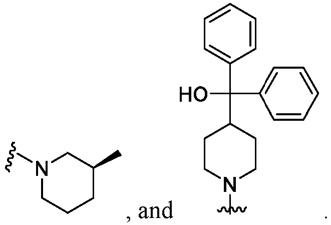

In various aspects of the invention, 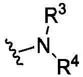 of formula (I) or (II) is selected from the group consisting of:

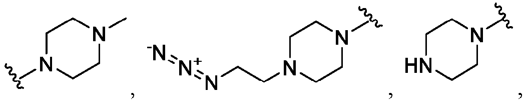

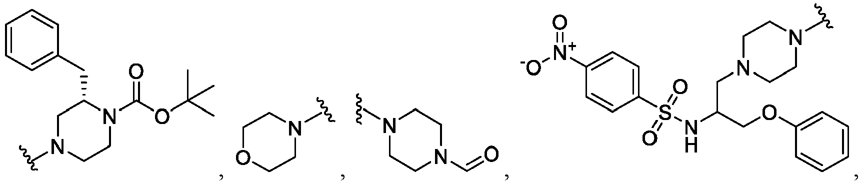

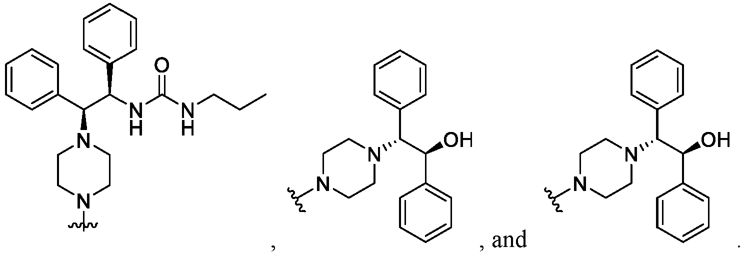

In various aspects of the invention, 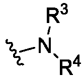 of formula (I) or (II) is

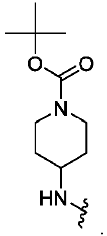

In various aspects of the invention, 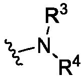 of formula (I) or (II) is 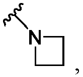 or

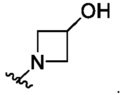

In various aspects of the invention, 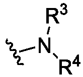 of formula (I) or (II) is selected from the group consisting of:

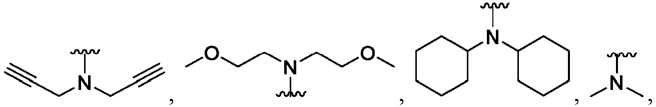

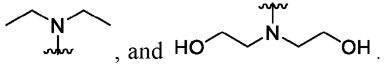

In various aspects of the invention, 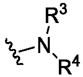 of formula (I) or (II) is selected from the group consisting of 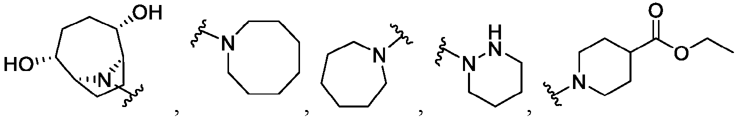 , and

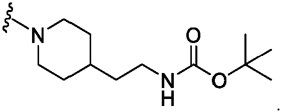

In various aspects of the invention, 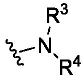 of formula (I) or (II) is selected from the group consisting of:

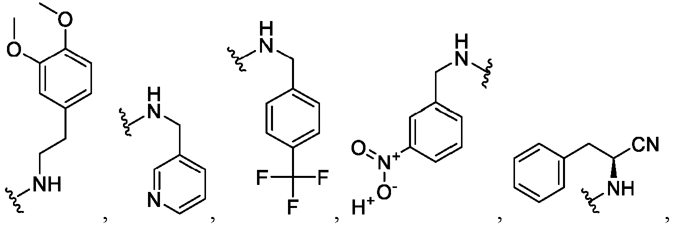

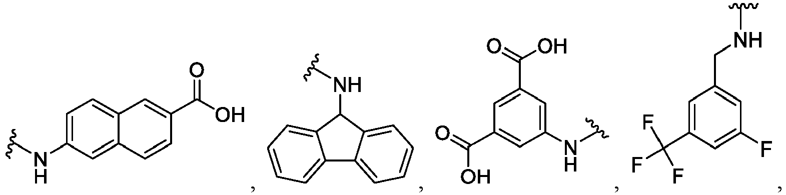

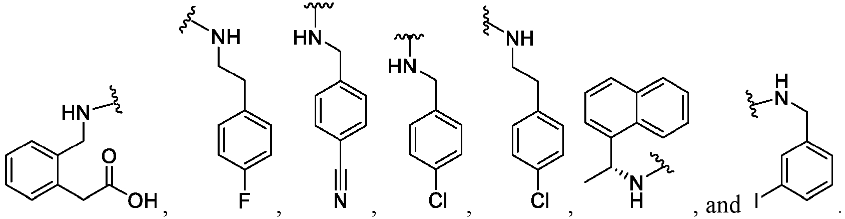

In various aspects of the invention, 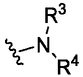 of formula (I) or (II) is selected from the group consisting:

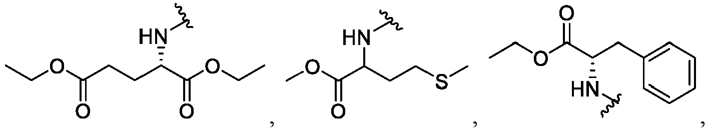

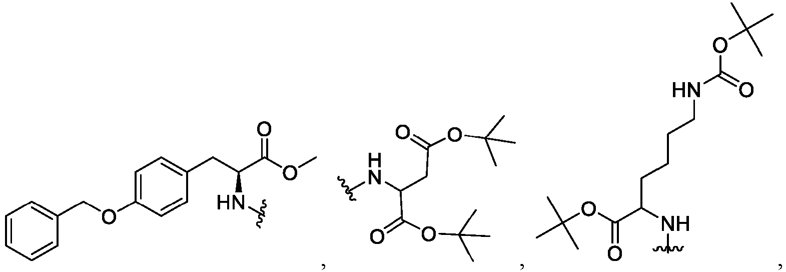

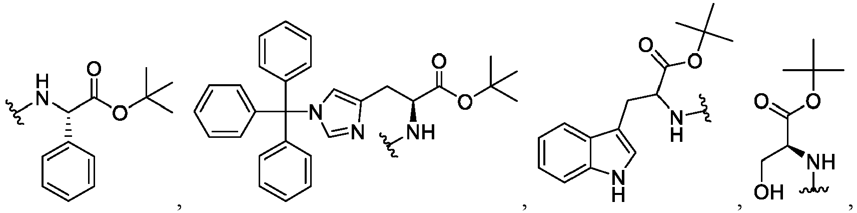

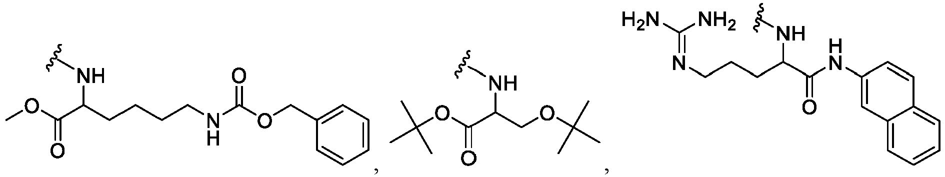

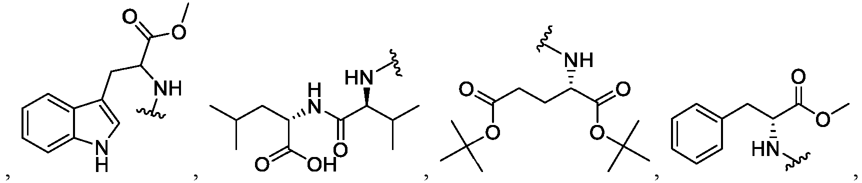

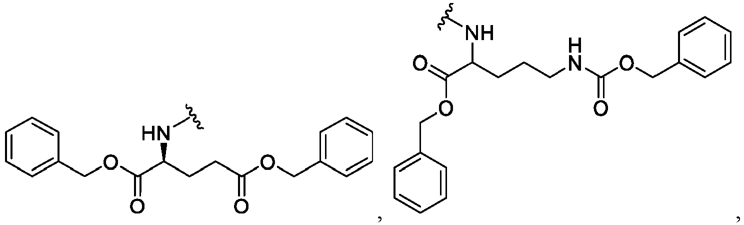

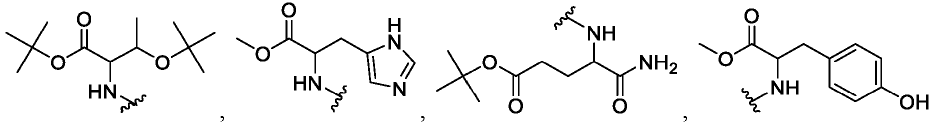

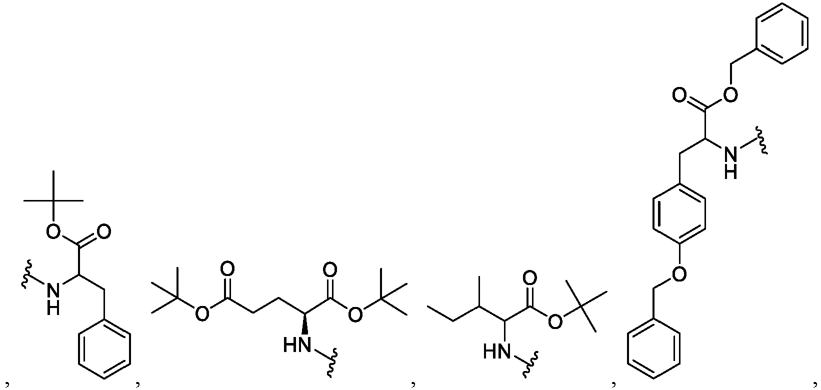

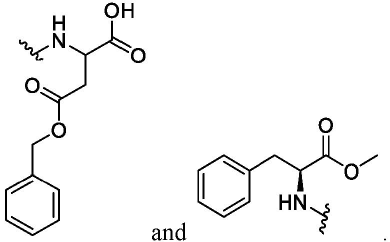

In various aspects of the invention, 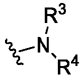 of formula (I) or (II) is selected from the group consisting of:

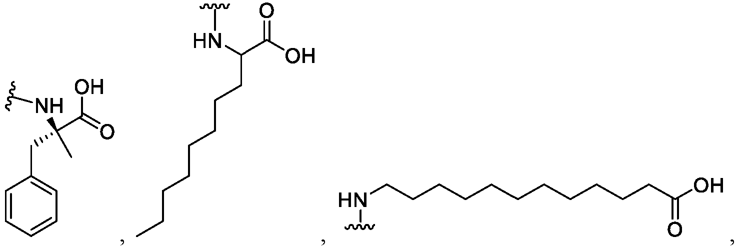

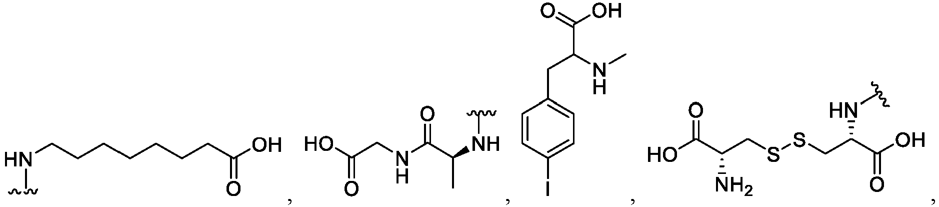

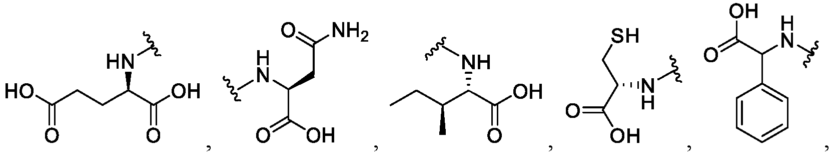

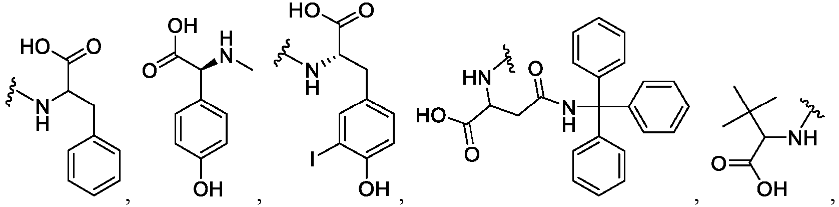

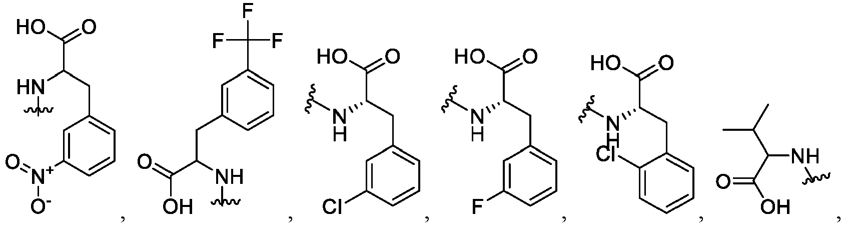

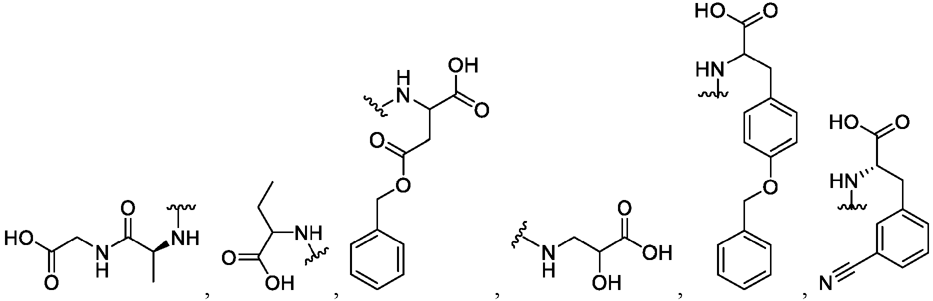

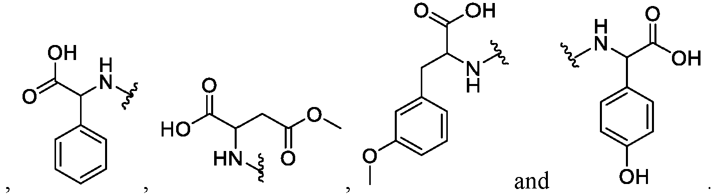

In various aspects of the invention, 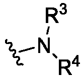 of formula (I) or (II) is

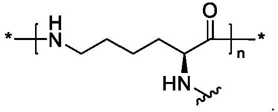

In various aspects of the invention, 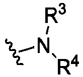 of formula (I) or (II) is 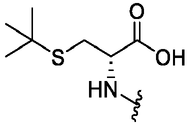 or

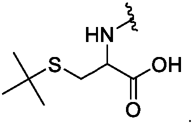

In various aspects of the invention, 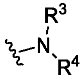 of formula (I) or (II) is selected from the group consisting of

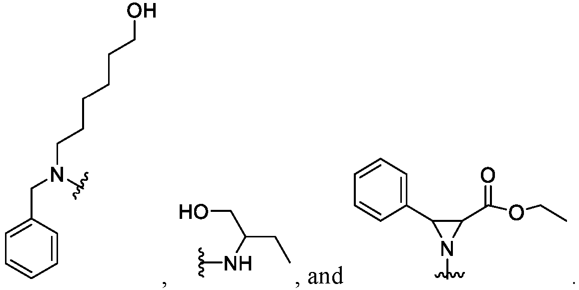

In various aspects of the invention, 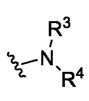 of formula (I) or (II) is selected from the group consisting of: 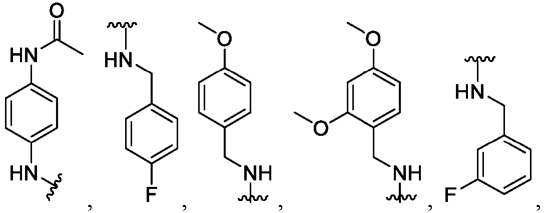 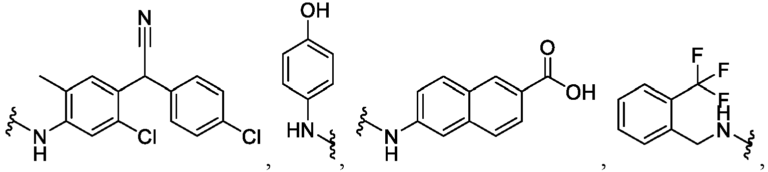 and

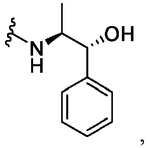

In various aspects of the invention, 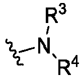 of formula (I) or (II) is selected from the group selected from:

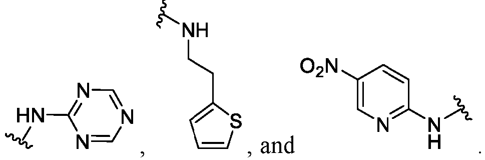

In various aspects of the invention, 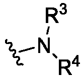 of formula (I) or (II) is 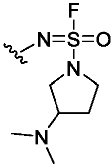 or

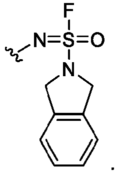

In various aspects of the invention, 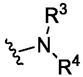 of formula (I) or (II) is selected from the group consisting of:

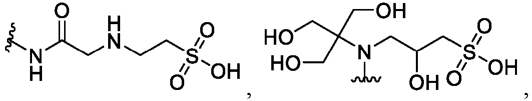

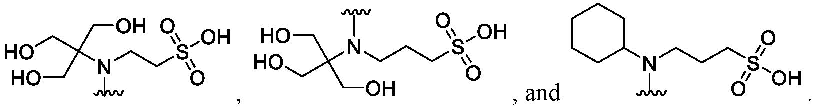

In various aspects of the invention, 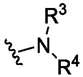 of formula (I) or (II) is

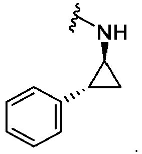

In various aspects of the invention, the compound of formula (I) or (II) is selected from the group consisting of:

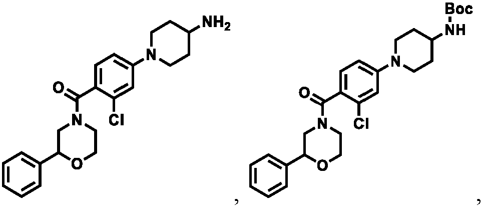

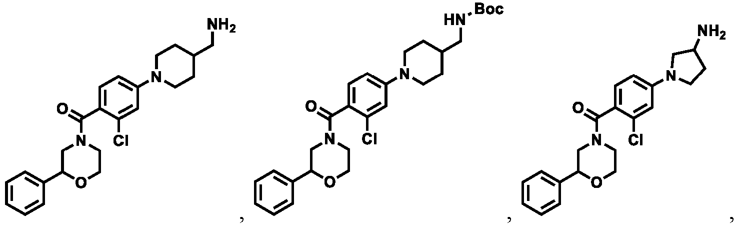

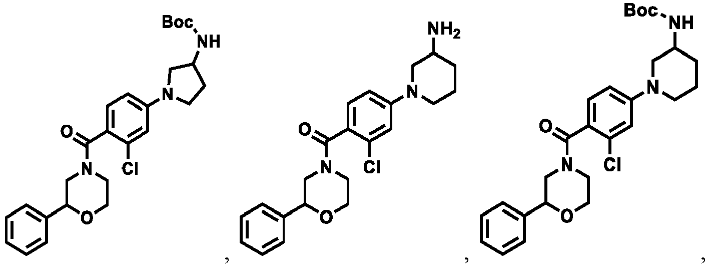

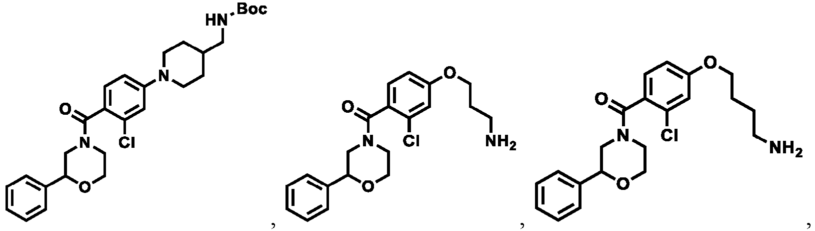

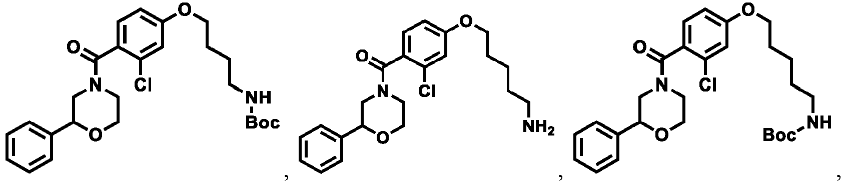

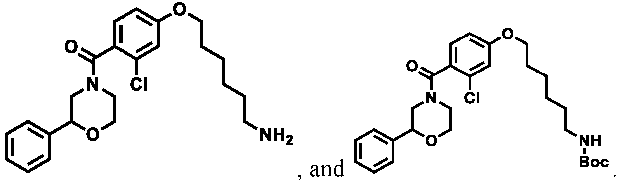

In various aspects of the invention, the compound of formula (I) or (II) is selected from the group consisting of: 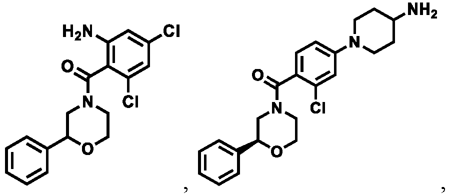 and

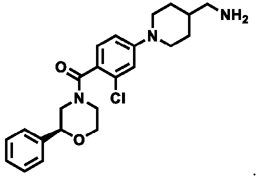

In various aspects of the invention, the compound of formula (I) or (II) is

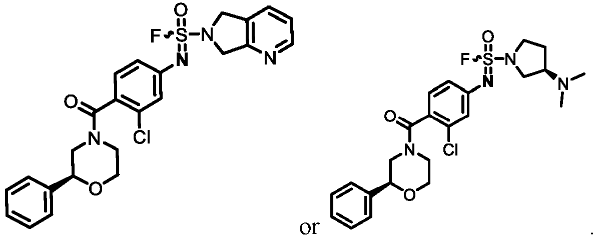

In various aspects of the invention, the compound of formula (I) or (II) is selected from the group consisting of:

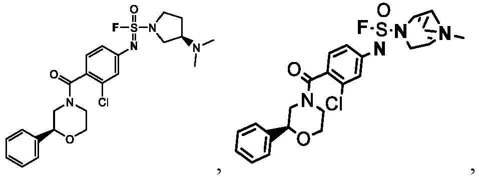

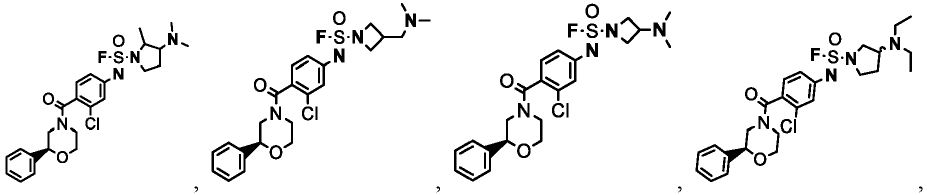

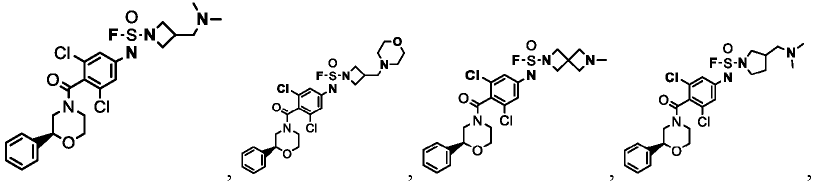

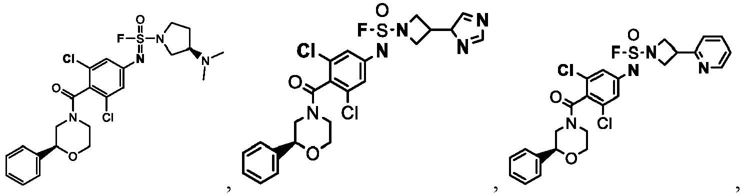

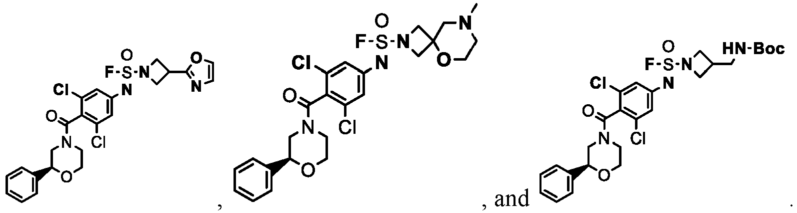

In various aspects of the invention, the compound of formula (I) or (II) is selected from the group consisting of: 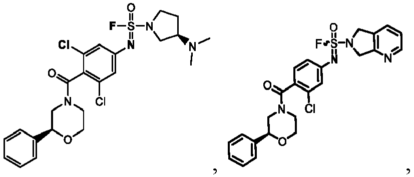 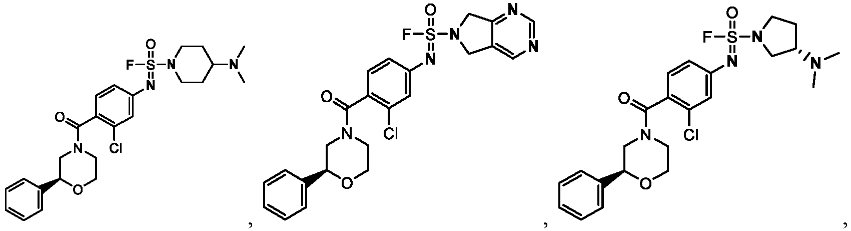 and

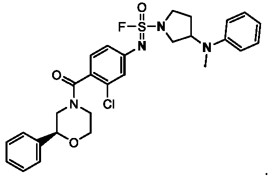

In various aspects of the invention, the compound of formula (I) or (II) is selected from the group consisting of: 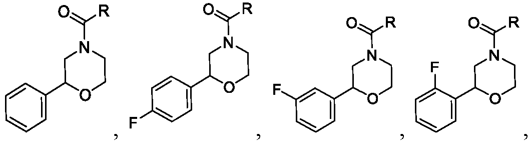 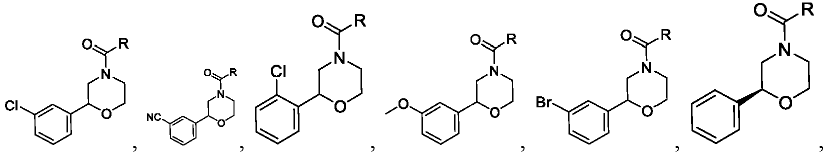 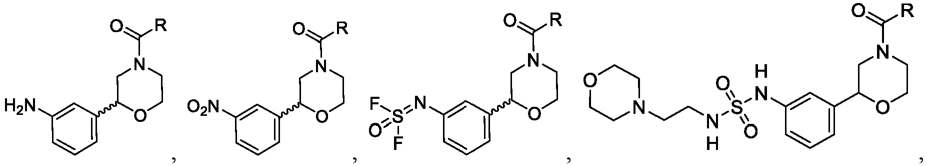 and 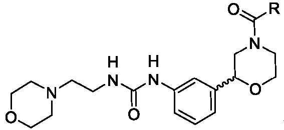 wherein R is

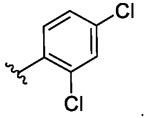

In various aspects of the invention, the compound of formula (I) or (II) is selected from the group consisting of:

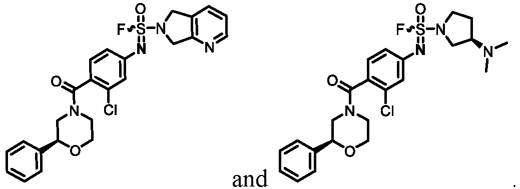

In various aspects of the invention, the compound of formula (I) or (II) is 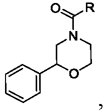 wherein R is selected from the group consisting of:

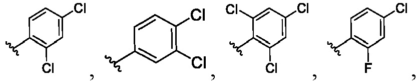

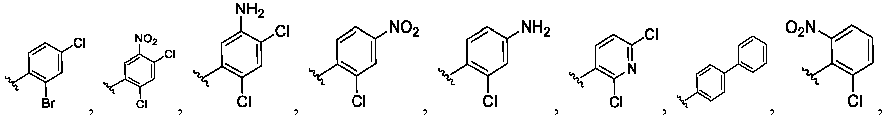

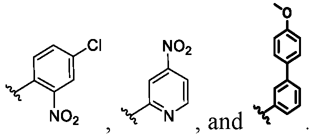

In various aspects of the invention, the compound of formula (I) or (II) is selected from the group consisting of:

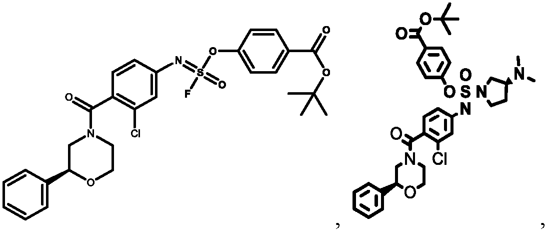

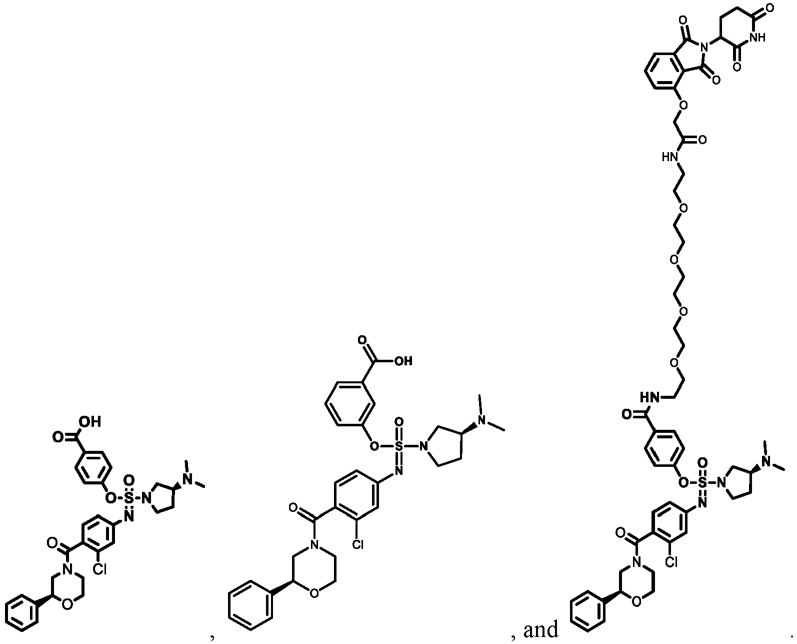

In various aspects of the invention, the compound of formula (I) or (II) is

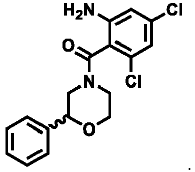

In various aspects of the invention, the compound of formula (I) or (II) is selected from the group consisting of: 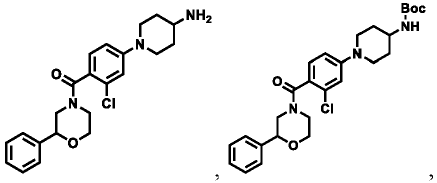 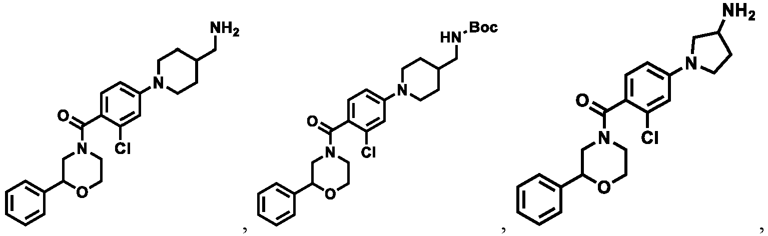 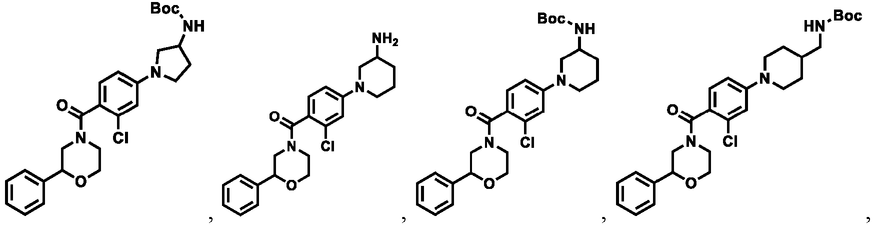 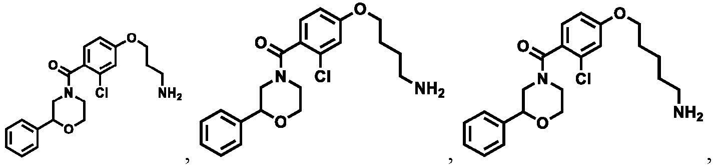 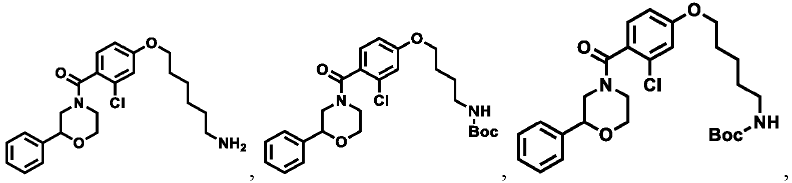 and

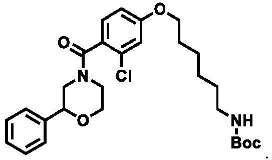

In various aspects of the invention, the compound of formula (I) or (II) is 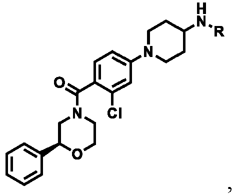 wherein R is selected from the group consisting of

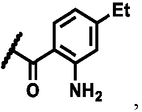

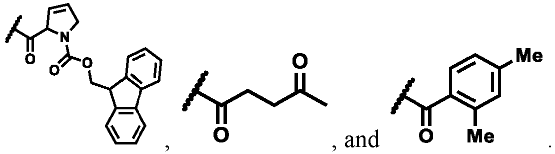

In various aspects of the invention, the compound of formula (I) or (II) is

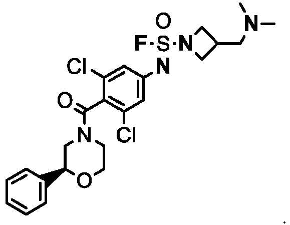

In various aspects of the invention, the compound of formula (I) or (II) is 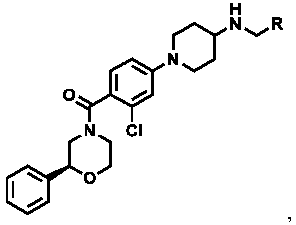 wherein R is selected from the group consisting of

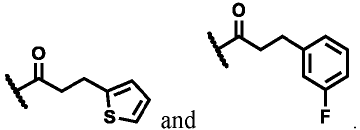

In various aspects of the invention, the compound of formula (I) or (II) is 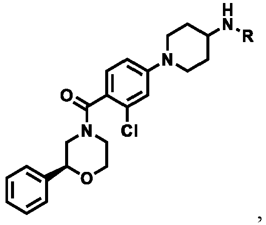 wherein R is selected from the group consisting of

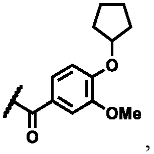

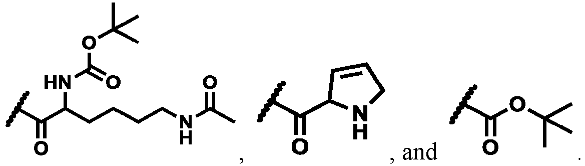

In various aspects of the invention, the compound of formula (I) or (II) is selected from the group consisting of: 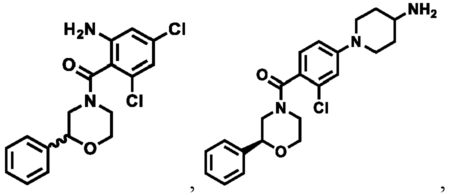 and

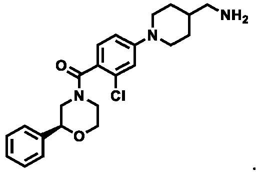

### EXEMPLARY PHARMACEUTICAL COMPOSITIONS

In various embodiments, the disclosure relates to a pharmaceutical composition comprising any one of the aforementioned compounds and a pharmaceutically acceptable carrier.

Patients, including but not limited to humans, can be treated by administering to the patient an effective amount of the active compound or a pharmaceutically acceptable prodrug or salt thereof in the presence of a pharmaceutically acceptable carrier or diluent. The active materials can be administered by any appropriate route, for example, orally, parenterally, intravenously, intradermally, subcutaneously, or topically, in liquid or solid form.

The concentration of active compound in the drug composition will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient can be administered at once or can be divided into a number of smaller doses to be administered at varying intervals of time.

In various embodiments, the mode of administration of the active compound is oral. Oral compositions will generally include an inert diluent or an edible carrier. They can be enclosed in gelatin capsules or compressed into tablets. For the purpose of oral therapeutic administration, the active compound can be incorporated with excipients and used in the form of tablets, troches or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, unit dosage forms can contain various other materials that modify the physical form of the dosage unit, for example, coatings of sugar, shellac, or other enteric agents.

The compound can be administered as a component of an elixir, suspension, syrup, wafer, chewing gum or the like. A syrup can contain, in addition to the active compound(s), sucrose or sweetener as a sweetening agent and certain preservatives, dyes and colorings and flavors.

The compound or a pharmaceutically acceptable prodrug or salts thereof can also be mixed with other active materials that do not impair the desired action, or with materials that supplement the desired action, such as antibiotics, antifungals, anti-inflammatories or other antivirals, including but not limited to nucleoside compounds. Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents, such as ethylenediaminetetraacetic acid; buffers, such as acetates, citrates or phosphates, and agents for the adjustment of tonicity, such as sodium chloride or dextrose. The parental preparation can be enclosed in ampoules, disposable syringes or multiple dose vials made of glass or plastic.

If administered intravenously, carriers include physiological saline and phosphate buffered saline (PBS).

In various embodiments, the active compounds are prepared with carriers that will protect the compound against rapid elimination from the body, such as a controlled release formulation, including but not limited to implants and microencapsulated delivery systems. Biodegradable, biocompatible polymers can be used, such as ethylene vinyl acetate, polyanhydrides, polyglycolic acid, collagen, polyorthoesters and polylactic acid. For example, enterically coated compounds can be used to protect cleavage by stomach acid. Methods for preparation of such formulations will be apparent to those skilled in the art. Suitable materials can also be obtained commercially.

Liposomal suspensions (including but not limited to liposomes targeted to infected cells with monoclonal antibodies to viral antigens) are also pharmaceutically acceptable carriers. These can be prepared according to methods known to those skilled in the art, for example, as described in U.S. Pat. No. 4,522,811. For example, liposome formulations can be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound is then introduced into the container. The container is then swirled by hand to free lipid material from the sides of the container and to disperse lipid aggregates, thereby forming the liposomal suspension.

### EXEMPLARY METHODS OF THE DISCLOSURE

In various embodiments, the disclosure relates to a method of treating hemagglutinin (HA)-mediated diseases or conditions comprising the step of: administering to a subject in need thereof a therapeutically effective amount of any one of the aforementioned compounds. In various embodiments, the HA-mediated disease is influenza. In various embodiments, the influenza is influenza A infections, such as but not limited to H1N1, H2N2, H3N2, H5N1, H7N7, H7N9, H1N2, H9N2, H7N2, H7N3, and H10N7. In various embodiments, the influenza is influenza B infections. In various embodiments, the influenza is influenza C infections.

### EXAMPLES

The disclosure now being generally described, it will be more readily understood by reference to the following examples, which are included merely for purposes of illustration of various aspects and embodiments of the present disclosure, and are not intended to limit the disclosure.

### Materials and Reagents

Fmoc-protected amino acids were purchased from Combi-Blocks and Oakwood Products. 5(6)-TAMRA, SE [5-(and-6)-carboxytetramethylrhodamine, succinimidyl ester] was purchased from AAT Bioquest. (S)-2-phenylmorpholine and (R)-2-phenylmorpholine were purchased from Astatech. Greiner Bio-One 384 wells and pyrrolidine were purchased from Fisher Scientific. CellTiter-Glo^{®} luminescent cell viability assay reagent was purchased from Promega Corp. All other reagents were of analytical grade and were used without further purification. Milli-Q water was used in all experiments unless otherwise stated.

### Synthesis

**P7 peptide.** P7 was prepared in a similar way as previously reported R. U. Kadam et al., Potent peptidic fusion inhibitors of influenza virus. Science 358, 496-502 (2017). The linear peptide was synthesized on a 0.3 mmol scale using 2-chlorotrityl chloride resin by manual Fmoc solid phase peptide synthesis. The first amino acid was loaded with an anhydrous DCM solution of 3 eq. Dde-L-Dap(Fmoc)-OH in the presence of 5 eq. N,N-diisopropylethylamine (DIPEA). The resin was then capped with freshly made solution of DCM/MeOH/DIPEA (17:2: 1) for 1 hour. The N-Dde protecting group was then removed by reaction with a 2% hydrazine hydrate solution in DMF, followed by N-Boc-β-alanine coupling. Standard Fmoc deprotection (20% Pyrrolidine in DMF) and coupling protocols (3 eq. Fmoc amino acid, HCTU and DIPEA) were used until the linear peptide was synthesized. The protected linear peptide was then cleaved from the resin using 20% hexafluoroisopropanol in DCM.

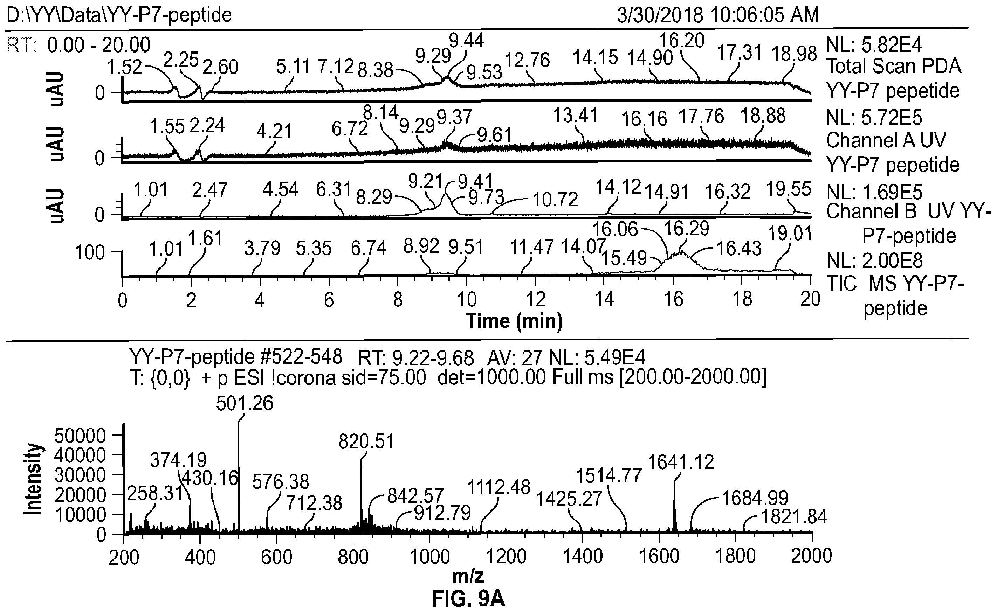

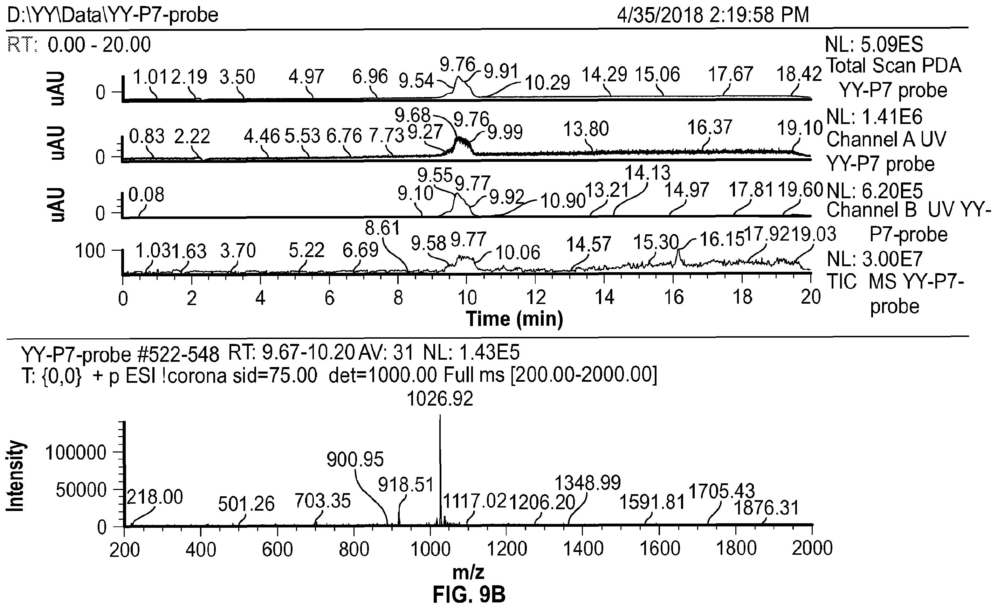

Finally, the lactam cyclization of crude linear peptide was performed at high dilution in DMF, with 3 eq. of HBTU, HObt and DIPEA. The reaction was performed at room temperature for 2 days, followed by the full side-chain deprotection with solution of TFA/triisopropylsilyl/H₂O (95:2.5:2.5). The final product was purified by reverse-phase HPLC (linear gradient from 90% water and 10% MeCN to 100% MeCN). The product was characterized by LC-MS. Calc'd for C₇₉H₁₀₆Cl₂N₁₅O₁₉ [M+H]⁺: 1640.70, found 1641.12 **(****Fig. 9****).**

**P7-TAMRA probe.** 1 eq. P7 peptide and 1.2 eq TAMRA succinimidyl ester (5(6)-TAMRA, SE) were dissolved in 1mL DMSO and then 3 eq. DIPEA was added to the solution. The solution was stirred in the dark at room temperature overnight. Water was then added to quench the reaction and purified by reverse-phase HPLC with 95% yield (linear gradient from 90% water and 10% MeCN to 100% MeCN). The product was characterized by LC-MS. Calc'd for C₁₀₄H₁₂₆Cl₂N₁₇O₂₃ [M+H]⁺: 2053.14, found (1)⁺=1026.92 **(****Fig. 9****).**

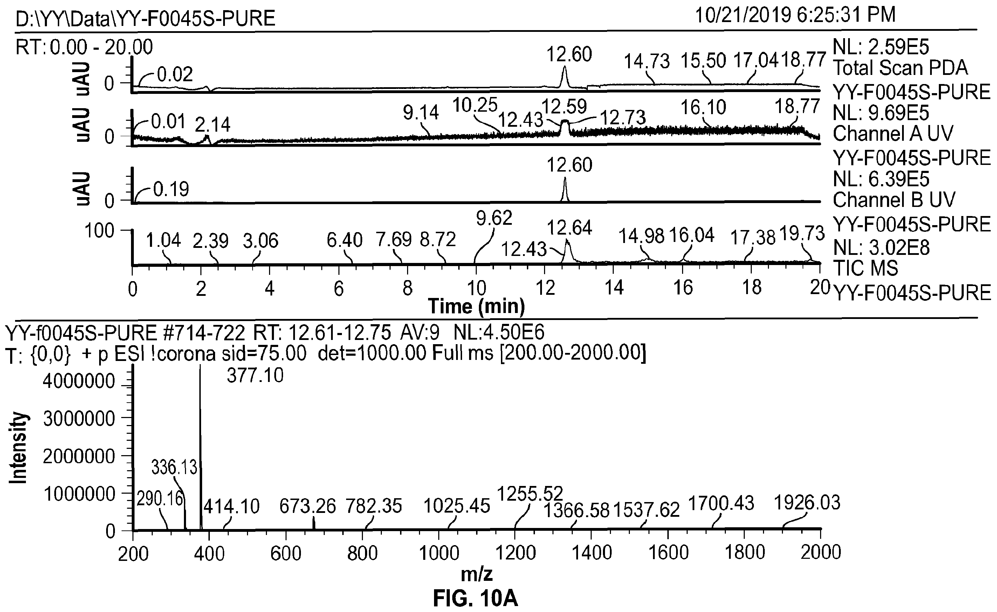

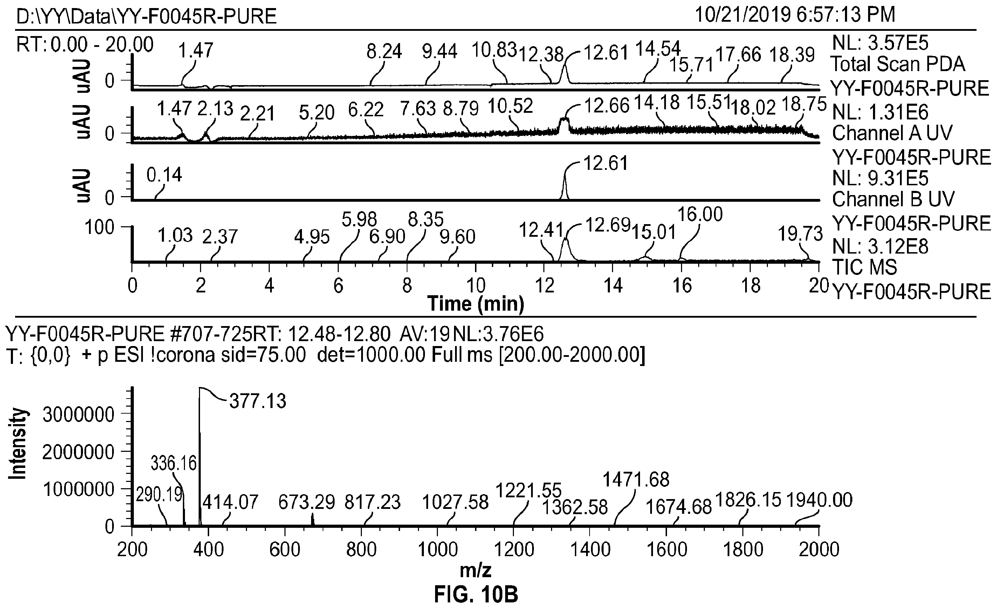

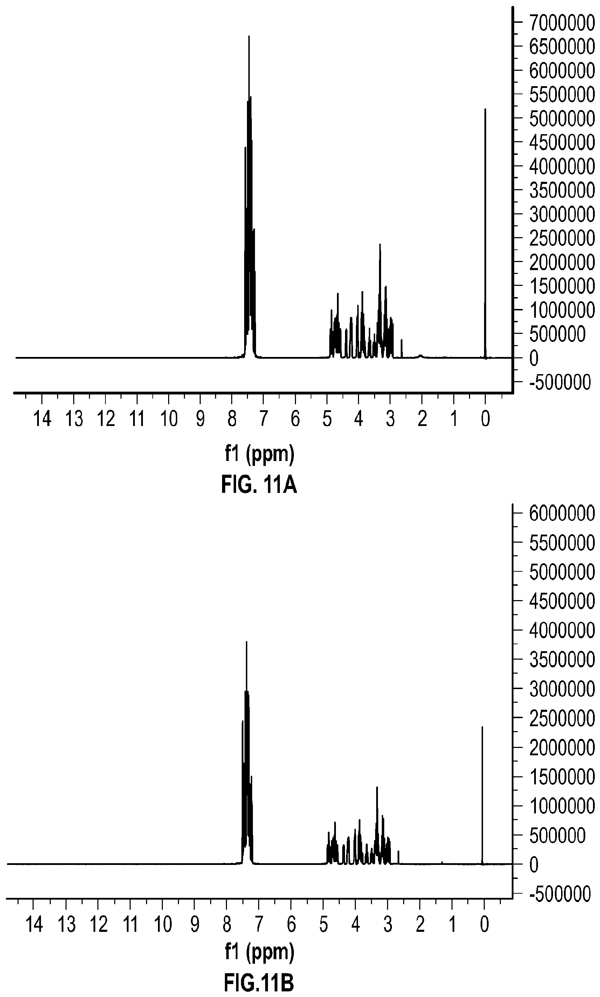

**F0045(S).** EDCI·HCl (264 mg, 1.38 mmol) and DIPEA (410 µl, 2.76 mmol) were added to a solution of (S)-2-phenylmorpholine (150 mg, 0.92 mmol), 2,4-dichlorobenzoic acid (193 mg, 1.01mmol) and HOBt (186 mg, 1.38 mmol) in DCM, and stirred for 24 hr at room temperature under nitrogen. The solvent was removed *in vacuo* and the residue purified by column chromatography on silica gel eluting with DCM:MeOH=100:1 to 100:5 (by volume), to furnish the title compound as a white foam (95% yield). ¹H NMR (500 MHz, CDCl₃) δ 7.52-7.18 (m, 8H, ArH), 4.89-4.66 (m, 1H, CH), 4.65-2.88 (m, 6H, CH₂). 13C NMR (500 MHz CDCl₃) δ 166.26, 138.75, 135.89, 134.05, 131.36, 129.77, 129.15, 128.64, 128.33, 127.92, 126.18, 78.54, 66.91, 53.65, 47.91. MS (ESI). Calc'd for C₁₇H₁₆Cl₂NO₂ [M+H]⁺: 336.05, found 336.13 **(****Figs. 10****,** **11****).**

**F0045(R).** EDCI·HCl (264 mg, 1.38 mmol) and DIPEA (410 µl, 2.76 mmol) were added to a solution of (R)-2-phenylmorpholine (150 mg, 0.92 mmol), 2,4-dichlorobenzoic acid (193 mg, 1.01 mmol) and HOBt (186 mg, 1.38 mmol) in DCM, and stirred for 24 hours at room temperature under nitrogen. The solvent was removed *in vacuo* and the residue purified by column chromatography on silica gel eluting with DCM:MeOH=100:1 to 100:5 (by volume), to furnish the title compound as a white foam (95% yield). ₁H NMR (500 MHz, CDCl₃) δ 7.52-7.18 (m, 8H, ArH), 4.89-4.66 (m, 1H, CH), 4.65-2.88 (m, 6H, CH₂). 13C NMR (500 MHz CDCl₃) δ 166.26, 138.75, 135.89, 134.05, 131.36, 129.77, 129.15, 128.64, 128.33, 127.92, 126.18, 78.54, 66.91, 53.65, 47.91. MS (ESI). Calc'd for C₁₇H₁₆Cl₂NO₂ [M+H]⁺: 336.05, found 336.16 **(****Figs. 10****,** **11****).**

### Methods

**LC-MS characterization.** Small molecules or peptides were dissolved in a H₂O/MeCN solution mixture, and an aliquot was taken for LC-MS analysis. Separation was achieved by gradient elution from 10-100% MeCN in water (constant 0.1 vol% formic acid) over 12 min, isocratic with 100% MeCN from 12 to 17 min and returned to initial conditions and equilibrated for 3 min. The LC chromatograms were recorded by monitoring absorption at 220 and 280 nm.

**Expression and purification of the influenza A hemagglutinin.** The hemagglutinin (HA) used for binding and crystallization studies were expressed using baculovirus expression system as described previously (2). Briefly, each HA was fused with a gp67 signal peptide at the N-terminus and to a BirA biotinylation site, thrombin cleavage site, foldon trimerization domain and His₆-tag at the C-terminus. Expressed HAs were purified using metal affinity chromatography using Ni-NTA resin. For binding studies, each HA was biotinylated using BirA and purified by gel filtration chromatography. For crystallization study, the HA was digested with trypsin (New England Biolabs) to produce uniformly cleaved (HA1/HA2), and to remove the trimerization domain and His₆-tag. The digested material was purified by gel filtration using Superdex 200 16/90 column on an ÄKTA (GE Healthcare Life Sciences).

**Surface Plasmon Resonance (SPR).** Direct binding of each compound to HA was measured by SPR using a Biacore 8K instrument (GE Healthcare) at 25 °C. Sensor chip SA (GE Healthcare) were used to create H1 biosensors by standard biotin-streptavidin coupling. 50 µg/ml HA diluted in PBS, pH 7.4 was injected for 15 min at 5 µl/min over a single flow cell. In total, six separate biotin-streptavidin-coupled H1 surfaces were generated during the course of this study with the following immobilization levels: H1/PR8 (5715 and 5042), H1/Mich15 (3081 and 3032), and H1/Cal04 (7547 and 8300) resonance units (RUs). All experiments were performed in a running buffer of PBS, pH 7.4, 0.005% (v/v) Tween-20, and 5% (v/v) DMSO (PBST-DMSO) using a flow rate of 30 µl/min. Solvent correction curves were obtained at the beginning and end by injecting varying DMSO concentrations (4.4, 4.6, 4.8, 5.0, 5.2, 5.4, 5.6, and 5.8% [v/v]). A reference flow cell was created on each sensor chip by injection of biotin.

To evaluate H1 binding, we diluted each compound from a 100 mM stock solution in neat DMSO to 20X of the desired concentrations, followed by dilution in a buffer containing 1.05X PBST to match the running buffer. Samples were injected in a dose-dependent manner over the H1 biosensor for 30 sec followed by 180 sec of dissociation phase and a subsequent wash step with a 50% (v/v) DMSO solution. The resulting sensorgrams were analyzed using Biacore 8K Evaluation Software as follows. Solvent correction curves were generated and applied to all datasets, and each sensorgram was double referenced by subtracting the nearest buffer blank injection. The signal just before injection stop of these corrected sensorgrams was treated as the H1-binding response for steady-state affinity analysis and fit using four-parameter variable slope nonlinear curve fitting in GraphPad Prism8 software.

**NanoDSF.** 10 µL of 0.2 mg/ml of HA protein in PBS, pH 7.4 was mixed with different concentrations of the sample (F0045(S), F0045(R)). DMSO and 3 µM P7 peptide were used as negative and positive controls. The mixture was then transferred to capillary and scanned in nanoDSF from 20 °C to 95 °C with 1 °C/min rate for protein unfold temperature.

***In vitro* human microsomal stability measurements.** Microsomal incubations were carried out in 96-well plates in 5 aliquots of 40 µL each (one for each time point). Liver microsomal incubation medium comprised of PBS (100 mM, pH 7.4), MgCl₂ (3.3 mM), NADPH (3 mM), glucose-6-phosphate (5.3 mM), glucose-6-phosphate dehydrogenase (0.67 units/ml) with 0.42 mg of liver microsomal protein per ml. In the control reactions the NADPH-cofactor system was substituted with PBS. Test compounds (2 µM, final solvent concentration 1.6%) were incubated with microsomes at 37 °C, shaking at 100 rpm. Each reaction was performed in duplicates. Five time points over 40 minutes were analyzed. The reactions were stopped by adding 12 volumes of 90% acetonitrile-water to incubation aliquots, followed by protein sedimentation by centrifuging at 5500 rpm for 3 min. Each reaction was performed in duplicates. Supernatants were analyzed using the HPLC system coupled with tandem mass spectrometer API 3000 (PE Sciex). The TurboIonSpray ion source was used in positive ion mode. Acquisition and analysis of the data were performed using Analyst 1.5.2 software (PE Sciex). The elimination constant (kₑ₁), half-life (t_{1/2}) and intrinsic clearance (Clᵢₙₜ) were determined by the plot of ln(area-under-the-curve) versus time, using linear regression analysis.

**Statistical analysis.** All data are shown as mean ± SD with n ≥ 3.

## Claims

1. A compound of formula (I) or (II): 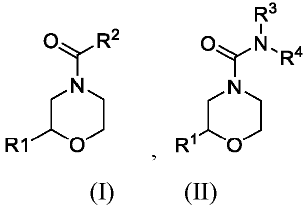 wherein
R¹ is phenyl;
R² is aryl, heterocyclyl, 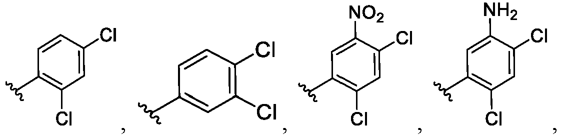 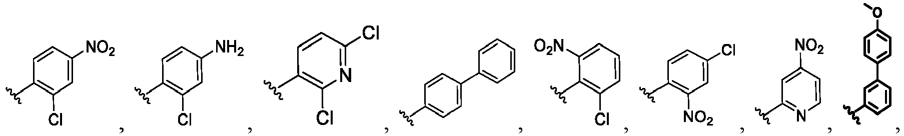 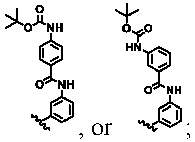
R³ is selected from H, alkyl, -alkylene-(heterocyclyl), -alkylene-(aryl), -alkylene-(cycloalkyl), -alkylene-O-R⁵, -alkylene-C(O)N(R⁵)₂, -alkylene-C(O)R⁵, -alkylene-C(O)₂R⁵, -alkylene-N(R⁵)₂, -alkylene-N(R⁵)C(O)R⁵, -alkylene-N(R⁵)C(O)N(R⁵)₂, -alkylene-N(R⁵)SO₂-R⁵, -alkylene-S-R⁵, -aryl, -arylene-(heterocyclyl), -arylene-alkyl, -arylene-(cycloalkyl), -arylene-O-R⁵, -arylene-C(O)N(R⁵)₂, -arylene-C(O)R⁵, -arylene-C(O)₂R⁵, -arylene-N(R⁵)₂, -arylene-N(R⁵)C(O)R⁵, -arylene-N(R⁵)C(O)N(R⁵)₂, -arylene-N(R⁵)SO₂-R⁵, -arylene-S-R⁵, -heterocyclyl, -heterocyclylene-(alkyl), -heterocyclylene-(aryl), -heterocyclylene-(cycloalkyl), -heterocyclylene-(cycloalkyl), -heterocyclylene-O-R⁵, -heterocyclylene-C(O)N(R⁵)₂, -heterocyclylene-C(O)R⁵, -heterocyclylene-C(O)₂R⁵, - heterocyclylene-N(R⁵)₂, -heterocyclylene-N(R⁵)C(O)R⁵, -heterocyclylene-N(R⁵)C(O)N(R⁵)₂, - heterocyclylene-N(R⁵)SO₂-R⁵, -heterocyclylene-S-R⁵, -cycloalkyl, -cycloalkylene-(alkyl), -cycloalkylene-(aryl), -cycloalkylene-(cycloalkyl), -cycloalkylene(heterocycloalkyl), -cycloalkylene-O-R⁵, -cycloalkylene-C(O)N(R⁵)₂, -cycloalkylene-C(O)R⁵, -cycloalkylene-C(O)₂R⁵, -cycloalkylene-N(R⁵)₂, -cycloalkylene-N(R⁵)C(O)R⁵, -cycloalkylene-N(R⁵)C(O)N(R⁵)₂, -cycloalkylene-N(R⁵)SO₂-R⁵, -alkylene-N(H)-alkylene-C(O)OR⁵, -alkylene-C(O)OR⁵, -arylene-alkylene-aryl, -C(O)R⁵, -C(O)₂R⁵, -alkylene-S-S-R⁵, -C(O)-alkylene-N(R⁵)₂, -C(O)₂R⁵, -aryl-alkylene-N(R⁵)C(O)-R⁵, -cycloalkylene-N(R⁵)S(O)(F)-R⁵, -SO₂-NR⁵, -S(O)(F)-NR⁵;
R⁴ is selected from alkyl, -alkylene-(heterocyclyl), -alkylene-(aryl), -alkylene-(cycloalkyl), -alkylene-O-R⁵, -alkylene-C(O)N(R⁵)₂, -alkylene-C(O)R⁵, -alkylene-C(O)₂R⁵, -alkylene-N(R⁵)₂, -alkylene-N(R⁵)C(O)R⁵, -alkylene-N(R⁵)C(O)N(R⁵)₂, -alkylene-N(R⁵)SO₂-R⁵, -alkylene-S-R⁵, -aryl, -arylene-(heterocyclyl), -arylene-alkyl, -arylene-(cycloalkyl), -arylene-O-R⁵, -arylene-C(O)N(R⁵)₂, -arylene-C(O)R⁵, -arylene-C(O)₂R⁵, -arylene-N(R⁵)₂, -arylene-N(R⁵)C(O)R⁵, -arylene-N(R⁵)C(O)N(R⁵)₂, -arylene-N(R⁵)SO₂-R⁵, -arylene-S-R⁵, -heterocyclyl, -heterocyclylene-(alkyl), -heterocyclylene-(aryl), -heterocyclylene-(cycloalkyl), -heterocyclylene-(cycloalkyl), -heterocyclylene-O-R⁵, -heterocyclylene-C(O)N(R⁵)₂, -heterocyclylene-C(O)R⁵, -heterocyclylene-C(O)₂R⁵, -heterocyclylene-N(R⁵)₂, heterocyclylene-N(R⁵)C(O)R⁵, -heterocyclylene-N(R⁵)C(O)N(R⁵)₂-, -heterocyclylene-N(R⁵)SO₂-R⁵, -heterocyclylene-S-R⁵, -cycloalkyl, -cycloalkylene-(alkyl), -cycloalkylene-(aryl), -cycloalkylene-(cycloalkyl), -cycloalkylene(heterocycloalkyl), -cycloalkylene-O-R⁵, -cycloalkylene-C(O)N(R⁵)₂, -cycloalkylene-C(O)R⁵, -cycloalkylene-C(O)₂R⁵, -cycloalkylene-N(R⁵)₂, -cycloalkylene-N(R⁵)C(O)R⁵, -cycloalkylene-N(R⁵)C(O)N(R⁵)₂, -cycloalkylene-N(R⁵)SO₂-R⁵, -alkylene-N(H)-alkylene-C(O)OR⁵, -alkylene-C(O)OR⁵, -arylene-alkylene-aryl, -C(O)R⁵, -C(O)₂R⁵, -alkylene-S-S-R⁵, -C(O)-alkylene-N(R⁵)₂, -C(O)₂R⁵, -aryl-alkylene-N(R⁵)C(O)-R⁵, -cycloalkylene-N(R⁵)S(O)(F)-R⁵, -SO₂-NR⁵, -S(O)(F)-NR⁵; or
R³ and R⁴ can be taken together to form a heterocyclyl; or 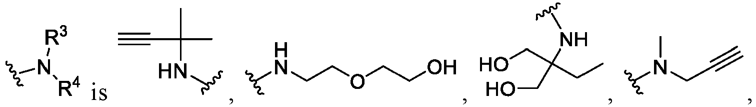 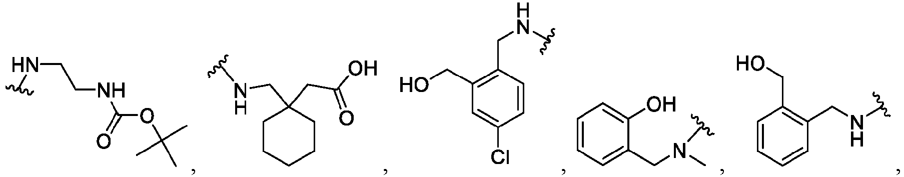 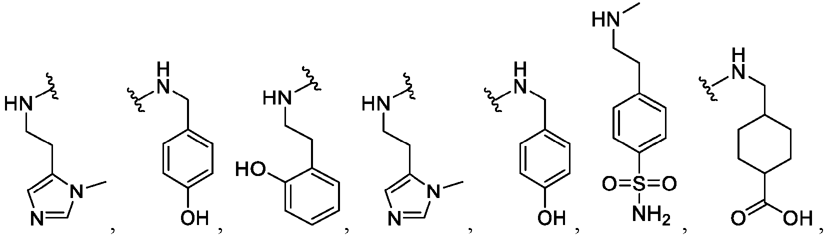 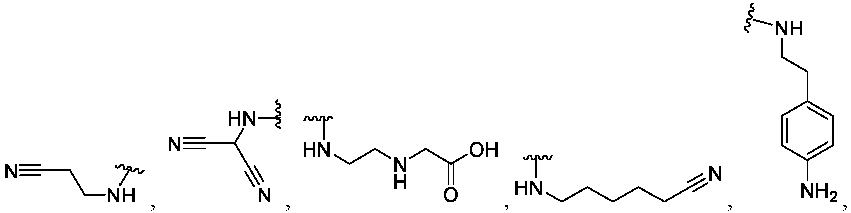 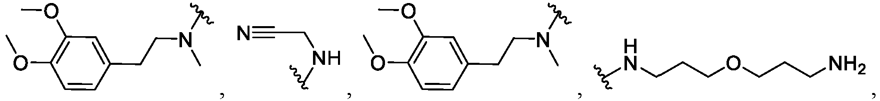 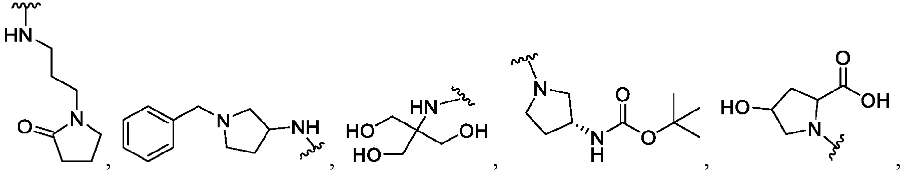 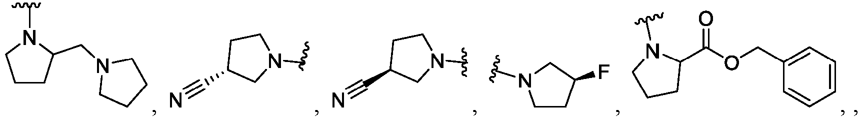 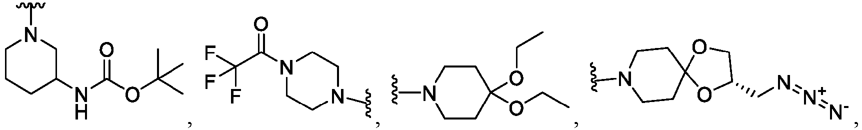 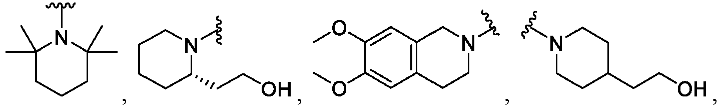 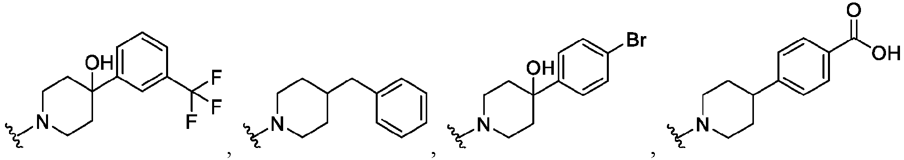 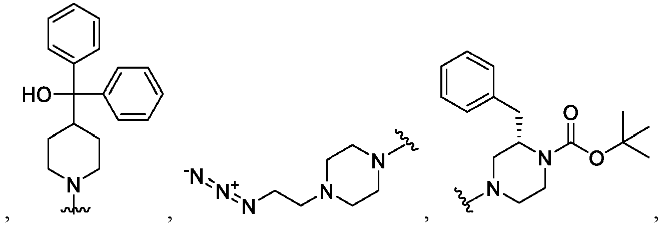 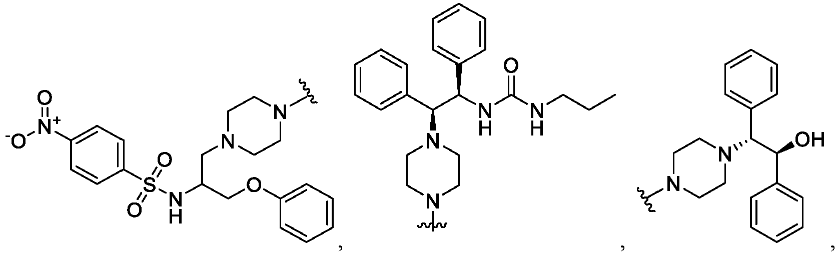 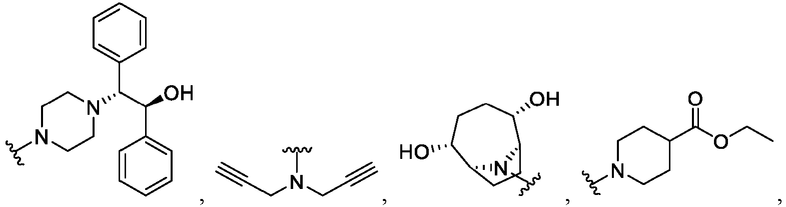 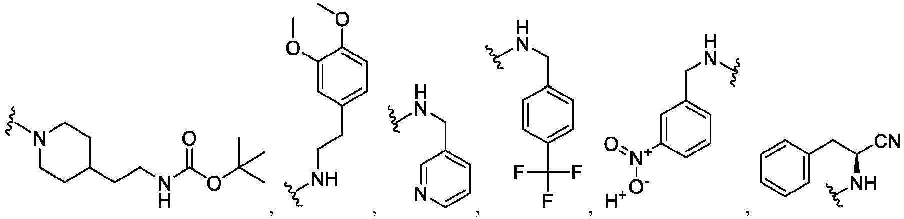 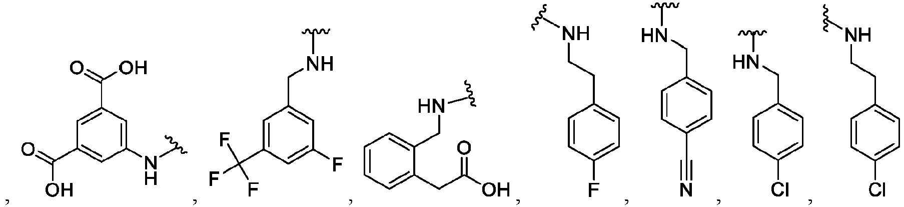 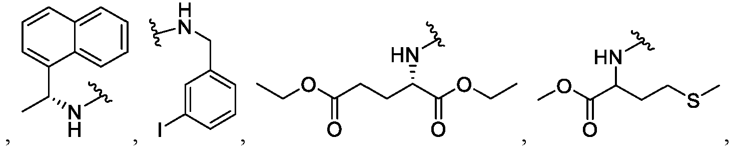 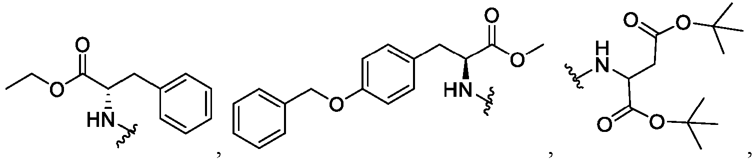 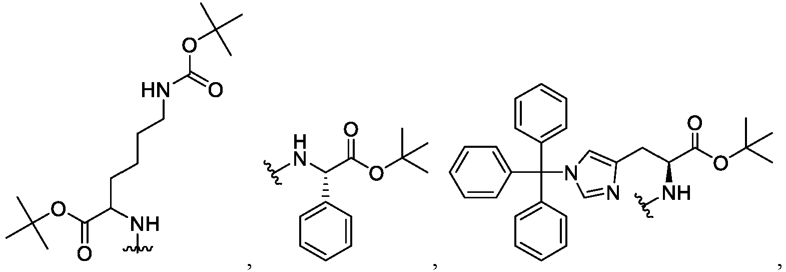 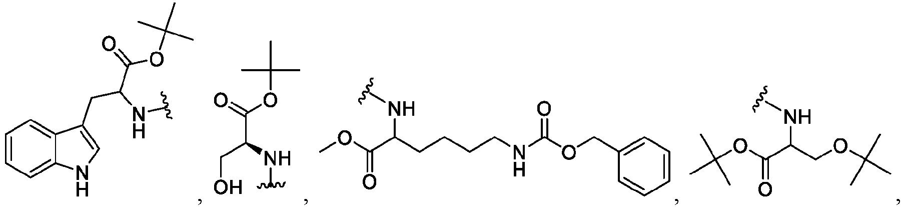 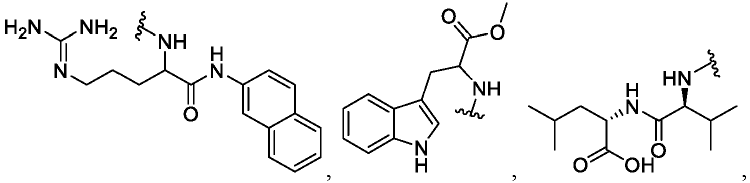 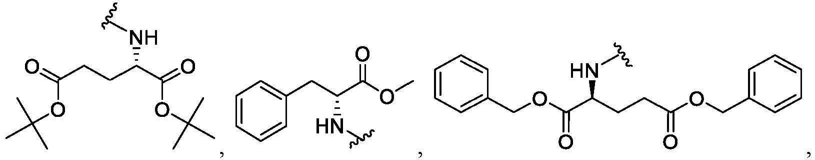 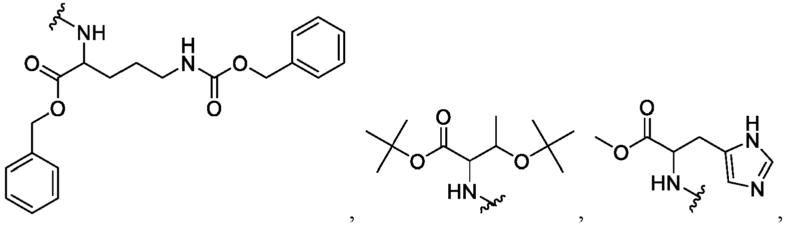 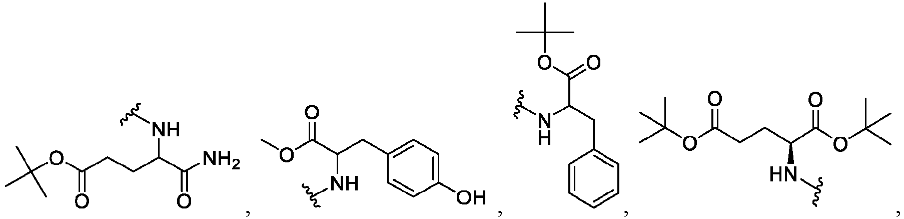 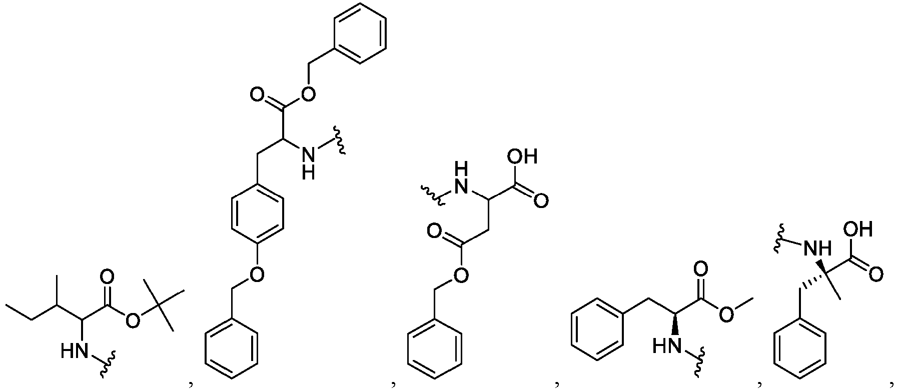 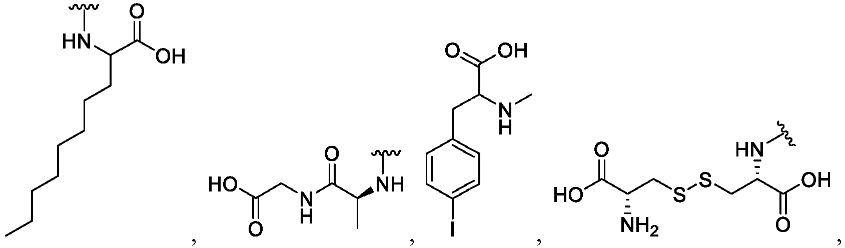 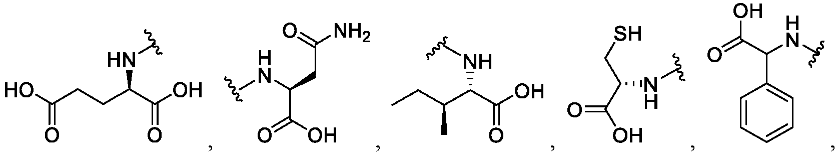 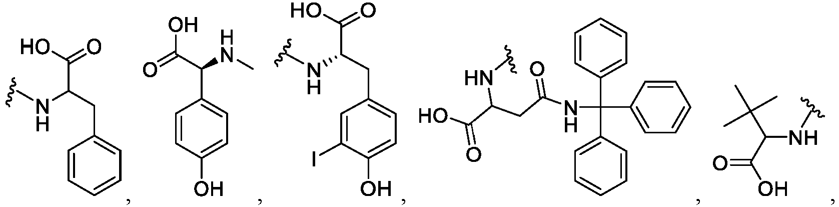 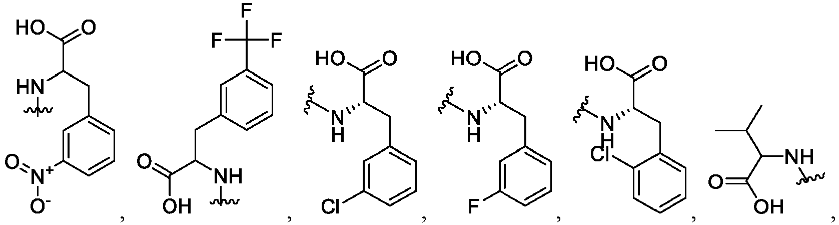 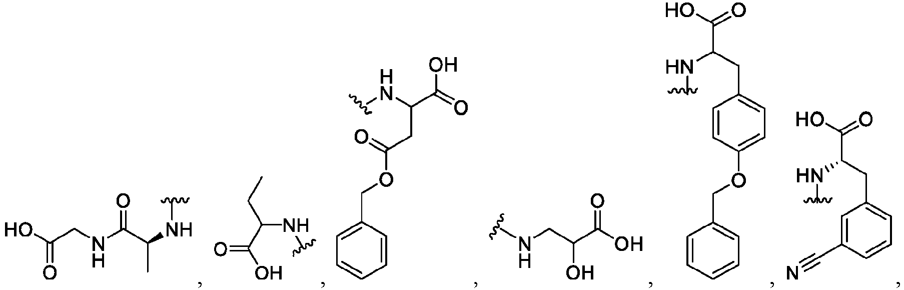 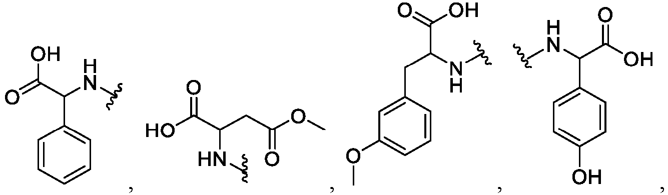 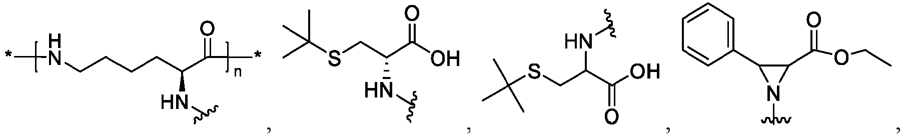 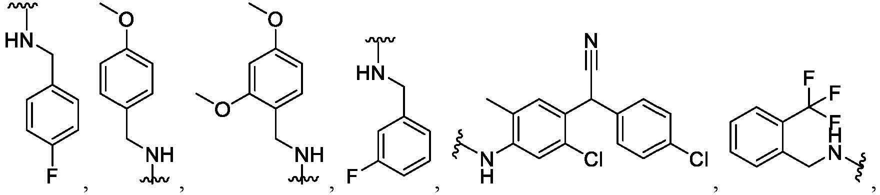 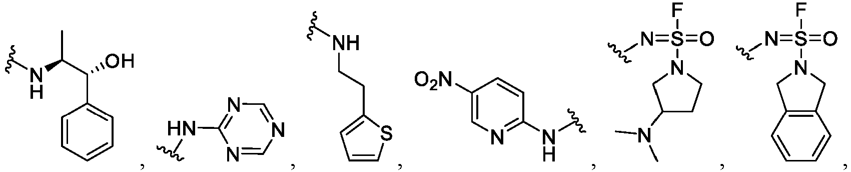 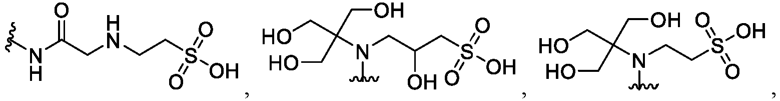 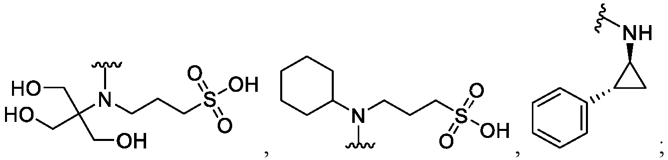 and
R⁵ is H, alkyl, aryl or -alkylene-aryl;
wherein, unless otherwise specified:
the term alkyl defines a branched or unbranched carbon chain moiety up to 30 carbon atoms;
the term alkylene defines an alkyl group having from 2 to 12 carbon atoms that contains two points of attachment to the rest of the compound on its longest carbon chain;
the term aryl defines a 5- to 12-membered aromatic ring, wherein each atom of the ring is carbon or one or more ring atoms are heteroatoms;
the term heterocyclyl defines a 3- to 12-membered ring structure, wherein one to four ring atoms are heteroatoms; and
the term cycloalkyl defines a mono-, bicyclic, bridged, spirocyclic or polycyclic saturated carbocyclic ring having from 3 to 12 carbon atoms;
wherein the compound of formula (II) is not

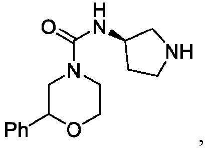

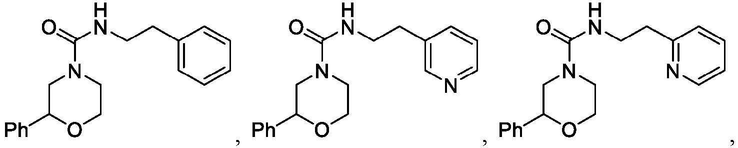

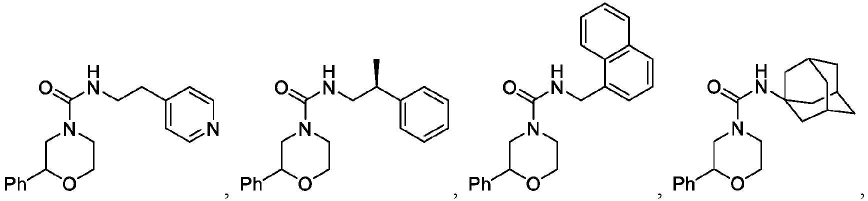

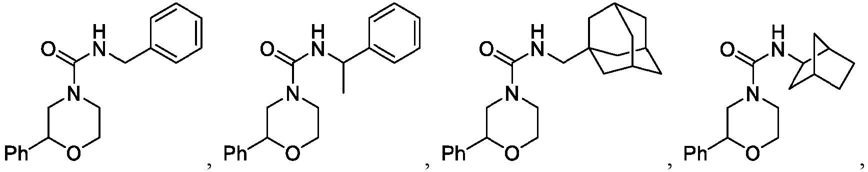

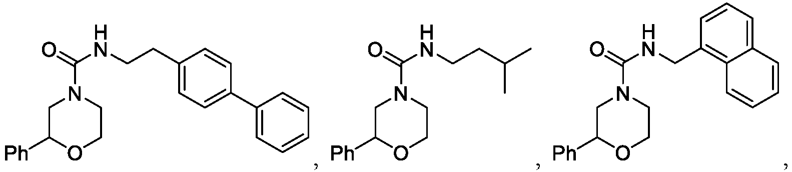

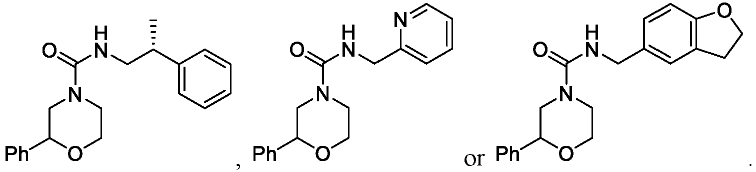

2. The compound of claim 1, wherein the compound is of formula (II)

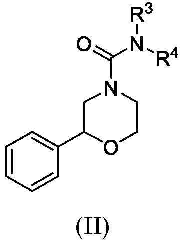

3. The compound of claim 1, wherein R² is selected from the group consisting of:

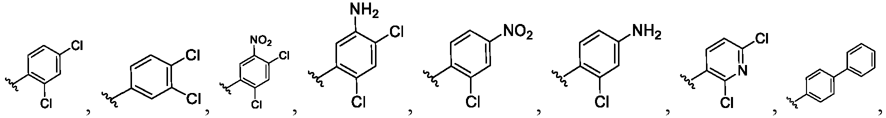

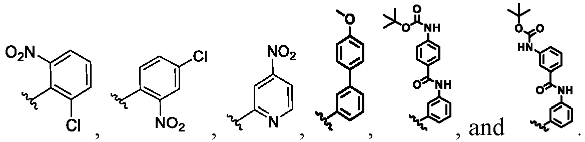

4. The compound of claim 1 or 2, wherein 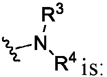
- selected from the group consisting of: 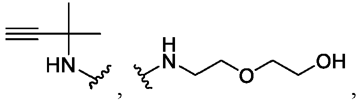 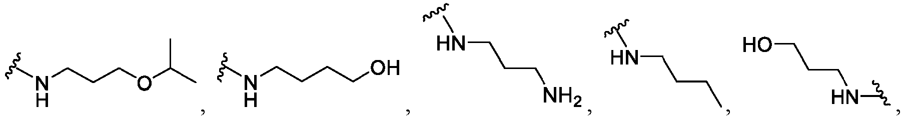 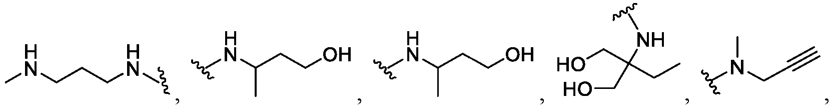 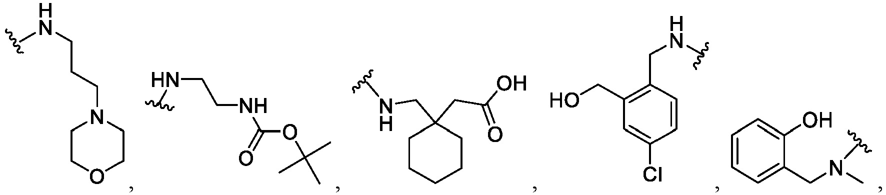 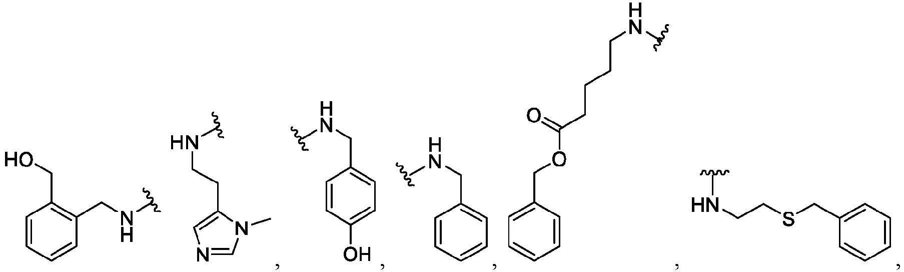 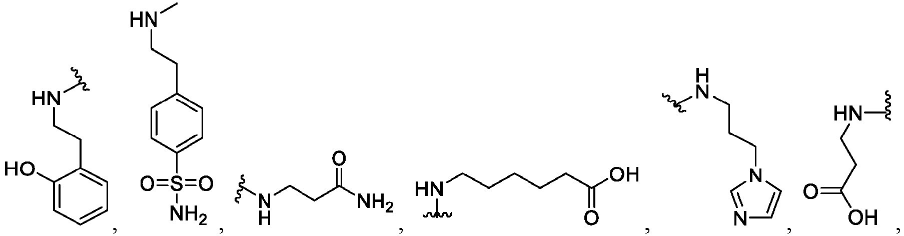 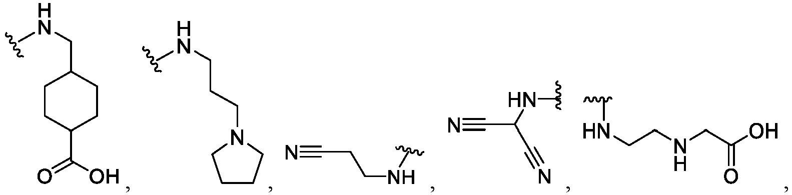 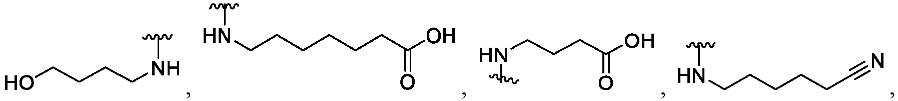 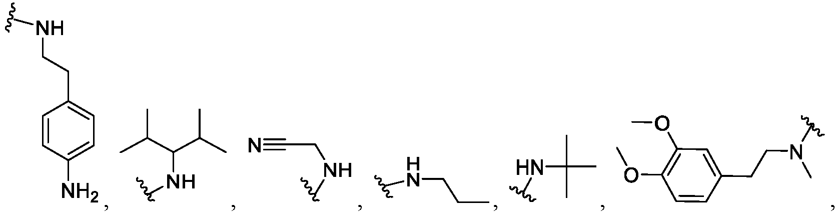 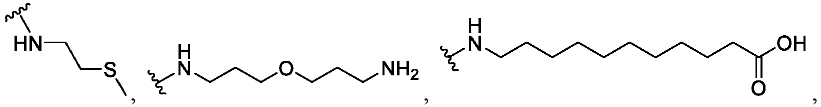 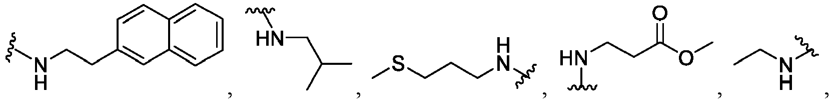 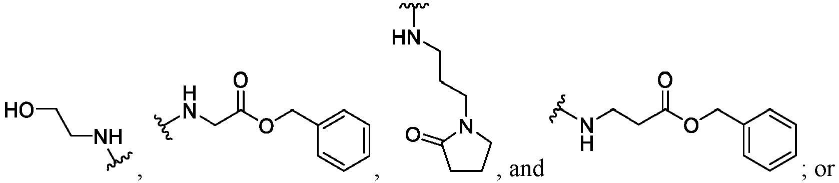
- selected from the group consisting of: 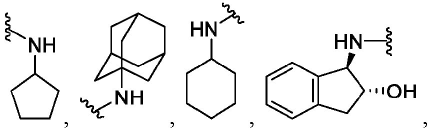 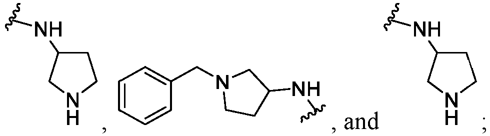 or
- 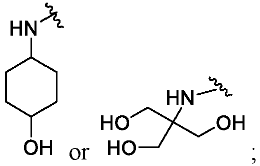 or
- selected from the group consisting of:

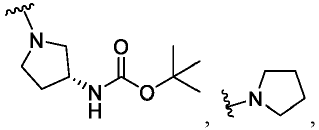

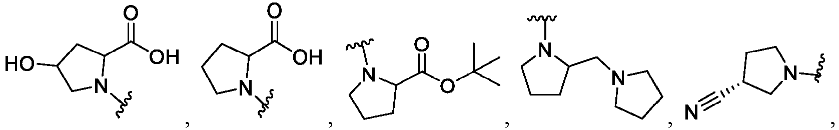

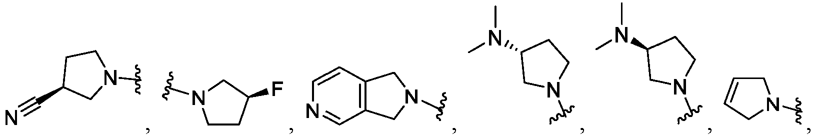

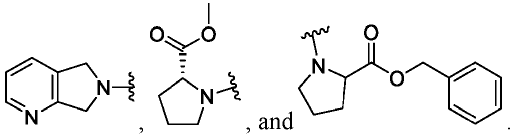

5. The compound of claim 1 or 2, wherein 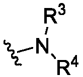 is:
- selected from the group consisting of: 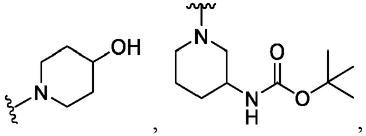 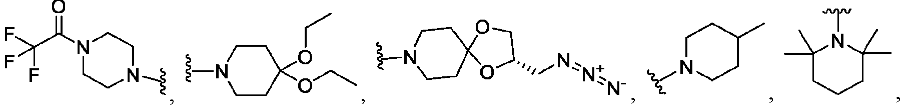 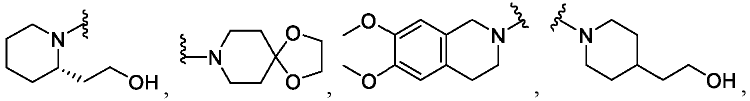 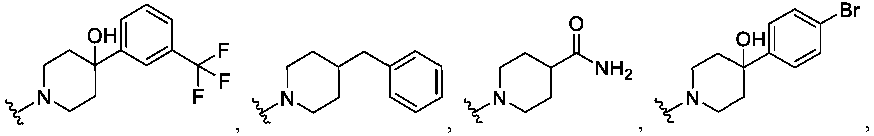 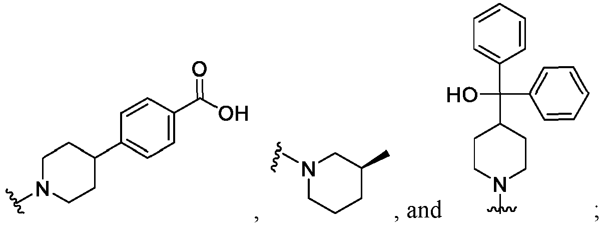 or
- selected from the group consisting of: 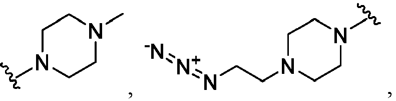 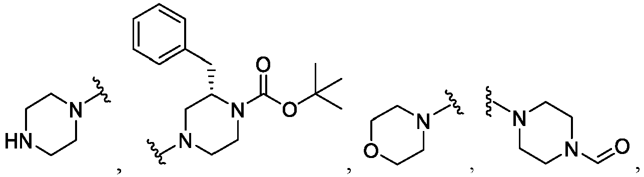 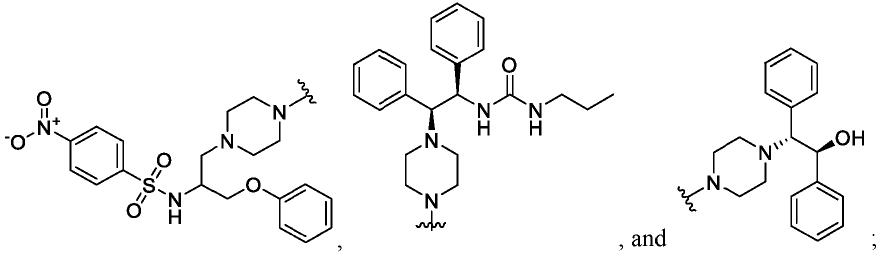 or
- 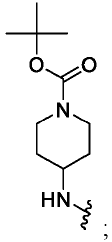 or
- 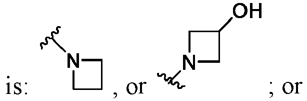
- selected from the group consisting of: 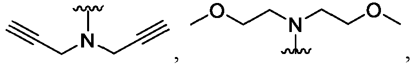 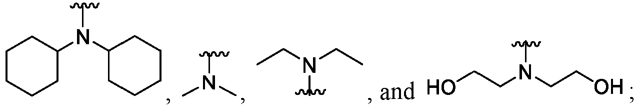 or
- is selected from the group consisting of

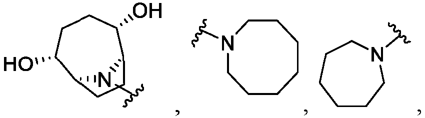

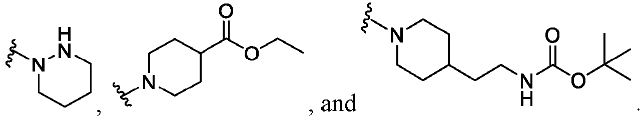

6. The compound of claim 1 or 2, wherein 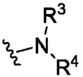 is:
- selected from the group consisting of: 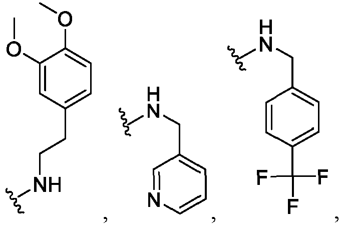 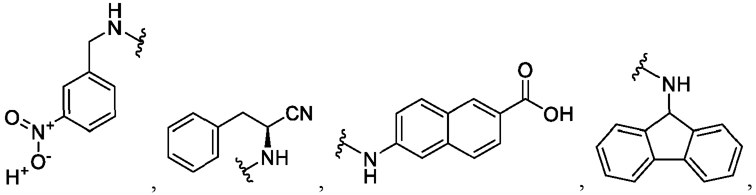 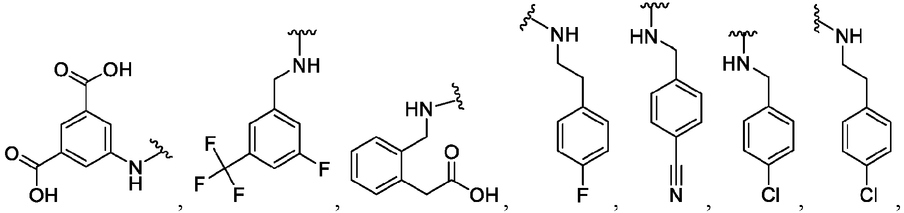 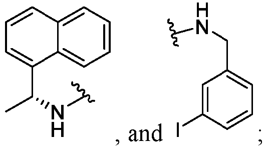 or
- selected from the group consisting: 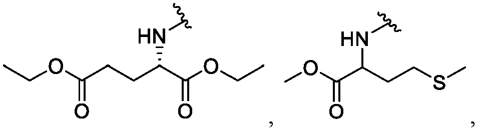 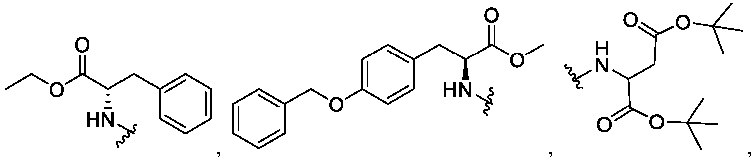 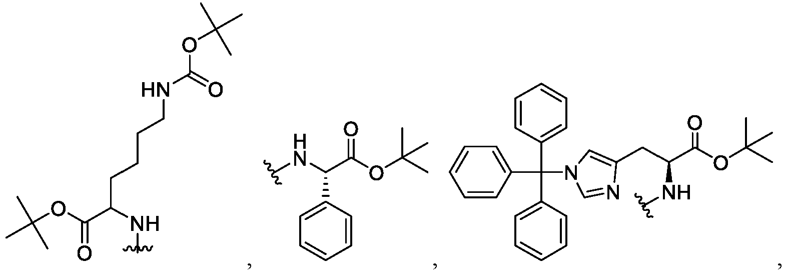 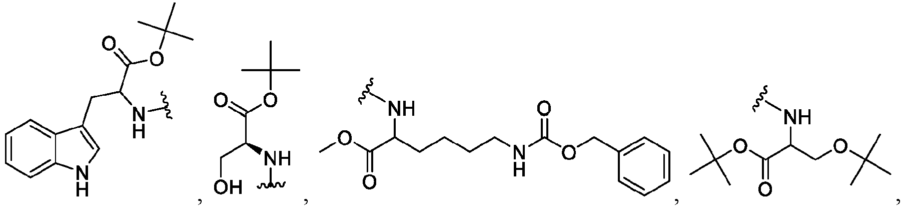 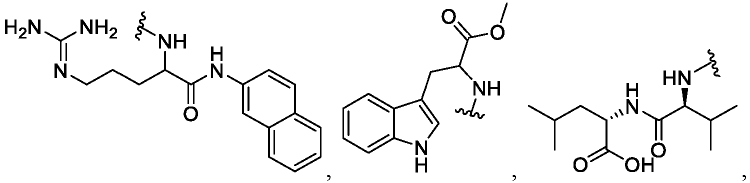 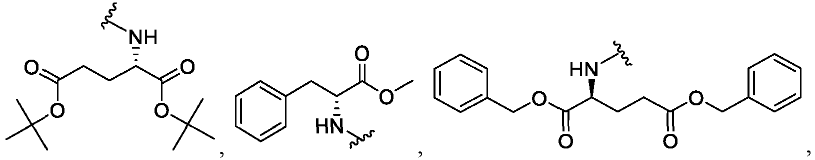 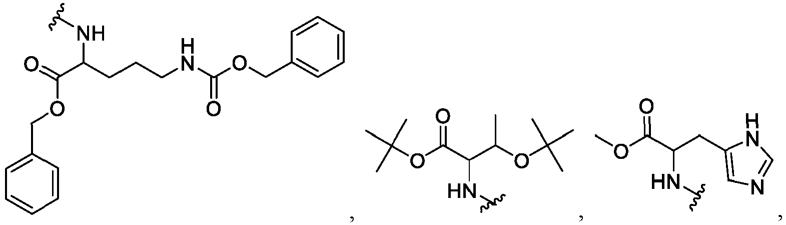 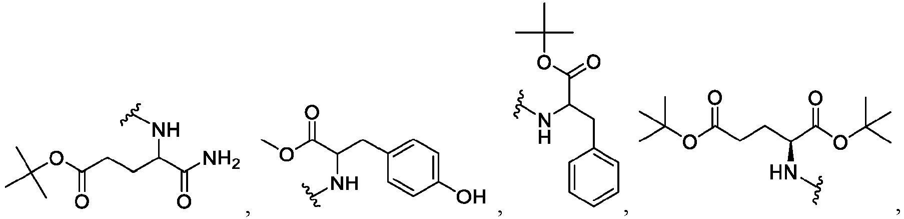 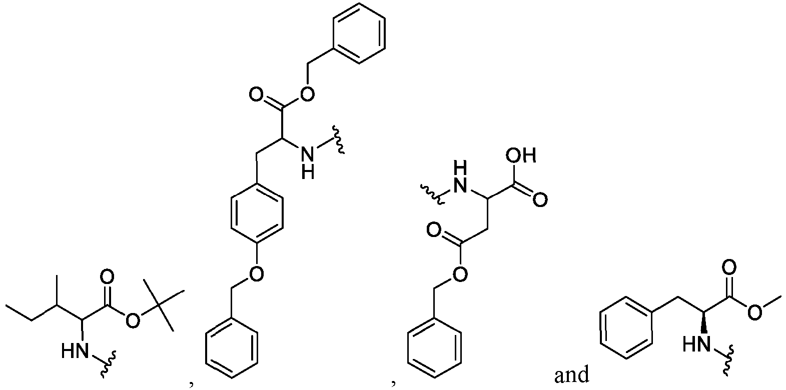 or
- selected from the group consisting of: 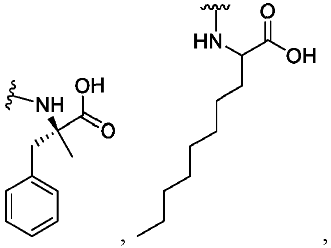 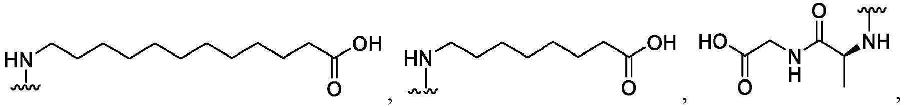 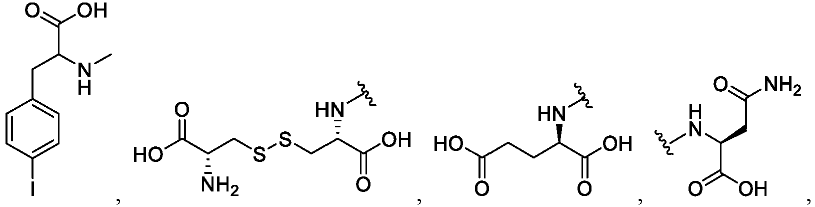 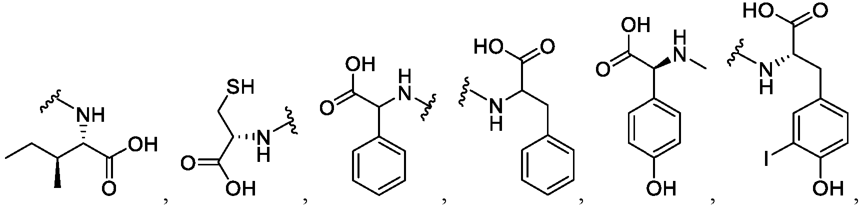 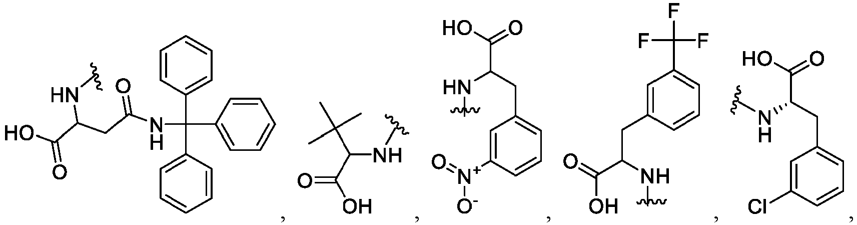 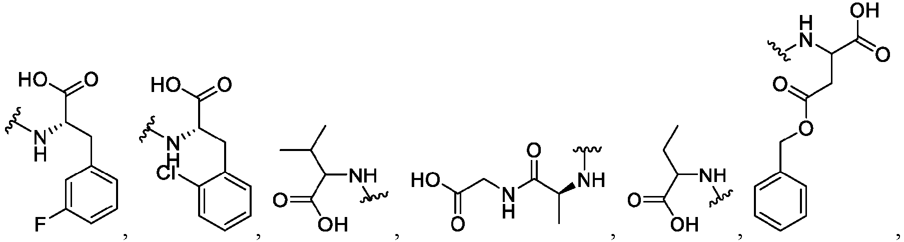 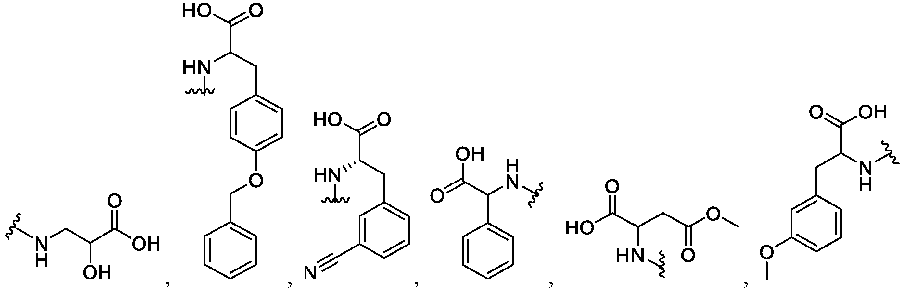 and

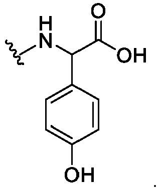

7. The compound of claim 1 or 2, wherein 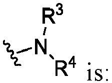 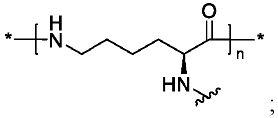 or 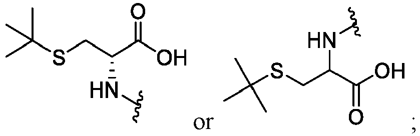 or
- s elected from the group consisting of 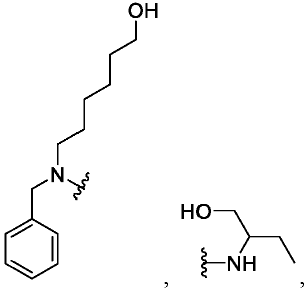 and 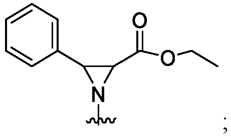 or
- is selected from the group consisting of: 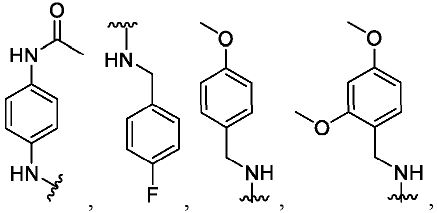 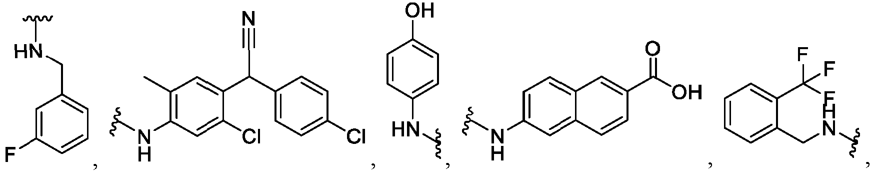 and 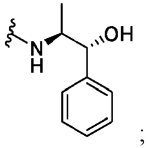 or
- is selected from the group selected from: 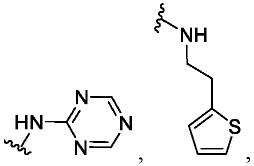 and 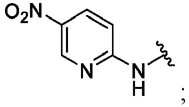 or
- 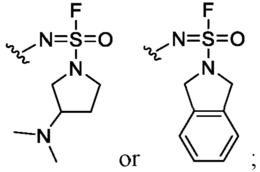 or
- selected from the group consisting of: 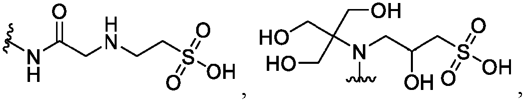 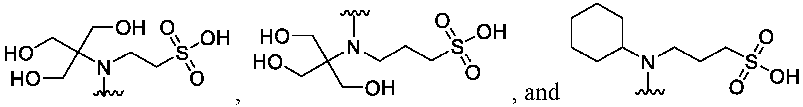 or
- is

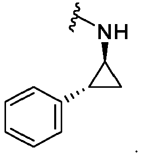

8. The compound of claim 1 or 2, wherein the compound of formula (I) or (II) is:
- selected from the group consisting of: 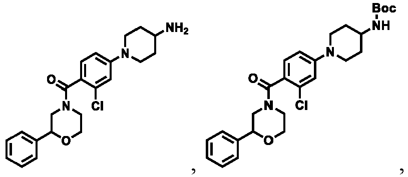 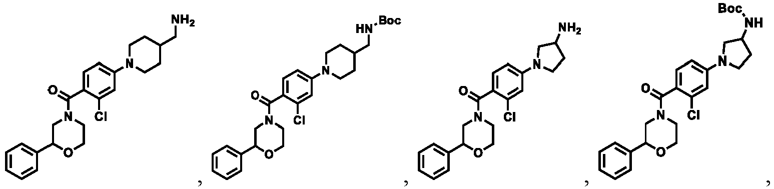 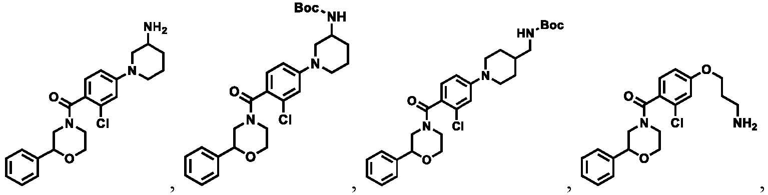 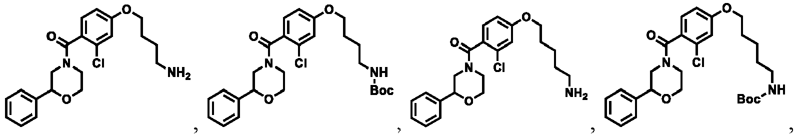 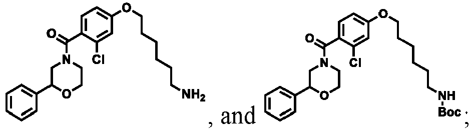 or
- selected from the group consisting of: 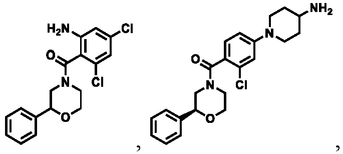 and 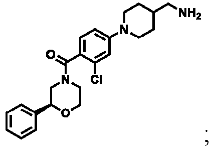 or
- 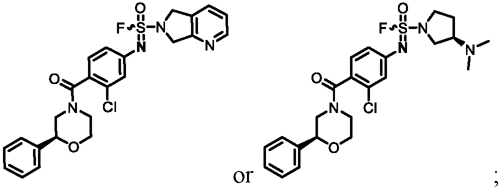 or
- selected from the group consisting of:

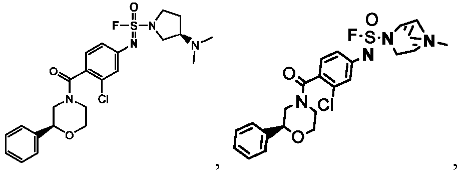

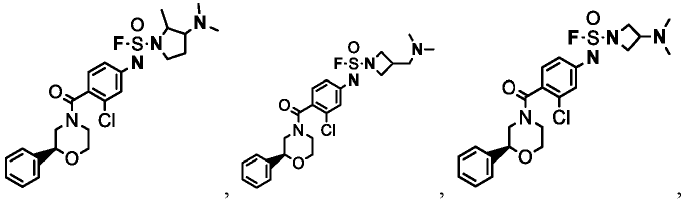

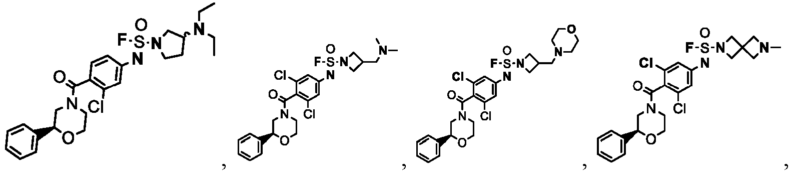

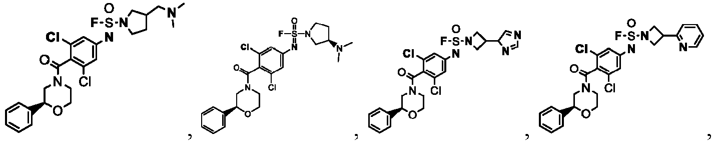

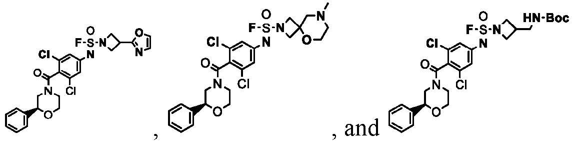

9. The compound of claim 1 or 2, wherein the compound of formula (I) or (II) is:
- selected from the group consisting of: 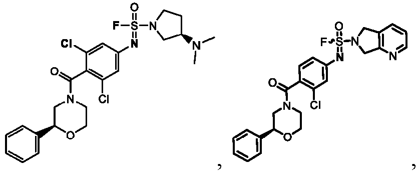 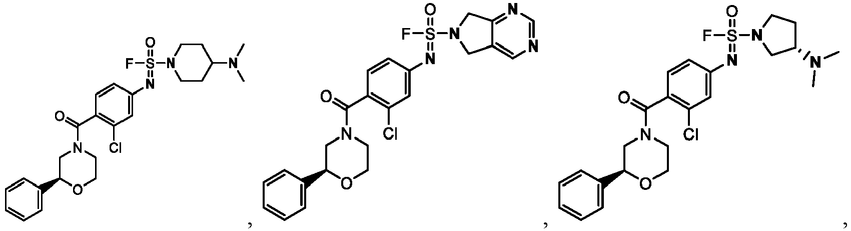 and 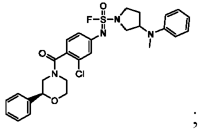 or
- selected from the group consisting of: 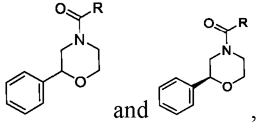 wherein R is 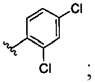 or
- selected from the group consisting of: 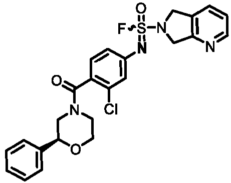 and 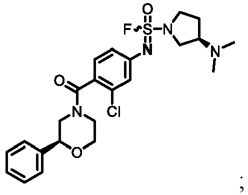 or
- 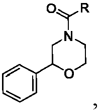 wherein R is selected from the group consisting of:

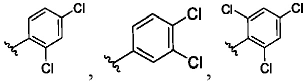

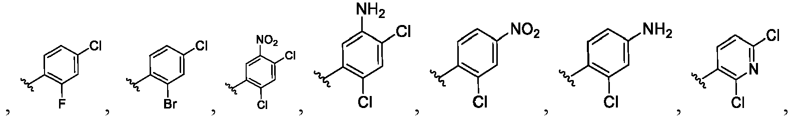

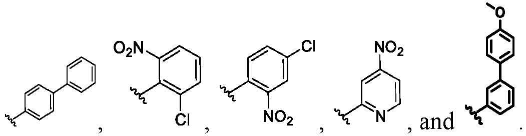

10. The compound of claim 1 or 2, wherein the compound of formula (I) or (II) is:
- selected from the group consisting of: 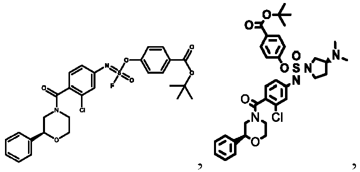 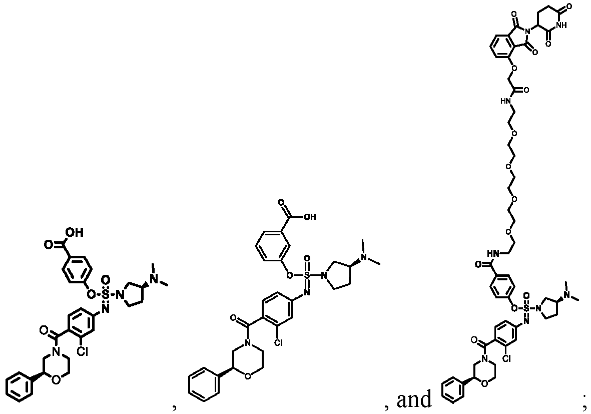 or
- 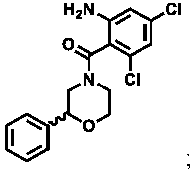 or is selected from the group consisting of: 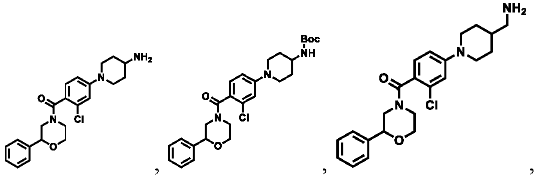 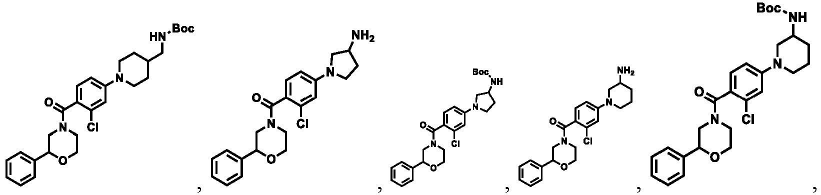 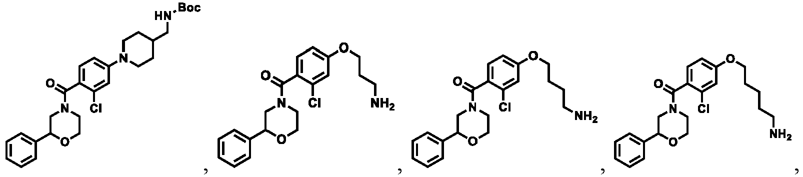 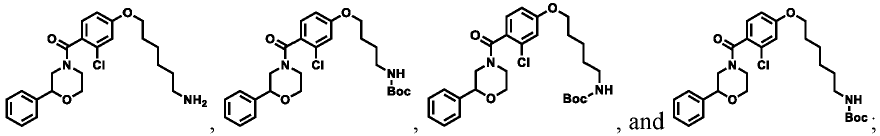 or
- 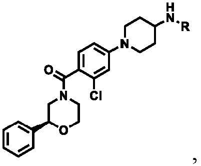 wherein R is selected from the group consisting of 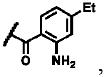 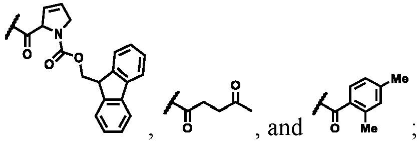 or
- 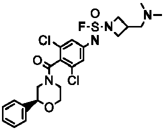 or
- 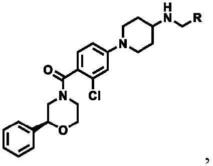 wherein R is selected from the group consisting of 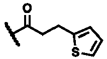 and 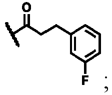 or
- 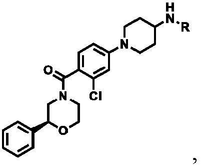 wherein R is selected from the group consisting of 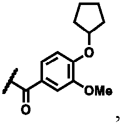 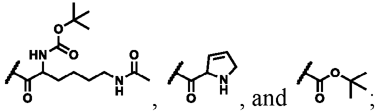 or
- selected from the group consisting of: 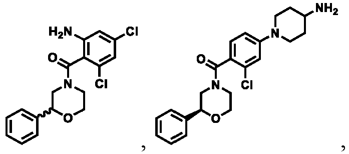 and

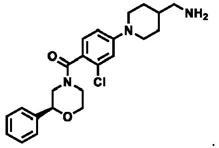

11. A pharmaceutical composition comprising a compound of any one of claims 1-10 and a pharmaceutical acceptable carrier.

12. The compound of any one of claims 1-10 or the pharmaceutical composition of claim 11, for use as a medicament.

13. A compound of formula (I) or (II): 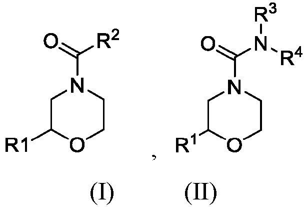 wherein
R¹ is phenyl;
R² is aryl, heterocyclyl, 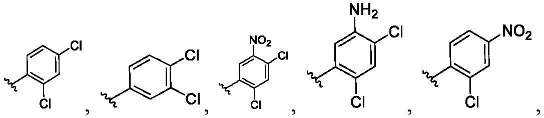 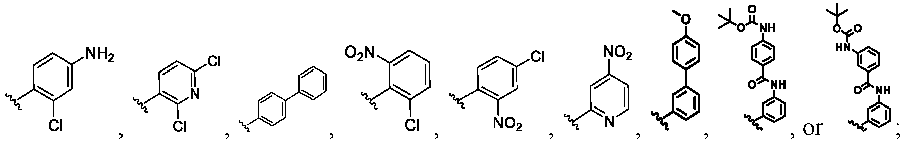
R³ is selected from H, alkyl, -alkylene-(heterocyclyl), -alkylene-(aryl), -alkylene-(cycloalkyl), -alkylene-O-R⁵, -alkylene-C(O)N(R⁵)₂, -alkylene-C(O)R⁵, -alkylene-C(O)₂R⁵, -alkylene-N(R⁵)₂, -alkylene-N(R⁵)C(O)R⁵, -alkylene-N(R⁵)C(O)N(R⁵)₂, -alkylene-N(R⁵)SO₂-R⁵, -alkylene-S-R⁵, -aryl, -arylene-(heterocyclyl), -arylene-alkyl, -arylene-(cycloalkyl), -arylene-O-R⁵, -arylene-C(O)N(R⁵)₂, -arylene-C(O)R⁵, -arylene-C(O)₂R⁵, -arylene-N(R⁵)₂, -arylene-N(R⁵)C(O)R⁵, -arylene-N(R⁵)C(O)N(R⁵)₂, -arylene-N(R⁵)SO₂-R⁵, -arylene-S-R⁵, -heterocyclyl, -heterocyclylene-(alkyl), -heterocyclylene-(aryl), -heterocyclylene-(cycloalkyl), -heterocyclylene-(cycloalkyl), -heterocyclylene-O-R⁵, -heterocyclylene-C(O)N(R⁵)₂, -heterocyclylene-C(O)R⁵, -heterocyclylene-C(O)₂R⁵, - heterocyclylene-N(R⁵)₂, -heterocyclylene-N(R⁵)C(O)R⁵, -heterocyclylene-N(R⁵)C(O)N(R⁵)₂, - heterocyclylene-N(R⁵)SO₂-R⁵, -heterocyclylene-S-R⁵, -cycloalkyl, -cycloalkylene-(alkyl), -cycloalkylene-(aryl), -cycloalkylene-(cycloalkyl), -cycloalkylene(heterocycloalkyl), -cycloalkylene-O-R⁵, -cycloalkylene-C(O)N(R⁵)₂, -cycloalkylene-C(O)R⁵, -cycloalkylene-C(O)₂R⁵, -cycloalkylene-N(R⁵)₂, -cycloalkylene-N(R⁵)C(O)R⁵, -cycloalkylene-N(R⁵)C(O)N(R⁵)₂, -cycloalkylene-N(R⁵)SO₂-R⁵, -alkylene-N(H)-alkylene-C(O)OR⁵, -alkylene-C(O)OR⁵, -arylene-alkylene-aryl, -C(O)R⁵, -C(O)₂R⁵, -alkylene-S-S-R⁵, -C(O)-alkyl, -C(O)-alkylene-N(R⁵)₂, -C(O)₂R⁵, -aryl-alkylene-N(R⁵)C(O)-R⁵, -cycloalkylene-N(R⁵)S(O)(F)-R⁵, -SO₂-NR⁵, -S(O)(F)-NR⁵;
R⁴ is selected from alkyl, -alkylene-(heterocyclyl), -alkylene-(aryl), -alkylene-(cycloalkyl), -alkylene-O-R⁵, -alkylene-C(O)N(R⁵)₂, -alkylene-C(O)R⁵, -alkylene-C(O)₂R⁵, -alkylene-N(R⁵)₂, -alkylene-N(R⁵)C(O)R⁵, -alkylene-N(R⁵)C(O)N(R⁵)₂, -alkylene-N(R⁵)SO₂-R⁵, -alkylene-S-R⁵, -aryl, -arylene-(heterocyclyl), -arylene-alkyl, -arylene-(cycloalkyl), -arylene-O-R⁵, -arylene-C(O)N(R⁵)₂, -arylene-C(O)R⁵, -arylene-C(O)₂R⁵, -arylene-N(R⁵)₂, -arylene-N(R⁵)C(O)R⁵, -arylene-N(R⁵)C(O)N(R⁵)₂, -arylene-N(R⁵)SO₂-R⁵, -arylene-S-R⁵, -heterocyclyl, -heterocyclylene-(alkyl), -heterocyclylene-(aryl), -heterocyclylene-(cycloalkyl), -heterocyclylene-(cycloalkyl), -heterocyclylene-O-R⁵, -heterocyclylene-C(O)N(R⁵)₂, -heterocyclylene-C(O)R⁵, -heterocyclylene-C(O)₂R⁵, -heterocyclylene-N(R⁵)₂, heterocyclylene-N(R⁵)C(O)R⁵, -heterocyclylene-N(R⁵)C(O)N(R⁵)₂-, -heterocyclylene-N(R⁵)SO₂-R⁵, -heterocyclylene-S-R⁵, -cycloalkyl, -cycloalkylene-(alkyl), -cycloalkylene-(aryl), -cycloalkylene-(cycloalkyl), -cycloalkylene(heterocycloalkyl), -cycloalkylene-O-R⁵, -cycloalkylene-C(O)N(R⁵)₂, -cycloalkylene-C(O)R⁵, -cycloalkylene-C(O)₂R⁵, -cycloalkylene-N(R⁵)₂, -cycloalkylene-N(R⁵)C(O)R⁵, -cycloalkylene-N(R⁵)C(O)N(R⁵)₂, -cycloalkylene-N(R⁵)SO₂-R⁵, -alkylene-N(H)-alkylene-C(O)OR⁵, -alkylene-C(O)OR⁵, -arylene-alkylene-aryl, -C(O)R⁵, -C(O)₂R⁵, -alkylene-S-S-R⁵, -C(O)-alkyl, -C(O)-alkylene-N(R⁵)₂, -C(O)₂R⁵, -aryl-alkylene-N(R⁵)C(O)-R⁵, -cycloalkylene-N(R⁵)S(O)(F)-R⁵, -SO₂-NR⁵, -S(O)(F)-NR⁵; or
R³ and R⁴ can be taken together to form a heterocyclyl; or 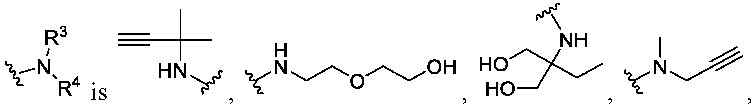 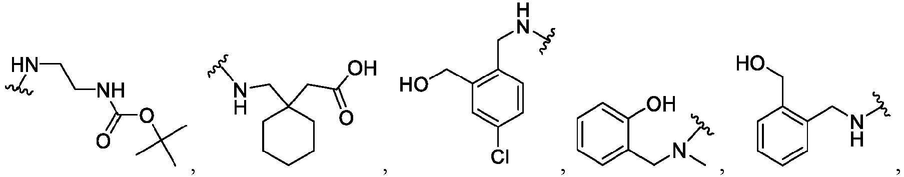 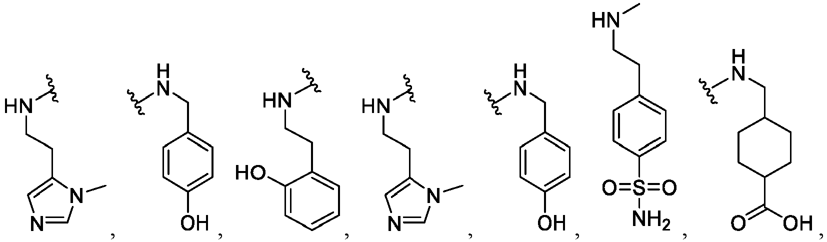 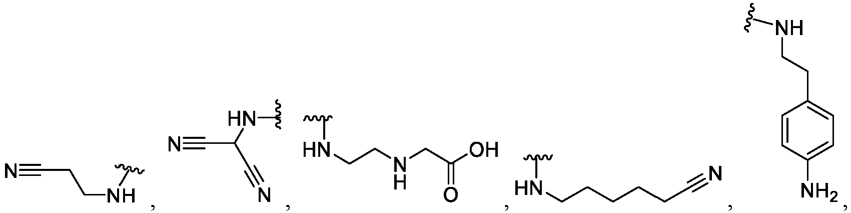 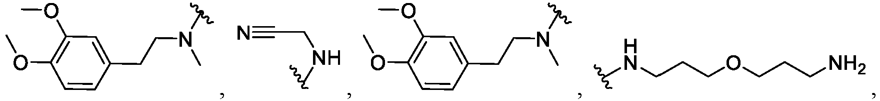 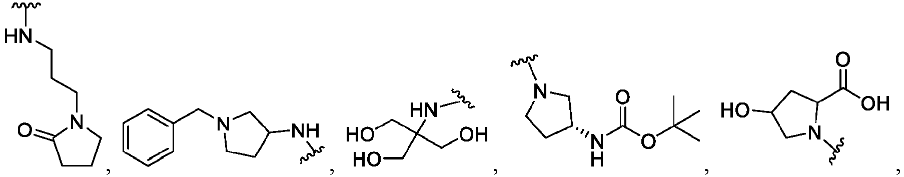 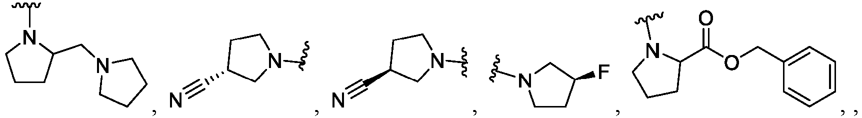 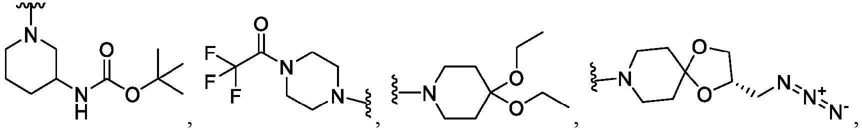 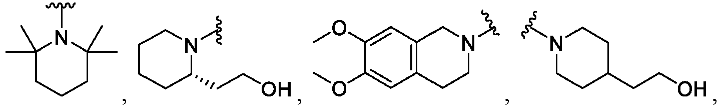 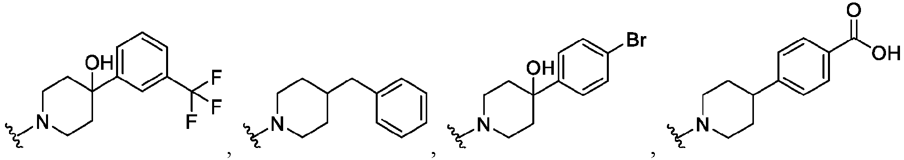 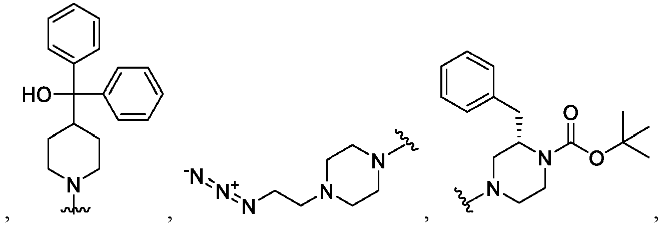 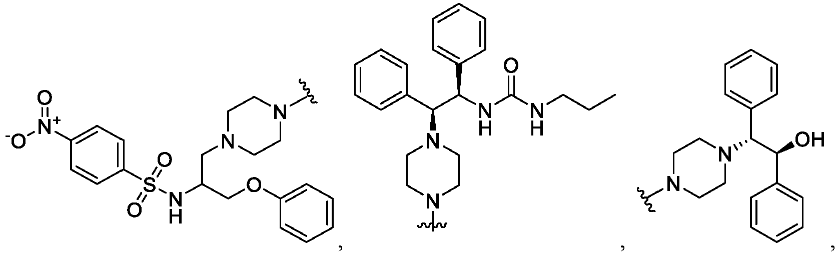 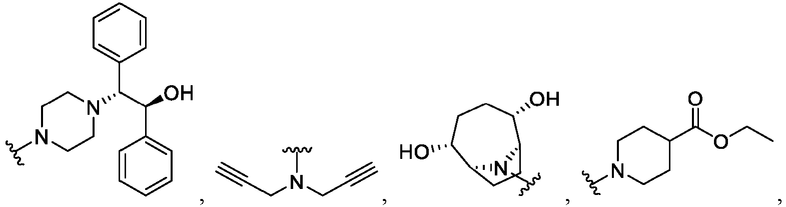 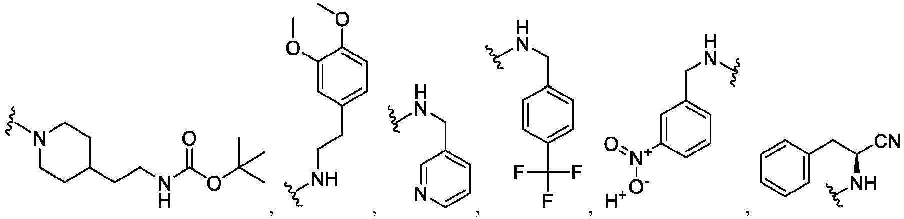 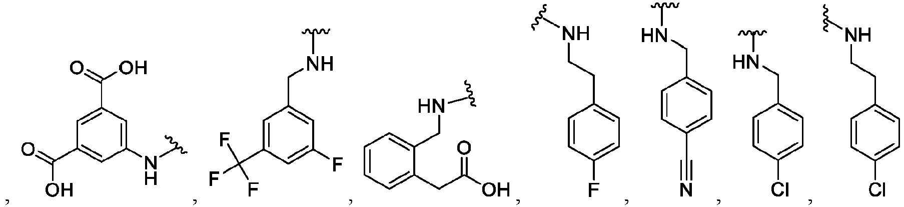 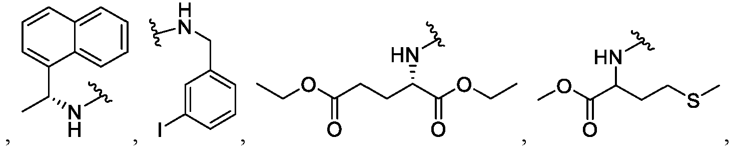 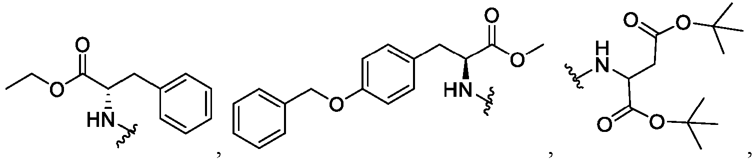 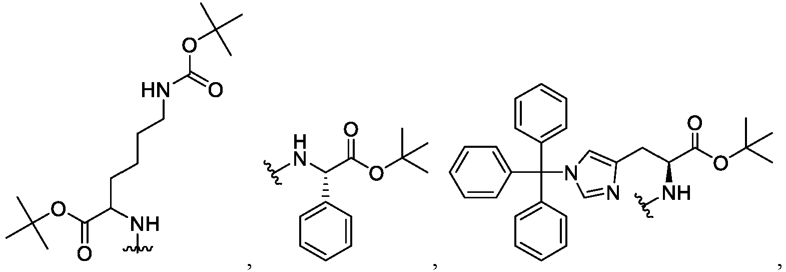 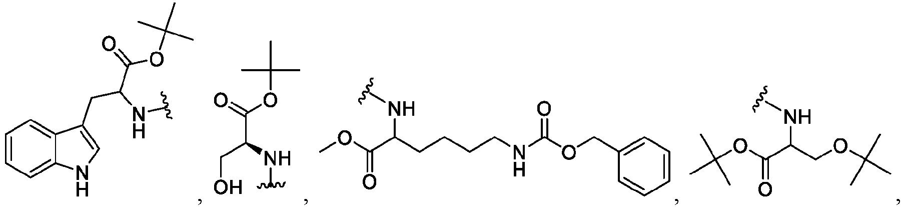 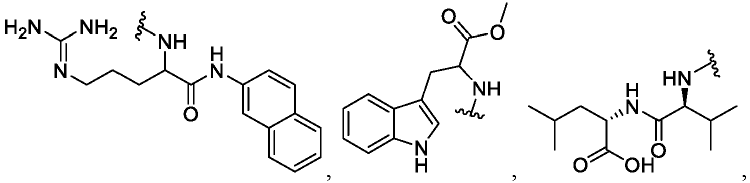 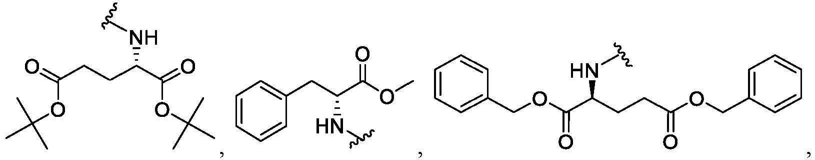 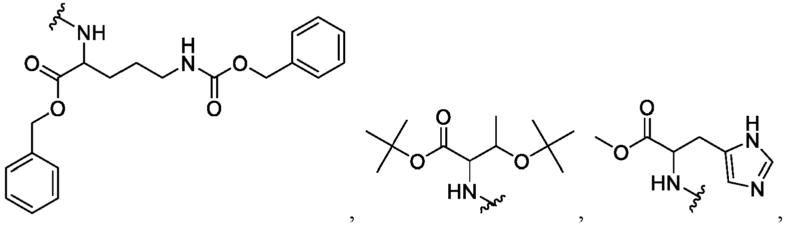 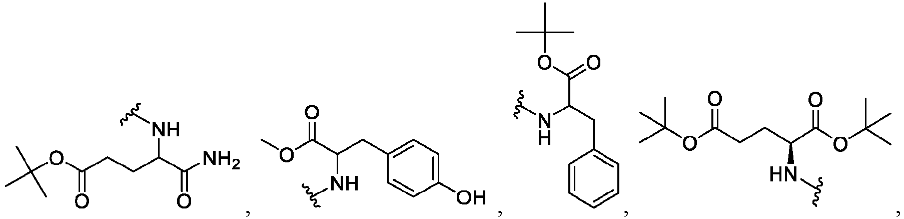 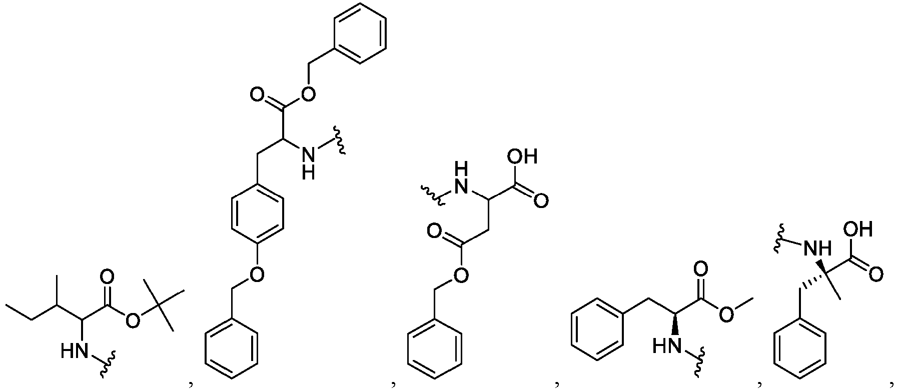 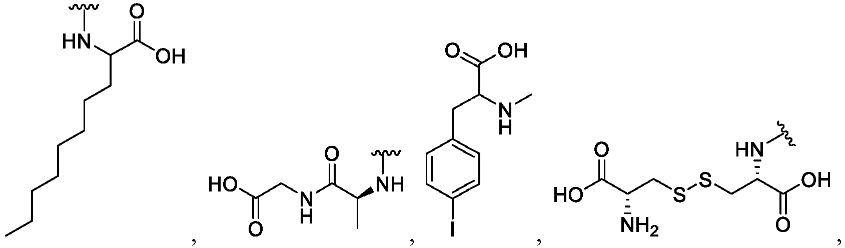 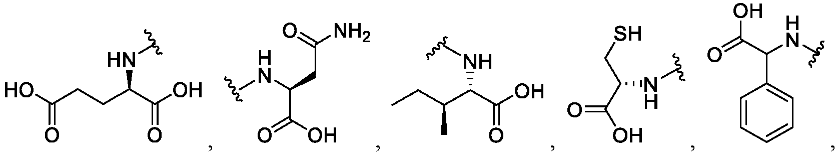 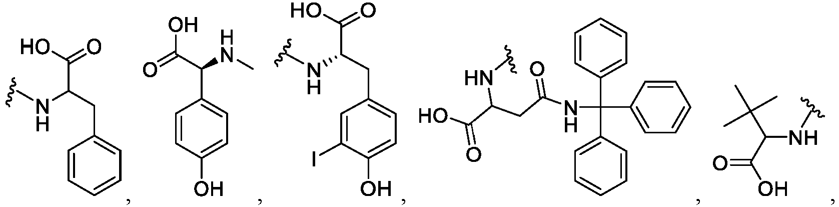 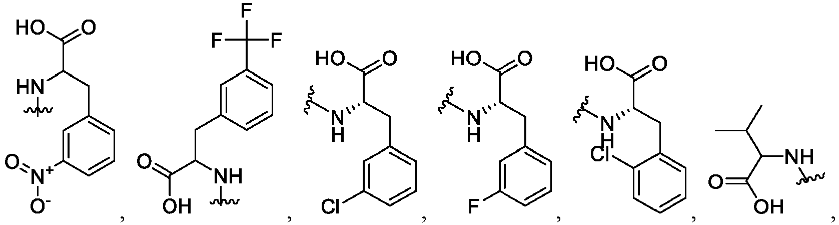 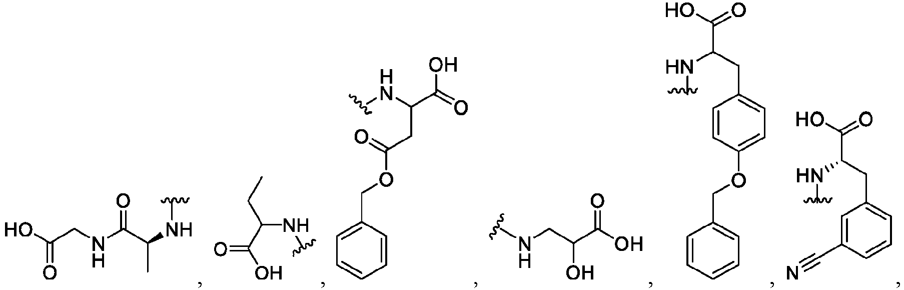 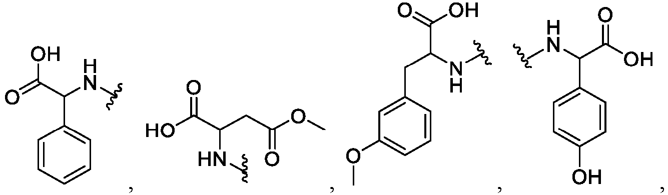 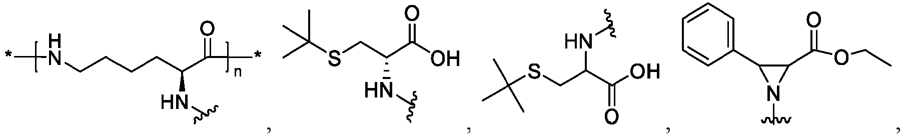 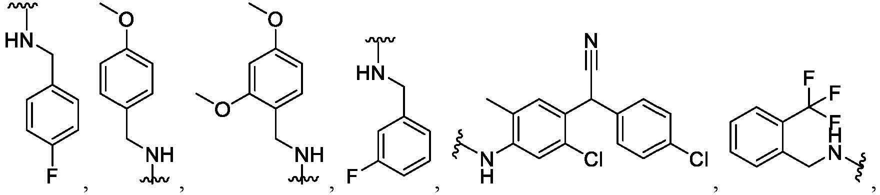 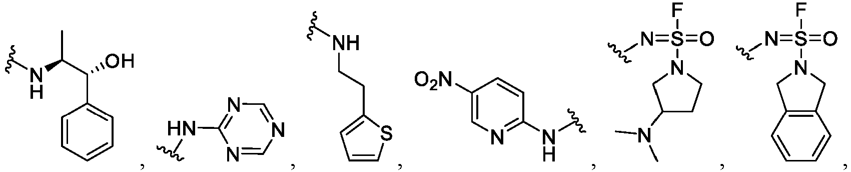 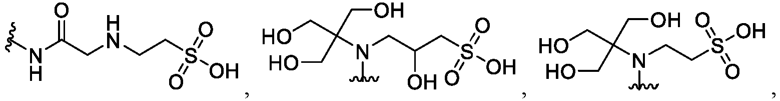 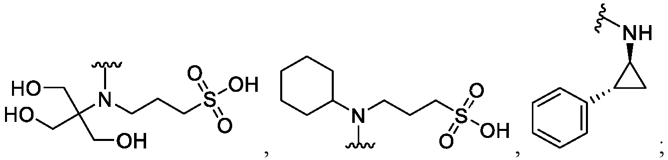 and
R⁵ is H, alkyl, aryl or -alkylene-aryl;
wherein, unless otherwise specified:
the term alkyl defines a branched or unbranched carbon chain moiety up to 30 carbon atoms;
the term alkylene defines an alkyl group having from 2 to 12 carbon atoms that contains two points of attachment to the rest of the compound on its longest carbon chain;
the term aryl defines a 5- to 12-membered aromatic ring, wherein each atom of the ring is carbon or one or more ring atoms are heteroatoms;
the term heterocyclyl defines a 3- to 12-membered ring structure, wherein one to four ring atoms are heteroatoms; and
the term cycloalkyl defines a mono-, bicyclic, bridged, spirocyclic or polycyclic saturated carbocyclic ring having from 3 to 12 carbon atoms;
or a pharmaceutical composition comprising the compound of formula (I) or formula (II) and a pharmaceutical acceptable carrier, for use in a method of treating or preventing an HA-mediated disease or condition.

14. The compound for use according to claim 13, wherein the HA-mediated disease is influenza.

15. The compound for use according to claim 14, wherein influenza is influenza A, influenza B or influenza C.

## Patentansprüche

1. Verbindung der Formel (I) oder (II): 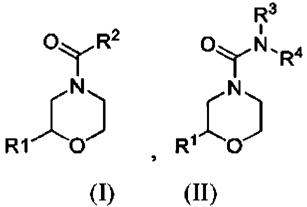 wobei
R¹ Phenyl ist;
R² Aryl, Heterocyclyl, 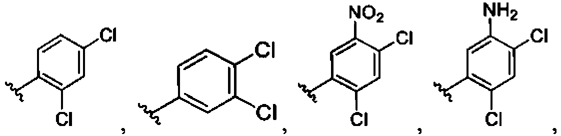 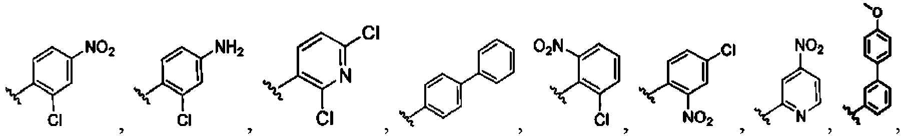 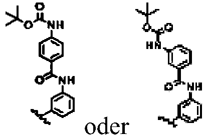 ist;
R³ ausgewählt ist aus H, Alkyl, -Alkylen-(heterocyclyl), -Alkylen-(aryl), -Alkylen-(cycloalkyl), -Alkylen-O-R⁵, -Alkylen-C(O)N(R⁵)₂, -Alkylen-C(O)R⁵, -Alkylen-C(O)₂R⁵, - Alkylen-N(R⁵)₂, -Alkylen-N(R⁵)C(O)R⁵, -Alkylen-N(R⁵)C(O)N(R⁵)₂, -Alkylen-N(R⁵)SO₂-R⁵, - Alkylen-S-R⁵, -Aryl, -Arylen-(heterocyclyl), -Arylen-alkyl, -Arylen-(cycloalkyl), -Arylen-O-R⁵, -Arylen-C(O)N(R⁵)₂, -Arylen-C(O)R⁵, -Arylen-C(O)₂R⁵, -Arylen-N(R⁵)₂, -Arylen-N(R⁵)C(O)R⁵, -Arylen-N(R⁵)C(O)N(R⁵)₂, -Arylen-N(R⁵)SO₂-R⁵, -Arylen-S-R⁵, -Heterocyclyl, -Heterocyclylen-(alkyl), -Heterocyclylen-(aryl), -Heterocyclylen-(cycloalkyl), -Heterocyclylen-(cycloalkyl), - Heterocyclylen-O-R⁵, -Heterocyclylen-C(O)N(R⁵)₂, -Heterocyclylen-C(O)R⁵, -Heterocyclylen-C(O)₂R⁵, -Heterocyclylen-N(R⁵)₂, -Heterocyclylen-N(R⁵)C(O)R⁵, -Heterocyclylen-N(R⁵)C(O)N(R⁵)₂, -Heterocyclylen-N(R⁵)SO₂-R⁵, -Heterocyclylen-S-R⁵, -Cycloalkyl, - Cycloalkylen-(alkyl), -Cycloalkylen-(aryl), -Cycloalkylen-(cycloalkyl), - Cycloalkylen(heterocycloalkyl), -Cycloalkylen-O-R⁵, -Cycloalkylen-C(O)N(R⁵)₂, -Cycloalkylen-C(O)R⁵, -Cycloalkylen-C(O)₂R⁵, -Cycloalkylen-N(R⁵)₂, -Cycloalkylen-N(R⁵)C(O)R⁵, - Cycloalkylen-N(R⁵)C(O)N(R⁵)₂, -Cycloalkylen-N(R⁵)SO₂-R⁵, -Alkylen-N(H)-alkylen-C(O)OR⁵, -Alkylen-C(O)OR⁵, -Arylen-alkylen-aryl, -C(O)R⁵, -C(O)₂R⁵, -Alkylen-S-S-R⁵, -C(O)-Alkylen-N(R⁵)₂, -C(O)₂R⁵, -Aryl-alkylen-N(R⁵)C(O)-R⁵, -Cycloalkylen-N(R⁵)S(O)(F)-R⁵, -SO₂-NR⁵, - S(O)(F)-NR⁵;
R⁴ ausgewählt ist aus Alkyl, -Alkylen-(heterocyclyl), -Alkylen-(aryl), -Alkylen-(cycloalkyl), -Alkylen-O-R⁵, -Alkylen-C(O)N(R⁵)₂, -Alkylen-C(O)R⁵, -Alkylen-C(O)₂R⁵, - Alkylen-N(R⁵)₂, -Alkylen-N(R⁵)C(O)R⁵, -Alkylen-N(R⁵)C(O)N(R⁵)₂, -Alkylen-N(R⁵)SO₂-R⁵, - Alkylen-S-R⁵, -Aryl, -Arylen-(heterocyclyl), -Arylen-alkyl, -Arylen-(cycloalkyl), -Arylen-O-R⁵, -Arylen-C(O)N(R⁵)₂, -Arylen-C(O)R⁵, -Arylen-C(O)₂R⁵, -Arylen-N(R⁵)₂, -Arylen-N(R⁵)C(O)R⁵, -Arylen-N(R⁵)C(O)N(R⁵)₂, -Arylen-N(R⁵)SO₂-R⁵, -Arylen-S-R⁵, -Heterocyclyl, -Heterocyclylen-(alkyl), -Heterocyclylen-(aryl), -Heterocyclylen-(cycloalkyl), -Heterocyclylen-(cycloalkyl), - Heterocyclylen-O-R⁵, -Heterocyclylen-C(O)N(R⁵)₂, -Heterocyclylen-C(O)R⁵, -Heterocyclylen-C(O)₂R⁵, -Heterocyclylen-N(R⁵)₂, Heterocyclylen-N(R⁵)C(O)R⁵, -Heterocyclylen-N(R⁵)C(O)N(R⁵)₂-, -Heterocyclylen-N(R⁵)SO₂-R⁵, -Heterocyclylen-S-R⁵, -Cycloalkyl, - Cycloalkylen-(alkyl), -Cycloalkylen-(aryl), -Cycloalkylen-(cycloalkyl), - Cycloalkylen(heterocycloalkyl), -Cycloalkylen-O-R⁵, -Cycloalkylen-C(O)N(R⁵)₂, -Cycloalkylen-C(O)R⁵, -Cycloalkylen-C(O)₂R⁵, -Cycloalkylen-N(R⁵)₂, -Cycloalkylen-N(R⁵)C(O)R⁵, - Cycloalkylen-N(R⁵)C(O)N(R⁵)₂, -Cycloalkylen-N(R⁵)SO₂-R⁵, -Alkylen-N(H)-alkylen-C(O)OR⁵, -Alkylen-C(O)OR⁵, -Arylen-alkylen-aryl, -C(O)R⁵, -C(O)₂R⁵, -Alkylen-S-S-R⁵, -C(O)-Alkylen-N(R⁵)₂, -C(O)₂R⁵, -Aryl-alkylen-N(R⁵)C(O)-R⁵, -Cycloalkylen-N(R⁵)S(O)(F)-R⁵, -SO₂-NR⁵, - S(O)(F)-NR⁵; oder
R³ und R⁴ zusammengenommen werden können, um ein Heterocyclyl zu bilden; oder 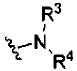 wie folgt ist: 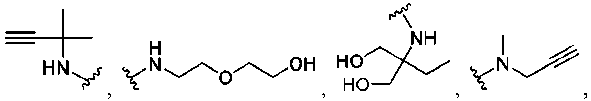 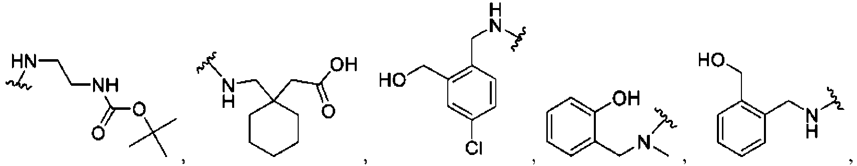 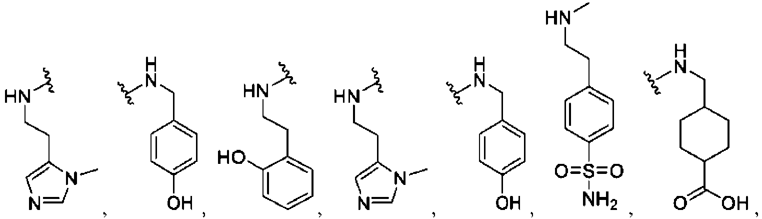 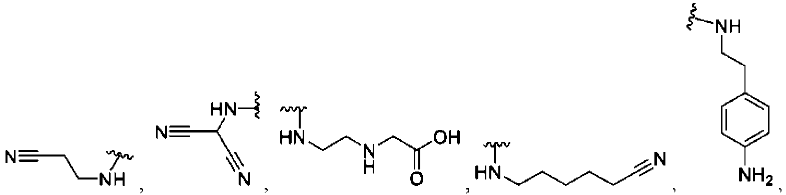 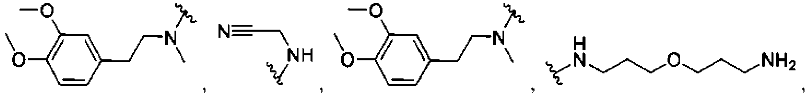 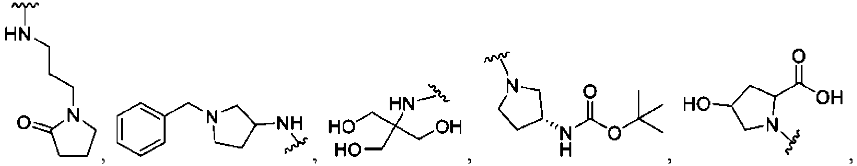 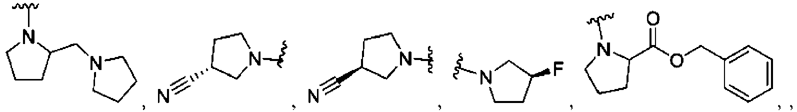 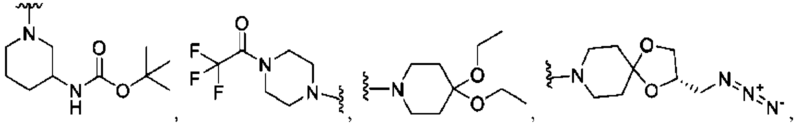 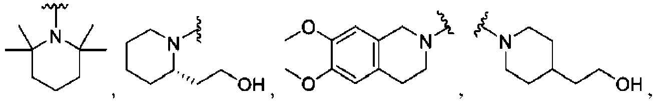 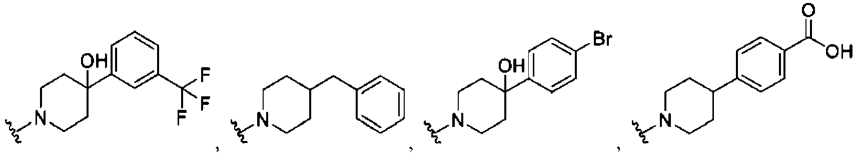 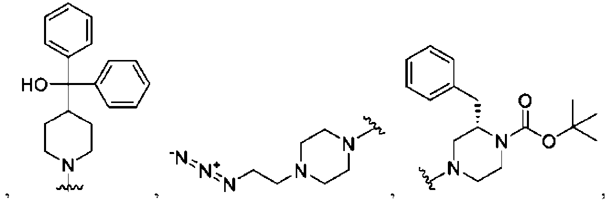 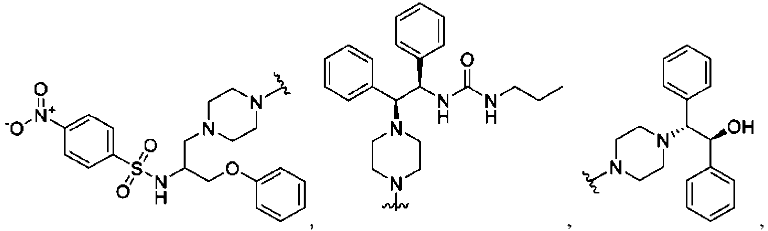 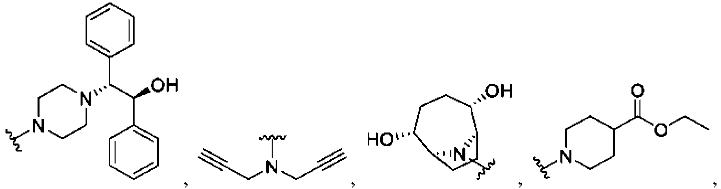 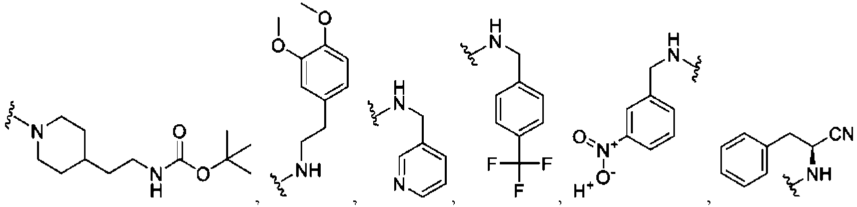 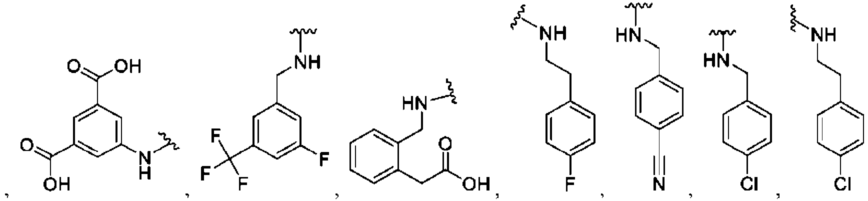 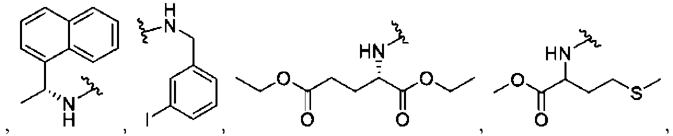 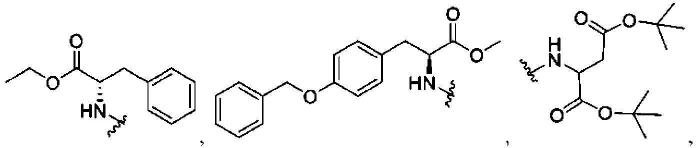 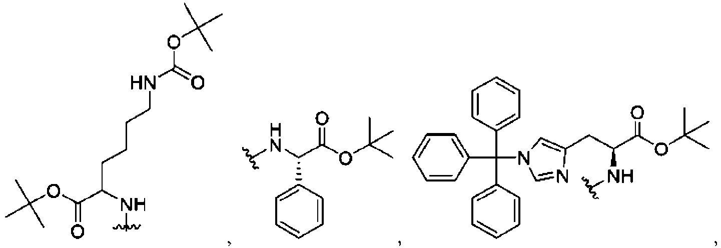 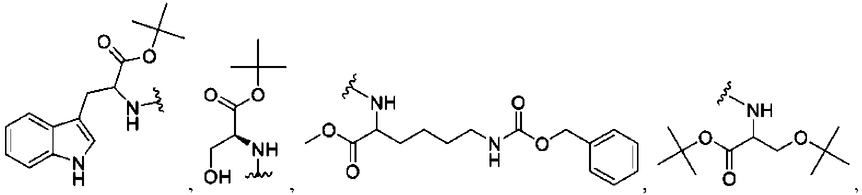 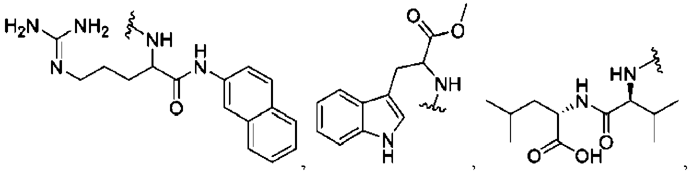 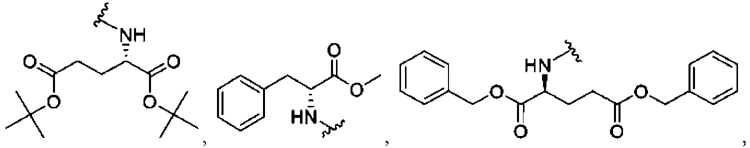 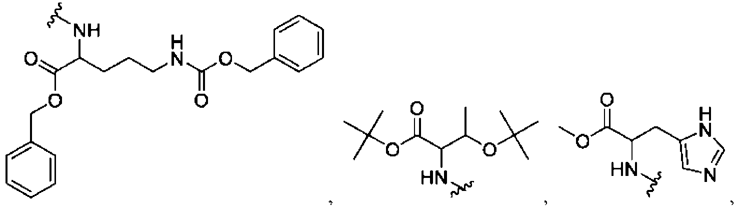 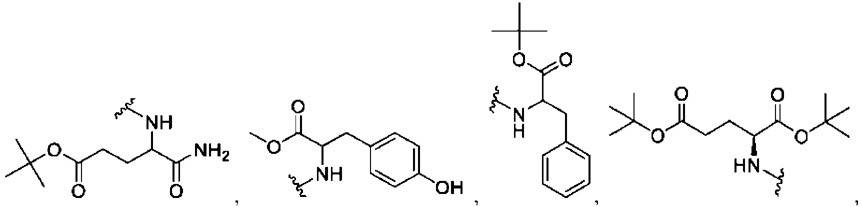 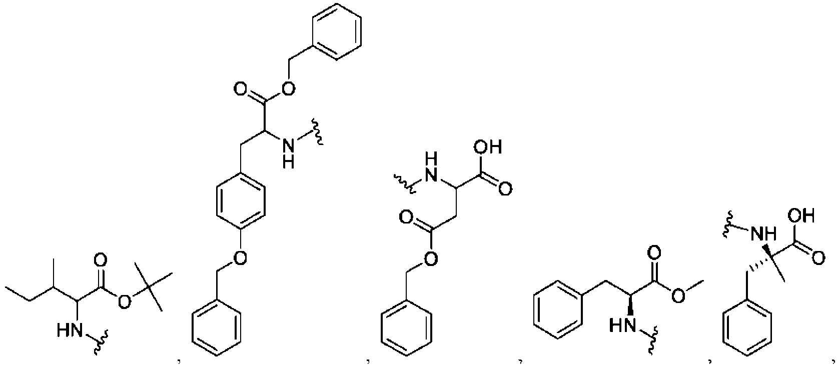 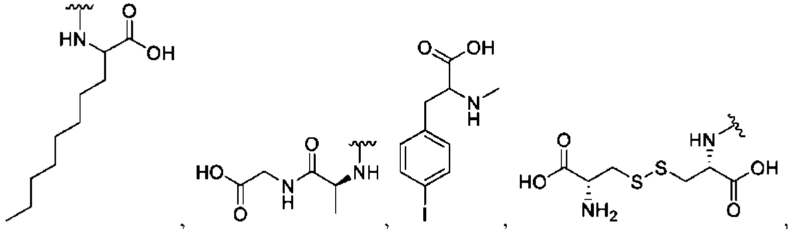 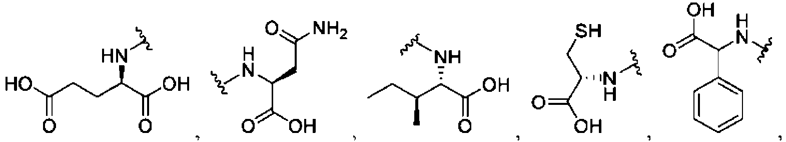 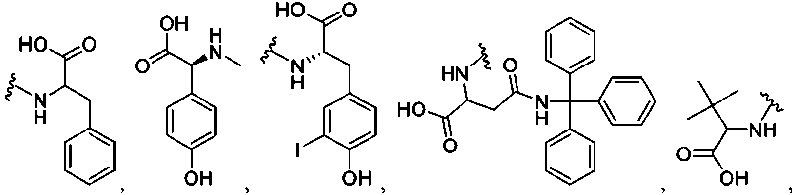 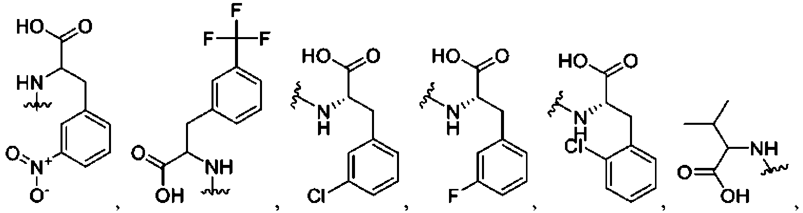 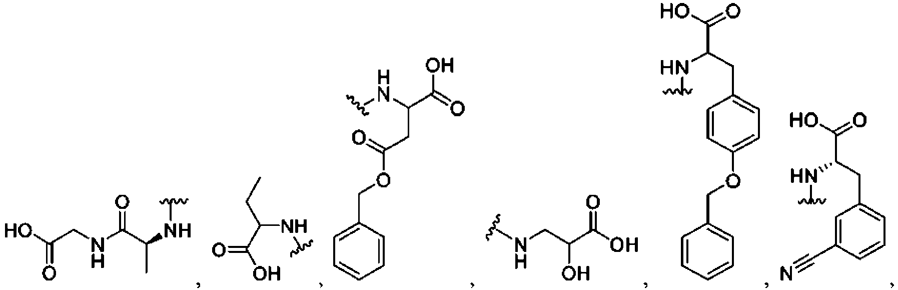 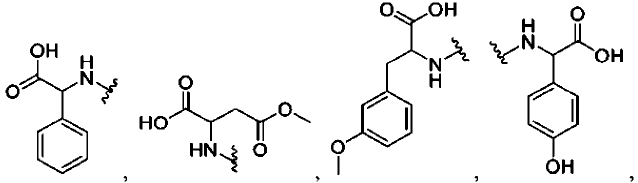 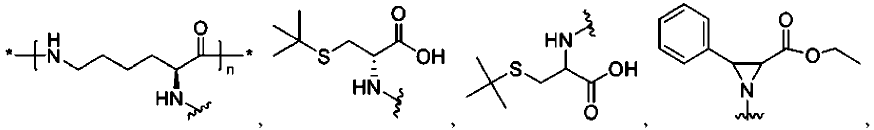 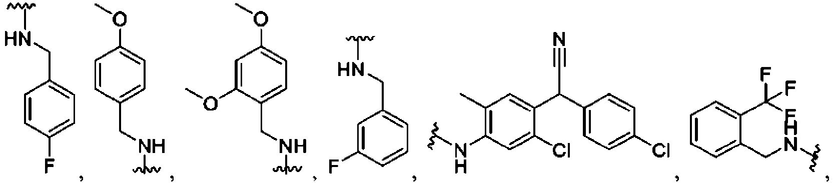 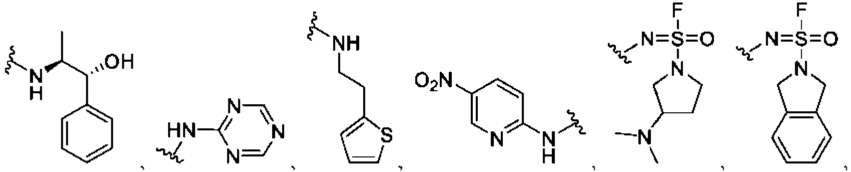 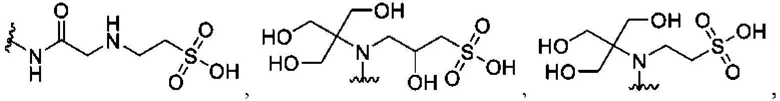 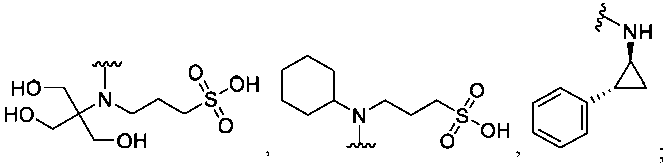 und
R⁵ H, Alkyl, Aryl oder -Alkylen-aryl ist;
wobei, sofern nicht anders spezifiziert:
der Begriff Alkyl eine verzweigte oder unverzweigte Kohlenstoffketteneinheit bis zu 30 Kohlenstoffatomen definiert;
der Begriff Alkylen eine Alkylgruppe mit 2 bis 12 Kohlenstoffatomen definiert, die zwei Verknüpfungspunkte zu dem Rest der Verbindung auf ihrer längsten Kohlenstoffkette enthält;
der Begriff Aryl einen 5- bis 12-gliedrigen aromatischen Ring definiert, wobei jedes Atom des Rings Kohlenstoff ist oder ein oder mehrere Ringatome Heteroatome sind;
der Begriff Heterocyclyl eine 3- bis 12-gliedrige Ringstruktur definiert, wobei ein bis vier Ringatome Heteroatome sind; und
der Begriff Cycloalkyl einen mono-, bicyclischen, verbrückten, spirocyclischen oder polycyclischen gesättigten carbocyclischen Ring mit 3 bis 12 Kohlenstoffatomen definiert;
wobei die Verbindung von Formel (II) nicht wie folgt ist:

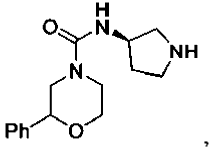

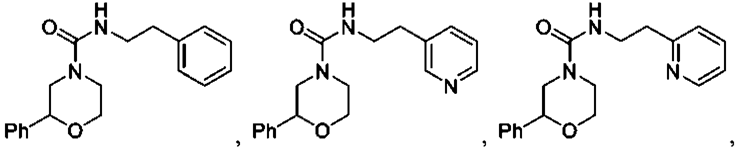

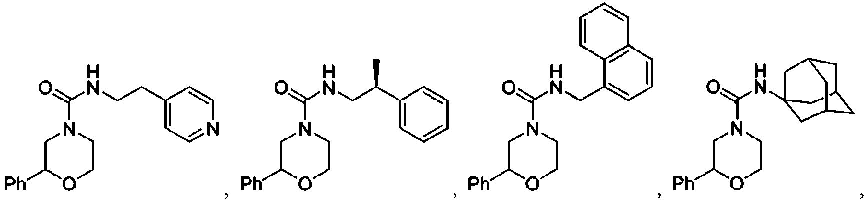

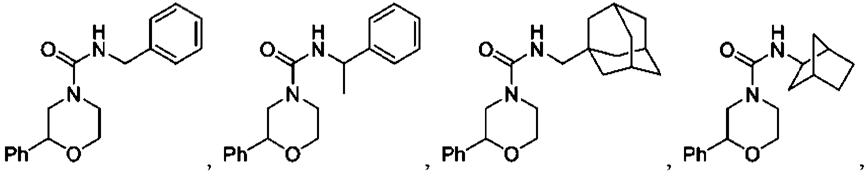

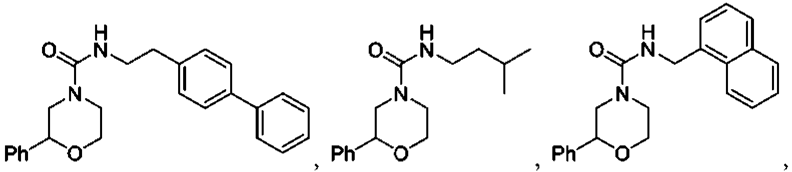

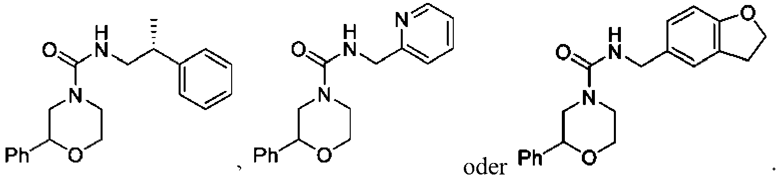

2. Verbindung nach Anspruch 1, wobei die Verbindung der Formel (II) ist:

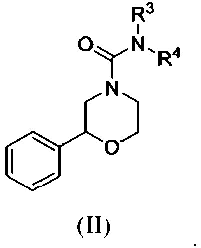

3. Verbindung nach Anspruch 1, wobei R² ausgewählt ist aus der Gruppe bestehend aus:

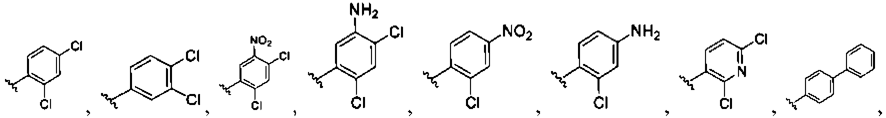

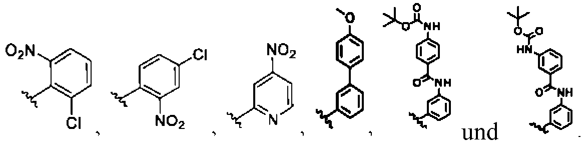

4. Verbindung nach Anspruch 1 oder 2, wobei 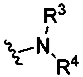 wie folgt ist:
- ausgewählt aus der Gruppe bestehend aus: 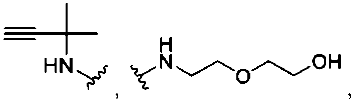 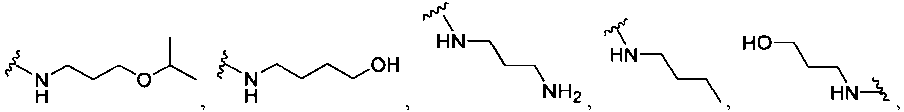 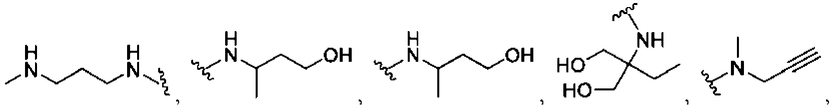 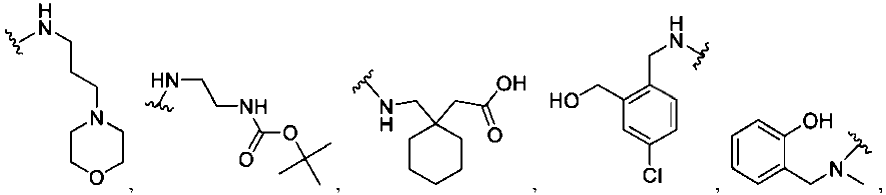 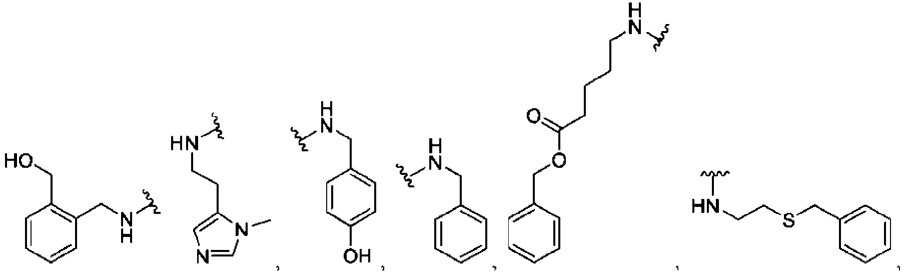 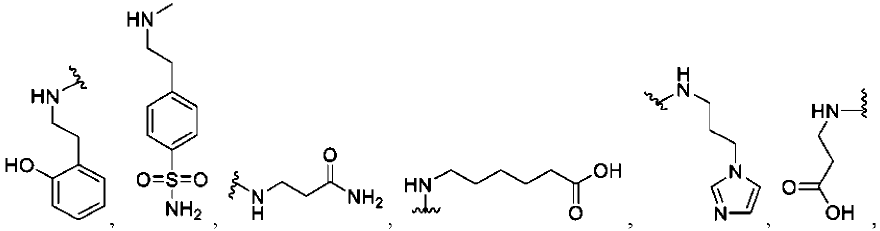 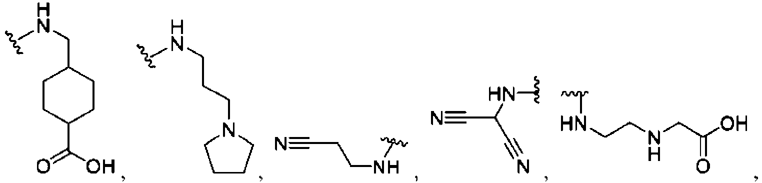 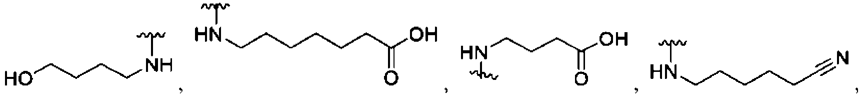 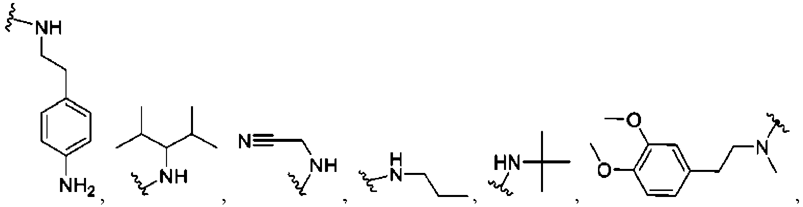 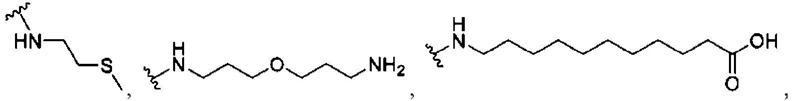 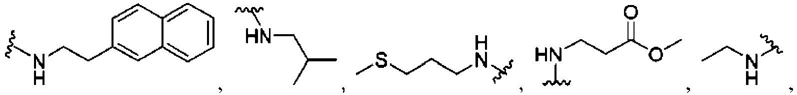 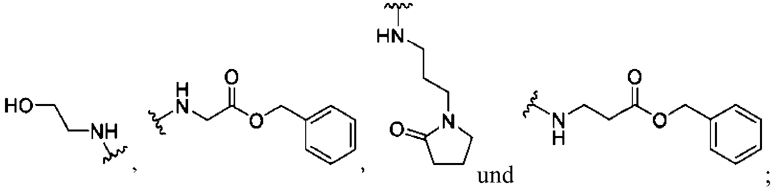 oder
- ausgewählt aus der Gruppe bestehend aus: 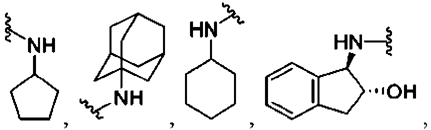 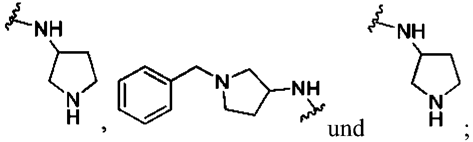 oder 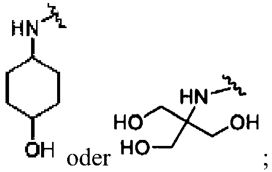 oder
- ausgewählt aus der Gruppe bestehend aus:

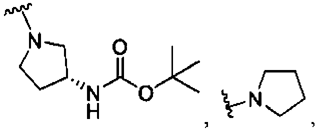

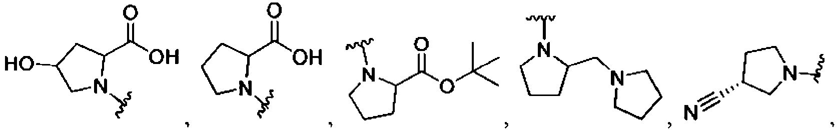

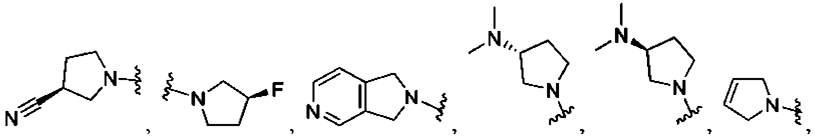

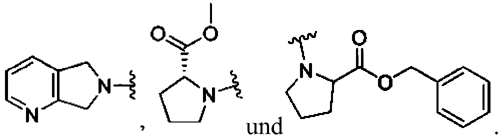

5. Verbindung nach Anspruch 1 oder 2, wobei 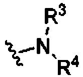 wie folgt ist:
- ausgewählt aus der Gruppe bestehend aus: 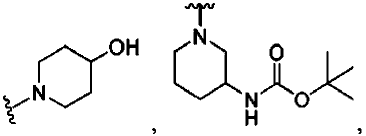 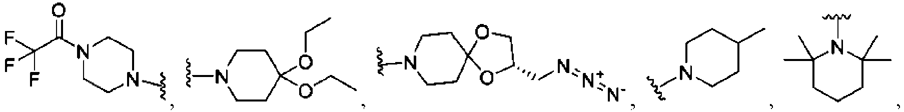 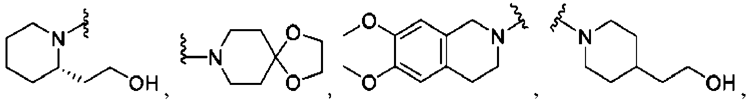 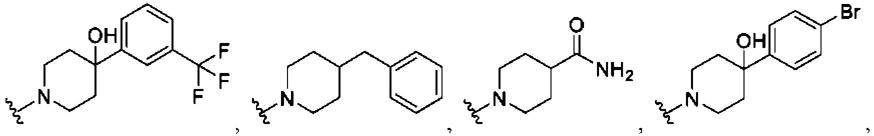 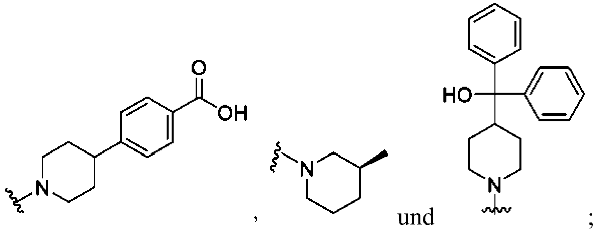 oder
- ausgewählt aus der Gruppe bestehend aus: 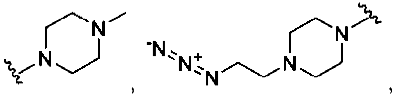 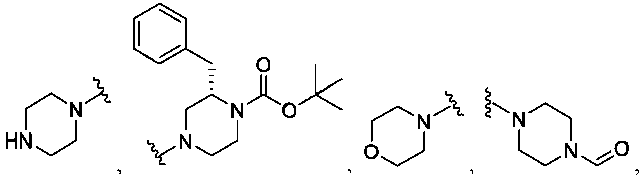 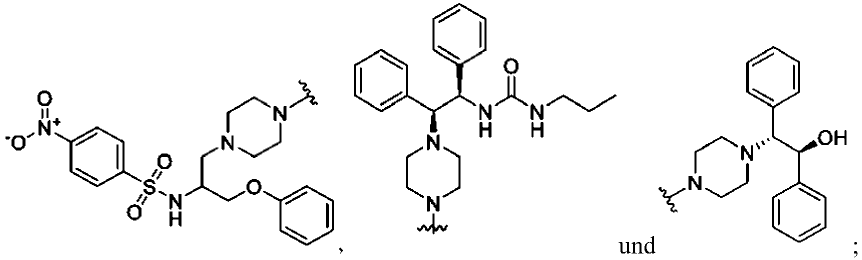 oder
- 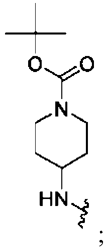 oder
- wie folgt ist: 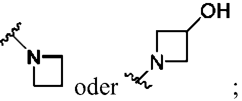 oder
- ausgewählt aus der Gruppe bestehend aus: 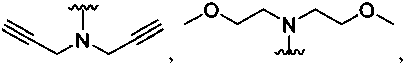 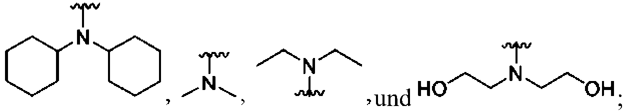 oder
- ausgewählt ist aus der Gruppe bestehend aus

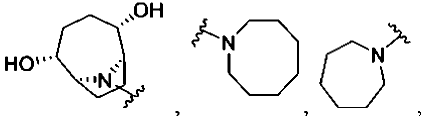

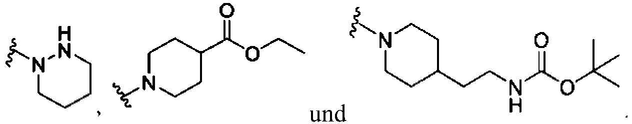

6. Verbindung nach Anspruch 1 oder 2, wobei 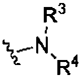 wie folgt ist:
- ausgewählt aus der Gruppe bestehend aus: 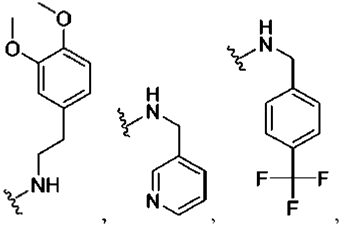 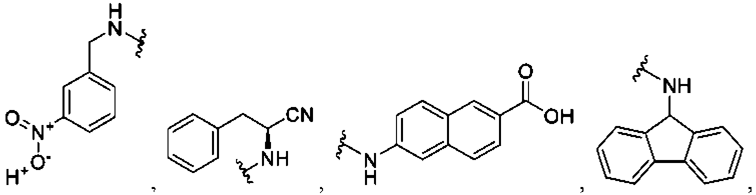 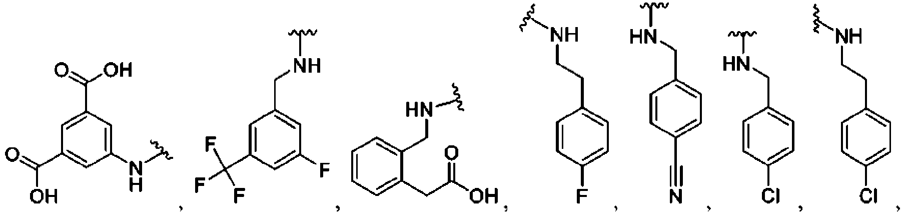 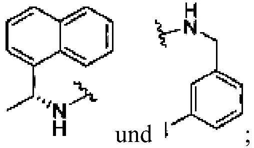 oder
- ausgewählt aus der Gruppe bestehend aus: 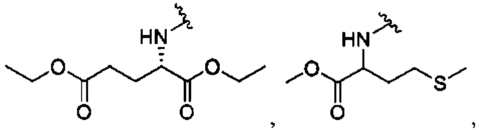 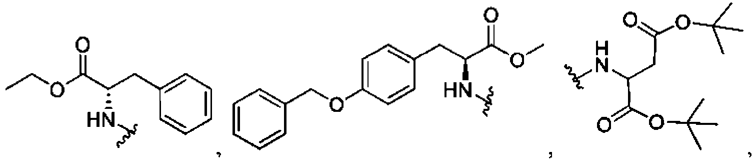 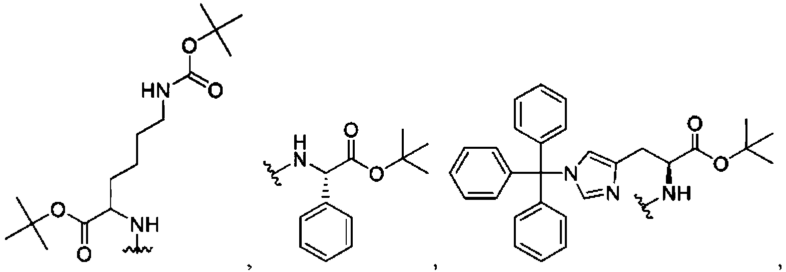 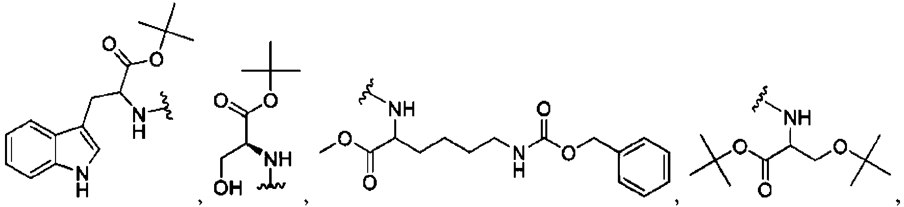 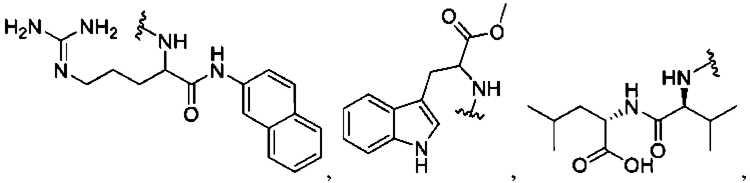 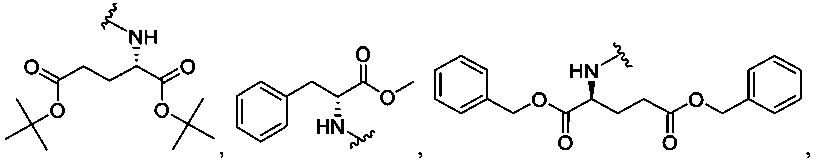 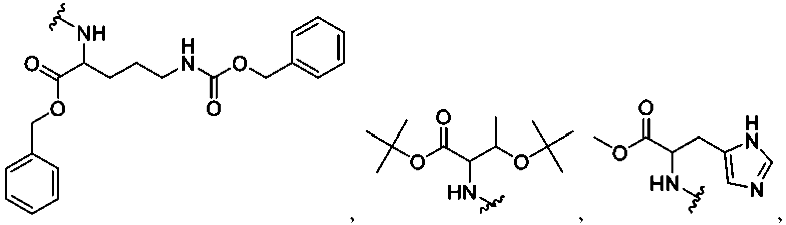 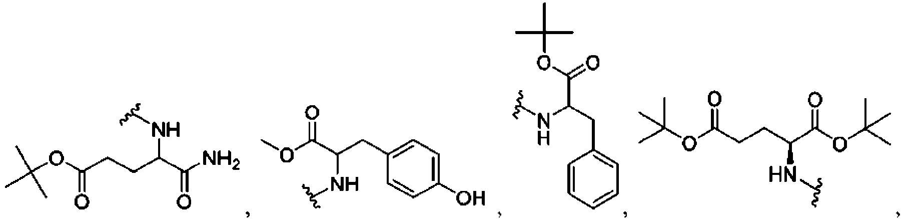 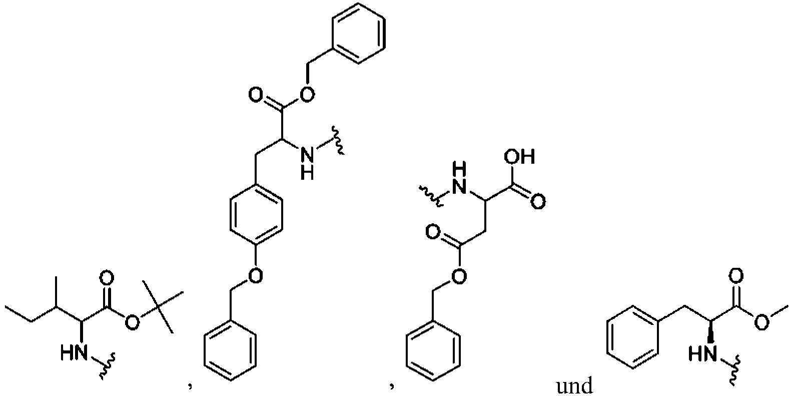 oder
- ausgewählt aus der Gruppe bestehend aus: 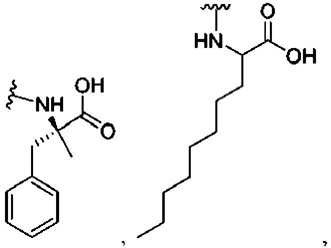 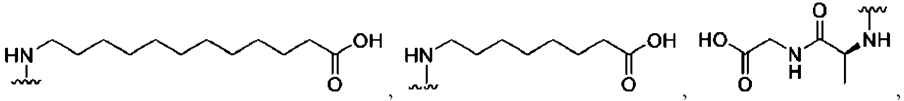 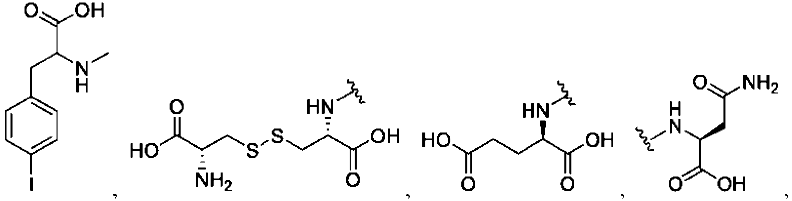 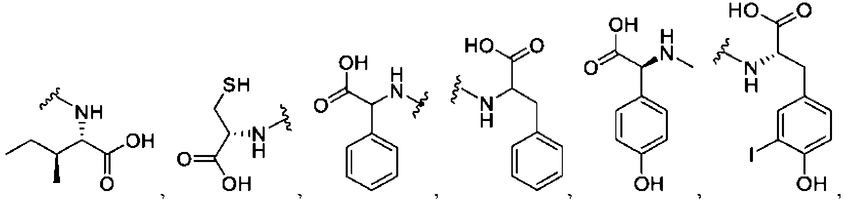 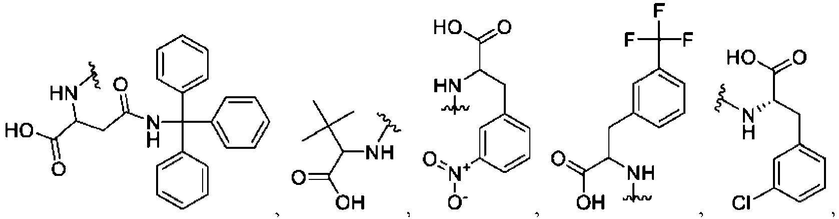 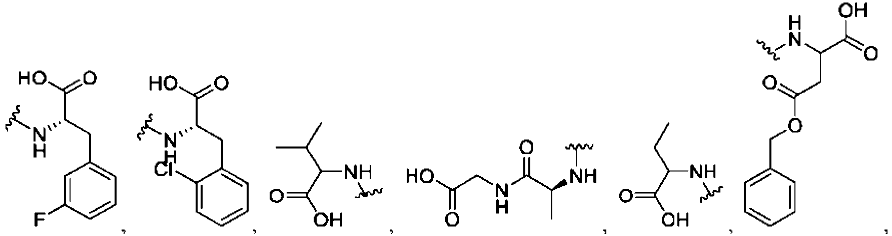 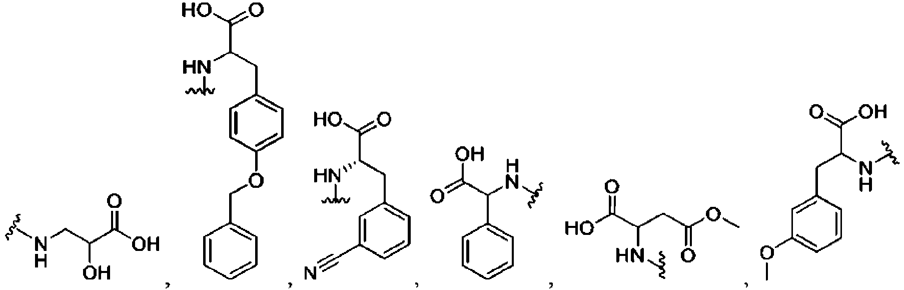 und

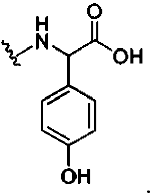

7. Verbindung nach Anspruch 1 oder 2, wobei 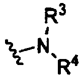 wie folgt ist:
- 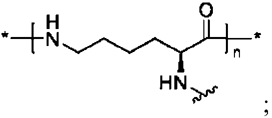 oder
- 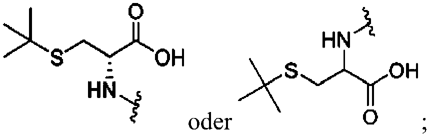 oder
- ausgewählt aus der Gruppe bestehend aus 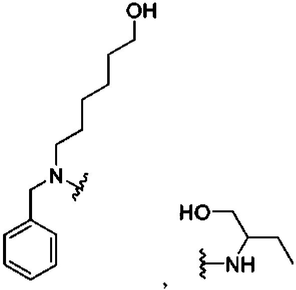 und 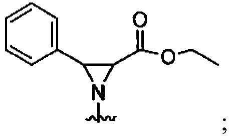 oder
- ausgewählt ist aus der Gruppe bestehend aus: 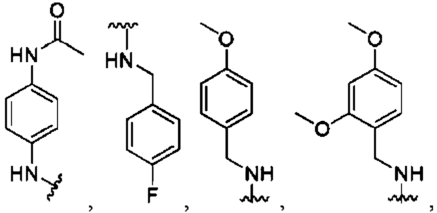 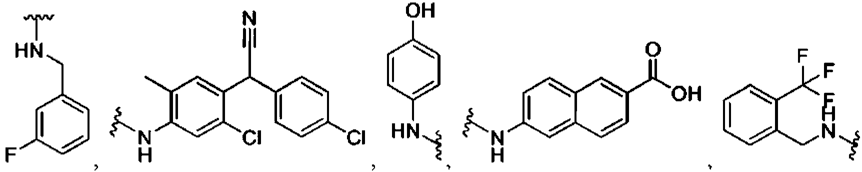 und 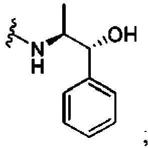 oder
- ausgewählt ist aus der Gruppe bestehend aus: 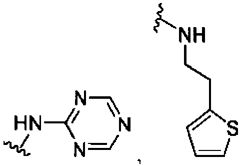 und 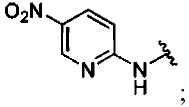 oder
- 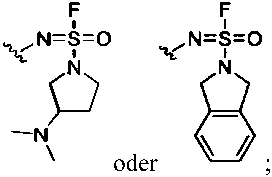 oder
- ausgewählt aus der Gruppe bestehend aus: 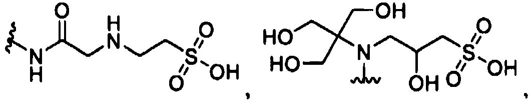 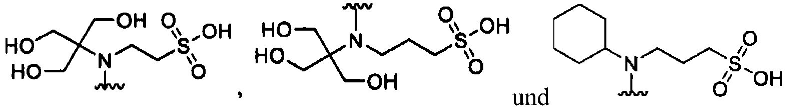 oder
- wie folgt ist:

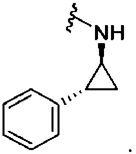

8. Verbindung nach Anspruch 1 oder 2, wobei die Verbindung der Formel (I) oder (II) wie folgt ist:
- ausgewählt aus der Gruppe bestehend aus: 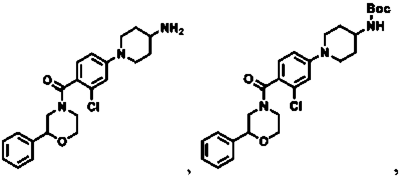 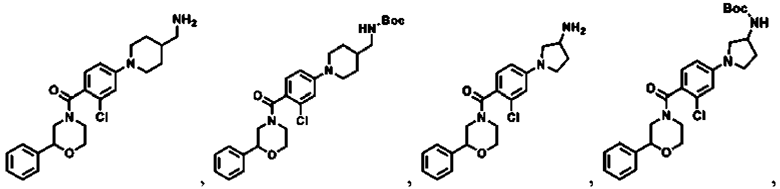 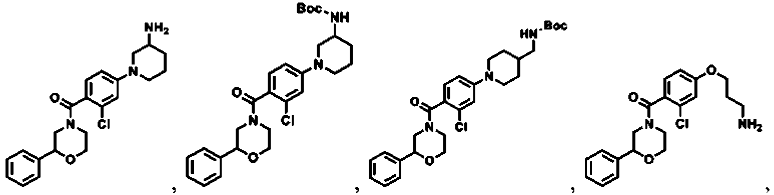 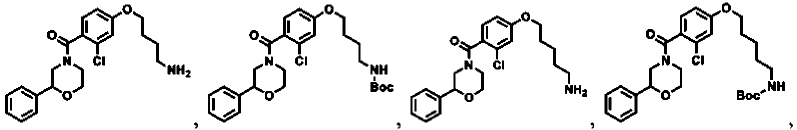 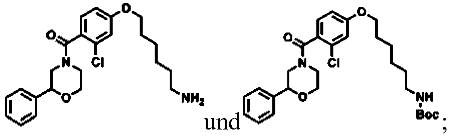 oder
- ausgewählt aus der Gruppe bestehend aus: 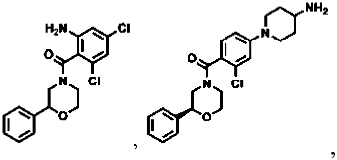 und 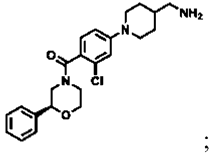 oder 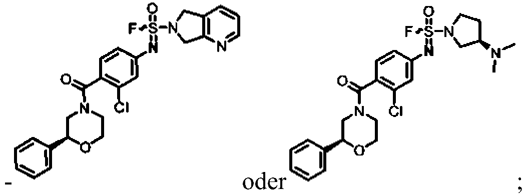 oder
- ausgewählt aus der Gruppe bestehend aus:

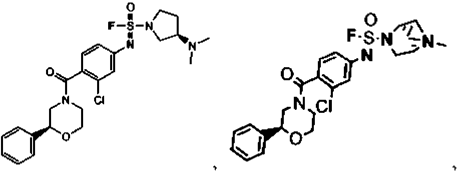

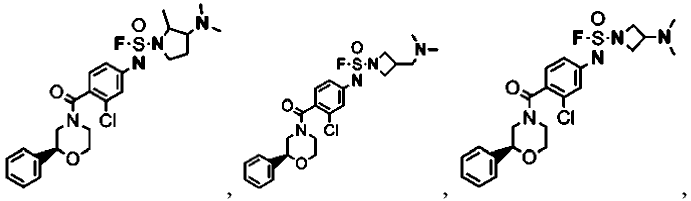

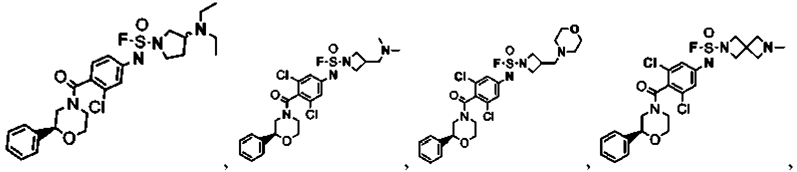

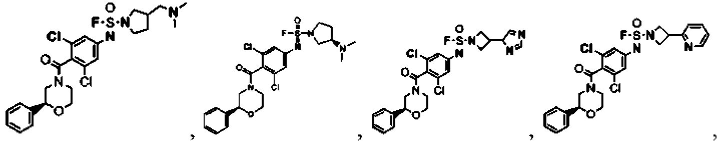

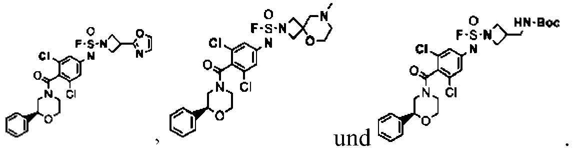

9. Verbindung nach Anspruch 1 oder 2, wobei die Verbindung der Formel (I) oder (II) wie folgt ist:
- ausgewählt aus der Gruppe bestehend aus: 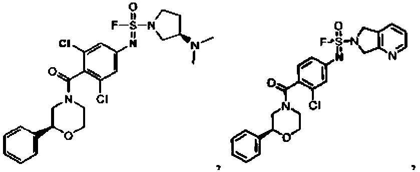 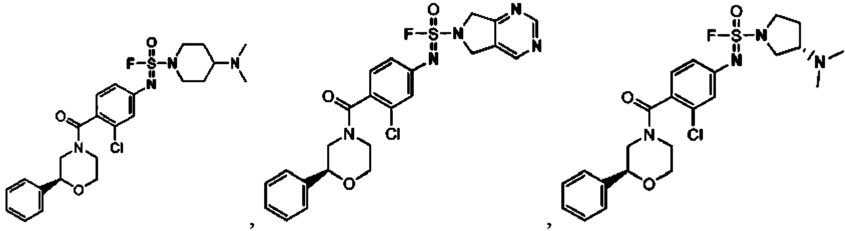 und 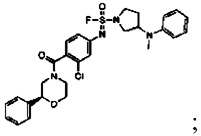 oder
- ausgewählt aus der Gruppe bestehend aus: 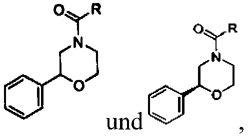 wobei R 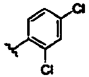 ist; oder
- ausgewählt aus der Gruppe bestehend aus: 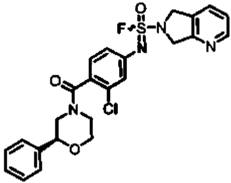 und 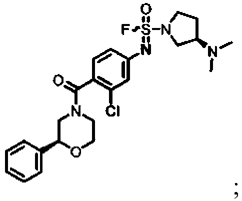 oder 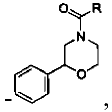 wobei R ausgewählt ist aus der Gruppe bestehend aus:

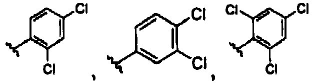

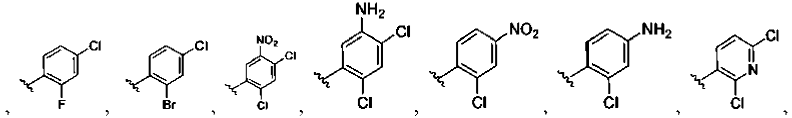

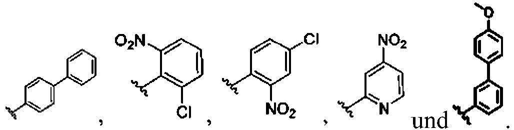

10. Verbindung nach Anspruch 1 oder 2, wobei die Verbindung der Formel (I) oder (II) wie folgt ist:
- ausgewählt aus der Gruppe bestehend aus: 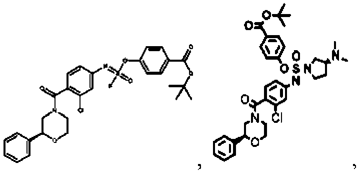 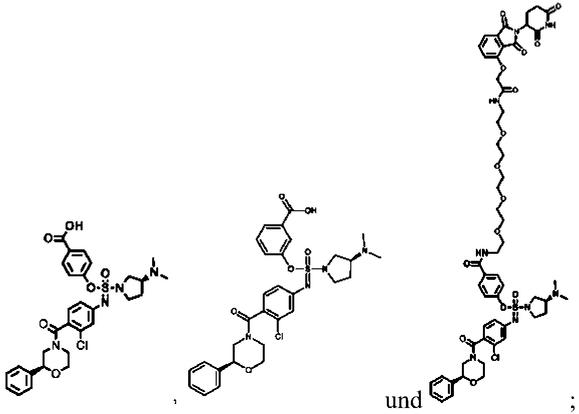 oder 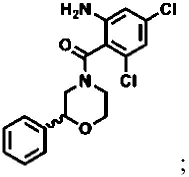 oder
- ausgewählt ist aus der Gruppe bestehend aus: 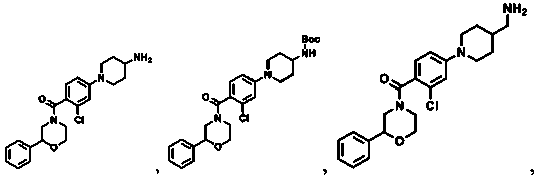 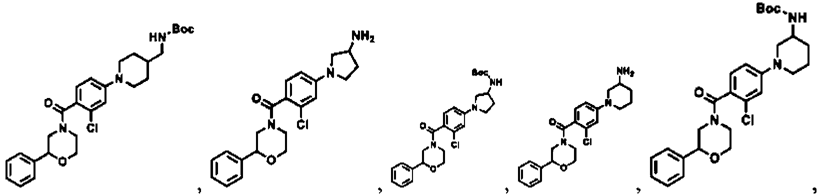 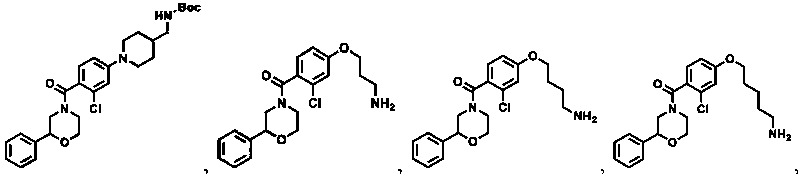 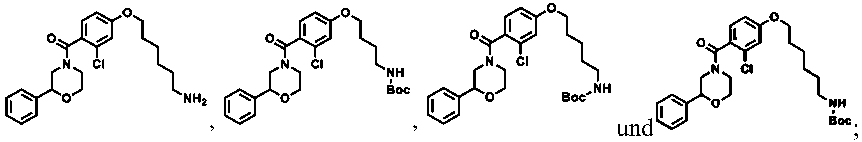 oder
- 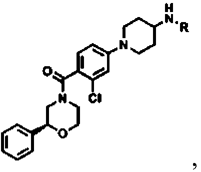 wobei R ausgewählt ist aus der Gruppe bestehend aus 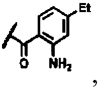 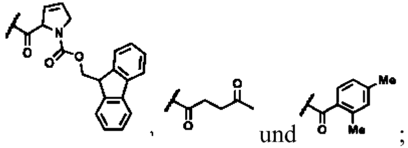 oder
- 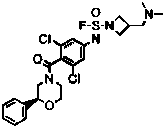 oder
- 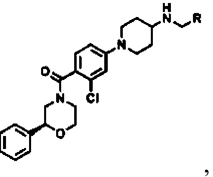 wobei R ausgewählt ist aus der Gruppe bestehend aus 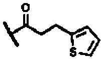 und 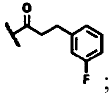 oder
- 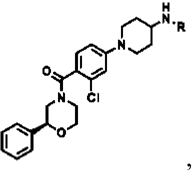 wobei R ausgewählt ist aus der Gruppe bestehend aus 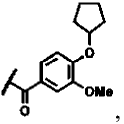 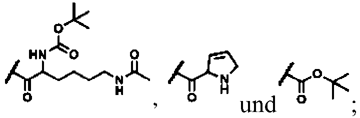 oder
- ausgewählt aus der Gruppe bestehend aus: 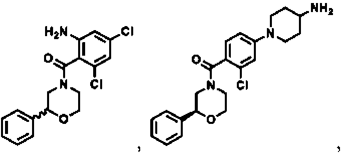 und

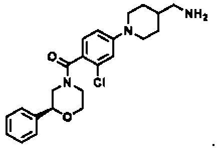

11. Pharmazeutische Zusammensetzung, umfassend eine Verbindung nach einem der Ansprüche 1-10 und einen pharmazeutisch verträglichen Träger.

12. Verbindung nach einem der Ansprüche 1-10 oder pharmazeutische Zusammensetzung nach Anspruch 11 zur Verwendung als Medikament.

13. Verbindung der Formel (I) oder (II): 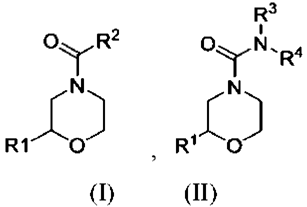 wobei
R¹ Phenyl ist;
R² Aryl, Heterocyclyl, 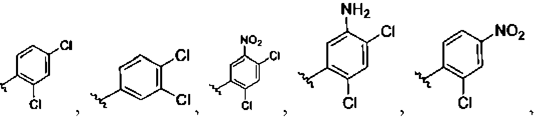 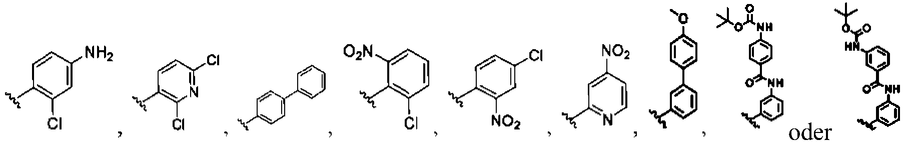 ist;
R³ ausgewählt ist aus **H,** Alkyl, -Alkylen-(heterocyclyl), -Alkylen-(aryl), -Alkylen-(cycloalkyl), -Alkylen-O-R⁵, -Alkylen-C(O)N(R⁵)₂, -Alkylen-C(O)R⁵, -Alkylen-C(O)₂R⁵, - Alkylen-N(R⁵)₂, -Alkylen-N(R⁵)C(O)R⁵, -Alkylen-N(R⁵)C(O)N(R⁵)₂, -Alkylen-N(R⁵)SO₂-R⁵, - Alkylen-S-R⁵, -Aryl, -Arylen-(heterocyclyl), -Arylen-alkyl, -Arylen-(cycloalkyl), -Arylen-O-R⁵, -Arylen-C(O)N(R⁵)₂, -Arylen-C(O)R⁵, -Arylen-C(O)₂R⁵, -Arylen-N(R⁵)₂, -Arylen-N(R⁵)C(O)R⁵, -Arylen-N(R⁵)C(O)N(R⁵)₂, -Arylen-N(R⁵)SO₂-R⁵, -Arylen-S-R⁵, -Heterocyclyl, -Heterocyclylen-(alkyl), -Heterocyclylen-(aryl), -Heterocyclylen-(cycloalkyl), -Heterocyclylen-(cycloalkyl), - Heterocyclylen-O-R⁵, -Heterocyclylen-C(O)N(R⁵)₂, -Heterocyclylen-C(O)R⁵, -Heterocyclylen-C(O)₂R⁵, -Heterocyclylen-N(R⁵)₂, -Heterocyclylen-N(R⁵)C(O)R⁵, -Heterocyclylen-N(R⁵)C(O)N(R⁵)₂, -Heterocyclylen-N(R⁵)SO₂-R⁵, -Heterocyclylen-S-R⁵, -Cycloalkyl, - Cycloalkylen-(alkyl), -Cycloalkylen-(aryl), -Cycloalkylen-(cycloalkyl), - Cycloalkylen(heterocycloalkyl), -Cycloalkylen-O-R⁵, -Cycloalkylen-C(O)N(R⁵)₂, -Cycloalkylen-C(O)R⁵, -Cycloalkylen-C(O)₂R⁵, -Cycloalkylen-N(R⁵)₂, -Cycloalkylen-N(R⁵)C(O)R⁵, - Cycloalkylen-N(R⁵)C(O)N(R⁵)₂, -Cycloalkylen-N(R⁵)SO₂-R⁵, -Alkylen-N(H)-alkylen-C(O)OR⁵, -Alkylen-C(O)OR⁵, -Arylen-alkylen-aryl, -C(O)R⁵, -C(O)₂R⁵, -Alkylen-S-S-R⁵, -C(O)-Alkyl, - C(O)-Alkylen-N(R⁵)₂, -C(O)₂R⁵, -Aryl-alkylen-N(R⁵)C(O)-R⁵, -Cycloalkylen-N(R⁵)S(O)(F)-R⁵, - SO₂-NR⁵, -S(O)(F)-NR⁵;
R⁴ ausgewählt ist aus Alkyl, -Alkylen-(heterocyclyl), -Alkylen-(aryl), -Alkylen-(cycloalkyl), -Alkylen-O-R⁵, -Alkylen-C(O)N(R⁵)₂, -Alkylen-C(O)R⁵, -Alkylen-C(O)₂R⁵, - Alkylen-N(R⁵)₂, -Alkylen-N(R⁵)C(O)R⁵, -Alkylen-N(R⁵)C(O)N(R⁵)₂, -Alkylen-N(R⁵)SO₂-R⁵, - Alkylen-S-R⁵, -Aryl, -Arylen-(heterocyclyl), -Arylen-alkyl, -Arylen-(cycloalkyl), -Arylen-O-R⁵, -Arylen-C(O)N(R⁵)₂, -Arylen-C(O)R⁵, -Arylen-C(O)₂R⁵, -Arylen-N(R⁵)₂, -Arylen-N(R⁵)C(O)R⁵, -Arylen-N(R⁵)C(O)N(R⁵)₂, -Arylen-N(R⁵)SO₂-R⁵, -Arylen-S-R⁵, -Heterocyclyl, -Heterocyclylen-(alkyl), -Heterocyclylen-(aryl), -Heterocyclylen-(cycloalkyl), -Heterocyclylen-(cycloalkyl), - Heterocyclylen-O-R⁵, -Heterocyclylen-C(O)N(R⁵)₂, -Heterocyclylen-C(O)R⁵, -Heterocyclylen-C(O)₂R⁵, -Heterocyclylen-N(R⁵)₂, Heterocyclylen-N(R⁵)C(O)R⁵, -Heterocyclylen-N(R⁵)C(O)N(R⁵)₂-, -Heterocyclylen-N(R⁵)SO₂-R⁵, -Heterocyclylen-S-R⁵, -Cycloalkyl, - Cycloalkylen-(alkyl), -Cycloalkylen-(aryl), -Cycloalkylen-(cycloalkyl), - Cycloalkylen(heterocycloalkyl), -Cycloalkylen-O-R⁵, -Cycloalkylen-C(O)N(R⁵)₂, -Cycloalkylen-C(O)R⁵, -Cycloalkylen-C(O)₂R⁵, -Cycloalkylen-N(R⁵)₂, -Cycloalkylen-N(R⁵)C(O)R⁵, - Cycloalkylen-N(R⁵)C(O)N(R⁵)₂, -Cycloalkylen-N(R⁵)SO₂-R⁵, -Alkylen-N(H)-alkylen-C(O)OR⁵, -Alkylen-C(O)OR⁵, -Arylen-alkylen-aryl, -C(O)R⁵, -C(O)₂R⁵, -Alkylen-S-S-R⁵, -C(O)-Alkyl, - C(O)-Alkylen-N(R⁵)₂, -C(O)₂R⁵, -Aryl-alkylen-N(R⁵)C(O)-R⁵, -Cycloalkylen-N(R⁵)S(O)(F)-R⁵, - SO₂-NR⁵, -S(O)(F)-NR⁵; oder
R³ und R⁴ zusammengenommen werden können, um ein Heterocyclyl zu bilden; oder 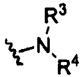 wie folgt ist: 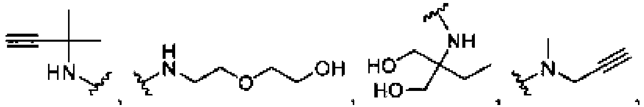 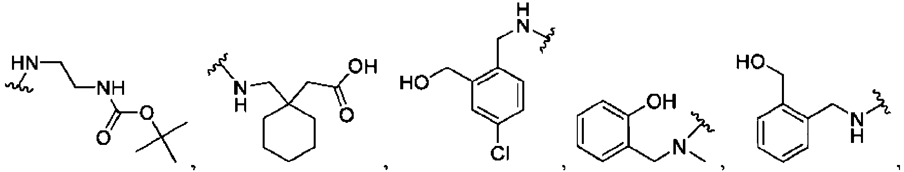 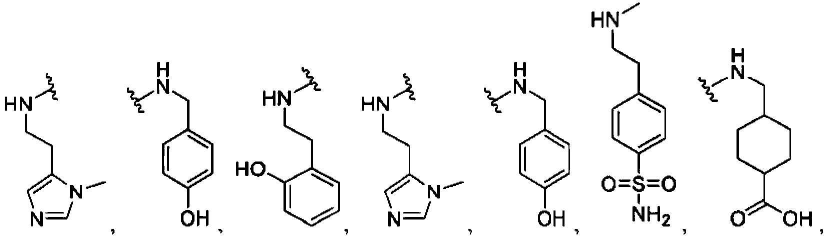 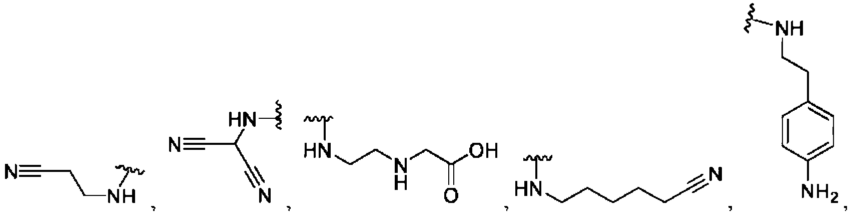 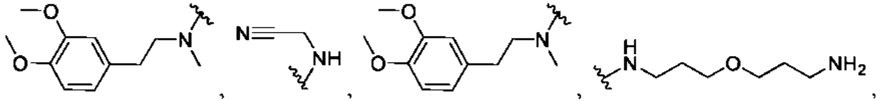 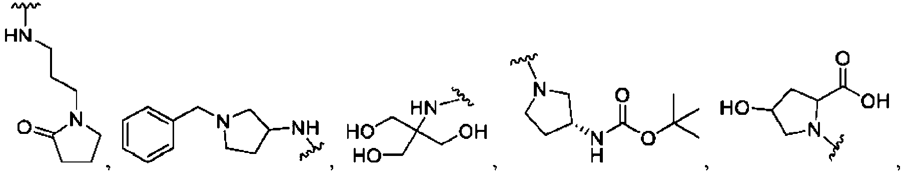 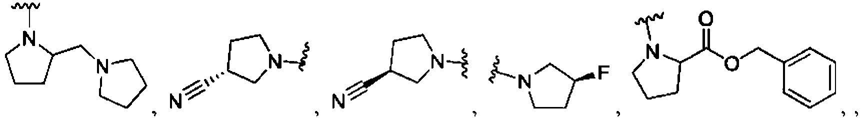 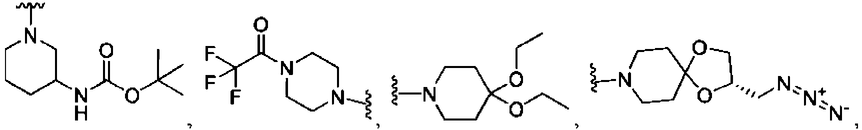 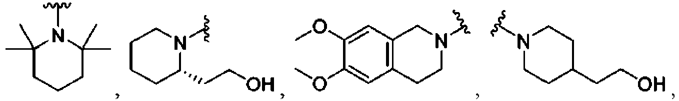 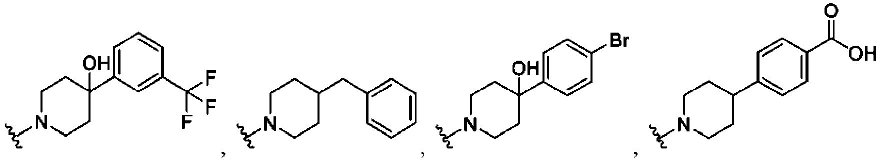 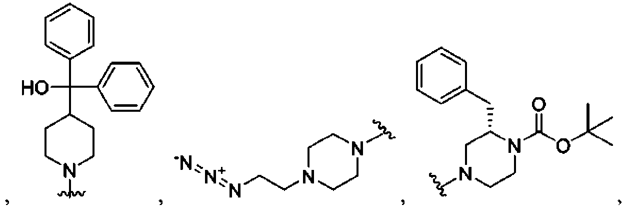 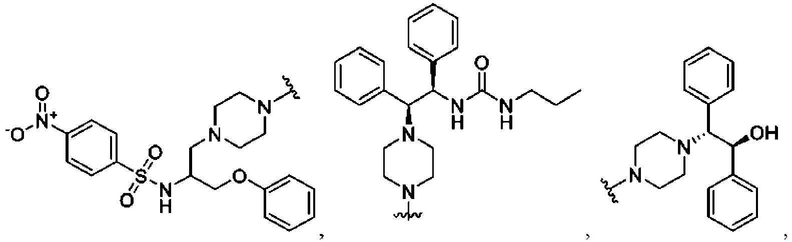 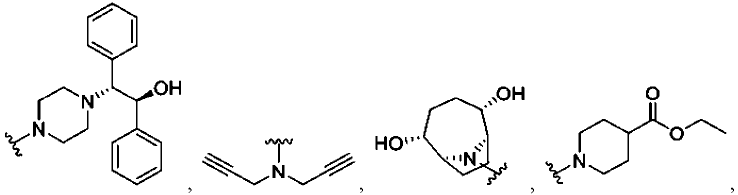 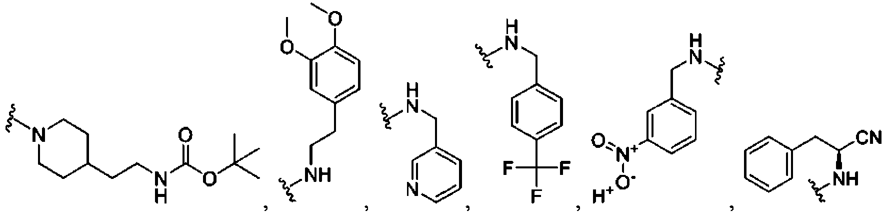 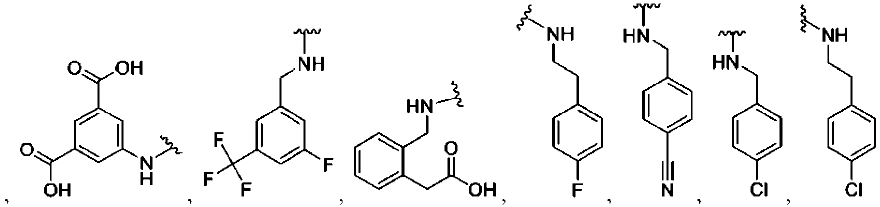 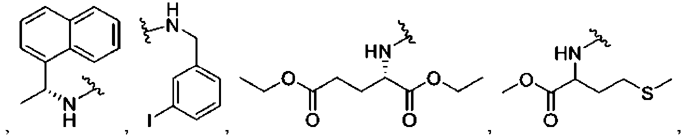 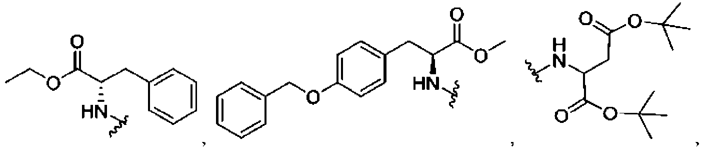 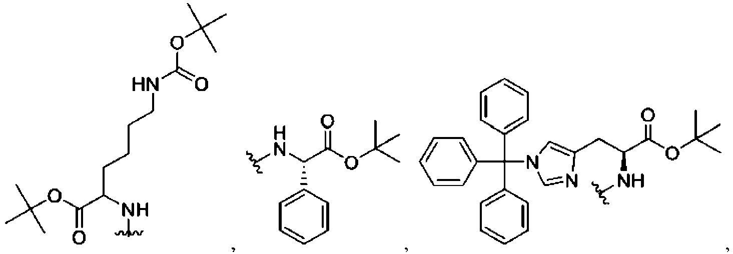 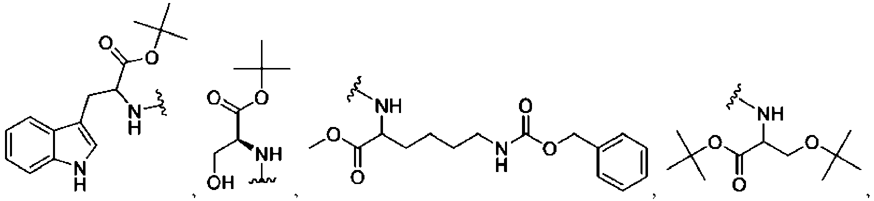 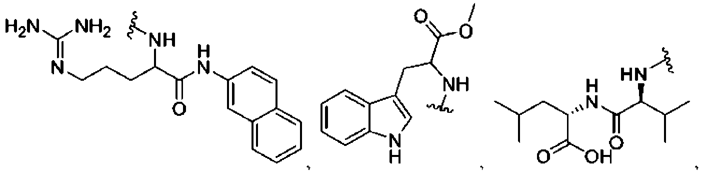 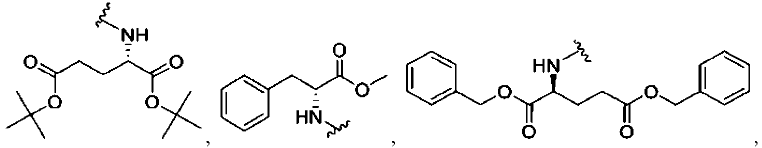 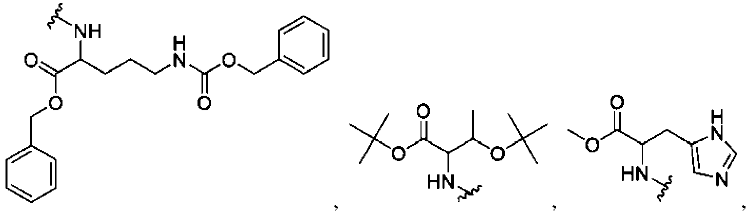 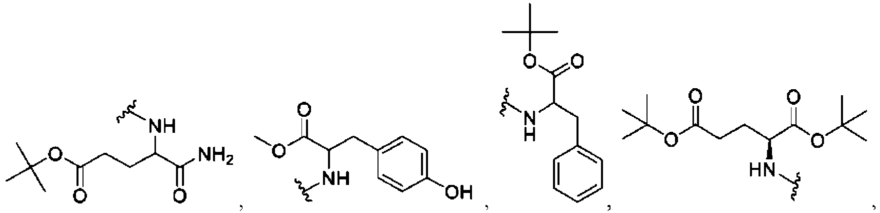 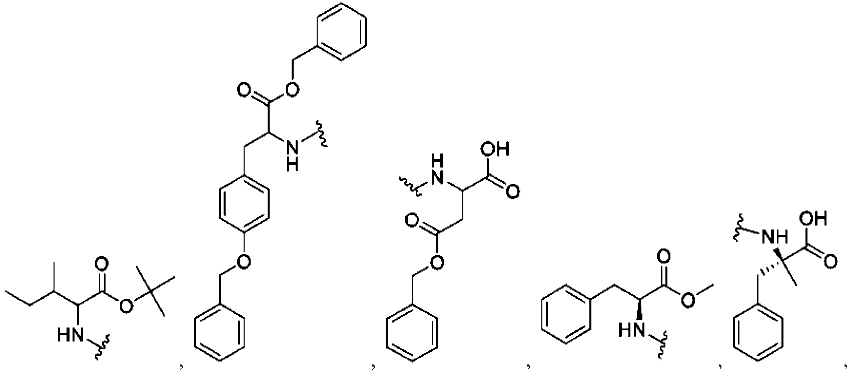 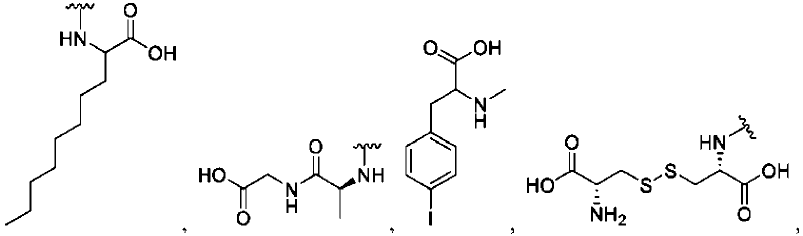 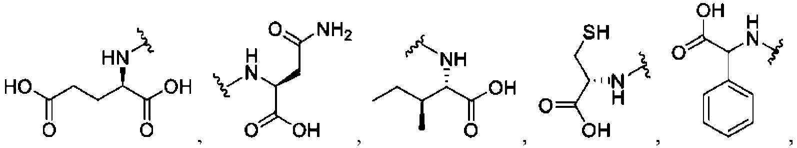 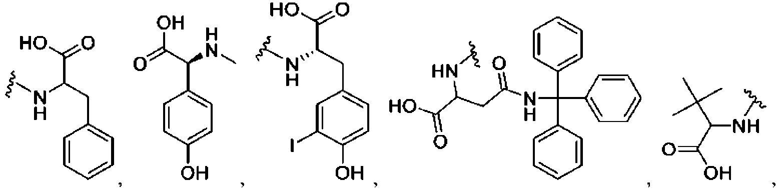 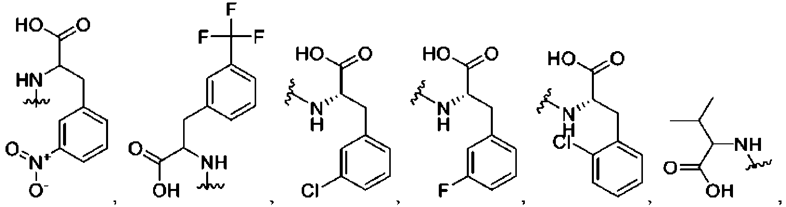 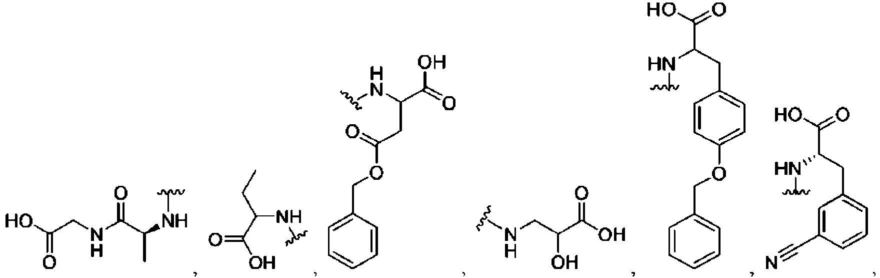 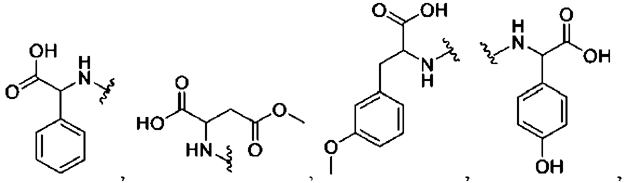 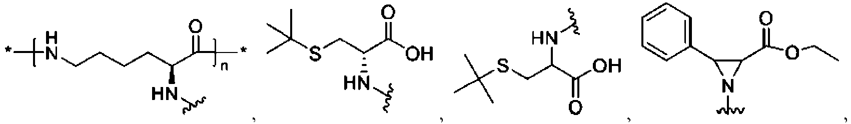 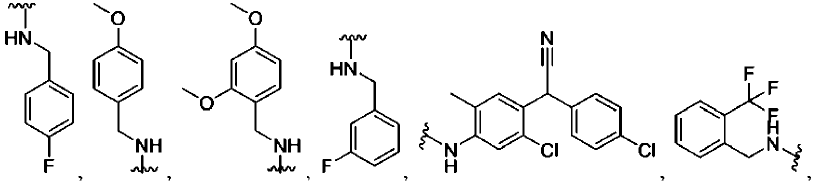 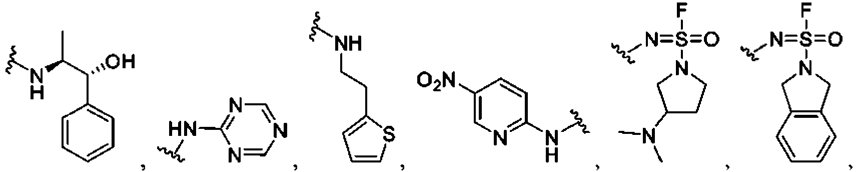 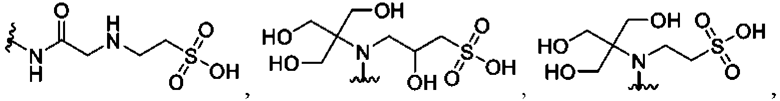 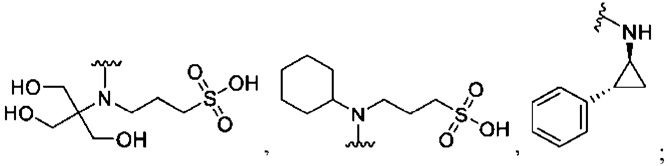 und
R⁵ H, Alkyl, Aryl oder -Alkylen-aryl ist;
wobei, sofern nicht anders spezifiziert:
der Begriff Alkyl eine verzweigte oder unverzweigte Kohlenstoffketteneinheit bis zu 30 Kohlenstoffatomen definiert;
der Begriff Alkylen eine Alkylgruppe mit 2 bis 12 Kohlenstoffatomen definiert, die zwei Verknüpfungspunkte zu dem Rest der Verbindung auf ihrer längsten Kohlenstoffkette enthält;
der Begriff Aryl einen 5- bis 12-gliedrigen aromatischen Ring definiert, wobei jedes Atom des Rings Kohlenstoff ist oder ein oder mehrere Ringatome Heteroatome sind;
der Begriff Heterocyclyl eine 3- bis 12-gliedrige Ringstruktur definiert, wobei ein bis vier Ringatome Heteroatome sind; und
der Begriff Cycloalkyl einen mono-, bicyclischen, verbrückten, spirocyclischen oder polycyclischen gesättigten carbocyclischen Ring mit 3 bis 12 Kohlenstoffatomen definiert;
oder eine pharmazeutische Zusammensetzung, umfassend die Verbindung der Formel (I) oder Formel (II) und einen pharmazeutisch verträglichen Träger, zur Verwendung in einem Verfahren zum Behandeln oder Verhindern einer/eines HA-vermittelten Erkrankung oder Zustands.

14. Verbindung zur Verwendung gemäß Anspruch 13, wobei die HA-vermittelte Erkrankung Influenza ist.

15. Verbindung zur Verwendung gemäß Anspruch 14, wobei Influenza Influenza A, Influenza B oder Influenza C ist.

## Revendications

1. Composé de formule (I) ou (II) : 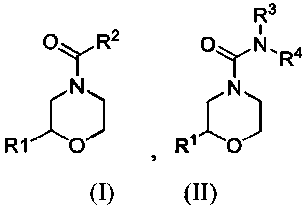 où
R¹ est un phényle ;
R² est un aryle, un hétérocyclyle, 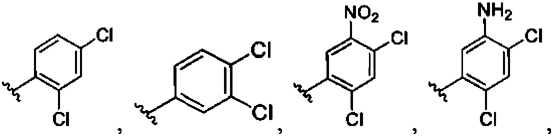 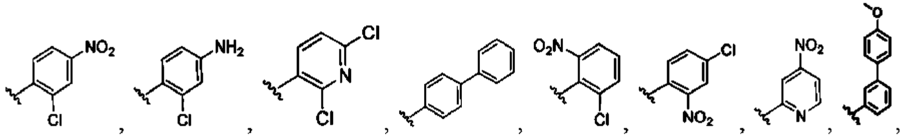 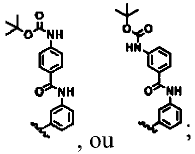
R³ est choisi parmi H, un alkyle, -alkylène-(hétérocyclyle), -alkylène-(aryle), -alkylène-(cycloalkyle), -alkylène-O-R⁵, -alkylène-C(O)N(R⁵)₂, -alkylène-C(O)R⁵, -alkylène-C(O)₂R⁵, - alkylène-N(R⁵)₂, -alkylène-N(R⁵)C(O)R⁵, -alkylène-N(R⁵)C(O)N(R⁵)₂, -alkylène-N(R⁵)SO₂-R⁵, - alkylène-S-R⁵, -aryle, -arylène-(hétérocyclyle), -arylène-alkyle, -arylène-(cycloalkyle), -arylène-O-R⁵, -arylène-C(O)N(R⁵)₂, -arylène-C(O)R⁵, -arylène-C(O)₂R⁵, -arylène-N(R⁵)₂, -arylène-N(R⁵)C(O)R⁵, -arylène-N(R⁵)C(O)N(R⁵)₂, -arylène-N(R⁵)SO₂-R⁵, -arylène-S-R⁵, -hétérocyclyle, -hétérocyclylène-(alkyle), -hétérocyclylène-(aryle), -hétérocyclylène-(cycloalkyle), - hétérocyclylène-(cycloalkyle), -hétérocyclylène-O-R⁵, -hétérocyclylène-C(O)N(R⁵)₂, - hétérocyclylène-C(O)R⁵, -hétérocyclylène-C(O)₂R⁵, -hétérocyclylène-N(R⁵)₂, -hétérocyclylène-N(R⁵)C(O)R⁵, -hétérocyclylène-N(R⁵)C(O)N(R⁵)₂, -hétérocyclylène-N(R⁵)SO₂-R⁵, - hétérocyclylène-S-R⁵, -cycloalkyle, -cycloalkylène-(alkyle), -cycloalkylène-(aryle), - cycloalkylène-(cycloalkyle), -cycloalkylène(hétérocycloalkyle), -cycloalkylène-O-R⁵, - cycloalkylène-C(O)N(R⁵)₂, -cycloalkylène-C(O)R⁵, -cycloalkylène-C(O)₂R⁵, -cycloalkylène-N(R⁵)₂, -cycloalkylène-N(R⁵)C(O)R⁵, -cycloalkylène-N(R⁵)C(O)N(R⁵)₂, -cycloalkylène-N(R⁵)SO₂-R⁵, -alkylène-N(H)-alkylène-C(O)OR⁵, -alkylène-C(O)OR⁵, -arylène-alkylène-aryle, - C(O)R⁵, -C(O)₂R⁵, -alkylène-S-S-R⁵, -C(O)-alkylène-N(R⁵)₂, -C(O)₂R⁵, -aryl-alkylène-N(R⁵)C(O)-R⁵, -cycloalkylène-N(R⁵)S(O)(F)-R⁵, -SO₂-NR⁵, -S(O)(F)-NR⁵ ;
R⁴ est choisi parmi un alkyle, -alkylène-(hétérocyclyle), -alkylène-(aryle), -alkylène-(cycloalkyle), -alkylène-O-R⁵, -alkylène-C(O)N(R⁵)₂, -alkylène-C(O)R⁵, -alkylène-C(O)₂R⁵, - alkylène-N(R⁵)₂, -alkylène-N(R⁵)C(O)R⁵, -alkylène-N(R⁵)C(O)N(R⁵)₂, -alkylène-N(R⁵)SO₂-R⁵, - alkylène-S-R⁵, -aryle, -arylène-(hétérocyclyle), -arylène-alkyle, -arylène-(cycloalkyle), -arylène-O-R⁵, -arylène-C(O)N(R⁵)₂, -arylène-C(O)R⁵, -arylène-C(O)₂R⁵, -arylène-N(R⁵)₂, -arylène-N(R⁵)C(O)R⁵, -arylène-N(R⁵)C(O)N(R⁵)₂, -arylène-N(R⁵)SO₂-R⁵, -arylène-S-R⁵, -hétérocyclyle, -hétérocyclylène-(alkyle), -hétérocyclylène-(aryle), -hétérocyclylène-(cycloalkyle), - hétérocyclylène-(cycloalkyle), -hétérocyclylène-O-R⁵, -hétérocyclylène-C(O)N(R⁵)₂, - hétérocyclylène-C(O)R⁵, -hétérocyclylène-C(O)₂R⁵, -hétérocyclylène-N(R⁵)₂, -hétérocyclylène-N(R⁵)C(O)R⁵, -hétérocyclylène-N(R⁵)C(O)N(R⁵)₂-, -hétérocyclylène-N(R⁵)SO₂-R⁵, - hétérocyclylène-S-R⁵, -cycloalkyle, -cycloalkylène-(alkyle), -cycloalkylène-(aryle), - cycloalkylène-(cycloalkyle), -cycloalkylène(hétérocycloalkyle), -cycloalkylène-O-R⁵, - cycloalkylène-C(O)N(R⁵)₂, -cycloalkylène-C(O)R⁵, -cycloalkylène-C(O)₂R⁵, -cycloalkylène-N(R⁵)₂, -cycloalkylène-N(R⁵)C(O)R⁵, -cycloalkylène-N(R⁵)C(O)N(R⁵)₂, -cycloalkylène-N(R⁵)SO₂-R⁵, -alkylène-N(H)-alkylène-C(O)OR⁵, -alkylène-C(O)OR⁵, -arylène-alkylène-aryle, - C(O)R⁵, -C(O)₂R⁵, -alkylène-S-S-R⁵, -C(O)-alkylène-N(R⁵)₂, -C(O)₂R⁵, -aryl-alkylène-N(R⁵)C(O)-R⁵, -cycloalkylène-N(R⁵)S(O)(F)-R⁵, -SO₂-NR⁵, -S(O)(F)-NR⁵ ; ou
R³ et R⁴ peuvent être pris ensemble pour former un hétérocyclyle ; ou 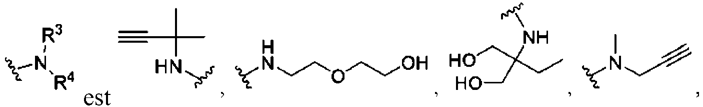 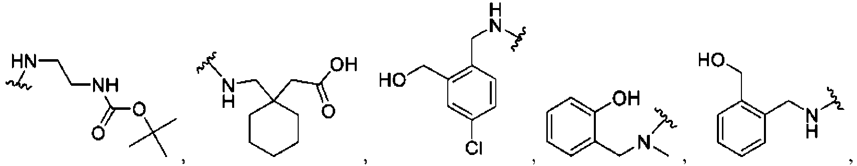 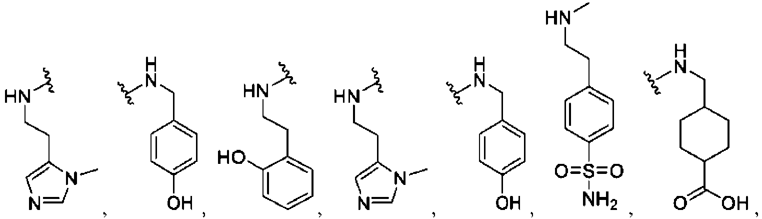 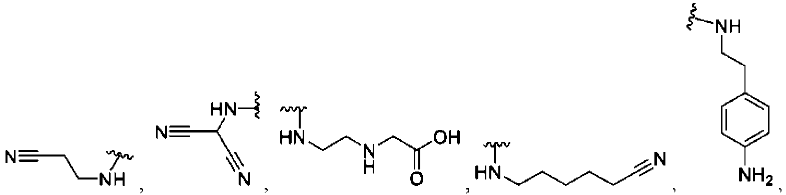 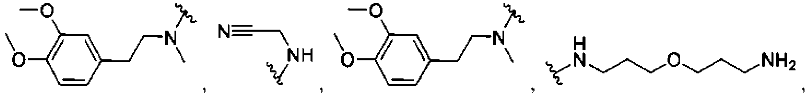 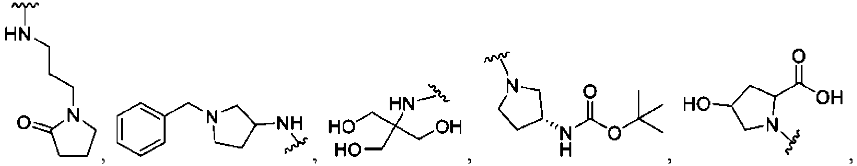 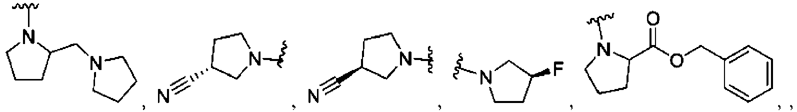 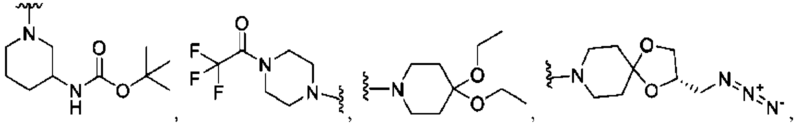 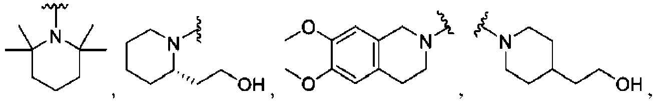 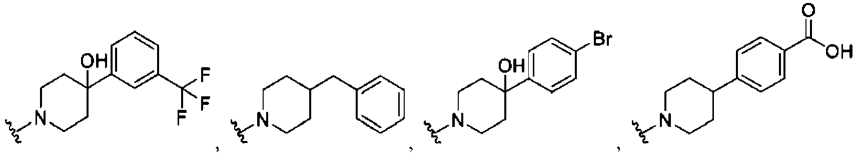 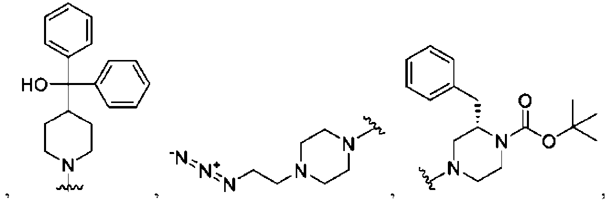 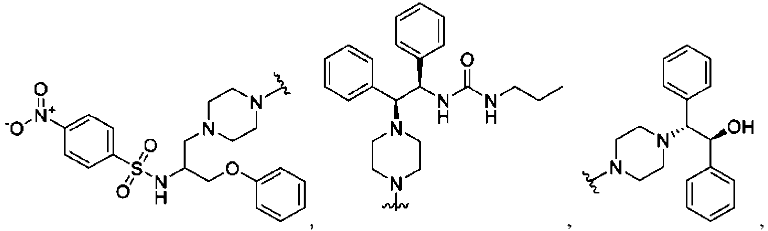 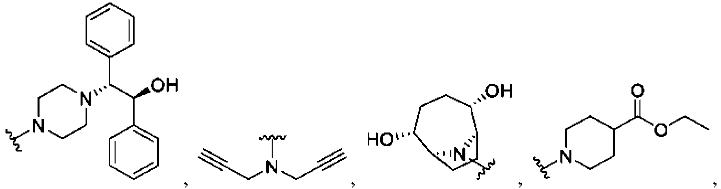 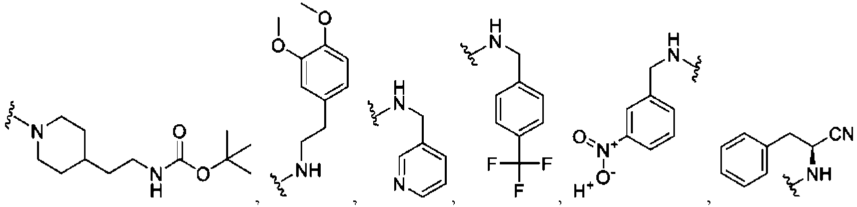 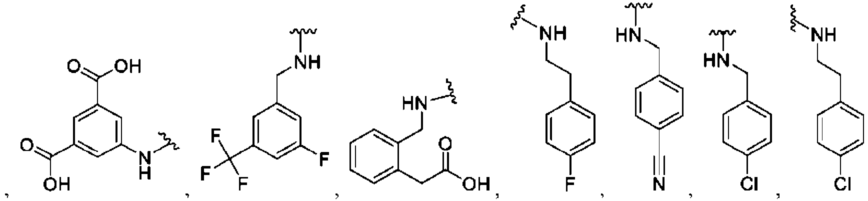 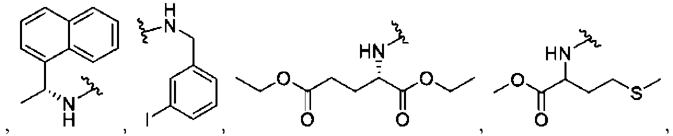 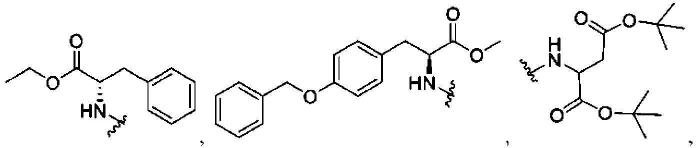 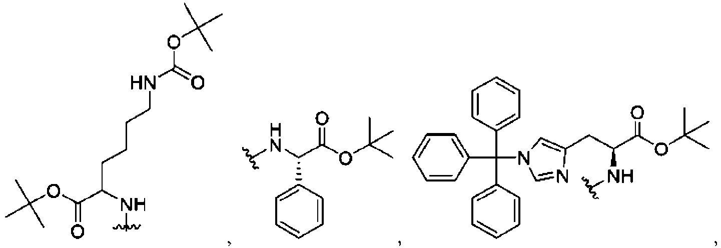 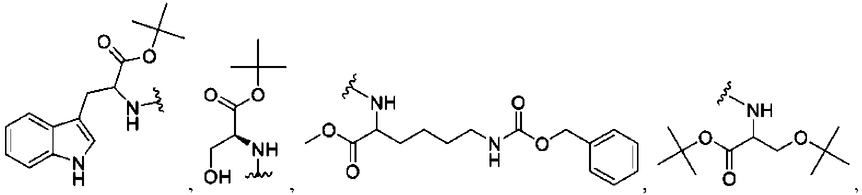 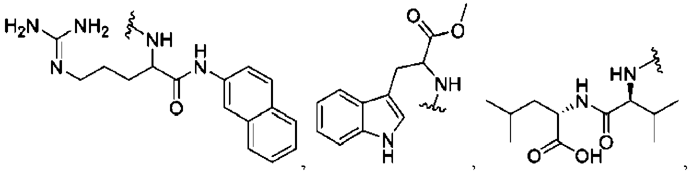 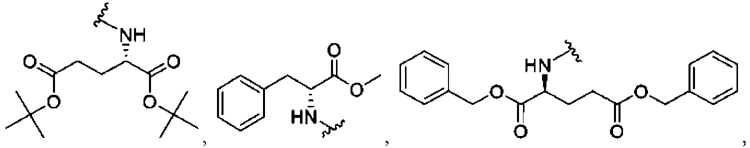 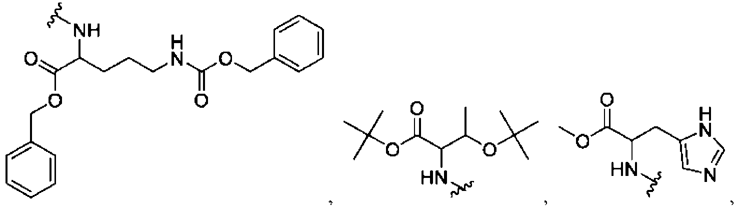 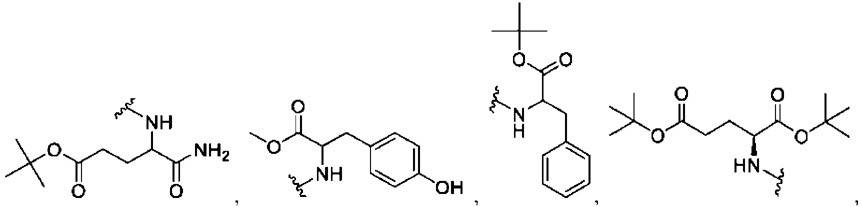 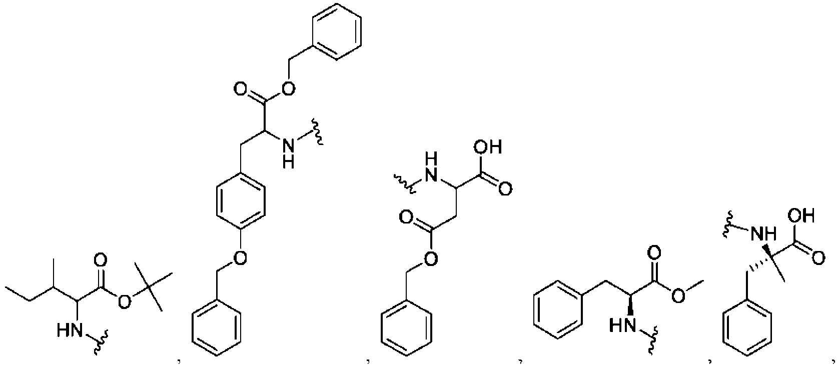 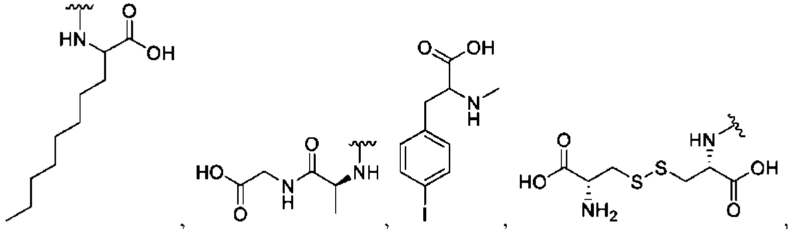 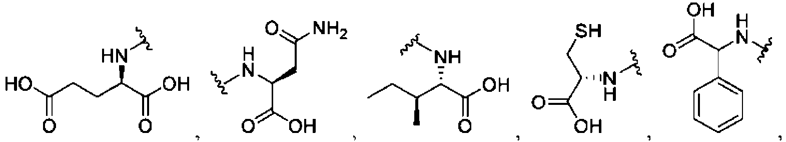 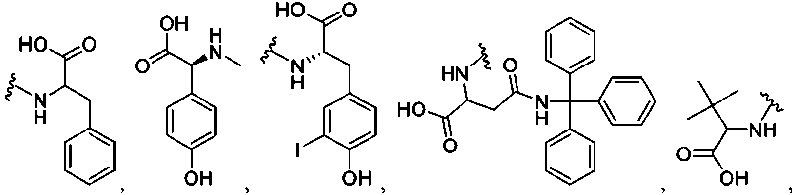 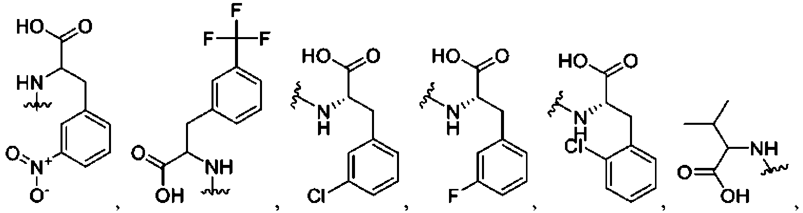 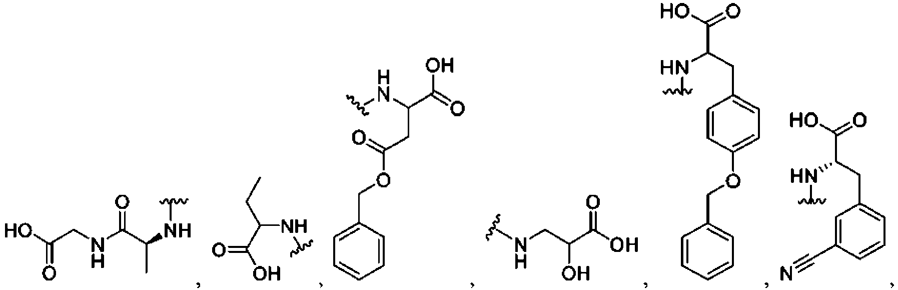 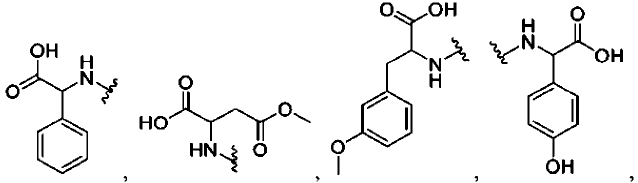 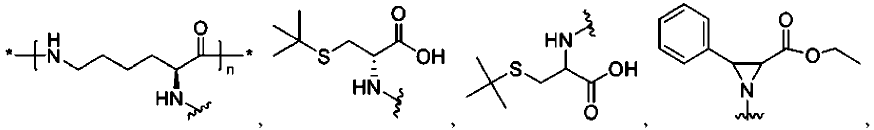 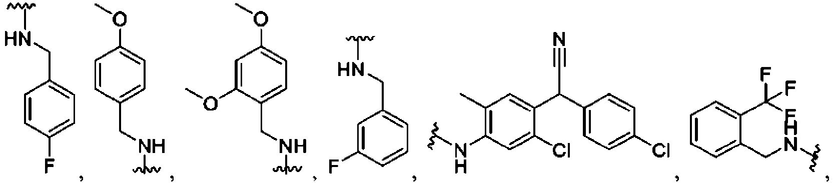 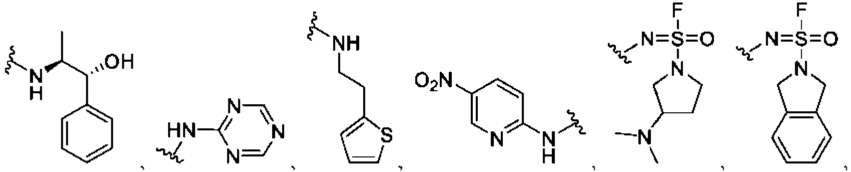 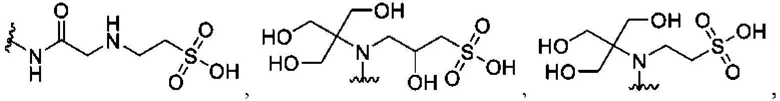 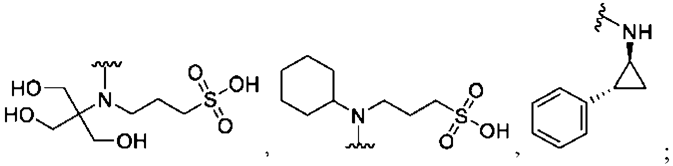 et
R⁵ est H, un alkyle, un aryle ou -alkylène-aryle ;
où, sauf indication contraire :
le terme alkyle définit une fraction de chaîne carbonée ramifiée ou non ramifiée jusqu'à 30 atomes de carbone ;
le terme alkylène définit un groupe alkyle comportant 2 à 12 atomes de carbone qui contient deux points de fixation au reste du composé sur sa chaîne carbonée la plus longue ;
le terme aryle définit un cycle aromatique de 5 à 12 chaînons, où chaque atome du cycle est un carbone ou un ou plusieurs atomes de cycle sont des hétéroatomes ;
le terme hétérocyclyle définit une structure cyclique de 3 à 12 chaînons, où un à quatre atomes de cycle sont des hétéroatomes ; et
le terme cycloalkyle définit un cycle carbocyclique saturé mono-, bicyclique, ponté, spirocyclique ou polycyclique comportant 3 à 12 atomes de carbone ;
où le composé de formule (II) n'est pas

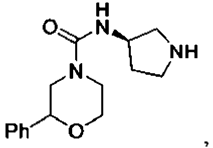

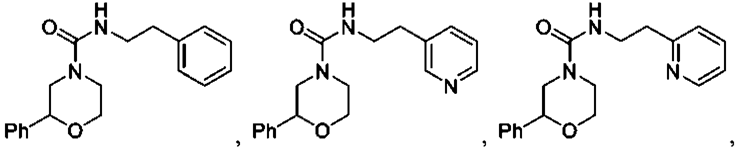

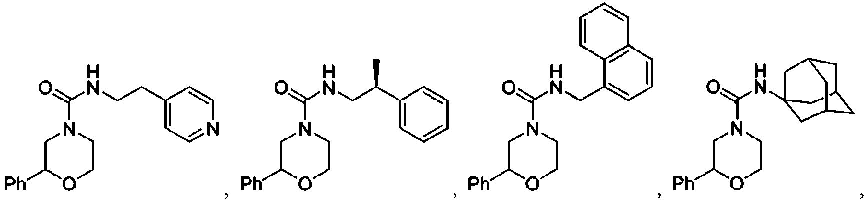

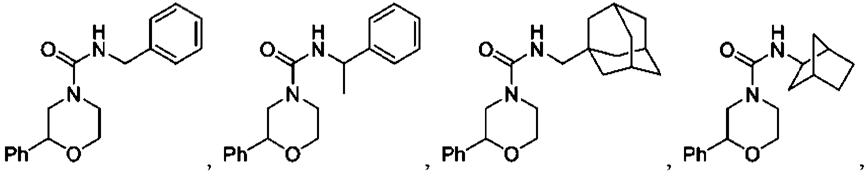

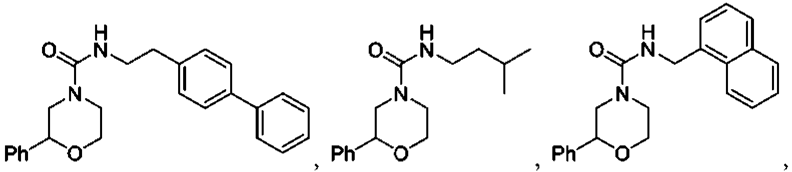

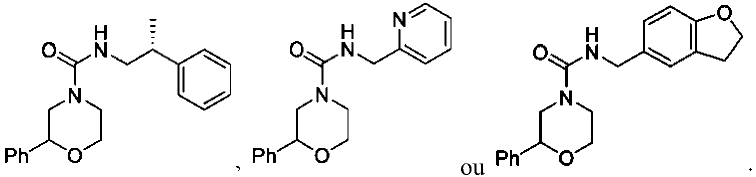

2. Composé de la revendication 1, ledit composé répondant à la formule (II)

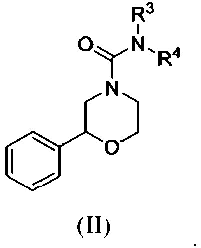

3. Composé de la revendication 1, dans lequel R² est choisi dans le groupe constitué par :

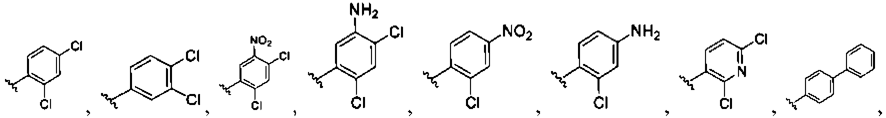

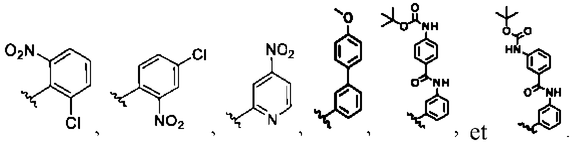

4. Composé de la revendication 1 ou 2, dans lequel 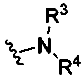 est :
- choisi dans le groupe constitué par : 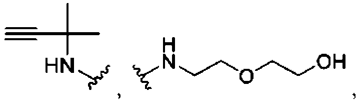 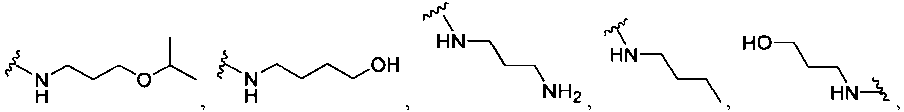 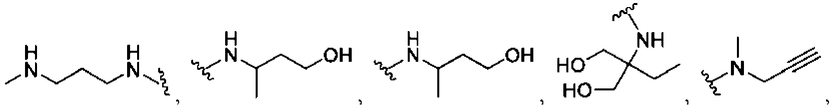 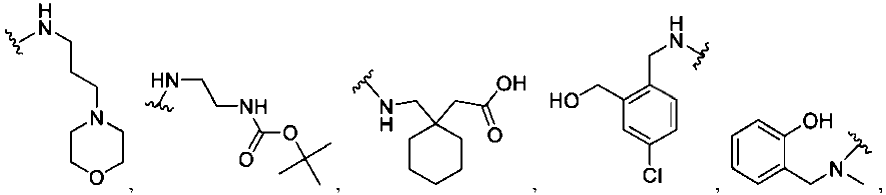 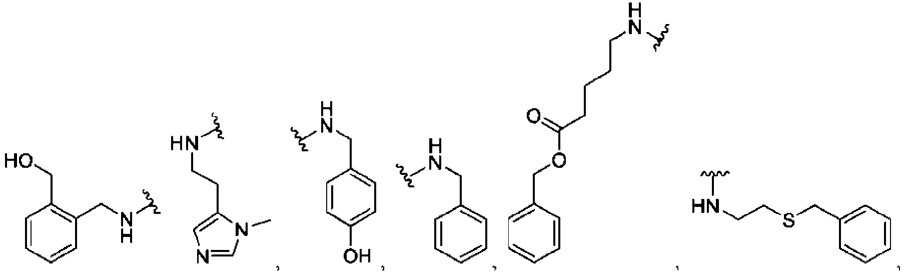 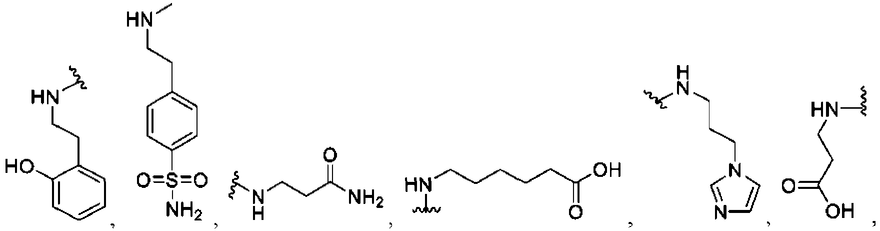 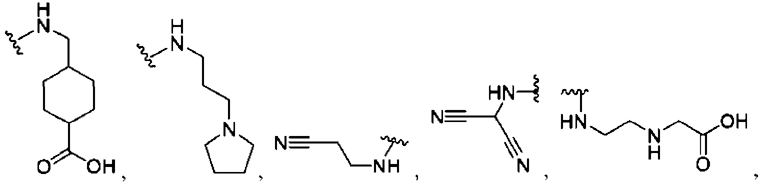 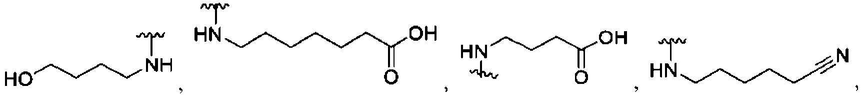 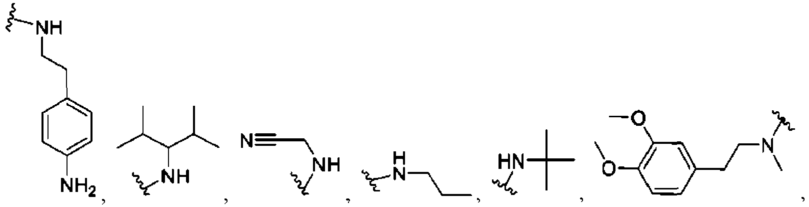 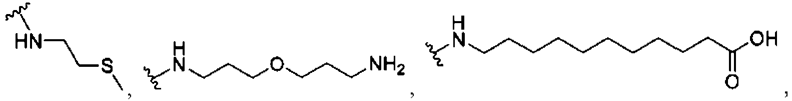 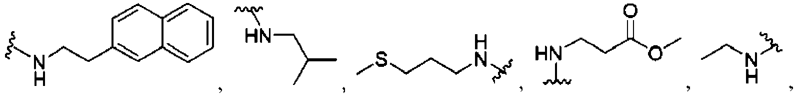 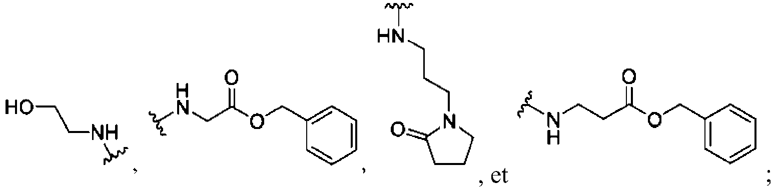 ou
- choisi dans le groupe constitué par : 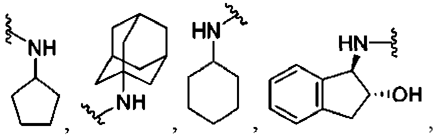 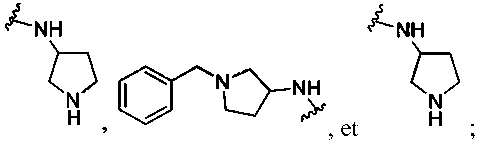 ou
- 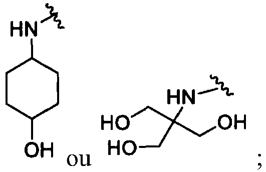 ou
- choisi dans le groupe constitué par :

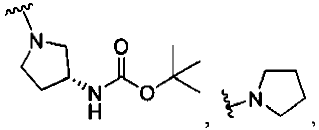

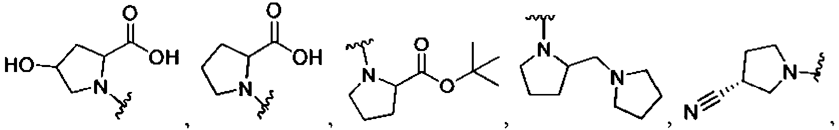

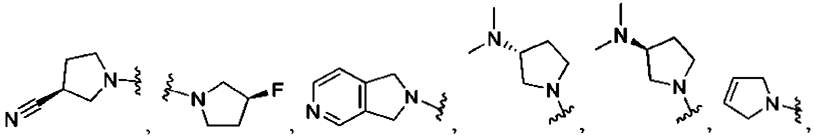

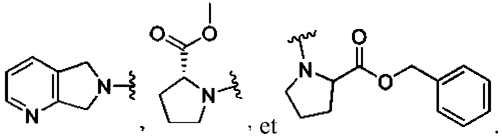

5. Composé de la revendication 1 ou 2, dans lequel 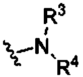 est :
- choisi dans le groupe constitué par : 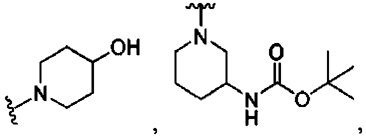 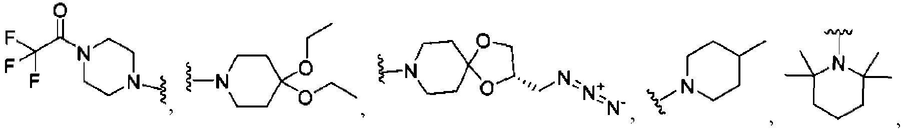 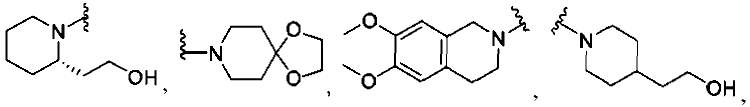 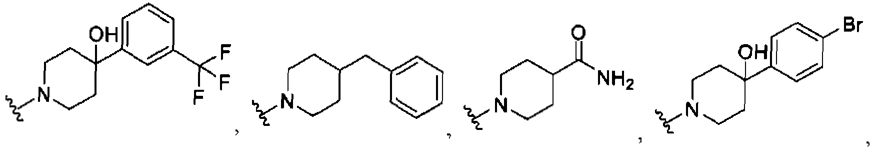 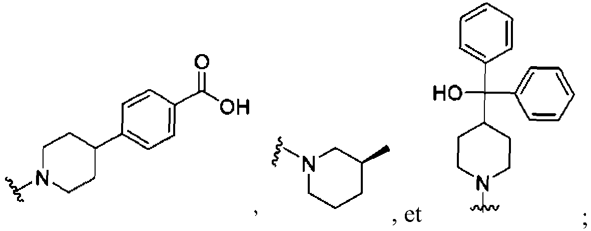 ou
- choisi dans le groupe constitué par : 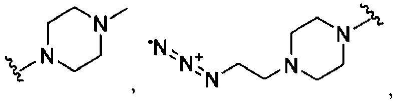 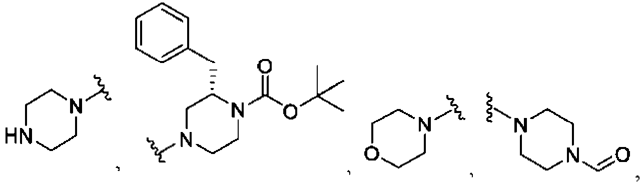 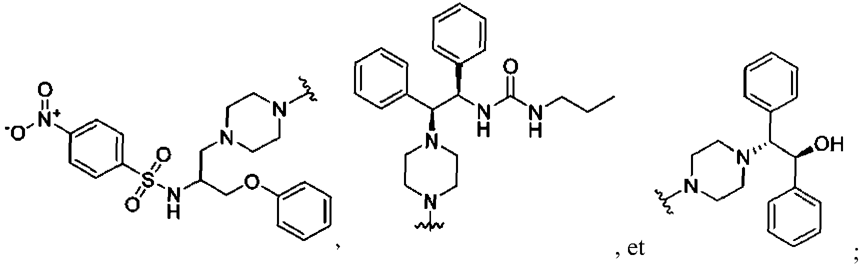 ou
- 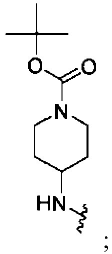 ou
- est : 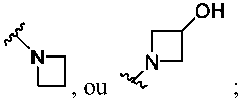 ou
- choisi dans le groupe constitué par : 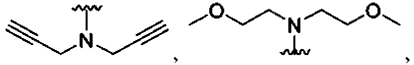 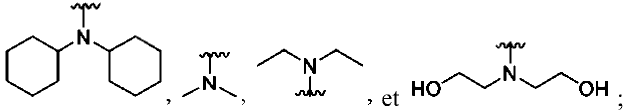 ou
- est choisi dans le groupe constitué par

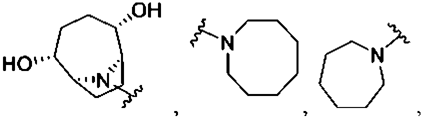

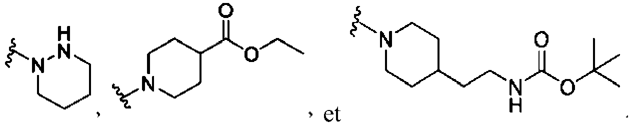

6. Composé de la revendication 1 ou 2, dans lequel 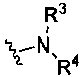 est :
- choisi dans le groupe constitué par : 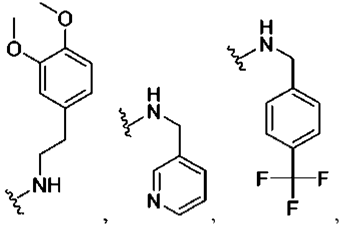 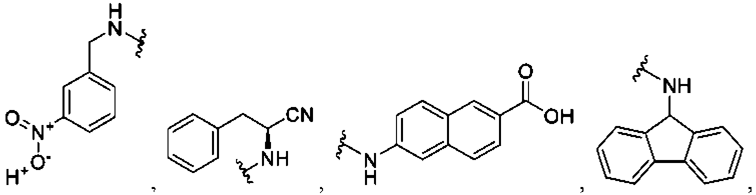 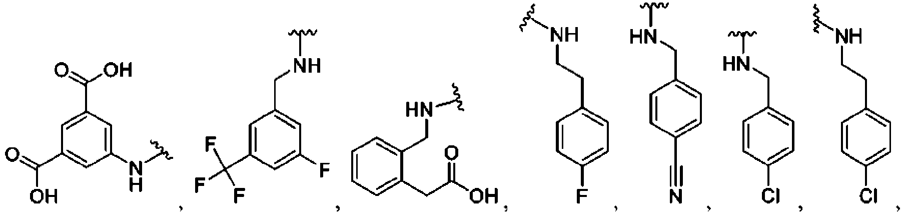 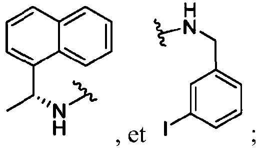 ou
- choisi dans le groupe constitué par : 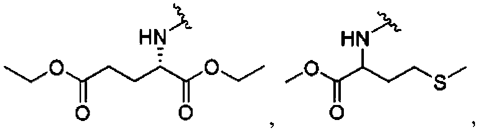 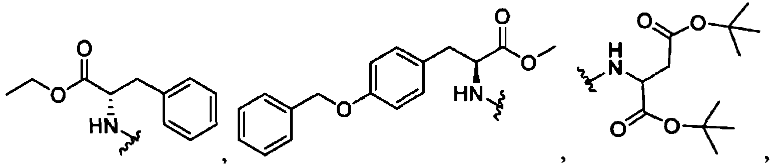 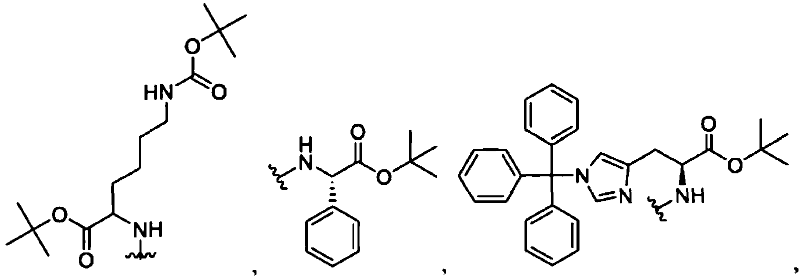 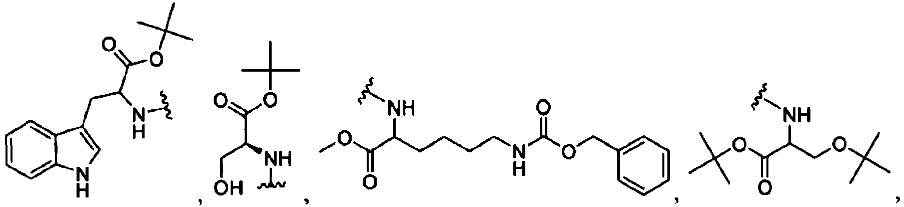 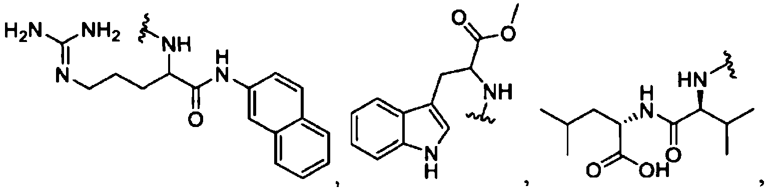 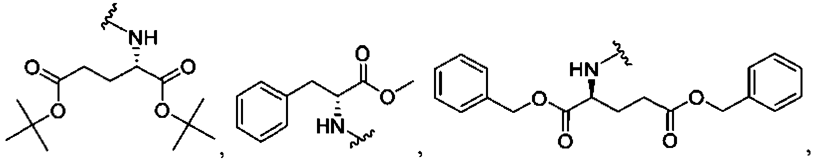 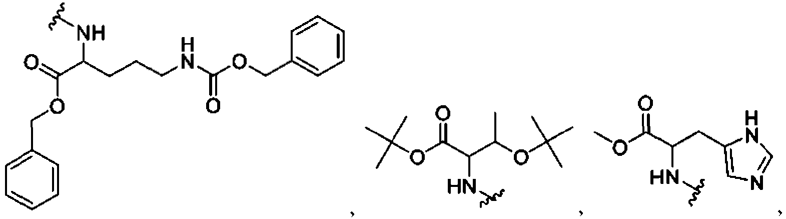 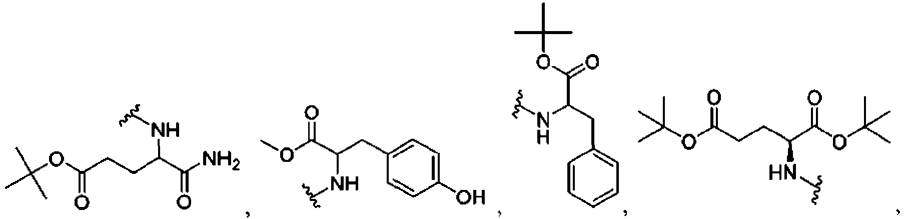 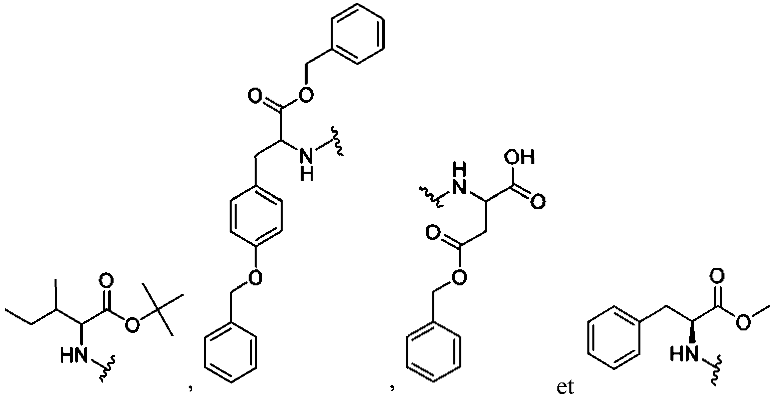 ou
- choisi dans le groupe constitué par : 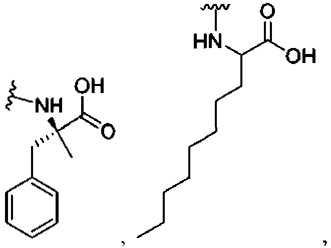 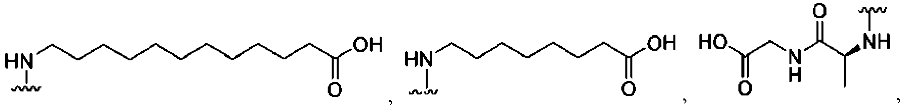 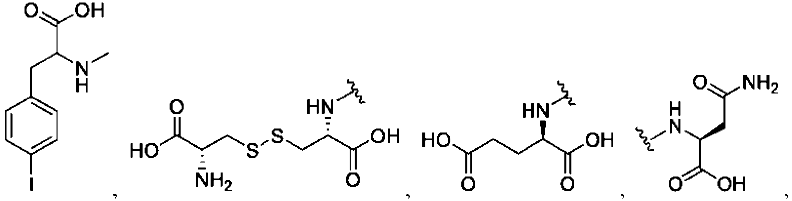 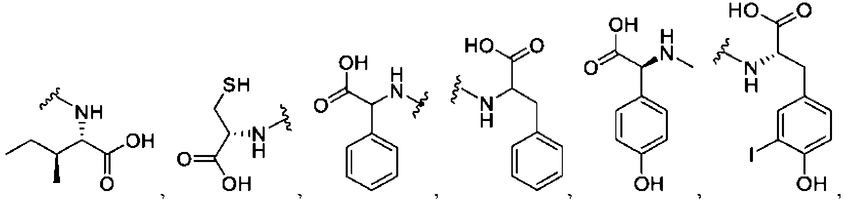 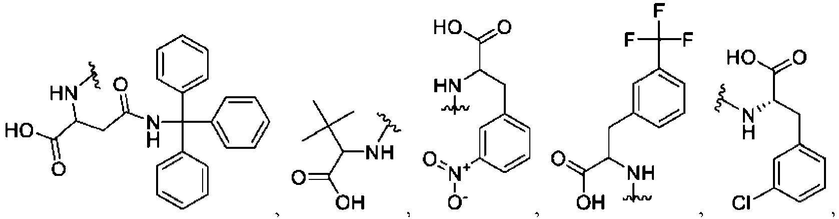 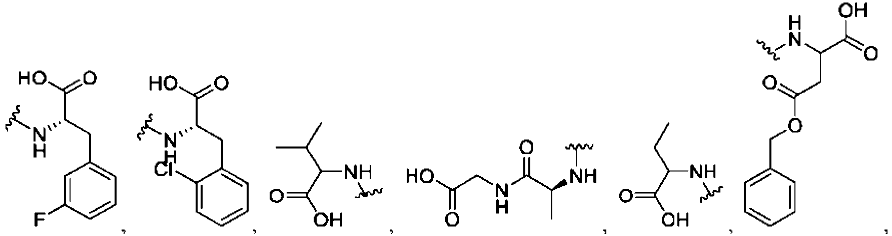 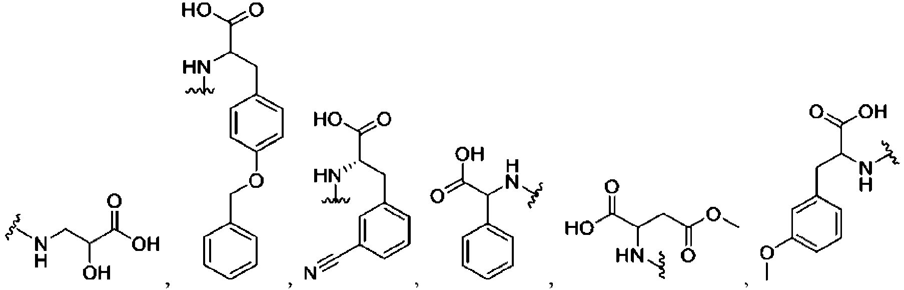 et

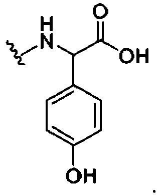

7. Composé de la revendication 1 ou 2, dans lequel 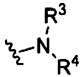 est :
- 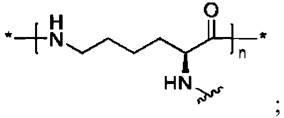 ou
- 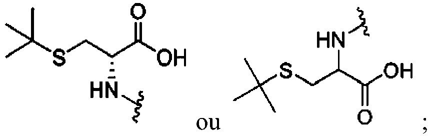 ou
- choisi dans le groupe constitué par 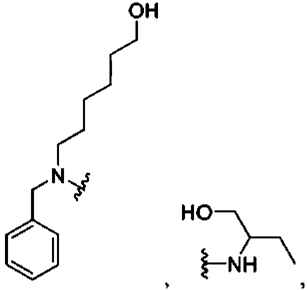 et 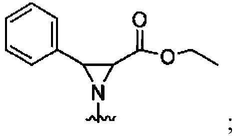 ou
- est choisi dans le groupe constitué par : 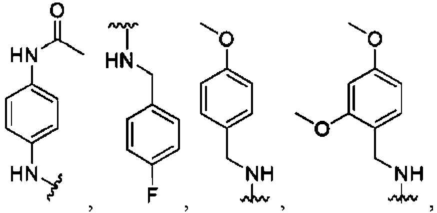 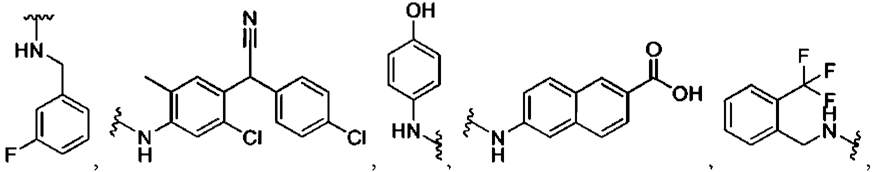 et 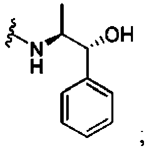 ou
- est choisi dans le groupe choisi parmi : 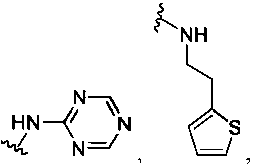 et 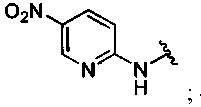 ou
- 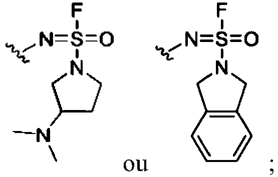 ou
- choisi dans le groupe constitué par : 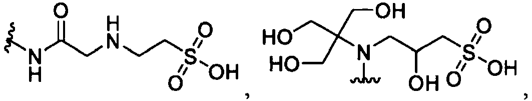 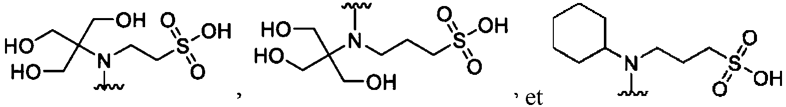 ou
- est

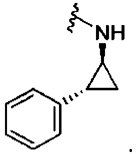

8. Composé de la revendication 1 ou 2, dans lequel le composé de formule (I) ou (II) est :
- choisi dans le groupe constitué par : 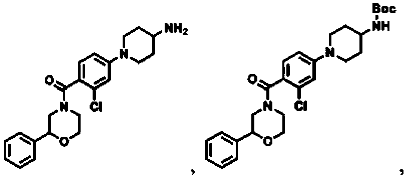 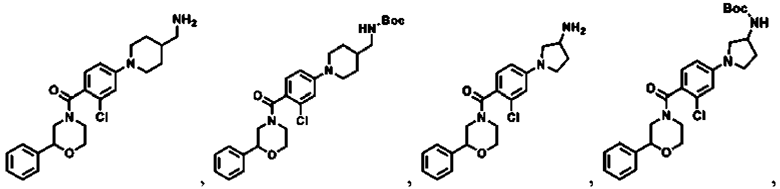 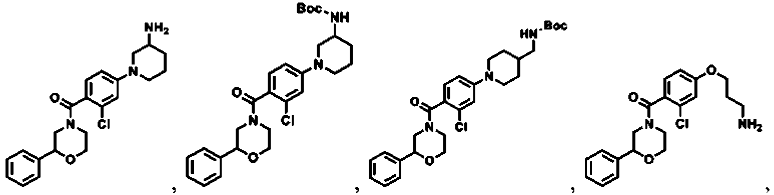 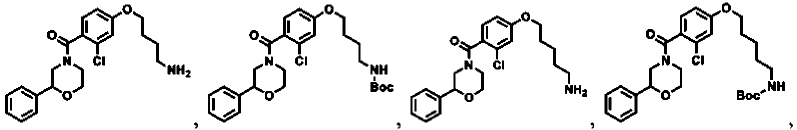 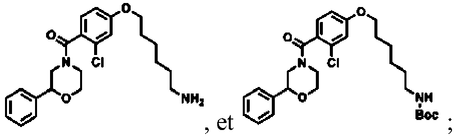 ou
- choisi dans le groupe constitué par : 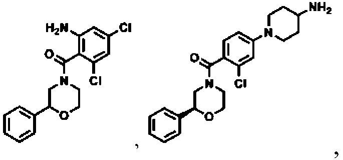 et 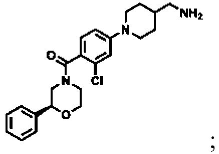 ou 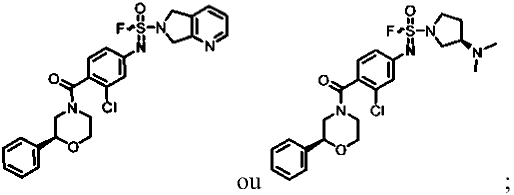 ou
- choisi dans le groupe constitué par :

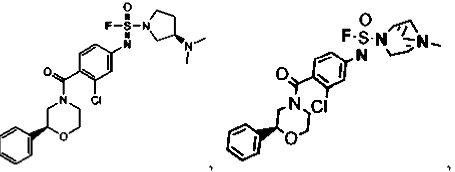

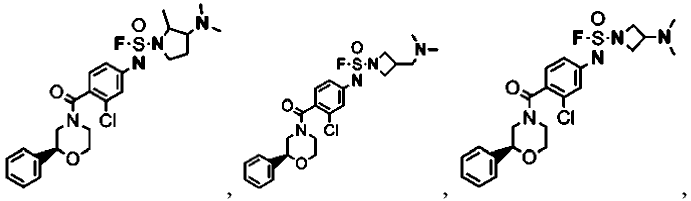

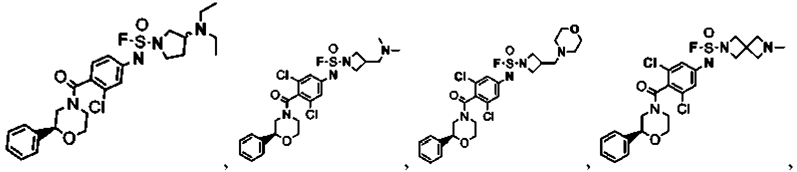

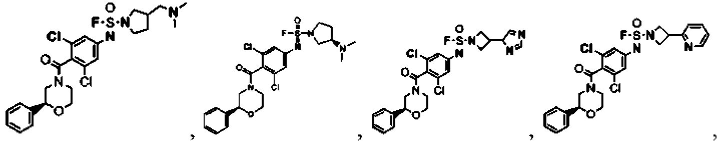

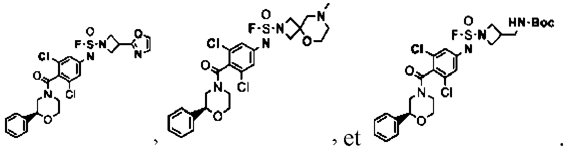

9. Composé de la revendication 1 ou 2, dans lequel le composé de formule (I) ou (II) est :
- choisi dans le groupe constitué par : 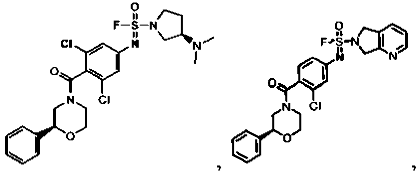 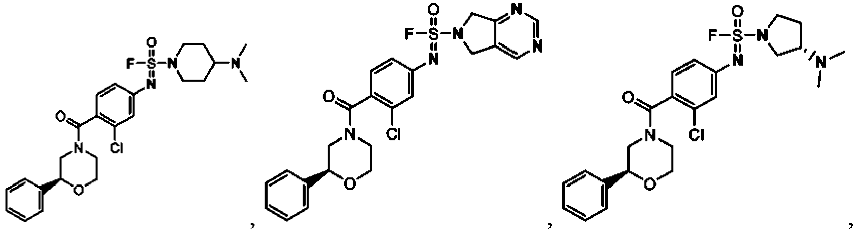 et 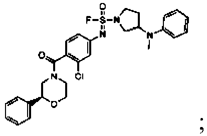 ou
- choisi dans le groupe constitué par : 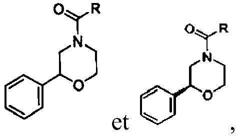 R étant 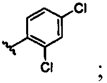 ou
- choisi dans le groupe constitué par : 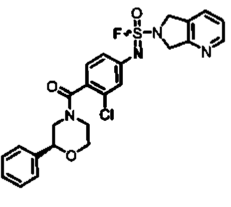 et 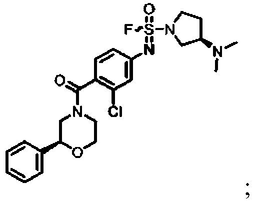 ou
- 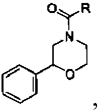 R étant choisi dans le groupe constitué par :

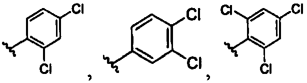

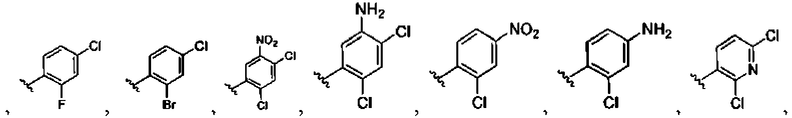

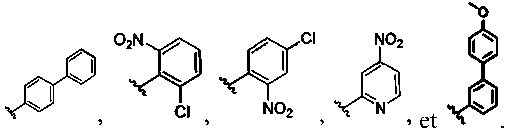

10. Composé de la revendication 1 ou 2, dans lequel le composé de formule (I) ou (II) est :
- choisi dans le groupe constitué par : 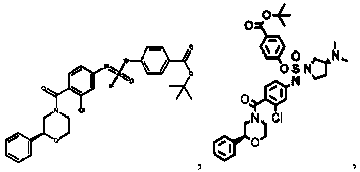 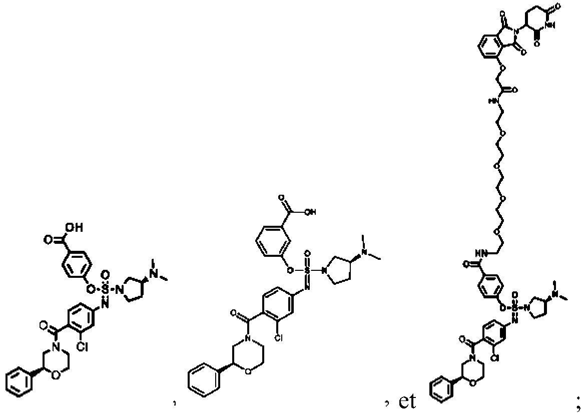 ou
- 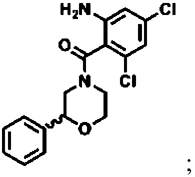 ou
- est choisi dans le groupe constitué par : 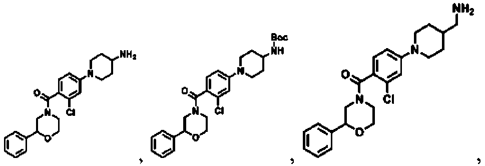 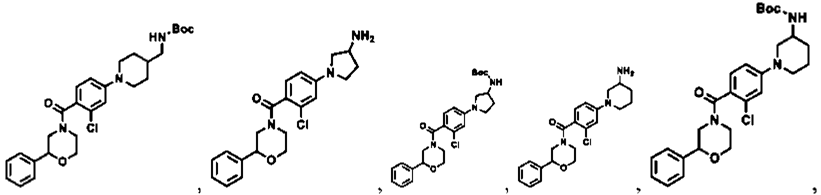 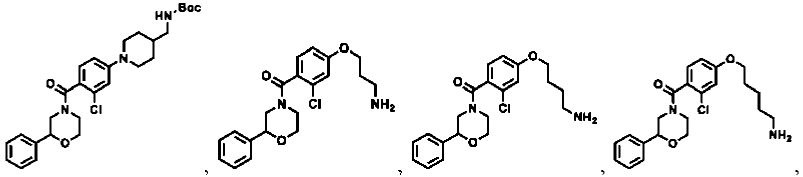 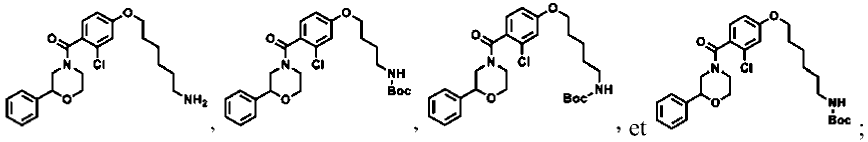 ou
- 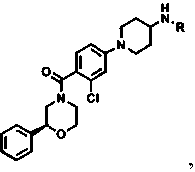 R étant choisi dans le groupe constitué par 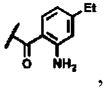 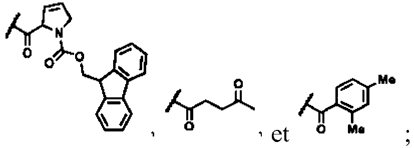 ou
- 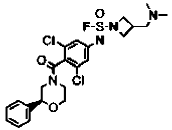 ou
- 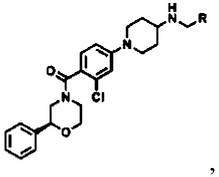 R étant choisi dans le groupe constitué par 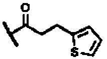 et 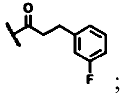 ou 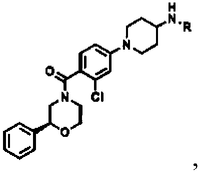 R étant choisi dans le groupe constitué par 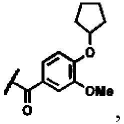 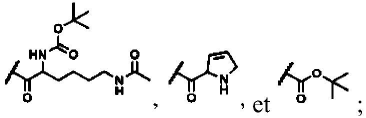 ou
- choisi dans le groupe constitué par : 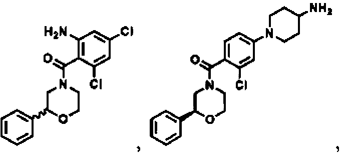 et

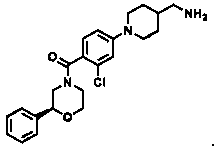

11. Composition pharmaceutique comprenant un composé de l'une quelconque des revendications 1 à 10 et un support acceptable pharmaceutiquement.

12. Composé de l'une quelconque des revendications 1 à 10 ou composition pharmaceutique de la revendication 11, destiné(e) à être utilisé(e) en tant que médicament.

13. Composé de formule (I) ou (II) : 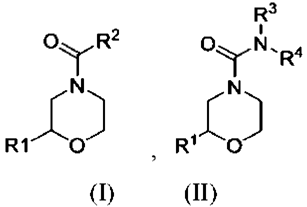 où
R¹ est un phényle ;
R²est un aryle, un hétérocyclyle, 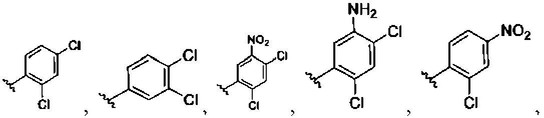 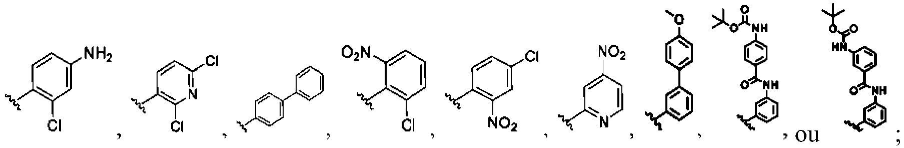
R³ est choisi parmi H, un alkyle, -alkylène-(hétérocyclyle), -alkylène-(aryle), -alkylène-(cycloalkyle), -alkylène-O-R⁵, -alkylène-C(O)N(R⁵)₂, -alkylène-C(O)R⁵, -alkylène-C(O)₂R⁵, - alkylène-N(R⁵)₂, -alkylène-N(R⁵)C(O)R⁵, -alkylène-N(R⁵)C(O)N(R⁵)₂, -alkylène-N(R⁵)SO₂-R⁵, - alkylène-S-R⁵, -aryle, -arylène-(hétérocyclyle), -arylène-alkyle, -arylène-(cycloalkyle), -arylène-O-R⁵, -arylène-C(O)N(R⁵)₂, -arylène-C(O)R⁵, -arylène-C(O)₂R⁵, -arylène-N(R⁵)₂, -arylène-N(R⁵)C(O)R⁵, -arylène-N(R⁵)C(O)N(R⁵)₂, -arylène-N(R⁵)SO₂-R⁵, -arylène-S-R⁵, -hétérocyclyle, -hétérocyclylène-(alkyle), -hétérocyclylène-(aryle), -hétérocyclylène-(cycloalkyle), - hétérocyclylène-(cycloalkyle), -hétérocyclylène-O-R⁵, -hétérocyclylène-C(O)N(R⁵)₂, - hétérocyclylène-C(O)R⁵, -hétérocyclylène-C(O)₂R⁵, -hétérocyclylène-N(R⁵)₂, -hétérocyclylène-N(R⁵)C(O)R⁵, -hétérocyclylène-N(R⁵)C(O)N(R⁵)₂, -hétérocyclylène-N(R⁵)SO₂-R⁵, - hétérocyclylène-S-R⁵, -cycloalkyle, -cycloalkylène-(alkyle), -cycloalkylène-(aryle), - cycloalkylène-(cycloalkyle), -cycloalkylène(hétérocycloalkyle), -cycloalkylène-O-R⁵, - cycloalkylène-C(O)N(R⁵)₂, -cycloalkylène-C(O)R⁵, -cycloalkylène-C(O)₂R⁵, -cycloalkylène-N(R⁵)₂, -cycloalkylène-N(R⁵)C(O)R⁵, -cycloalkylène-N(R⁵)C(O)N(R⁵)₂, -cycloalkylène-N(R⁵)SO₂-R⁵, -alkylène-N(H)-alkylène-C(O)OR⁵, -alkylène-C(O)OR⁵, -arylène-alkylène-aryle, - C(O)R⁵, -C(O)₂R⁵, -alkylène-S-S-R⁵, -C(O)-alkyle, -C(O)-alkylène-N(R⁵)₂, -C(O)₂R⁵, -aryl-alkylène-N(R⁵)C(O)-R⁵, -cycloalkylène-N(R⁵)S(O)(F)-R⁵, -SO₂-NR⁵, -S(O)(F)-NR⁵ ;
R⁴ est choisi parmi un alkyle, -alkylène-(hétérocyclyle), -alkylène-(aryle), -alkylène-(cycloalkyle), -alkylène-O-R⁵, -alkylène-C(O)N(R⁵)₂, -alkylène-C(O)R⁵, -alkylène-C(O)₂R⁵, - alkylène-N(R⁵)₂, -alkylène-N(R⁵)C(O)R⁵, -alkylène-N(R⁵)C(O)N(R⁵)₂, -alkylène-N(R⁵)SO₂-R⁵, - alkylène-S-R⁵, -aryle, -arylène-(hétérocyclyle), -arylène-alkyle, -arylène-(cycloalkyle), -arylène-O-R⁵, -arylène-C(O)N(R⁵)₂, -arylène-C(O)R⁵, -arylène-C(O)₂R⁵, -arylène-N(R⁵)₂, -arylène-N(R⁵)C(O)R⁵, -arylène-N(R⁵)C(O)N(R⁵)₂, -arylène-N(R⁵)SO₂-R⁵, -arylène-S-R⁵, -hétérocyclyle, -hétérocyclylène-(alkyle), -hétérocyclylène-(aryle), -hétérocyclylène-(cycloalkyle), - hétérocyclylène-(cycloalkyle), -hétérocyclylène-O-R⁵, -hétérocyclylène-C(O)N(R⁵)₂, - hétérocyclylène-C(O)R⁵, -hétérocyclylène-C(O)₂R⁵, -hétérocyclylène-N(R⁵)₂, -hétérocyclylène-N(R⁵)C(O)R⁵, -hétérocyclylène-N(R⁵)C(O)N(R⁵)₂-, -hétérocyclylène-N(R⁵)SO₂-R⁵, - hétérocyclylène-S-R⁵, -cycloalkyle, -cycloalkylène-(alkyle), -cycloalkylène-(aryle), - cycloalkylène-(cycloalkyle), -cycloalkylène(hétérocycloalkyle), -cycloalkylène-O-R⁵, - cycloalkylène-C(O)N(R⁵)₂, -cycloalkylène-C(O)R⁵, -cycloalkylène-C(O)₂R⁵, -cycloalkylène-N(R⁵)₂, -cycloalkylène-N(R⁵)C(O)R⁵, -cycloalkylène-N(R⁵)C(O)N(R⁵)₂, -cycloalkylène-N(R⁵)SO₂-R⁵, -alkylène-N(H)-alkylène-C(O)OR⁵, -alkylène-C(O)OR⁵, -arylène-alkylène-aryle, - C(O)R⁵, -C(O)₂R⁵, -alkylène-S-S-R⁵, -C(O)-alkyle, -C(O)-alkylène-N(R⁵)₂, -C(O)₂R⁵, -aryl-alkylène-N(R⁵)C(O)-R⁵, -cycloalkylène-N(R⁵)S(O)(F)-R⁵, -SO₂-NR⁵, -S(O)(F)-NR⁵ ; ou
R³ et R⁴ peuvent être pris ensemble pour former un hétérocyclyle ; ou 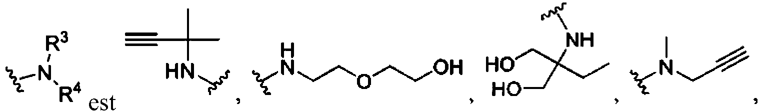 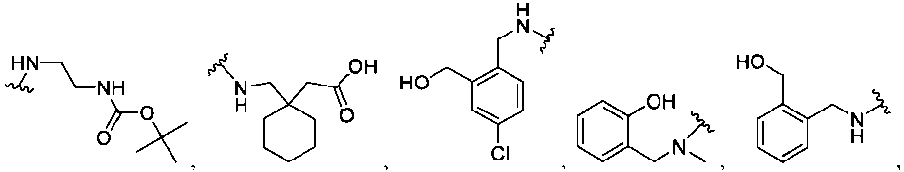 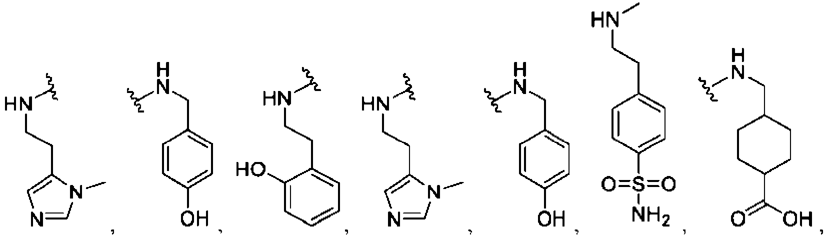 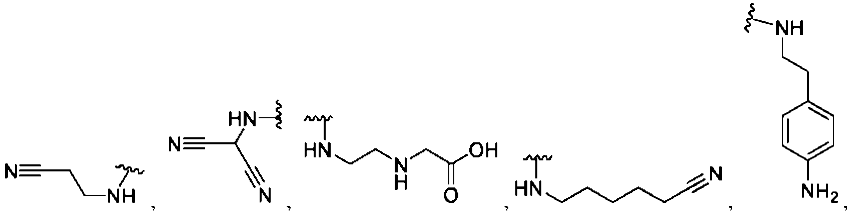 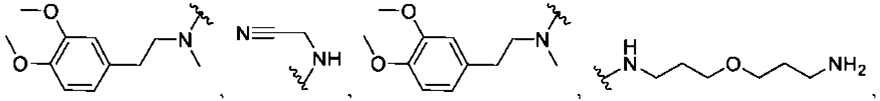 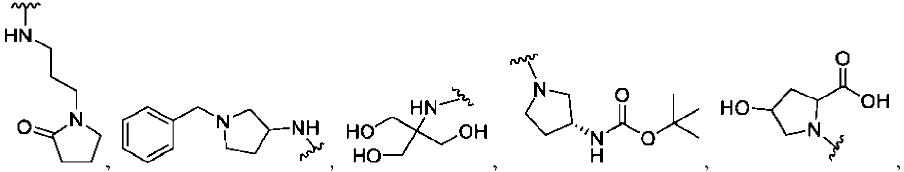 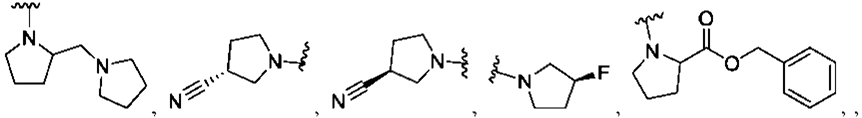 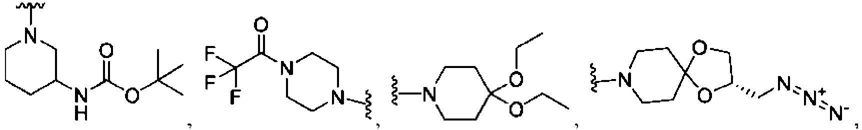 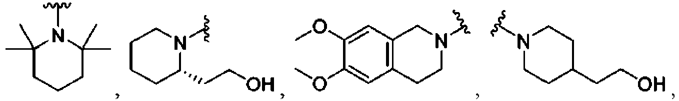 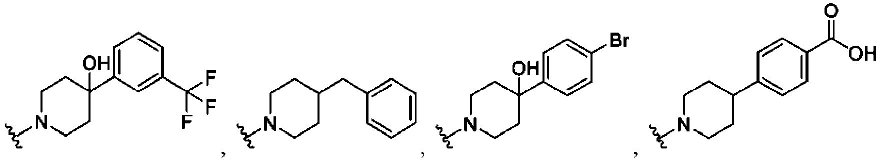 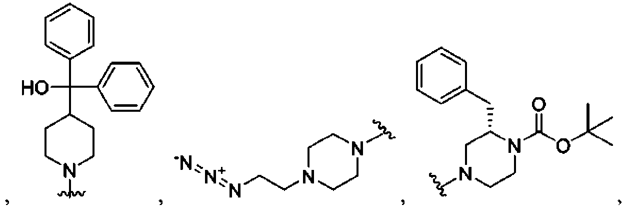 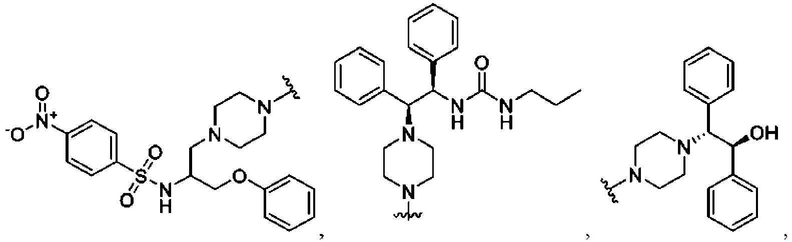 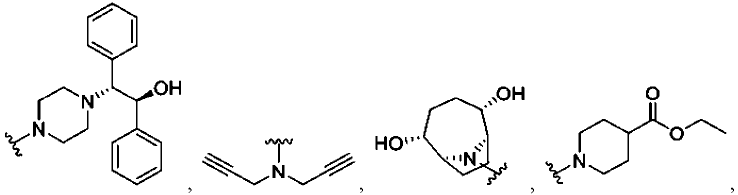 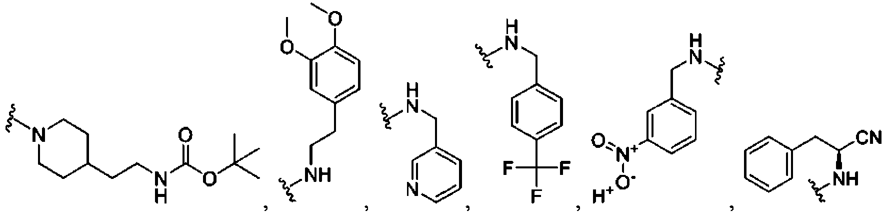 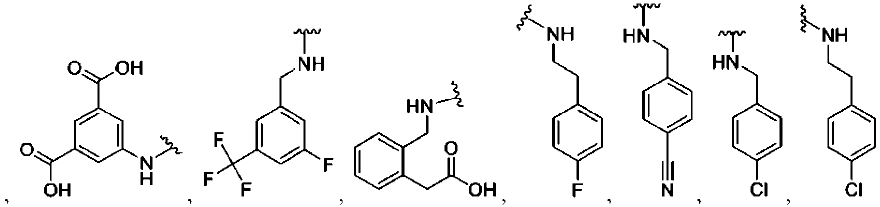 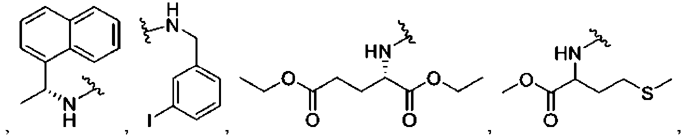 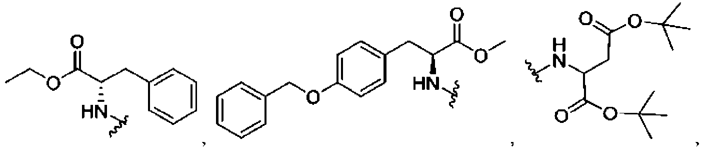 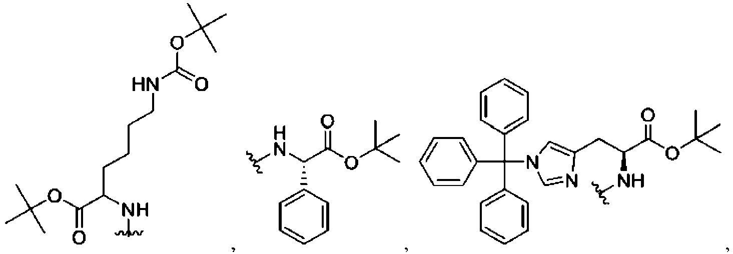 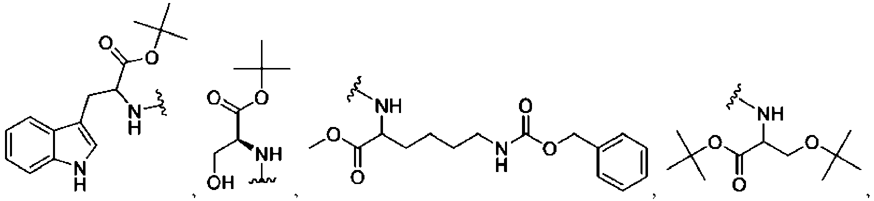 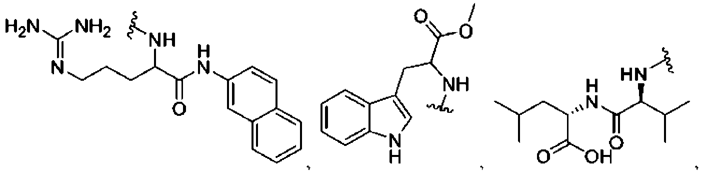 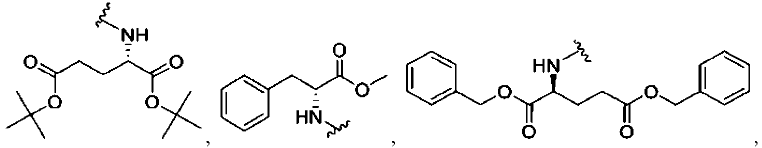 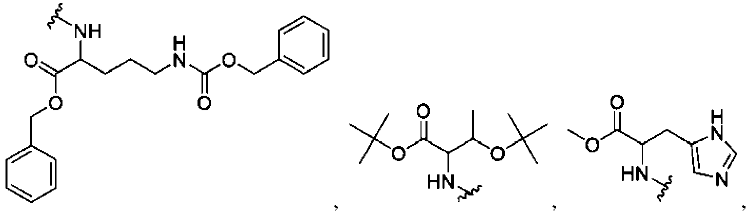 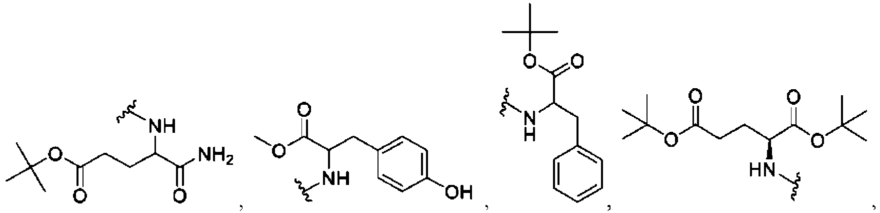 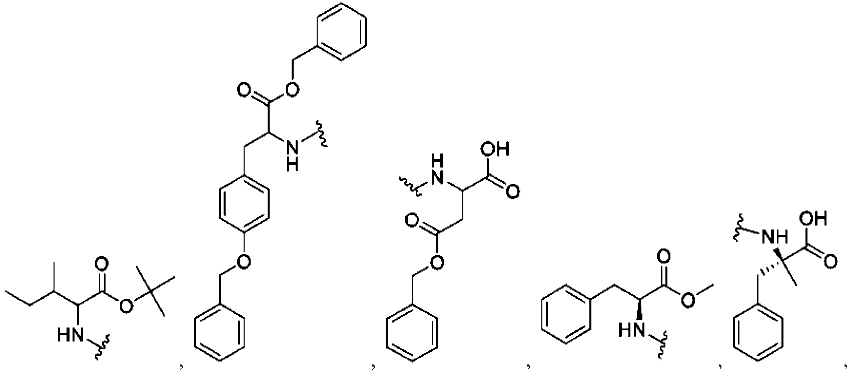 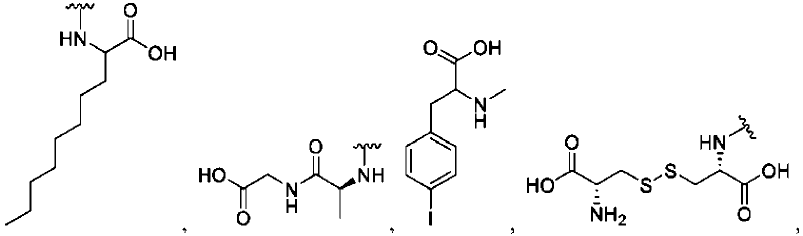 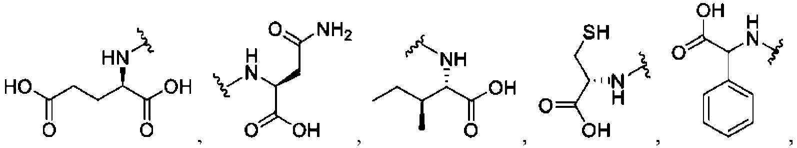 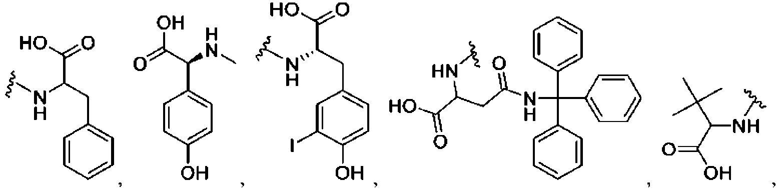 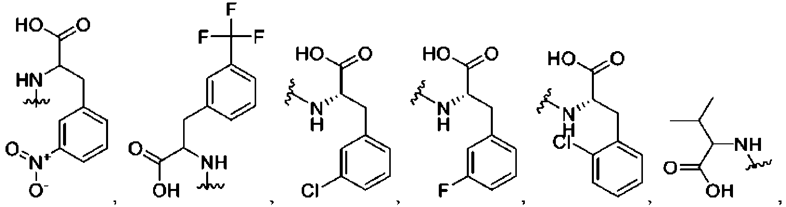 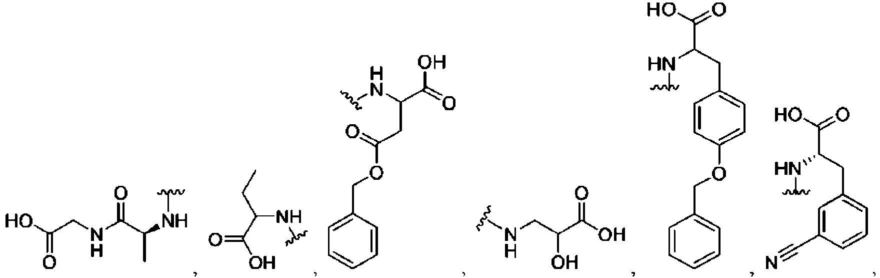 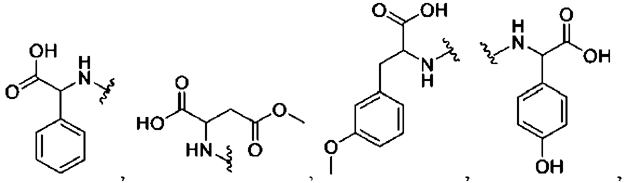 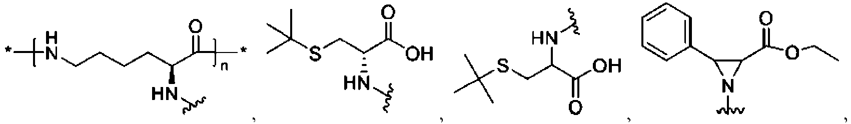 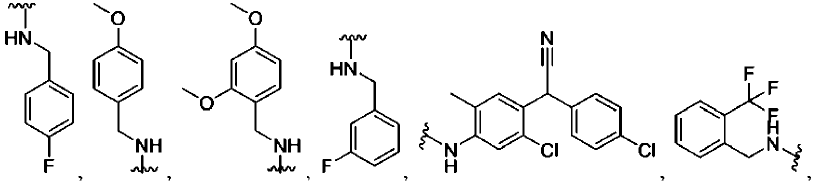 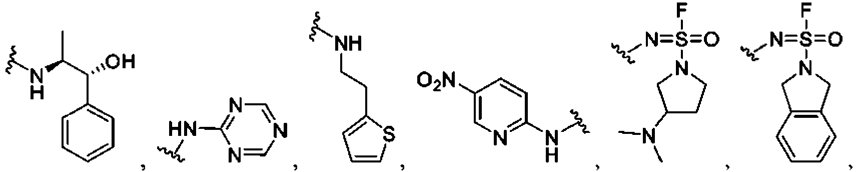 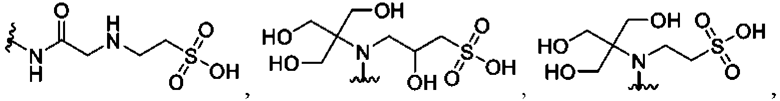 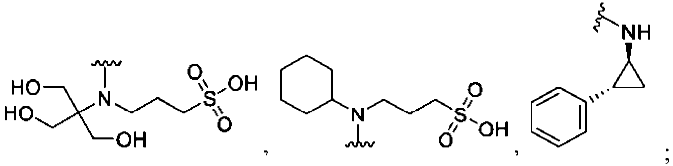 et
R⁵ est H, un alkyle, un aryle ou -alkylène-aryle ;
où, sauf indication contraire :
le terme alkyle définit une fraction de chaîne carbonée ramifiée ou non ramifiée jusqu'à 30 atomes de carbone ;
le terme alkylène définit un groupe alkyle comportant 2 à 12 atomes de carbone qui contient deux points de fixation au reste du composé sur sa chaîne carbonée la plus longue ;
le terme aryle définit un cycle aromatique de 5 à 12 chaînons, où chaque atome du cycle est un carbone ou un ou plusieurs atomes de cycle sont des hétéroatomes ;
le terme hétérocyclyle définit une structure cyclique de 3 à 12 chaînons, où un à quatre atomes de cycle sont des hétéroatomes ; et
le terme cycloalkyle définit un cycle carbocyclique saturé mono-, bicyclique, ponté, spirocyclique ou polycyclique comportant 3 à 12 atomes de carbone ;
ou une composition pharmaceutique comprenant le composé de formule (I) ou de formule (II) et un support acceptable pharmaceutiquement, destinée à être utilisée dans un procédé de traitement ou de prévention d'une maladie ou d'une affection médiée par HA.

14. Composé destiné à être utilisé selon la revendication 13, ladite maladie médiée par HA étant la grippe.

15. Composé destiné à être utilisé selon la revendication 14, ladite grippe étant la grippe A, la grippe B ou la grippe C.
